# EUROPÄISCHE PATENTSCHRIFT

(11) **EP 2 331 506 B1**
(45) Veröffentlichungstag und Bekanntmachung des Hinweises auf die Patenterteilung: **03.07.2013**
(21) Anmeldenummer: 09782238.1
(22) Anmeldetag: 26.08.2009
(51) Int. Cl.: C07D 213/89, C07D 213/68, C07D 239/34, C07D 239/47, C07D 401/12, C07D 403/04, C07D 403/06, C07D 405/12, C07D 401/06, C07D 403/12, A61P 11/00, A61K 31/505, A61K 31/444, A61K 31/4433, A61K 31/443, A61K 31/4439, C07D 213/74, C07D 239/46

(54) **NEUE BENZAMIDE, DEREN HERSTELLUNG UND DEREN VERWENDUNG ALS ARZNEIMITTEL**
NOVEL BENZAMIDES, PRODUCTION THEREOF, AND USE THEREOF AS MEDICAMENTS
NOUVEAUX BENZAMIDES, LEUR PRÉPARATION ET LEUR UTILISATION COMME MÉDICAMENTS

(30) Priorität: 02.09.2008 EP 08163525
(43) Veröffentlichungstag der Anmeldung: 15.06.2011
(73) Patentinhaber: Boehringer Ingelheim International GmbH, 55216 Ingelheim am Rhein (DE)
(72) Erfinder: WAGNER, Holger, 55216 Ingelheim am Rhein (DE); LANGKOPF, Elke, 55216 Ingelheim am Rhein (DE); HIMMELSBACH, Frank, 55216 Ingelheim am Rhein (DE); GOEGGEL, Rolf, 55216 Ingelheim am Rhein (DE); JUNG, Birgit, 55216 Ingelheim am Rhein (DE)
(74) Vertreter: Simon, Elke Anna Maria
(86) Internationale Anmeldenummer: PCT/EP2009/061024
(87) Internationale Veröffentlichungsnummer: WO 2010/026095

(56) Entgegenhaltungen:
- WO-A-01/47897
- WO-A-2005/085202

## Beschreibung

Gegenstand der vorliegenden Erfindung sind substituierte Benzoesäureamide der allgemeinen Formel I 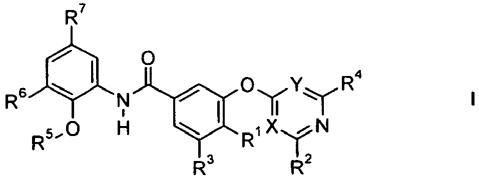 wobei die Reste und Gruppen R¹, R², R³, R⁴, R⁵, R⁶, R⁷, X und Y nachfolgend definiert sind, einschließlich deren Tautomere, deren Stereoisomere, deren Gemische und deren Salze. Ein weiterer Gegenstand dieser Erfindung betrifft Arzneimittel enthaltend eine erfindungsgemäße Verbindung der Formel I sowie die Verwendung einer erfindungsgemäßen Verbindung zur Herstellung eines Arzneimittels zur Behandlung von Atemwegserkrankungen. Darüber hinaus sind Verfahren zur Herstellung eines Arzneimittels sowie einer erfindungsgemäßen Verbindung Gegenstand dieser Erfindung.

In der Literatur werden Verbindungen, die eine Hemmwirkung auf das Enzym p38 Mitogenaktivierte Protein (MAP)-Kinase besitzen, u.a. zur Behandlung von Atemwegserkrankungen, insbesondere von chronisch obstruktiver Bronchitis (COPD) und Asthma, vorgeschlagen (siehe z.B. Journal of Allergy and Clinical Immunology 2007, 119, 1055-1062 und Drug Discovery Today 2007, 12, 479-486 sowie darin zitierte Literatur).

In der internationalen Offenlegungsschrift WO 2005/085202 (Merck) werden Verbindungen der allgemeinen Formel 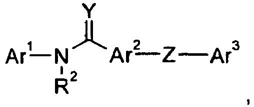 worin Ar¹, Ar², Ar³, Z, Y und R² wie darin definiert sind, als Inhibitoren von Tyrosin- und Raf-Kinasen beschrieben, die u.a. zur Behandlung von Krankheiten, die durch Angiogenese verursacht, vermittelt und/oder propargiert werden, eingesetzt werden können.

In der internationalen Offenlegungsschrift WO 2005/086904 (Amgen) werden Verbindungen der allgemeinen Formel 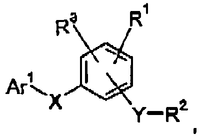 worin R¹, R², R³, X, Y und Ar¹ wie darin definiert sind, als Modulatoren des PPARγ-Rezeptors beansprucht.

Die WO 01/47897 betrifft N-heterocyclische Verbindungen der folgenden allgemeinen Formel, 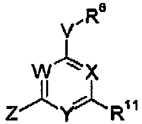 welche die Produktion von Cytokinen durch Inhibition der p38-Kinase blockieren.

Den Erfindern ist nicht bekannt, dass Benzamide der vorliegenden allgemeinen Struktur als Inhibitoren des Enzyms p38 MAP-Kinase beschrieben sind.

### Aufgabe der Erfindung

Der vorliegenden Erfindung liegt die Aufgabe zugrunde, neue substituierte Benzoesäureamide aufzuzeigen, insbesondere solche, die eine Aktivität auf das Enzym p38 MAP-Kinase besitzen. Eine weitere Aufgabe der vorliegenden Erfindung besteht im Aufzeigen von substituierten Benzoesäureamiden, die *in vitro* und/oder *in vivo* eine Hemmwirkung auf das Enzym p38 MAP-Kinase besitzen und geeignete pharmakologische und/oder pharmakokinetische Eigenschaften aufweisen, um diese als Arzneimittel verwenden zu können.

Ferner ist es eine Aufgabe der vorliegenden Erfindung, neue Arzneimittel bereit zu stellen, welche zur Prophylaxe und/oder Behandlung von Atemwegserkrankungen, insbesondere von COPD und Asthma geeignet sind.

Ebenfalls eine Aufgabe dieser Erfindung ist es, ein Verfahren zur Herstellung der erfindungsgemäßen Verbindungen bereit zu stellen.

Weitere Aufgaben der vorliegenden Erfindung ergeben sich für den Fachmann unmittelbar aus den vorhergehenden und nachfolgenden Ausführungen.

### Gegenstand der Erfindung

Ein erster Gegenstand der vorliegenden Erfindung sind Heteroaryloxy-substituierte Benzoesäureamide der allgemeinen Formel I 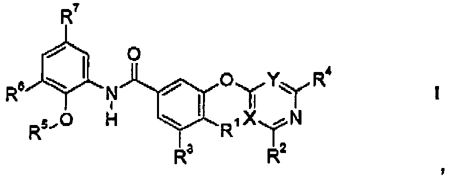 in der
- R¹: L₁-R₈ ist,
wobei L₁ ausgewählt ist aus einer Bindung, O, CO, NH, CONH, NHCO, SO, SO₂, SO₂NH, NHSO₂ und C₁-C₄-Alkylen,
wobei R₈ ausgewählt ist aus Wasserstoff, Halogen, C₁₋₆-Alkyl, C₂₋₆-Alkinyl, C₂₋₆-Alkenyl, C₃₋₈-Cycloalkyl, C₃₋₈-Heterocycloalkyl, C₆₋₁₀-Aryl, C₅₋₁₀-Heteroaryl, Cyan, Carboxy, C₁₋₄-Alkyloxycarbonyl, Nitro, Amino, Di-(C₁₋₄-alkyl)-amino, Hydroxy, C₁₋₆-Alkylsulfanyl,
wobei R₈ teilweise oder vollständig fluoriert und/oder mit einem oder mehreren Substituenten L substituiert sein kann, und
wobei in Heterocycloalkyl-Resten eine Methylengruppe durch CO, SO oder SO₂ substituiert sein können, und
wobei R¹ nicht Wasserstoff ist, wenn R³ die Bedeutung Wasserstoff hat, und
- R²: L₂-R₉ ist,
wobei L₂ ausgewählt ist aus einer Bindung, O, CO, NH, CONH, NHCO, SO, SO₂, SO₂NH, NHSO₂ und C₁-C₄-Alkylen
wobei R₉ ausgewählt ist aus Wasserstoff, Halogen, C₁₋₆-Alkyl, C₂₋₆-Alkinyl, C₂₋₆-Alkenyl, C₃₋₈-Cycloalkyl, C₄₋₈-Cycloalkenyl, C₃₋₈-Heterocycloalkyl, C₆₋₁₀-Aryl, C₅₋₁₀-Heteroaryl, Cyan, Carboxy, C₁₋₄-Alkyloxycarbonyl, Nitro, Amino, Di-(C₁₋₄-alkyl)-amino, Hydroxy, C₁₋₆-Alkylsulfanyl,
wobei R₉ teilweise oder vollständig fluoriert und/oder mit einem oder mehreren Substituenten L substituiert sein kann, und
wobei in den genannten Alkyl-Resten optional eine oder zwei CH₂-Gruppe gegen O und/oder NR^{N} ausgetauscht sind,
wobei in den genannten Cycloalkyl-Resten eine oder zwei CH₂-Gruppen unabhängig voneinander durch NR^{N}, O, S, CO, SO oder SO₂ und eine oder zwei CH-Gruppen durch N ersetzt sein können, und
- R³: L₃-R₁₀ ist
wobei L₃ ausgewählt ist aus einer Bindung, O, CO, NH, CONH, NHCO, SO, SO₂, SO₂NH, NHSO₂ und C₁-C₄-Alkylen
wobei R₁₀ ausgewählt ist aus Wasserstoff, Halogen, C₁₋₆-Alkyl, C₂₋₆-Alkinyl, C₂₋₆₋Alkenyl, C₃₋₆-Cycloalkyl, C₄₋₆-Cycloalkenyl, C₃₋₈-Heterocycloalkyl, C₅₋₁₀-Aryl, C₅₋₁₀-Heteroaryl, Cyan, Carboxy, C₁₋₄-Alkyloxycarbonyl, Nitro, Amino, Di-(C₁₋₄-alkyl)-amino, Hydroxy, C₁₋₈-Alkylsulfanyl
wobei R₁₀ teilweise oder vollständig fluoriert und/oder mit einem oder mehreren Substituenten L substituiert sein kann, und
wobei R³ nicht Wasserstoff ist, wenn R¹ die Bedeutung Wasserstoff hat,
- R⁴: L₄-R₁₁ ist
wobei L₄ ausgewählt ist aus einer Bindung, O, CO, NH, CONH, NHCO,
wobei R₁₁ ausgewählt ist aus Wasserstoff, Halogen, Amino, C₁₋₆-Alkyl, C₃₋₈-Cycloalkyl, C₃₋₈-Heterocycloalkyl, Trifluoromethyl, Difluormethyl, Cyan, Carboxy, C₁₋₄-Alkyloxycarbonyl, Di-(C₁₋₄-alkyl)-amino, C₁₋₄-Alkylsulfanyl,
- R⁵: C₁₋₆-Alkyl
- R⁶: ausgewählt ist aus Dimethylamino-C₁₋₃-alkyl, Cyclopropylamino-C₁₋₃-alkyl, Pyrrolidinyl-C₁₋₃-alkyl, Hydroxy-C₁₋₃-alkyl, Methylsulfanyl-C₁₋₃-alkyl, Methylsulfinyl-C₁₋₃-alkyl, Methylsulfonyl-C₁₋₃-alkyl, C₁₋₄-Alkylcarbonylamino, C₃₋₅-Cycloalkyl-carbonylamino, C₁₋₄-Alkylsulfonylamino, C₃₋₅-Cycloalkylsulfonylamino, C₁₋₃-Alkylsulfinyl und C₁₋₃-Alkylsulfonyl,
- R⁷: C₁₋₆-Alkyl, Trifluormethyl, Pentafluorethyl,
- X,Y: unabhängig voneinander N oder C-R₁₃, wobei X und Y nicht beide N sein können und R₁₃ ausgewählt ist aus Wasserstoff, Fluor, Chlor, C₁₋₄-Alkyl, Trifluormethyl, OH, C₁₋₄-Alkyloxy, Trifluormethoxy, Difluormethoxy und Cyan,
- R^{N}: ausgewählt ist aus H, C₁₋₄-Alkyl, 1,1,1-Trifluorethyl, Cyanmethyl, Acetyl, Methylsulfonyl, C₆₋₁₀-Arylmethyl,
- L: ausgewählt ist aus der Gruppe bestehend aus Halogen, Cyan, C₁₋₆-Alkyl, C₁₋₆-Alkyloxy, Amino, C₁₋₃-Alkylamino, Di-(C₁₋₃-Alkyl)-amino, C₁₋₄-Alkylcarbonylamino, Difluormethyl, C₁₋₄-Alkylsulfonylamino Trifluormethyl, Hydroxy, C₁₋₃-Alkoxy, Difluormethoxy, Trifluormethoxy, Acetylamino, C₆₋₁₀-Aryl und C₅₋₁₀-Heteroaryl.

In einer bevorzugten Ausführungsform ist
R¹ ausgewählt aus Wasserstoff, Halogen, C₁₋₆-Alkyl, C₂₋₆-Alkinyl, C₂₋₆-Alkenyl, C₃₋₇-Cycloalkyl, C₃₋₇-Cycloalkyl-C₁₋₃-alkyl, C₆₋₁₀-Aryl, C₅₋₁₀-Heteroaryl, C₆₋₁₀-Aryl-C₁₋₃-alkyl, C₅₋₁₀-Heteroaryl-C₁₋₃-alkyl, C₁₋₄-Alkylcarbonyl, Cyan, Aminocarbonyl, C₁₋₄-Alkyl-aminocarbonyl, Di-(C₁₋₃-Alkyl)-aminocarbonyl, Pyrrolidin-1-ylcarbonyl, Piperidin-1-ylcarbonyl, Morpholin-4-ylcarbonyl, Piperazin-1-ylcarbonyl, 4-(C₁₋₄-Alkyl)-piperazin-1-ylcarbonyl, Carboxy, C₁₋₄-Alkyloxycarbonyl, Nitro, Amino, C₁₋₄-Alkylamino, Di-(C₁₋₃-alkyl)-amino, Pyrrolidin-1-yl, Piperidin-1-yl, Morpholin-4-yl, Piperazin-1-yl, 4-(C₁₋₄-Alkyl)piperazin-1-yl, C₁₋₄-Alkylcarbonylamino, C₁₋₄-Alkylsulfonylamino, Hydroxy, C₁₋₆-Alkyloxy, C₃₋₇-Cycloalkyloxy, C₆₋₁₀-Aryloxy, C₁₋₄-Alkylsulfanyl, C₁₋₄-Alkylsulfinyl und C₁₋₄-Alkylsulfonyl,
wobei Alkyl-, Alkenyl-, Alkinyl-, Cycloalkyl- und Heterocycloalkyl-Reste teilweise oder vollständig fluoriert und/oder ein- oder zweifach mit gleichen oder verschiedenen Substituenten ausgewählt aus Cyan, Hydroxy, C₁₋₃-Alkyloxy, Acetylamino, Methylsulfonylamino und C₁₋₃-Alkyl substituiert sein können, und
wobei in Cycloalkyl-Resten ein oder zwei Methylengruppen unabhängig voneinander durch O, S, CO, SO oder SO₂ substituiert sein können, und
wobei in N-Heterocycloalkyl-Resten eine Methylengruppe durch CO oder SO₂ substituiert sein kann,
wobei R¹ nicht Wasserstoff ist, wenn R³ die Bedeutung Wasserstoff hat, und
R² ausgewählt aus Wasserstoff, C₁₋₈-Alkyl, C₂₋₆-Alkenyl, C₂₋₆-Alkinyl, C₃₋₇-Cycloalkyl-C₁₋₄-alkyl, C₁₋₈Alkylcarbonyl-C₁₋₃-alkyl, C₆₋₁₀-Aryl-C₁₋₄-alkyl, C₅₋₁₀-Heteroaryl-C₁₋₄-alkyl, C₃₋₇-Cycloalkyl, C₁₋₆-Alkylcarbonyl, C₃₋₇-Cycloalkyl-carbonyl. Cyan, Aminocarbonyl, Carboxy, Amino, C₁₋₄-Alkylcarbonylamino, C₁₋₄-Alkylsulfonylamino, C₁₋₄-Alkyloxy, C₆₋₁₀-Aryl. C₅₋₁₀-Heteroaryl, Hydroxy,
wobei alle genannten Alkyl-, Alkenyl-, Alkinyl- und Cycloalkyl-Reste teilweise oder vollständig fluoriert und/oder ein-, zwei- oder dreifach mit gleichen oder verschiedenen Substituenten ausgewählt aus C₁₋₃-Alkyl, Cyan, Hydroxy, C₁₋₃-Alkyloxy, Amino, C₁₋₃-Alkylamino, Di-(C₁₋₃-Alkyl)amino, Acetylamino, Methylsulfonylamino, C₆₋₁₀-Aryl und C₅₋₁₀₋Heteroaryl substituiert sein können.
wobei in den genannten Alkyl-Resten optional eine oder zwei CH₂-Gruppe gegen O und/oder NR^{N} ausgetauscht sind,
wobei in den genannten Cycloalkyl-Resten eine oder zwei CH₂-Gruppen unabhängig voneinander durch NR^{N}, O, S, CO, SO oder SO₂ und eine oder zwei CH-Gruppen durch N ersetzt sein können, und
R³ ausgewählt aus Wasserstoff, Halogen, C₁₋₄-Alkyl, C₂₋₆-Alkinyl, C₂₋₆₋Alkenyl, C₃₋₇-Cycloalkyl-C₁₋₃-alkyl, C₃₋₆-Cycloalkyl, Trifluormethyl, Difluormethyl, Hydroxy, C₁₋₄-Alkyloxy, Trifluormethoxy, Difluormethoxy, Cyan, Aminocarbonyl, C₁₋₄-Alkyl-aminocarbonyl. Di-(C₁₋₃₋Alkyl)-aminocarbonyl, Carboxy, C₁₋₄-Alkyloxycarbonyl, Amino, C₁₋₄-Alkylamino, Di-(C₁₋₃-alkyl)-amino, C₁₋₄-Alkylcarbonylamino, C₁₋₄-Alkylsulfonylamino, wobei R³ nicht Wasserstoff ist, wenn R¹ die Bedeutung Wasserstoff hat,
R⁴ ausgewählt aus Wasserstoff, C₁₋₄-Alkyl, C₃₋₆-Cycloalkyl, Trifluoromethyl, Difluormethyl, Cyan, Carboxy, Aminocarbonyl, C₁₋₃-Alkylaminocarbonyl, Di-(C₁₋₃alkyl)aminocarbonyl, C₁₋₄₋Alkyloxycarbonyl, Amino, C₁₋₃-Alkylamino, Di-(C₁₋₃-alkyl)amino, Hydroxy, C₁₋₄-Alkyloxy, C₁₋₄-Alkylsulfanyl,
R⁵ C₁₋₄-Alkyl ist,
R⁶ ausgewählt ist aus Dimethylamino-C₁₋₃-alkyl, Cyclopropylamino-C₁₋₃-alkyl, Pyrrolidinyl-C₁₋₃-alkyl, Hydroxy-C₁₋₃-alkyl, Methylsulfanyl-C₁₋₃-alkyl, Methylsulfinyl-C₁₋₃-alkyl, Methylsulfonyl-C₁₋₃-alkyl, C₁₋₄-Alkylcarbonylamino, C₃₋₅-Cycloalkyl-carbonylamino, C₁₋₄-Alkylsulfonylamino, C₃₋₅-Cycloalkylsulfonylamino, C₁₋₃-Alkylsulfinyl und C₁₋₃-Alkylsulfonyl,
R⁷ ausgewählt aus C₄-Alkyl, Trifluormethyl, Pentafluorethyl,
X,Y unabhängig voneinander N oder CR₁₃, wobei X und Y in der selbenBedeutung N ausgeschlossen ist und R₁₃ ausgewählt ist aus Wasserstoff, Fluor, Chlor, C₁₋₄-Alkyl, Trifluormethyl, Hydroxy, C₁₋₄-Alkyloxy, Trifluormethoxy, Difluormethoxy, Cyano,
R^{N} ausgewählt aus H, C₁₋₄-Alkyl, 1,1,1-Trifluorethyl, Cyanmethyl, Acetyl, Methylsulfonyl, C₆₋₁₀-Arylmethyl,
L ausgewählt aus der Gruppe bestehend aus Fluor, Chlor, Brom, C₁₋₃-Alkyl, Difluormethyl, Trifluormethyl, Hydroxy, C₁₋₃-Alkoxy, Difluormethoxy, Trifluormethoxy, Acetylamino, Methylsulfonylamino und Cyan,
wobei unter den bei der Definition der vorstehend genannten Reste erwähnten C₆₋₁₀-Arylgruppen Phenyl- oder Naphthylgruppen zu verstehen sind, welche unabhängig von einander ein- oder zweifach mit gleichen oder verschiedenen Resten L substituiert sein können; und
unter den bei der Definition der vorstehend erwähnten Reste erwähnten Heteroaryl-gruppen eine Pyrrolyl-, Furanyl-, Thienyl-, Pyridyl-, Indoxyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinyl-gruppe zu verstehen ist, in der eine bis drei Methingruppen durch Stickstoffatome ersetzt sind,
wobei die vorstehend erwähnten Heteroarylgruppen unabhängig voneinander ein- oder zweifach mit gleichen oder verschiedenen Resten L substituiert sein können;
wobei, soweit nichts anderes erwähnt wurde, die vorstehend erwähnten Alkylgruppen geradkettig oder verzweigt sein können,
deren Tautomere, deren Stereoisomere, deren Gemische und deren Salze, insbesondere deren physiologisch verträglichen Salze.

In einer weiteren bevorzugten Ausführungsform ist
R¹ ausgewählt aus Wasserstoff, Fluor, Chlor, Brom, C₁₋₄-Alkyl, Trifluormethyl, C₃₋₆-Cycloalkyl, Cyan, Aminocarbonyl, C₁₋₄-Alkyl-aminocarbonyl, Di-(C₁₋₃-Alkyl)-aminocarbonyl, Carboxy, C₁₋₄-Alkyloxycarbonyl, Amino, C₁₋₄-Alkylamino, Di-(C₁₋₃-alkyl)-amino, Pyrrolidin-1-yl, Piperidin-1-yl, Morpholin-4-yl, Acetylamino, Methylsulfonylamino, Hydroxy, C₁₋₄-Alkyloxy, Trifluormethoxy, Difluormethoxy und C₁₋₄-Alkylsulfonyl,
wobei die genannten Alkyl-, Cycloalkyl- und N-Heterocycloalkyl-Reste ein- oder zweifachmit gleichen oder verschiedenen Substituenten ausgewählt aus Cyan, Hydroxy, C₁₋₃-Alkyloxy, Acetylamino, Methylsulfonylamino und C₁₋₃-Alkyl substituiert sein können, und wobei in den genannten Cycloalkyl-Resten ein oder zwei Methylengruppen unabhängig voneinander durch O, CO oder SO₂ und in den genannten N-Heterocycloalkyl-Resten eine Methylengruppe durch CO oder SO₂ substituiert sein können, und wobei R¹ nicht Wasserstoff ist, wenn R³ die Bedeutung Wasserstoff hat, bedeutet.

In einer weiteren bevorzugten Ausführungsform ist
R¹ ausgewählt aus Wasserstoff, Fluor, Chlor, Brom, C₁₋₄-Alkyl, Trifluormethyl, C₃₋₆-Cycloalkyl, Cyan, Aminocarbonyl, C₁₋₄-Alkyl-aminocarbonyl, Di-(C₁₋₃-Alkyl)-aminocarbonyl, Carboxy, C₁₋₄-Alkyloxycarbonyl, Amino, C₁₋₄-Alkylamino, Di-(C₁₋₃-alkyl)-amino, Pyrrolidin-1-yl, Piperidin-1-yl, Morpholin-4-yl, Acetylamino, Methylsulfonylamino, Hydroxy, C₁₋₄- Alkyloxy, Trifluormethoxy, Difluormethoxy und C₁₋₄-Alkylsulfonyl, wobei R¹ nicht Wasserstoff ist, wenn R³ die Bedeutung Wasserstoff hat.

In einer weiteren bevorzugten Ausführungsform ist
R¹ ausgewählt aus Wasserstoff, Fluor, Chlor, Brom, Methyl, Ethyl und Trifluormethyl, wobei R¹ nicht Wasserstoff ist, wenn R³ die Bedeutung Wasserstoff hat.

In einer weiteren bevorzugten Ausführungsform ist
R² ausgewählt aus Wasserstoff, C₁₋₄-Alkyl, C₃₋₆-Cycloalkyl-C₁₋₃-alkyl, C₁₋₃-Alkyl-amino-C₁₋₃-alkyl, C₃₋₆-Cycloalkylamino-C₁₋₃-alkyl, Pyrrolidinyl-C₁₋₃-alkyl, Piperidin-C₁₋₃-alkyl, Azepanyl-C₁₋₃-alkyl, Piperazinyl-C₁₋₃-alkyl, Morpholinyl-C₁₋₃-alkyl, Homopiperazinyl-C₁₋₃-alkyl, Pyrrolidin-3-ylamino-C₁₋₃-alkyl, Piperidin-3-ylamino-C₁₋₃-alkyl, Piperidin-4-ylamino-C₁₋₃-alkyl, C₃₋₆-Cycloalkyl,Trifluormethyl,
Cyan, Aminocarbonyl, C₁₋₄-Alkyl-aminocarbonyl, Di-(C₁₋₃-Alkyl)-aminocarbonyl, Piperazin-1-ylcarbonyl, Morpholin-4-ylcarbonyl, Carboxy, C₁₋₄-Alkyloxycarbonyl,
Amino, C₁₋₄-Alkylamino, C₃₋₆-Cycloalkylamino, C₃₋₆-Cycloalkyl-C₁₋₃-alkylamino, Pyrrolidin-1-yl, Piperidin-1-yl, Piperazin-1-yl, Morpholin-4-yl, C₁₋₃-Alkyl-amino-C₂₋₃-alkylamino, Pyrrolidin-3-ylamino, Piperidin-3-ylamino, Piperidin-4-ylamino, Pyrrolidinyl-C₁₋₃-alkyl-amino, Piperidin-C₁₋₃-alkyl-amino, Piperazinyl-C₁₋₃-alkyl-amino, Morpholinyl-C₁₋₃-alkyl-amino, Homopiperazinyl-C₁₋₃-alkyl-amino, Acetylamino, Methylsulfonylamino,
Hydroxy, C₁₋₄-Alkyloxy, Trifluormethoxy, Difluormethoxy, C₃₋₆-Cycloalkyloxy, C₃₋₆-Cycloalkyl-C₁₋₃-alkyloxy, C₁₋₃-Alkylamino-C₂₋₃-alkyloxy, Di-(C₁₋₃-Alkyl)-amino-C₂₋₃-alkyloxy, Pyrrolidinyl-C₂₋₃-alkyloxy, Piperidin-C₂₋₃-alkyloxy, Piperazinyl-C₂₋₃-alkyloxy, Morpholinyl-C₂₋₃-alkyloxy, Homopiperazinyl-C₂₋₃-alkyloxy,
wobei die oben genannten Alkyl-, Cycloalkyl- und N-Heterocycloalkyl-Reste ein-oder zweifach mit gleichen oder verschiedenen Substituenten ausgewählt aus C₁₋₃-Alkyl, Cyan, Amino, C₁₋₃-Alkylamino, Di-(C₁₋₃-alkyl)amino, Acetylamino, Methylsulfonylamino, Hydroxy und C₁₋₃-Alkyloxy substituiert sein können, und
wobei in den oben genannten Cycloalkyl-Resten ein oder zwei Methylen-gruppen unabhängig voneinander durch O und/oder CO und in den genannten N-Heterocycloalkyl-Resten eine Methylengruppe durch CO oder SO₂ substituiert sein können, und wobei optional alle in den oben genannten Resten beinhalteten NH-Gruppen durch N-Me, N-Et, N-*i*Pr, N-Acetyl und N-SO₂Me ersetzt sind.

In einer weiteren bevorzugten Ausführungsform ist
R² ausgewählt aus Wasserstoff, C₁₋₄-Alkyl, C₁₋₃-Alkylamino-C₁₋₃-alkyl, Tetrahydrofuran-3-ylamino-C₁₋₃-alkyl, Pyrrolidinyl-C₁₋₃-alkyl, Piperidinyl-C₁₋₃-alkyl, Azepanyl-C₁₋₃-alkyl, Piperazinyl-C₁₋₃-alkyl,4-(1,1,1-Trifluorethyl)-piperazin-1-yl-C₁₋₃-alkyl, 4-Cyanmethyl-piperazin-1-yl-C₁₋₃-alkyl, Homopiperazinyl-C₁₋₃-alkyl, Morpholinyl-C₁₋₃-alkyl, N-(Pyrrolidin-3-yl)-N-(C₁₋₃-alkyl)-amino-C₁₋₃-alkyl, Pyrrolidin-3-ylamino-C₁₋₃-alkyl, N-[Di-(C₁₋₃-alkyl)amino-C₂₋₃-alkyl]-N-(C₁₋₃-alkyl)-amino-C₁₋₃-alkyl, N-[Di-(C₁₋₃-alkyl)amino-C₂₋₃-alkyl]-amino-C₁₋₃-alkyl, N-(Piperidinyl)-N-(C₁₋₃-alkyl)-amino-C₁₋₃-alkyl, Piperidinylamino-C₁₋₃-alkyl, C₁₋₃-Alkyl- aminocarbonyl, Piperazin-1-ylcarbonyl, Morpholin-4-ylcarbonyl, Amino, C₁₋₃-Alkylamino, C₅₋₆-Cycloalkylamino, Pyrrolidin-1-yl, Piperidin-1-yl, Piperazin-1-yl, Morpholin-4-yl, Pyrrolidin-3-ylamino, N-(C₁₋₃-Alkyl)-N-(pyrrolidin-3-yl)-amino, Piperidin-4-ylamino, N-(C₁₋₃-Alkyl)-N-(piperidin-4-yl)-amino, Di-(C₁₋₃-alkyl)-amino-C₂₋₃-alkylamino, N-[Di-(C₁₋₃-alkyl)-amino-C₂₋₃-alkyl]-N-(C₁₋₃-alkyl)-amino, Pyrrolidin-1-yl-C₂₋₃-alkyl-amino, Hydroxy-C₂₋₃-alkyl-amino, Hydroxy,
wobei in den zuvor genannten N- und O-Heterocycloalkyl-Resten optional eine CH₂-Gruppe durch C=O ersetzt ist und jeder dieser Cyclen optional mit einem Rest ausgewählt aus C₁₋₃-alkyl, Amino, C₁₋₃-Alkyl-amino, Di-(C₁₋₃-alkyl)-amino und Hydroxy substituiert ist, bedeutet.

In einer weiteren bevorzugten Ausführungsform ist
R² ausgewählt aus Wasserstoff, Methyl, Methylaminomethyl, 3-Hydroxypyrrolidin-1-ylmethyl, 3-Dimethylaminopyrrolidin-1-ylmethyl, N-(1-Methylpyrrolidin-3-yl)-N-methyl-aminomethyl, Piperidin-4-ylmethyl, N-Methyl-piperidin-4-ylmethyl, Azepan-4-ylmethyl, 1-Methyl-azepan-4-ylmethyl, N-(2-Dimethylaminoethyl)-N-methyl-aminomethyl, N-(2-Dimethyl-aminoethyl)-aminomethyl, N-(1-Methylpyrrolidin-3-yl)-aminomethyl, 1-Methylpiperidin-4-ylaminomethyl, 1-Methylpiperidin-3-yl-aminomethyl, Piperazin-1-ylmethyl, 4-Methyl-piperazin-1-ylmethyl, 4-(1,1,1-Trifluorethyl)-piperazin-1-ylmethyl, 4-Cyanmethyl-piperazin-1-ylmethyl, Piperazin-2-on-4-ylmethyl, Morpholin-4-ylmethyl, Tetrahydrofuran-3-ylaminomethyl, Homo-piperazin-1-ylmethyl, 4-Methylhomopiperazin-1-ylmethyl, Methylaminocarbonyl, Piperazin-1-ylcarbonyl, 4-Methylpiperazin-1-ylcarbonyl, Morpholin-4-ylcarbonyl, Methylamino, 4-Dimethylaminocyclohexyl-amino, 3-Dimethyl-amino-pyrrolidin-1-yl, Pyrrolidin-3-ylamino, N-Methyl-N-pyrrolidin-3-yl-amino, N-Methyl-N-(1-methylpyrrolidin-3-yl)-amino, Piperidin-4-ylamino, 1-Methyl-piperidin-4-ylamino, N-Methyl-N-piperidin-4-yl-amino, 4-Dimethylamino-piperidin-1-yl, Piperazin-1-yl, Piperazin-2-on-4-yl, Morpholin-4-yl, 2-(Dimethylamino)ethyl-amino, 2-(Pyrrolidin-1-yl)ethylamino, 2-Hydroxy-ethylamino, N-(2-Dimethylaminoethyl)-N-methyl-amino, Hydroxy.

In einer weiteren bevorzugten Ausführungsform ist R³ ausgewählt aus Wasserstoff, C₁₋₃₋Alkyl, C₃₋₆-Cycloalkyl, Fluor, Chlor, Brom, Trifluormethyl, Difluormethyl, Hydroxy, C₁₋₃-Alkyloxy, Trifluormethoxy, Difluormethoxy und Cyan, wobei R³ nicht Wasserstoff ist, wenn R¹ die Bedeutung Wasserstoff hat.

In einer weiteren bevorzugten Ausführungsform ist R³ ausgewählt aus Wasserstoff, C₁₋₃₋Alkyl, Fluor, Chlor, Trifluormethyl, C₁₋₃-Alkyloxy und Cyan , wobei R³ nicht Wasserstoff ist, wenn R¹ die Bedeutung Wasserstoff hat

In einer weiteren bevorzugten Ausführungsform ist R³ ausgewählt aus Wasserstoff, Methyl, Fluor und Chlor, wobei R³ nicht Wasserstoff ist, wenn R¹ die Bedeutung Wasserstoff hat.

In einer weiteren bevorzugten Ausführungsform ist R⁴ ausgewählt aus Wasserstoff, C₁₋₃₋Alkyl, Triftuoromethyl, Carboxy, Aminocarbonyl, Methylaminocarbonyl, Dimethylamino-carbonyl, Amino, C₁₋₃-Alkylamino, Di-(C₁₋₃-alkyl)-amino, Hydroxy und C₁₋₃-Alkyloxy .

In einer weiteren bevorzugten Ausführungsform ist R⁴ ausgewählt aus Wasserstoff, Methyl, Dimethylamino, Hydroxy und Methoxy.

In einer weiteren bevorzugten Ausführungsform ist R⁴ Wasserstoff.

In einer weiteren bevorzugten Ausführungsform ist R⁵ ausgewählt aus Methyl, Ethyl, n-Propyl und Isopropyl.

In einer weiteren bevorzugten Ausführungsform ist R⁵ ausgewählt aus Methyl, Ethyl und Isopropyl.

In einer weiteren bevorzugten Ausführungsform ist R⁵ Methyl.

In einer weiteren bevorzugten Ausführungsform ist R⁶ ausgewählt aus Dimethylaminomethyl, Cyclopropylaminomethyl, Pyrrolidin-1-ylamino, Hydroxymethyl, Methylsulfanylmethyl, Methylsulfinylmethyl, Methylsulfonylmethyl, n-Butylcarbonyl-amino, Isopropylcarbonyl-amino, Isobutylcarbonylamino, Cyclopropylcarbonylamino, Methylsulfonylamino, Isopropylsulfonylamino, n-Butylsulfonylamino, Isobutylsulfonylamino, Cyclopropylsuffonylamino, Methylsulfinyl und Methylsulfonyl.

In einer weiteren bevorzugten Ausführungsform ist R⁶ Methylsulfonylamino.

In einer weiteren bevorzugten Ausführungsform ist R⁷ ausgewählt aus Isobutyl, tert-Butyl, Trifluormethyl und Pentafluorethyl.

In einer weiteren bevorzugten Ausführungsform ist R⁷ ausgewählt aus tert-Butyl, Trifluormethyl und Pentafluorethyl.

In einer weiteren bevorzugten Ausführungsform ist R⁷ tert-Butyl.

In einer weiteren bevorzugten Ausführungsform sind X und Y unabhängig voneinander ausgewählt aus N, C-H, C-F, C-Cl, C-C₁₋₃-alkyl und C₁₋₃-alkyloxy, mit der Einschränkung, dass X und Y nicht in der selben Bedeutung N vorkommen.

In einer weiteren bevorzugten Ausführungsform sind X und Y unabhängig voneinander ausgewählt aus N, C-H, C-F und C-Me, mit der Einschränkung, dass X und Y nicht in der selben Bedeutung N vorkommen.

In einer weiteren bevorzugten Ausführungsform sind X und Y CH und CH, N und CH oder CH und N.

Die erfindungsgemäßen Verbindungen der allgemeinen Formel I und ihre physiologisch verträglichen Salze weisen wertvolle pharmakologische Eigenschaften auf, insbesondere eine Hemmwirkung auf das Enzym p38 MAP-Kinase.

Gegenstand der vorliegenden Erfindung sind auch die physiologisch verträglichen Salze der erfindungsgemäßen Verbindungen mit anorganischen oder organischen Säuren. Gegenstand der Erfindung sind die jeweiligen Verbindungen der Formel I in Form ihrer pharmakologisch verträglichen Salze. Diese pharmakologisch verträglichen Salze der Verbindungen der Formel I können ebenfalls in Form ihrer jeweiligen Hydrate (z.B. Monohydrate, Dihydrate etc.) sowie in Form Ihrer jeweiligen Solvate vorliegen. Unter einem Hydrat der Verbindung nach Formel I versteht man im Rahmen der Erfindung ein kristallines, kristallwasserhaltiges Salz der Verbindung nach Formel I. Unter einem Solvat der Verbindung nach Formel I versteht man im Rahmen der Erfindung ein kristallines Salz der Verbindung nach Formel I, welches Lösungsmittelmoleküle-Moleküle (z.B. Ethanol, Methanol etc) im Kristallgitter enthalten.

Dem Fachmann sind Standardverfahren zur Gewinnung von Hydraten und Solvaten (z.B. das Umkristallisieren aus dem entsprechenden Lösungsmittel bzw. aus Wasser) bekannt.

Daher ist die Verwendung der erfindungsgemäßen Verbindungen, einschließlich der physiologisch verträglichen Salze, als Arzneimittel ebenfalls ein Gegenstand dieser Erfindung.

Ein weiterer Gegenstand dieser Erfindung sind Arzneimittel, enthaltend mindestens eine erfindungsgemäße Verbindung oder ein erfindungsgemäßes physiologisch verträgliches Salz neben gegebenenfalls einem oder mehreren inerten Trägerstoffen und/oder Verdünnungsmitteln.

Ebenfalls ein Gegenstand dieser Erfindung ist die Verwendung mindestens einer erfindungsgemäßen Verbindung oder eines physiologisch verträglichen Salzes solch einer Verbindung zur Herstellung eines Arzneimittels, das zur Behandlung oder Prophylaxe von Erkrankungen oder Zuständen geeignet ist, die durch Inhibierung des Enzyms p38 MAP-Kinase beeinflussbar sind.

Ein weiterer Gegenstand dieser Erfindung ist die Verwendung mindestens einer erfindungsgemäßen Verbindung zur Herstellung eines Arzneimittels, das zur Behandlung von Atemwegserkrankungen geeignet ist.

Ein weiterer Gegenstand dieser Erfindung ist die Verwendung mindestens einer erfindungsgemäßen Verbindung zur Herstellung eines Arzneimittels zur Inhibition des Enzyms p38 MAP-Kinase.

Ferner ist ein Verfahren zur Herstellung eines erfindungsgemäßen Arzneimittels Gegenstand dieser Erfindung, dadurch gekennzeichnet, dass auf nicht-chemischem Wege eine erfindungsgemäße Verbindung in einen oder mehrere inerte Trägerstoffe und/oder Verdünnungsmittel eingearbeitet wird.

Gegenstand der vorliegenden Erfindung ist auch ein Verfahren zur Herstellung der erfindungsgemäßen Verbindungen der allgemeinen Formel I, dadurch gekennzeichnet, dass
a) zur Herstellung von Verbindungen der allgemeinen Formel I, die wie vor- und nachstehend definiert ist,
   eine Verbindung der allgemeinen Formel II, 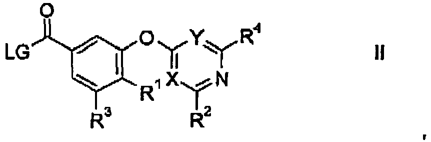 in der R¹ bis R⁴, X und Y die in den Ansprüchen 1 bis 6 und 10 angegebenen Bedeutungen besitzen und LG
   Fluor, Chlor, Brom, Cyan, C₁₋₁₀-Alkoxy, C₁₋₆-Alkylsulfanyl, C₂₋₄-Alkenyl-oxy, C₂₋₄-Alkinyloxy, Benzotriazol-1-yloxy, [1,2,3]Triazolo[4,5-b]pyridin-3-yloxy, C₅₋₁₀-Heteroaryl (über N an II gebunden), Succinyl-N-oxy, C₁₋₄-Alkylcarbonyloxy, Di-(C₁₋₄-alkyl)aminocarbonyloxy, Pyrrol-1-ylcarbonyloxy, Piperidin-1-yl-carbonyloxy, Morpholin-4-ylcarbonyloxy, Tri-(C₁₋₄-alkyl)carbamimidoyloxy, N,N,N',N'-Tetra-(C₁₋₄-alkyl)-uronium-O-yl, N,N-Dicyclohexyluron-O-yl, N-(3-Dimethylaminopropyl)-N'-ethyl-uronyl, Di-(C₁₋₄-alkyloxy)-phosphoryloxy, Bis(di-C₁₋₄-alkylamino)-phosphoryloxy, Dipyrrolidinyl-phosphoryloxy, C₆₋₁₀-Arylsulfanyl, C₅₋₁₀-Heteroarylsulfanyl, C₆₋₁₀-Aryloxy oder C₅₋₁₀-Heteroaryloxy,
   wobei alle in der Definition erwähnten Akyl-, Alkenyl- und Alkinyl-Gruppen mit Fluor, Chlor, C₁₋₃-Alkyl und/oder C₁₋₃-Alkoxy einfach oder mehrfach substituiert sein können,
   wobei alle in der Definition erwähnten Aryl-Gruppen für Phenyl oder Naphthyl und alle Heteroaryl-Gruppen für Pyridinyl, Pyrimidinyl, Triazinyl, Imidazolyl, Pyrazolyl, Triazolyl, Tetrazolyl stehen, die, sowohl Aryl- als auch Heteroaryl-Gruppen, optional einfach oder mehrfach mit identischen oder unterschiedlichen Resten ausgewählt von Fluor, Chlor, Brom, C₁₋₃-Alkyl, C₁₋₃-Alkyloxy, Nitro, Cyan und Di-(C₁₋₃-alkyl)amino substituiert sind, ist,
   mit einem Anilin der allgemeinen Formel 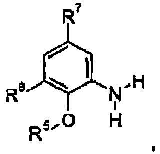 in der R⁵, R⁶ und R⁷ die in den Ansprüchen 1, 2, 7, 8 und 9 angegebenen Bedeutungen besitzen
   optional in Gegenwart einer Base und/oder eines Additivs wie z.B. Triethylamin, Pyridin, Ethyldiisopropylamin, 4-Dimethylaminopyridin, Kaliumcarbonat oder 1-Hydroxybenzotriazol,
   zur Reaktion gebracht wird; und
   erforderlichenfalls ein bei den vorstehend unter a) und b) beschriebenen Umsetzungen verwendeter Schutzrest wieder abgespalten wird und/oder
   falls gewünscht eine so erhaltene Verbindung der allgemeinen Formel I in ihre Stereoisomere aufgetrennt wird und/oder
   falls gewünscht eine so erhaltene Verbindung der allgemeinen Formel I in ihre Salze, insbesondere für die pharmazeutische Anwendung in ihre physiologisch verträglichen Salze, überführt wird.
   In einer bevorzuigten Ausführungsform des Verfahrens wird eine Verbindung der allgemeinen Formel III 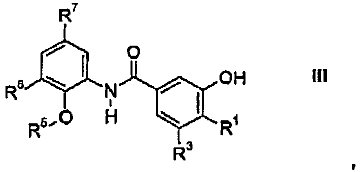 in der R¹, R³, R⁵, R⁶ und R⁷ die in den Ansprüchen 1, 2, 3, 5, 7, 8 und 9 angegebenen Bedeutungen besitzen,
   mit einer Verbindung der allgemeinen Formel IV 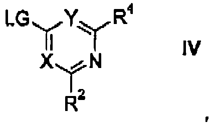 in der R² und R⁴ die in den Ansprüchen 1, 2, 4, 6 und 10 angegebenen Bedeutungen besitzen und LG eine Austrittsgruppe darstellt, insbesondere bedeutet LG
   F, Cl, Br, I, O-C₁₋₆-Alkyl, O-C₆₋₁₀-Aryl, S(O)ₙ-C₁₋₄-Alkyl, S(O)ₘ-C₅₋₁₀-Aryl, OSO₂-C₁₋₄-Alkyl, OSO₂-C₆₋₁₀-Aryl, NO₂,
   wobei alle erwähnten Alkyl-Gruppen optional mit Fluor, C₁₋₃-Alkyl und/oder C₁₋₃- Alkoxy einfach oder mehrfach substituiert sind, und
   wobei alle erwähnten Aryl-Gruppen für Phenyl oder Naphthyl stehen, die optional einfach oder mehrfach mit identischen oder unterschiedlichen Resten ausgewählt aus Fluor, Chlor, C₁₋₃-Alkyl, C₁₋₃-Alkyloxy, Nitro und Cyan substituiert sind,
   wobei n und m unabhängig voneinander 0, 1 oder 2 sein können,
   in Gegenwart einer Base, z.B. NaH, KH, KO*t*Bu, NaO*t*Bu, NaOMe, NaOEt, NaO*iP*r, KF, K₂CO₃, Cs₂CO₃, Pyridin, 4-Dimethylaminopyridin, NEt₃ oder EtN*i*Pr₂,
   optional in Gegenwart eines Katalysators, z.B. eines Cu- oder Pd-Komplexes,
   umgesetzt wird; und
   erforderlichenfalls ein bei den vorstehend unter a) und b) beschriebenen Umsetzungen verwendeter Schutzrest wieder abgespalten wird und/oder
   falls gewünscht eine so erhaltene Verbindung der allgemeinen Formel I in ihre Stereoisomere aufgetrennt wird und/oder
   falls gewünscht eine so erhaltene Verbindung der allgemeinen Formel I in ihre Salze, insbesondere für die pharmazeutische Anwendung in ihre physiologisch verträglichen Salze, überführt wird.

### Detailierte Beschreibung der Erfindung

Sofern nicht anders angegeben besitzen die Gruppen, Reste und Substituenten, insbesondere R¹ bis R⁷, R^{N}, X, Y und L die zuvor und nachfolgend angegebenen Bedeutungen.

Kommen Reste, Substituenten oder Gruppen in einer Verbindung mehrfach vor, so können diese gleiche oder verschiedene Bedeutungen aufweisen.

Bevorzugte Ausführungsformen der Erfindung sind durch die folgenden Definitionen wiedergegeben:
***a)*** Die Definitionen ***(aⁱ)*** für R¹ gemäß ihrer zunehmenden Präferenz, beginnend mit bevorzugt ***(a¹)*** über besonders bevorzugt ***(a²)*** zu ganz besonders bevorzugt ***(a³),*** sind wie folgt:
   ***(a¹):*** Bevorzugt bedeutet R¹ Wasserstoff, Fluor, Chlor, Brom, C₁₋₄-Alkyl, Trifluormethyl, C₃₋₆-Cycloalkyl, Cyan, Aminocarbonyl, C₁₋₄-Alkyl-aminocarbonyl, Di-(C₁₋₃-Alkyl)-aminocarbonyl, Carboxy, C₁₋₄-Alkyloxycarbonyl, Amino, C₁₋₄-Alkylamino, Di-(C₁₋₃-alkyl)-amino, Pyrrolidin-1-yl, Piperidin-1-yl, Morpholin-4-yl, Acetylamino, Methylsulfonylamino, Hydroxy, C₁₋₄-Alkyloxy, Trifluormethoxy, Difluormethoxy und C₁₋₄-Alkylsulfonyl,
      wobei die genannten Alkyl-, Cycloalkyl- und N-Heterocycloalkyl-Reste ein- oder zweifach mit gleichen oder verschiedenen Substituenten ausgewählt aus Cyan, Hydroxy, C₁₋₃-Alkyloxy, Acetylamino, Methylsulfonylamino und C₁₋₃-Alkyl substituiert sein können, und
      wobei in den genannten Cycloalkyl-Resten ein oder zwei Methylengruppen unabhängig voneinander durch O, CO oder SO₂ und in den genannten N-Heterocycloalkyl-Resten eine Methylengruppe durch CO oder SO₂ substituiert sein können und
      wobei R¹ nicht Wasserstoff ist, wenn R³ die Bedeutung Wasserstoff hat.
   ***(a²):*** Besonders bevorzugt bedeutet R¹ Wasserstoff, Fluor, Chlor, Brom, C₁₋₄-Alkyl, Trifluormethyl, C₃₋₆-Cycloalkyl, Cyan, Aminocarbonyl, C₁₋₄-Alkyl-aminocarbonyl, Di-(C₁₋₃-Alkyl)-aminocarbonyl, Carboxy, C₁₋₄-Alkyloxycarbonyl, Amino, C₁₋₄-Alkylamino, Di-(C₁₋₃-alkyl)-amino, Pyrrolidin-1-yl, Piperidin-1-yl, Morpholin-4-yl, Acetylamino, Methylsulfonylamino, Hydroxy, C₁₋₄-Alkyloxy, Trifluormethoxy, Difluormethoxy und C₁₋₄-Alkylsulfonyl, wobei R¹ nicht Wasserstoff ist, wenn R³ die Bedeutung Wasserstoff hat.
   ***(a³):*** Ganz besonders bevorzugt bedeutet R¹ Wasserstoff, Fluor, Chlor, Brom, Methyl, Ethyl und Trifluormethyl, wobei R¹ nicht Wasserstoff ist, wenn R³ die Bedeutung Wasserstoff hat.
***b)*** Die Definitionen ***(bⁱ)*** für R² gemäß ihrer zunehmenden Präferenz, beginnend mit bevorzugt ***(b¹)*** über besonders bevorzugt ***(b²)*** zu ganz besonders bevorzugt ***(b³),*** sind wie folgt:
   ***(b¹):*** Bevorzugt bedeutet R² Wasserstoff, C₁₋₄-Alkyl, C₃₋₆-Cycloalkyl-C₁₋₃-alkyl, C₁₋₃-Alkyl-amino-C₁₋₃-alkyl, C₃₋₆-Cycloalkylamino-C₁₋₃-alkyl, Pyrrolidinyl-C₁₋₃-alkyl, Piperidin-C₁₋₃-alkyl, Azepanyl-C₁₋₃-alkyl, Piperazinyl-C₁₋₃-alkyl, Morpholinyl-C₁₋₃-alkyl, Homopiperazinyl-C₁₋₃-alkyl, Pyrrolidin-3-ylamino-C₁₋₃-alkyl, Piperidin-3-ylamino-C₁₋₃-alkyl, Piperidin-4-ylamino-C₁₋₃-alkyl, Trifluormethyl, C₃₋₆-Cycloalkyl,
      Cyan, Aminocarbonyl, C₁₋₄-Alkyl-aminocarbonyl, Di-(C₁₋₃-Alkyl)-aminocarbonyl, Piperazin-1-ylcarbonyl, Morpholin-4-ylcarbonyl, Carboxy, C₁₋₄-Alkyloxycarbonyl,
      Amino, C₁₋₄-Alkylamino, C₃₋₆-Cycloalkylamino, C₃₋₆-Cycloalkyl-C₁₋₃-alkylamino, Pyrrolidin-1-yl, Piperidin-1-yl, Piperazin-1-yl, Morpholin-4-yl, C₁₋₃-Alkyl-amino-C₂₋₃-alkylamino, Pyrrolidin-3-ylamino, Piperidin-3-ylamino, Piperidin-4-ylamino, Pyrrolidinyl-C₁₋₃-alkyl-amino, Piperidin-C₁₋₃-alkyl-amino, Piperazinyl-C₁₋₃-alkyl-amino, Morpholinyl-C₁₋₃-alkyl-amino, Homopiperazinyl-C₁₋₃-alkyl-amino, Acetylamino, Methylsulfonylamino,
      Hydroxy, C₁₋₄-Alkyloxy, Trifluormethoxy, Difluormethoxy, C₃₋₆-Cycloalkyloxy, C₃₋₆-Cycloalkyl-C₁₋₃-alkyloxy, C₁₋₃-Alkylamino-C₂₋₃-alkyloxy, Di-(C₁₋₃-Alkyl)-amino-C₂₋₃-alkyloxy, Pyrrolidinyl-C₂₋₃-alkyloxy, Piperidin-C₂₋₃-alkyloxy, Piperazinyl-C₂₋₃-alkyloxy, Morpholinyl-C₂₋₃-alkyloxy, Homopiperazinyl-C₂₋₃-alkyloxy,
      wobei die oben genannten Alkyl-, Cycloalkyl- und N-Heterocycloalkyl-Reste ein- oder zweifach mit gleichen oder verschiedenen Substituenten ausgewählt aus C₁₋₃-Alkyl, Cyan, Amino, C₁₋₃-Alkylamino, Di-(C₁₋₃-alkyl)amino, Acetylamino, Methylsulfonylamino, Hydroxy und C₁₋₃-Alkyloxy substituiert sein können, und
      wobei in den oben genannten Cycloalkyl-Resten ein oder zwei Methylengruppen unabhängig voneinander durch O und/oder CO und in den genannten N- Heterocycloalkyl-Resten eine Methylengruppe durch CO oder SO₂ substituiert sein können, und
      wobei optional alle in den oben genannten Resten beinhalteten NH-Gruppen durch NR^{N} ersetzt sind.
   ***(b²):*** Besonders bevorzugt bedeutet R² Wasserstoff, C₁₋₄-Alkyl, C₁₋₃-Alkylamino-C₁₋₃-alkyl, Tetrahydrofuran-3-ylamino-C₁₋₃-alkyl, Pyrrolidinyl-C₁₋₃-alkyl, Piperidinyl-C₁₋₃-alkyl, Azepanyl-C₁₋₃-alkyl, Piperazinyl-C₁₋₃-alkyl, 4-(1,1,1-Trifluorethyl)-piperazin-1-yl-C₁₋₃-alkyl, 4-Cyanmethyl-piperazin-1-yl-C₁₋₃-alkyl, Homopiperazinyl-C₁₋₃-alkyl, Morpholinyl-C₁₋₃-alkyl, N-(Pyrrolidin-3-yl)-N-(C₁₋₃-alkyl)-amino-C₁₋₃-alkyl, Pyrrolidin-3-ylamino-C₁₋₃-alkyl, N-[Di-(C₁₋₃-alkyl)amino-C₂₋₃-alkyl]-N-(C₁₋₃-alkyl)-amino-C₁₋₃-alkyl,
      N-[Di-(C₁₋₃-alkyl)amino-C₂₋₃-alkyl]-amino-C₁₋₃-alkyl, N-(Piperidinyl)-N-(C₁₋₃-alkyl)-amino-C₁₋₃-alkyl, Piperidinylamino-C₁₋₃-alkyl, C₁₋₃-Alkylaminocarbonyl, Piperazin-1-ylcarbonyl, Morpholin-4-ylcarbonyl, Amino, C₁₋₃-Alkylamino, C₅₋₆-Cycloalkylamino, Pyrrolidin-1-yl, Piperidin-1-yl, Piperazin-1-yl, Morpholin-4-yl, Pyrrolidin-3-ylamino, N-(C₁₋₃-Alkyl)-N-(pyrrolidin-3-yl)-amino, Piperidin-4-ylamino, N-(C₁₋₃-Alkyl)-N-(piperidin-4-yl)-amino, Di-(C₁₋₃-alkyl)-amino-C₂₋₃-alkylamino, N-[Di-(C₁₋₃-alkyl)-amino-C₂₋₃-alkyl]-N-(C₁₋₃-alkyl)-amino, Pyrrolidin-1-yl-C₂₋₃-alkyl-amino, Hydroxy-C₂₋₃-alkyl-amino, Hydroxy,
      wobei in den zuvor genannten Heterocycloalkyl-Resten optional eine CH₂-Gruppe durch C=O ersetzt ist und jeder dieser Cyclen optional mit einem Rest ausgewählt aus C₁₋₃-alkyl, Amino, C₁₋₃-Alkyl-amino, Di-(C₁₋₃-alkyl)-amino und Hydroxy substituiert ist.
   ***(b³):*** Ganz besonders bevorzugt bedeutet R² Wasserstoff, Methyl, Methylaminomethyl, 3-Hydroxypyrrolidin-1-ylmethyl, 3-Dimethylaminopyrrolidin-1-ylmethyl, N-(1-Methylpyrrolidin-3-yl)-N-methyl-aminomethyl, Piperidin-4-ylmethyl, N-Methyl-piperidin-4-ylmethyl, Azepan-4-ylmethyl, 1-Methyl-azepan-4-ylmethyl, N-(2-Dimethylaminoethyl)-N-methyl-aminomethyl, N-(2-Dimethylaminoethyl)-aminomethyl, N-(1-Methylpyrrolidin-3-yl)-aminomethyl, 1-Methylpiperidin-4-ylaminomethyl, 1-Methylpiperidin-3-ylaminomethyl, Piperazin-1-ylmethyl, 4-Methylpiperazin-1-ylmethyl, 4-(1,1,1-Trifluorethyl)-piperazin-1-ylmethyl, 4-Cyanmethyl-piperazin-1-ylmethyl, Piperazin-2-on-4-ylmethyl, Morpholin-4-ylmethyl, Tetrahydrofuran-3-ylaminomethyl, Homopiperazin-1-ylmethyl, 4-Methylhomopiperazin-1-ylmethyl, Methylaminocarbonyl, Piperazin-1-ylcarbonyl, 4-Methylpiperazin-1-ylcarbonyl, Morpholin-4-ylcarbonyl, Methylamino, 4-Dimethylaminocyclohexyl-amino, 3-Dimethylamino-pyrrolidin-1-yl, Pyrrolidin-3-ylamino, N-Methyl-N-pyrrolidin-3-yl-amino, N-Methyl-N-(1-methylpyrrolidin-3-yl)-amino, Piperidin-4-ylamino, 1-Methyl-piperidin-4-ylamino, N-Methyl-N-piperidin-4-yl-amino, 4-Dimethylamino-piperidin-1-yl, Piperazin-1-yl, Piperazin-2-on-4-yl, Morpholin-4-yl, 2-(Dimethylamino)ethyl-amino, 2-(Pyrrolidin-1-yl)ethylamino, 2-Hydroxy-ethylamino, N-(2-Dimethylaminoethyl)-N-methyl-amino, Hydroxy.
c) Die Definitionen ***(cⁱ)*** für R³ gemäß ihrer zunehmenden Präferenz, beginnend mit bevorzugt ***(c¹)*** über besonders bevorzugt ***(c²)*** zu ganz besonders bevorzugt ***(c³),*** sind wie folgt:
   ***(c¹):*** Bevorzugt bedeutet R³ Wasserstoff, C₁₋₃-Alkyl, C₃₋₆-Cycloalkyl, Fluor, Chlor, Brom, Trifluormethyl, Difluormethyl, Hydroxy, C₁₋₃-Alkyloxy, Trifluormethoxy, Difluormethoxy und Cyan, wobei R³ nicht Wasserstoff ist, wenn R¹ die Bedeutung Wasserstoff hat.
   ***(c²):*** Besonders bevorzugt bedeutet R³ Wasserstoff, C₁₋₃-Alkyl, Fluor, Chlor, Trifluormethyl, C₁₋₃-Alkyloxy und Cyan, wobei R³ nicht Wasserstoff ist, wenn R¹ die Bedeutung Wasserstoff hat.
   ***(c³):*** Ganz besonders bevorzugt bedeutet R³ Wasserstoff, Methyl, Fluor, Chlor, wobei R³ nicht Wasserstoff ist, wenn R¹ die Bedeutung Wasserstoff hat.
***d)*** Die Definitionen ***(dⁱ)*** für R⁴ gemäß ihrer zunehmenden Präferenz, beginnend mit bevorzugt ***(d¹)*** über besonders bevorzugt ***(d²)*** zu ganz besonders bevorzugt ***(d³),*** sind wie folgt:
   ***(d¹):*** Bevorzugt bedeutet R⁴ Wasserstoff, C₁₋₃-Alkyl, Trifluormethyl, Carboxy, Aminocarbonyl, Methylaminocarbonyl, Dimethylaminocarbonyl, Amino, C₁₋₃-Alkylamino, Di-(C₁₋₃-alkyl)-amino, Hydroxy, C₁₋₃-Alkyloxy.
   ***(d²):*** Besonders bevorzugt bedeutet R⁴ Wasserstoff, Methyl, Dimethylamino, Hydroxy, Methoxy.
   ***(d³):*** Ganz besonders bevorzugt bedeutet R⁴ Wasserstoff.
***e)*** Die Definitionen ***(eⁱ)*** für X und Y (X/Y) gemäß ihrer zunehmenden Präferenz, beginnend mit bevorzugt ***(e¹)*** über besonders bevorzugt ***(e²)*** zu ganz besonders bevorzugt ***(e³),*** sind wie folgt:
   ***(e¹):*** Bevorzugt bedeuten X/Y CR/CR, N/CR und CR/N, wobei R Wasserstoff, Fluor, Chlor, C₁₋₃-Alkyl und C₁₋₃-Alkyloxy ist.
   ***(e²):*** Besonders bevorzugt bedeuten X/Y CR/CR, N/CR und CR/N, wobei R Wasserstoff, Fluor oder Methyl ist.
   ***(e³):*** Ganz besonders bevorzugt bedeuten X/Y CH/CH, N/CH und CH/N.
***f)*** Die Definitionen ***(fⁱ)*** für R^{N} gemäß ihrer zunehmenden Präferenz, beginnend mit bevorzugt ***(f¹)*** über besonders bevorzugt ***(f²)*** zu ganz besonders bevorzugt ***(f³),*** sind wie folgt:
   ***(f¹):*** Bevorzugt bedeutet R^{N} Wasserstoff, Methyl, Ethyl, Isopropyl, Acetyl, Methylsulfonyl, 1,1,1-Trifluorethyl und Cyanmethyl.
   ***(f²):*** Besonders bevorzugt bedeutet R^{N} Wasserstoff, Methyl, Acetyl, Methylsulfonyl, 1,1,1-Trifluorethyl und Cyanmethyl.
   ***(f³):*** Ganz besonders bevorzugt bedeutet R^{N} Wasserstoff, Methyl, 1,1,1-Trifluorethyl und Cyanmethyl.
g) Die Definitionen ***(gⁱ)*** für L gemäß ihrer zunehmenden Präferenz, beginnend mit bevorzugt ***(g¹)*** über besonders bevorzugt ***(g²)*** zu ganz besonders bevorzugt ***(g³),*** sind wie folgt:
   ***(g¹):*** Bevorzugt bedeutet L Fluor, Chlor, Brom, C₁₋₃-Alkyl, Difluormethyl, Trifluormethyl, C₁₋₃-Alkoxy, Difluormethoxy, Trifluormethoxy, Acetylamino und Cyan.
   ***(g²):*** Besonders bevorzugt bedeutet L Fluor, Chlor, Methyl, Ethyl, Trifluormethyl, Methoxy, Acetylamino und Cyan.
   ***(g³):*** Ganz besonders bevorzugt bedeutet L Fluor, Methyl, Methoxy, Acetylamino und Cyan.
h) Die Definitionen ***(h¹)*** für R⁵ gemäß ihrer zunehmenden Präferenz, beginnend mit bevorzugt ***(h¹)*** über besonders bevorzugt ***(h²)*** zu ganz besonders bevorzugt ***(h³),*** sind wie folgt:
   ***(h¹):*** Bevorzugt bedeutet-R⁵ Methyl, Ethyl, n-Propyl und Isopropyl.
   ***(h²):*** Besonders bevorzugt bedeutet R⁵ Methyl, Ethyl und Isopropyl.
   ***(h³):*** Ganz besonders bevorzugt bedeutet R⁵ Methyl.
***j)*** Die Definitionen ***(jⁱ)*** für R⁶ gemäß ihrer zunehmenden Präferenz, beginnend mit bevorzugt ***(j¹)*** über besonders bevorzugt ***(j²)*** zu ganz besonders bevorzugt ***(j³),*** sind wie folgt:
   ***(j¹):*** Bevorzugt bedeutet R⁶ Dimethylamino-C₁₋₃-alkyl, Cyclopropylamino-C₁₋₃-alkyl, Pyrrolidin-1-yl-C₁₋₃-alkyl, Hydroxy-C₁₋₃-alkyl, Methylsulfanyl-C₁₋₃-alkyl, Methylsulfinyl-C₁₋₃-alkyl, Methylsulfonyl-C₁₋₃-alkyl, C₁₋₄-Alkylcarbonylamino, C₃₋₅-Cycloalkylcarbonylamino, C₁₋₄-Alkylsulfonylamino, C₃₋₅-Cycloalkylsulfonylamino, C₁₋₃-Alkylsulfinyl und C₁₋₃-Alkylsulfonyl.
   ***(j²):*** Besonders bevorzugt bedeutet R⁶ Dimethylaminomethyl, Cyclopropylaminomethyl, Pyrrolidin-1-ylmethyl, Hydroxymethyl, Methylsulfanylmethyl, Methylsulfinylmethyl, Methyl-sulfonylmethyl, n-Butylcarbonylamino, Isopropylcarbonylamino, Isobutylcarbonylamino, Cyclopropylcarbonylamino, Methylsulfonylamino, Isopropylsulfonylamino, n-Butylsulfonyl-amino, Isobutylsulfonylamino, Cyclopropylsulfonylamino, Methylsulfinyl und Methylsulfonyl.
   ***(j³):*** Ganz besonders bevorzugt bedeutet R⁶ Methylsulfonylamino.
***k)*** Die Definitionen ***(kⁱ)*** für R⁷ gemäß ihrer zunehmenden Präferenz, beginnend mit bevorzugt ***(k¹)*** über besonders bevorzugt ***(k²)*** zu ganz besonders bevorzugt ***(k³),*** sind wie folgt:
   ***(k¹):*** Bevorzugt bedeutet R⁷ Isobutyl, tert-Butyl, Trifluormethyl und Pentafluorethyl.
   ***(k²):*** Besonders bevorzugt bedeutet R⁷ tert-Butyl, Trifluormethyl und Pentafluorethyl.
   ***(k³):*** Ganz besonders bevorzugt bedeutet R⁷ tert-Butyl.

Jedes **aⁱ, bⁱ, cⁱ, dⁱ, eⁱ, fⁱ, gⁱ, hⁱ, jⁱ, kⁱ** repräsentiert eine definierte, individuelle Ausführungsform der jeweiligen Gruppe so wie oben dargestellt. Gemäß der oben dargestellten Definitionen ist jede einzelne bevorzugte Ausführungsform durch den Ausdruck **(aⁱbⁱcⁱdⁱeⁱfⁱgⁱhⁱjⁱkⁱ)** vollständig charakterisiert, wobei der Index i jeweils für eine individuelle Ausführungsform steht und die einzelnen Indices i unabhängig voneinander einstellbar sind. Alle individuellen Ausführungsformen, die von dem Ausdruck in Klammem umfasst sind, wobei die Indices i gemäß der oben dargestellten Definitionen beliebig einstell- und kombinierbar sind, sollen von der vorliegenden Erfindung umfasst sein.

In der nachfolgenden Tabelle 1 sind exemplarisch, mit aufsteigender Präferenz von der ersten zur letzten Reihe, die bevorzugten Ausführungsformen E-1 bis E-14 aufgelistet. Demgemäß hat die Ausführungsform E-14, dargestellt in der letzten Reihe von Tabelle 1, die höchste Präferenz.

**Tabelle 1.**

| | R¹ | R² | R³ | R⁴ | X/Y | R^{N} | L | R⁵ | R⁶ | R⁷ |
|---|---|---|---|---|---|---|---|---|---|---|
| **E-1** | ***a¹*** | ***b¹*** | ***c¹*** | ***d¹*** | ***e¹*** | ***f¹*** | ***g¹*** | ***h¹*** | ***j¹*** | ***k¹*** |
| **E-2** | ***a¹*** | ***b²*** | ***c²*** | ***d²*** | ***e²*** | ***f²*** | ***g²*** | ***h²*** | ***j²*** | ***k²*** |
| **E-3** | ***a²*** | ***b¹*** | ***c²*** | ***d²*** | ***e²*** | ***f²*** | ***g²*** | ***h²*** | ***j²*** | ***k²*** |
| **E-4** | ***a²*** | ***b²*** | ***c²*** | ***d²*** | ***e²*** | ***f²*** | ***g²*** | ***h²*** | ***j²*** | ***k²*** |
| **E-5** | ***a²*** | ***b²*** | ***c³*** | ***d²*** | ***e³*** | ***f³*** | ***g³*** | ***h²*** | ***j²*** | ***k²*** |
| **E-6** | ***a²*** | ***b²*** | ***c³*** | ***d³*** | ***e²*** | ***f³*** | ***g³*** | ***h²*** | ***j²*** | ***k²*** |
| **E-7** | ***a²*** | ***b²*** | ***c²*** | ***d³*** | ***e³*** | ***f³*** | ***g³*** | ***h²*** | ***j²*** | ***k²*** |
| **E-8** | ***a¹*** | ***b¹*** | ***c³*** | ***d³*** | ***e³*** | ***f³*** | ***g³*** | ***h²*** | ***j²*** | ***k²*** |
| **E-9** | ***a²*** | ***b¹*** | ***c³*** | ***d³*** | ***e³*** | ***f³*** | ***g³*** | ***h²*** | ***j²*** | ***k²*** |
| **E-10** | ***a¹*** | ***b²*** | ***c³*** | ***d³*** | ***e³*** | ***f³*** | ***g³*** | ***h²*** | ***j²*** | ***k²*** |
| **E-11** | ***a²*** | ***b²*** | ***c³*** | ***d³*** | ***e³*** | ***f³*** | ***g³*** | ***h²*** | ***j²*** | ***k²*** |
| **E-12** | ***a²*** | ***b³*** | ***c³*** | ***d³*** | ***e³*** | ***f³*** | ***g³*** | ***h²*** | ***j²*** | ***k²*** |
| **E-13** | ***a³*** | ***b²*** | ***c³*** | ***d³*** | ***e³*** | ***f³*** | ***g³*** | ***h²*** | ***j²*** | ***k²*** |
| **E-14** | ***a³*** | ***b³*** | ***c³*** | ***d³*** | ***e³*** | ***f³*** | ***g³*** | ***h³*** | ***j³*** | ***k³*** |

einschließlich deren Tautomere, deren Stereoisomere und deren Gemische.

Bevorzugte Verbindungen der allgemeinen Formel I sind ausgewählt aus der Gruppe :
- N-(5-tert-Butyl-3-methansulfonylamino-2-methoxy-phenyl)-4-methyl-3-(pyridin-4-yloxy)-benzamid 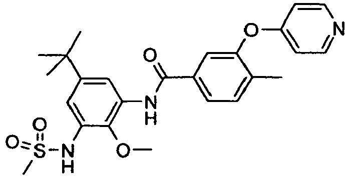
- N-(5-tert-Butyl-3-methansulfonylamino-2-methoxy-phenyl)-4-methyl-3-(2-methylamino-pyrimidin-4-yloxy)-benzamid 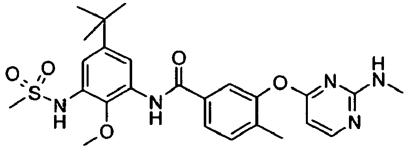
- N-(5-tert-Butyl-3-methansulfonylamino-2-methoxy-phenyl)-4-methyl-3-(6-methylamino-pyrimidin-4-yloxy)-benzamid 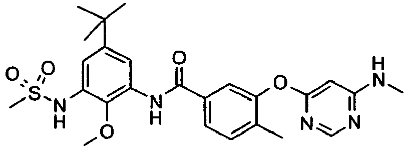
- N-(5-tert-Butyl-3-methansulfonylamino-2-methoxy-phenyl)-4-methyl-3-[2-(2-pyrrolidin-1-yl-ethylamino)-pyrimidin-4-yloxy]-benzamid 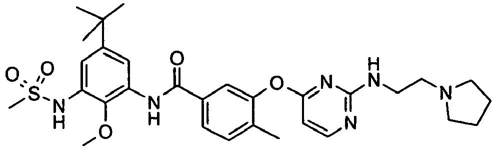
- N-(5-tert-Butyl-3-methansulfonylamino-2-methoxy-phenyl)-3-[6-(2-hydroxy-ethylamino)-pyrimidin-4-yloxy]-4-methyl-benzamid 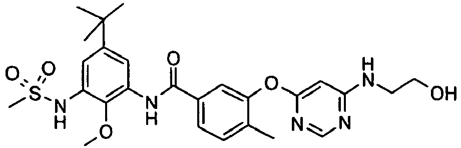
- N-(5-tert-Butyl-3-methansulfonylamino-2-methoxy-phenyl)-3-[6-(2-dimethylamino-ethylamino)-pyrimidin-4-yloxy]-4-methyl-benzamid 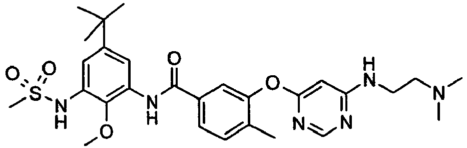
- N-(5-tert-Butyl-3-methansulfonylamino-2-methoxy-phenyl)-4-methyl-3-[6-(3-oxo-piperazin-1-yl)-pyrimidin-4-yloxy]-benzamid 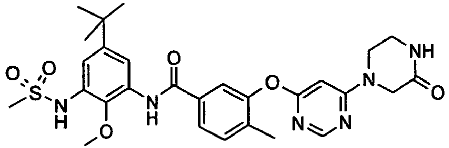
- N-(5-tert-Butyl-3-methansulfonylamino-2-methoxy-phenyl)-4-methyl-3-(6-pyrrolidin-1-yl-pyrimidin-4-yloxy)-benzamid 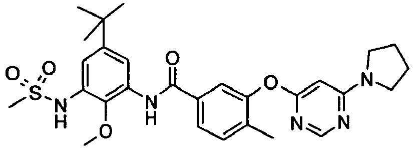
- N-(5-tert-Butyl-3-methansulfonylamino-2-methoxy-phenyl)-4-methyl-3-(6-morpholin-4-yl-pyrimidin-4-yloxy)-benzamid 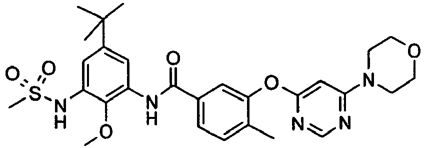
- (R)-N-(5-tert-Butyl-3-methansulfonylamino-2-methoxy-phenyl)-3-[2-(3-dimethylamino-pyrrolidin-1-yl)-pyrimidin-4-yloxy]-4-methyl-benzamid 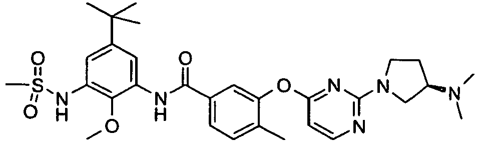
- (S)-N-(5-tert-Butyl-3-methansulfonylamino-2-methoxy-phenyl)-3-[2-(3-dimethylamino-pyrrolidin-1-yl)-pyrimidin-4-yloxy]-4-methyl-benzamid 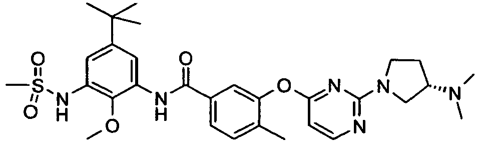
- N-(5-tert-Butyl-3-methansulfonylamino-2-methoxy-phenyl)-3-[2-(4-dimethylamino-piperidin-1-yl)-pyrimidin-4-yloxy]-4-methyl-benzamid 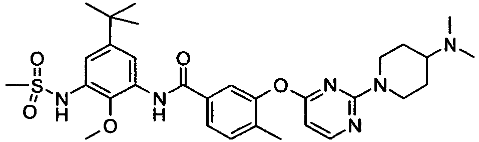
- N-(5-tert-Butyl-3-methansulfonylamino-2-methoxy-phenyl)-4-methyl-3-[2-(1-methyl-piperidin-4-ylamino)-pyrimidin-4-yloxy]-benzamid 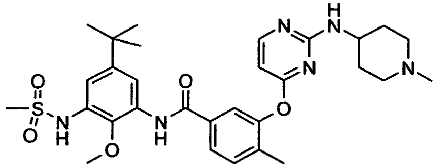
- N-(5-tert-Butyl-3-methansulfonylamino-2-methoxy-phenyl)-4-methyl-3-(2-piperazin-1-yl-pyrimidin-4-yloxy)-benzamid 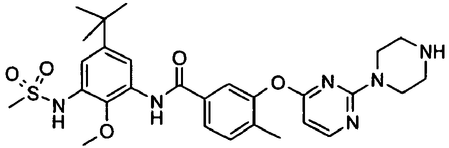
- N-(5-tert-Butyl-3-methansulfonylamino-2-methoxy-phenyl)-4-methyl-3-(6-piperazin-1-yl-pyrimidin-4-yloxy)-benzamid 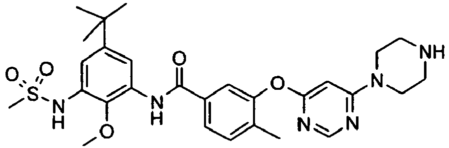
- N-(5-tert-Butyl-3-methansulfonylamino-2-methoxy-phenyl)-4-methyl-3-[6-(piperazin-1-carbonyl)-pyrimidin-4-yloxy]-benzamid 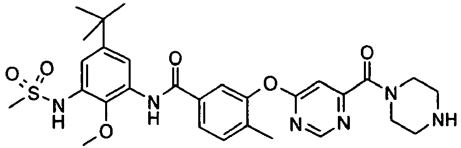
- N-(5-tert-Butyl-3-methansulfonylamino-2-methoxy-phenyl)-4-chlor-3-[6-(piperazin-1-carbonyl)-pyrimidin-4-yloxy]-benzamid 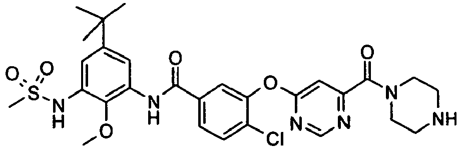
- N-(5-tert-Butyl-3-methansulfonylamino-2-methoxy-phenyl)-3-(2-piperazin-1-yl-pyrimidin-4-yloxy)-4-trifluormethyl-benzamid 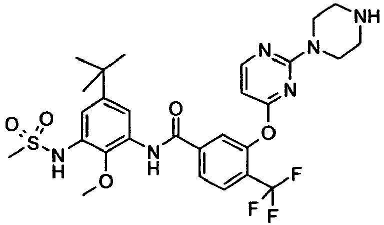
- N-(5-tert-Butyl-3-methansulfonylamino-2-methoxy-phenyl)-4-chlor-3-(2-piperazin-1-yl-pyrimidin-4-yloxy)-benzamid 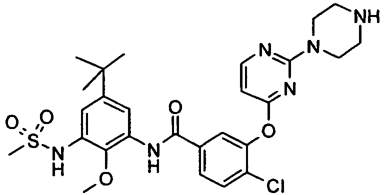
- N-(5-tert-Butyl-3-methansulfonylamino-2-methoxy-phenyl)-4-methyl-3-(2-piperazin-1-ylmethyl-pyrimidin-4-yloxy)-benzamid 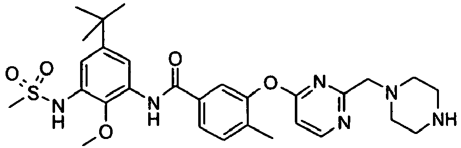
- N-(5-tert-Butyl-3-methansulfonylamino-2-methoxy-phenyl)-4-methyl-3-(6-piperazin-1-ylmethyl-pyrimidin-4-yloxy)-benzamid 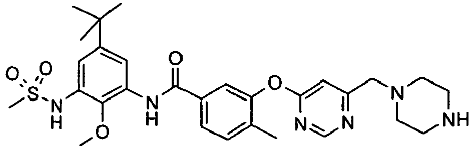
- N-(5-tert-Butyl-3-methansulfonylamino-2-methoxy-phenyl)-4-methoxy-3-(2-piperazin-1-yl-pyrimidin-4-yloxy)-benzamid 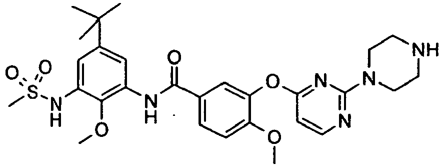
- N-(5-tert-Butyl-3-methansulfonylamino-2-methoxy-phenyl)-4-methyl-3-(2-piperazin-1-ylmethyl-pyridin-4-yloxy)-benzamid 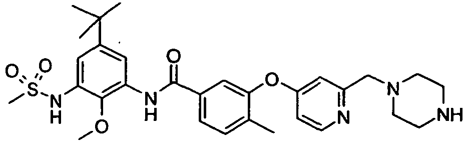
- N-(5-tert-Butyl-3-methansulfonylamino-2-methoxy-phenyl)-4-fluor-3-(2-piperazin-1-yl-pyrimidin-4-yloxy)-benzamid 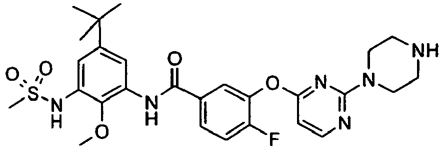
- N-(5-tert-Butyl-3-methansulfonylamino-2-methoxy-phenyl)-4-brom-3-(2-piperazin-1-yl-pyrimidin-4-yloxy)-benzamid 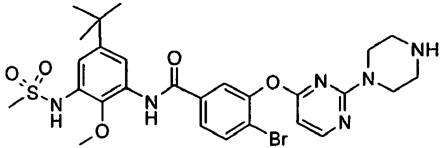
- N-(5-tert-Butyl-3-methansulfonylamino-2-methoxy-phenyl)-3-chlor-4-methyl-5-(2-piperazin-1-yl-pyrimidin-4-yloxy)-benzamid 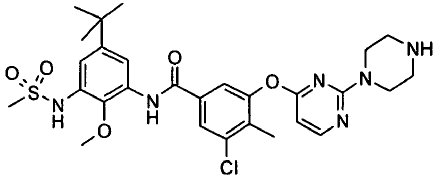
- N-(5-tert-Butyl-3-methansulfonylamino-2-methoxy-phenyl)-3-fluor-4-methyl-5-(2-piperazin-1-yl-pyrimidin-4-yloxy)-benzamid 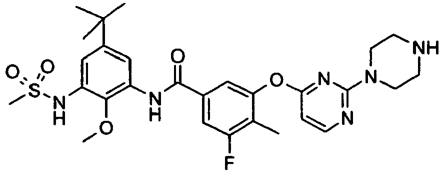
- N-(5-tert-Butyl-3-methansulfonylamino-2-methoxy-phenyl)-4-methyl-3-[2-(N-methyl-N-piperidin-4-yl-amino)-pyrimidin-4-yloxy]-benzamid 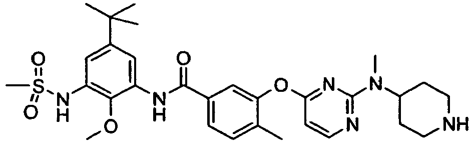
- N-(5-tert-Butyl-3-methansulfonylamino-2-methoxy-phenyl)-4-ethyl-5-(2-piperazin-1-yl-pyrimidin-4-yloxy)-benzamid 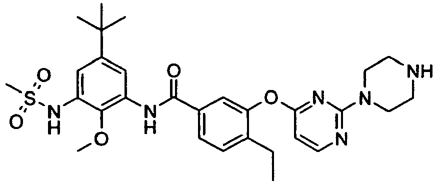
- N-(5-tert-Butyl-3-methansulfonylamino-2-methoxy-phenyl)-4-methyl-3-(2-(piperidin-4-ylamino)-pyrimidin-4-yloxy]-benzamid 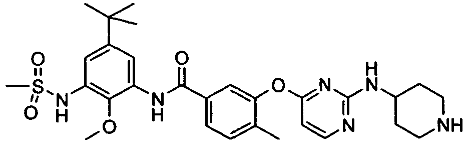
- N-(5-tert-Butyl-3-methansulfonylamino-2-methoxy-phenyl)-4-methyl-3-[6-(piperidin-4-ylamino)-pyrimidin-4-yloxy]-benzamid 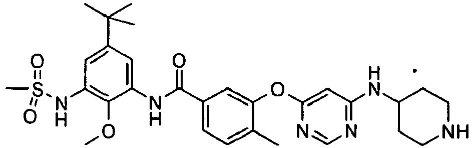
- N-(5-tert-Butyl-3-methansulfonylamino-2-methoxy-phenyl)-4-methyl-3-(6-piperidin-4-ylmethyl-pyrimidin-4-yloxy)-benzamid 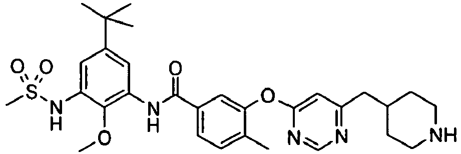
- N-(5-tert-Butyl-3-methansulfonylamino-2-methoxy-phenyl)-4-methyl-3-(2-piperidin-4-ytmethyl-pyrimidin-4-yloxy)-benzamid 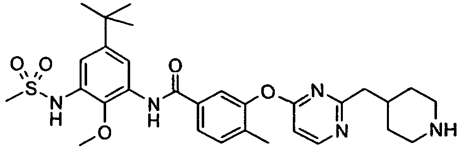
- N-(5-tert-Butyl-3-methansulfonylamino-2-methoxy-phenyl)-4-methyl-3-(2-piperidin-4-ylmethyl-pyridin-4-yloxy)-benzamid 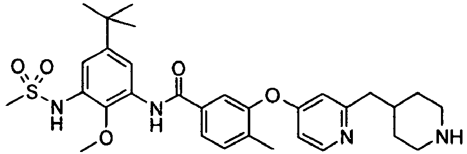
- N-(5-tert-Butyl-3-methansulfonylamino-2-methoxy-phenyl)-3-methyl-5-[2-(piperazin-1-carbonyl)-pyrimidin-4-yloxy]-benzamid 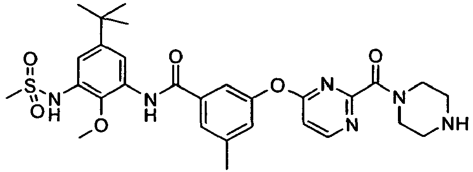
- N-(5-tert-Butyl-3-methansulfonylamino-2-methoxy-phenyl)-3-methyl-5-(2-piperazin-1-ylmethyl-pyrimidin-4-yloxy)-benzamid 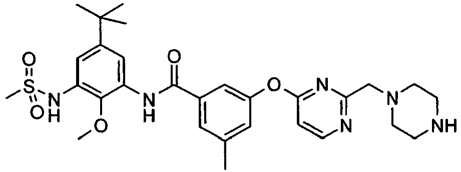
- N-(5-tert-Butyl-3-methansulfonylamino-2-methoxy-phenyl)-3-methyl-5-(pyridin-4-yloxy)-benzamid 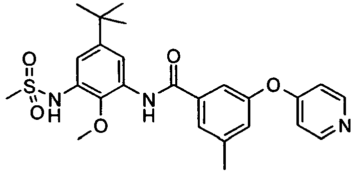
- 6-[5-(5-tert-Butyl-3-methansulfonylamino-2-methoxy-phenylaminocarbonyl)-2 -methyl-phenoxy]-pyrimidin-4-carbonsäure-methylamid 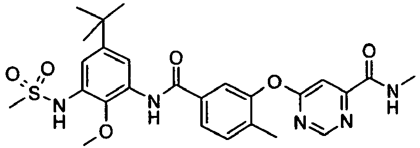
- N-(5-tert-Butyl-3-methansulfonylamino-2-methoxy-phenyl)-4-methyl-3-[6-(morpholin-4-carbonyl)-pyrimidin-4-yloxy]-benzamid 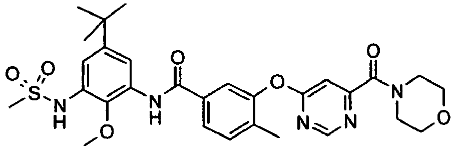
- N-(5-tert-Butyl-3-methansulfonylamino-2-methoxy-phenyl)-4-methyl-3-[6-(4-methyl-piperazin-1-carbonyl)-pyrimidin-4-yloxy]-benzamid 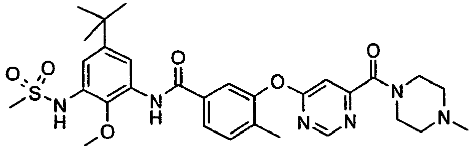
- N-(5-tert-Butyl-3-methansulfonylamino-2-methoxy-phenyl)-4-methyl-3-(6-morpholin-4-ylmethyl-pyrimidin-4-yloxy)-benzamid 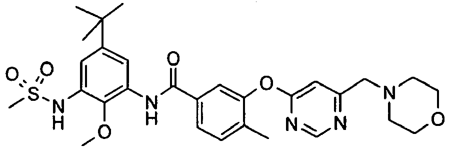
- N-(5-tert-Butyl-3-methansulfonylamino-2-methoxy-phenyl)-4-methyl-3-(6-methylaminomethyl-pyrimidin-4-yloxy)-benzamid 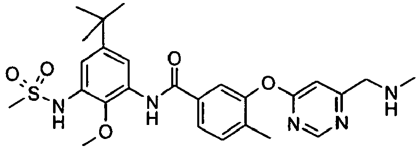
- N-(5-tert-Butyl-3-methansulfonylamino-2-methoxy-phenyl)-4-methyl-3-[6-(4-methyl-piperazin-1-ylmethyl)-pyrimidin-4-yloxy]-benzamid 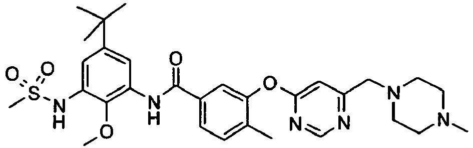
- N-(5-tert-Butyl-3-methansulfonylamino-2-methoxy-phenyl)-4-methyl-3-(2-morpholin-4-ylmethyl-pyrimidin-4-yloxy)-benzamid 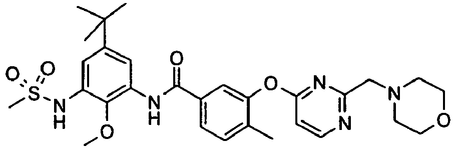
- N-(5-tert-Butyl-3-methansulfonylamino-2-methoxy-phenyl)-4-methyl-3-(2-methylaminomethyl-pyrimidin-4-yloxy)-benzamid 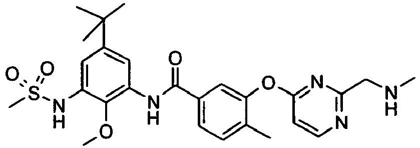
- N-(5-tert-Butyl-3-methansulfonylamino-2-methoxy-phenyl)-4-methyl-3-[2-(3-oxo-piperazin-1-ylmethyl)-pyrimidin-4-yloxy]-benzamid 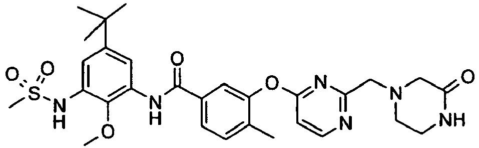
- N-(5-tert-Butyl-3-methansulfonylamino-2-methoxy-phenyl)-4-methyl-3-[2-(4-methyl-piperazin-1-ylmethyl)-pyrimidin-4-yloxy]-benzamid 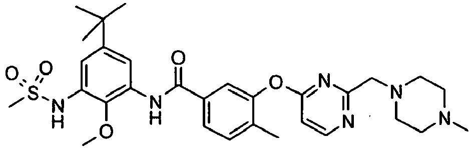
- N-(5-tert-Butyl-3-methansulfonylamino-2-methoxy-phenyl)-4-methyl-3-[6-(3-oxo-piperazin-1-ylmethyl)-pyrimidin-4-yloxy]-benzamid 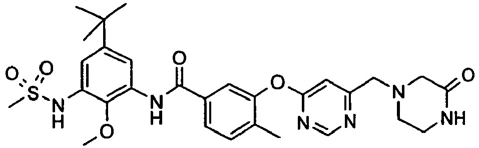
- N-(5-tert-Butyl-3-methansulfonylamino-2-methoxy-phenyl)-4-methyl-3-(6-piperazin-1-ylmethyl-pyrimidin-4-yloxy)-benzamid 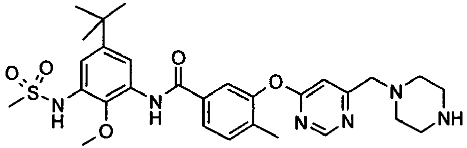
- N-(5-tert-Butyl-3-methansulfonylamino-2-methoxy-phenyl)-4-methyl-3-(2-morpholin-4-ylmethyl-pyridin-4-yloxy)-benzamid 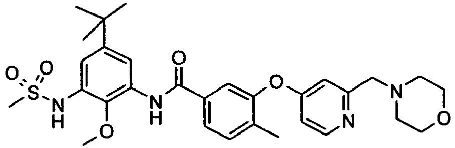
- N-(5-tert-Butyl-3-methansulfonylamino-2-methoxy-phenyl)-4-methyl-3-[2-(4-methyl-piperazin-1-ylmethyl)-pyridin-4-yloxy]-benzamid 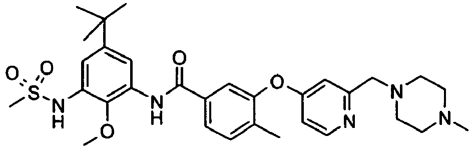
- (S)-N-(5-tert-Butyl-3-methansulfonylamino-2-methoxy-phenyl)-4-methyl-3-{2-[(tetrahydro-furan-3-ylamino)-methyl]-pyridin-4-yloxy}-benzamid 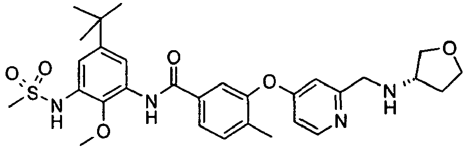
- (*R*)-N-(5-tert-Butyl-3-methansulfonylamino-2-methoxy-phenyl)-3-[2-(3-hydroxy-pyrrolidin-1-ylmethyl)-pyridin-4-yloxy]-4-methyl-benzamid 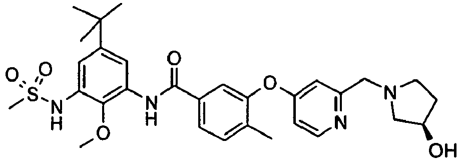
- (*S*)-N-(5-tert-Butyl-3-methansulfonylamino-2-methoxy-phenyl)-3-[2-(3-hydroxy-pyrrolidin-1-ylmethyl)-pyridin-4-yloxy]-4-methyl-benzamid 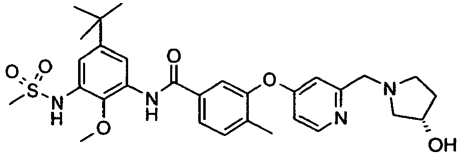
- N-(5-tert-Butyl-3-methansulfonylamino-2-methoxy-phenyl)-4-methyl-3-(2-methylaminomethyl-pyridin-4-yloxy)-benzamid 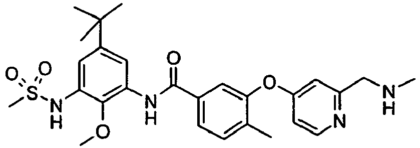
- (*S*)-N-(5-tert-Butyl-3-methansulfonylamino-2-methoxy-phenyl)-4-methyl-3-{2-[(tetrahydro-furan-3-ylamino)-methyl]-pyridin-4-yloxy}-benzamid 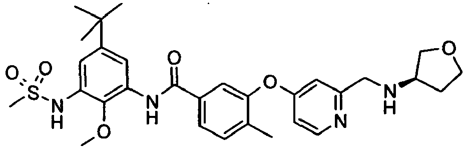
- N-(5-tert-Butyl-3-methansulfonylamino-2-methoxy-phenyl)-4-methyl-3-[2-(4-methyl-homopiperazin-1-ylmethyl)-pyrimidin-4-yloxy]-benzamid 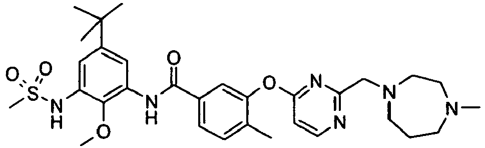
- N-(5-tert-Butyl-3-methansulfonylamino-2-methoxy-phenyl)-4-methyl-3-{2-[(1-methyl-piperidin-4-ylamino)-methyl]-pyrimidin-4-yloxy}-benzamid 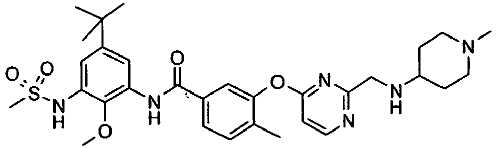
- N-(5-tert-Butyl-3-methansulfonylamino-2-methoxy-phenyl)-4-methyl-3-{2-[(1-methyl-piperidin-3-ylamino)-methyl]-pyrimidin-4-yloxy}-benzamid 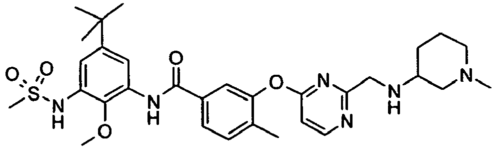
- N-(5-tert-Butyl-3-methansulfonylamino-2-methoxy-phenyl)-4-methyl-3-(2-{[methyl-(1-methyl-pyrrolidin-3-yl)-amino]-methyl}-pyrimidin-4-yloxy)-benzamid 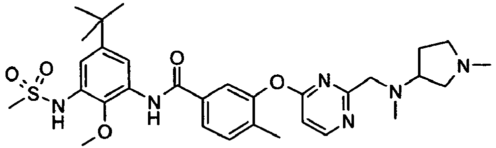
- N-(5-tert-Butyl-3-methansulfonylamino-2-methoxy-phenyl)-4-methyl-3-[2-(4-methyl-homopiperazin-1-ylmethyl)-pyridin-4-yloxy]-benzamid 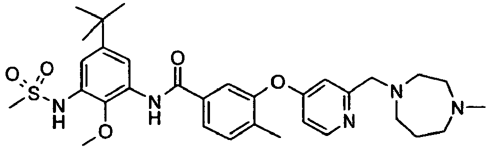
- (*S*)-N-(5-tert-Butyl-3-methansulfonylamino-2-methoxy-phenyl)-3-[2-(3-dimethylamino-pyrrolidin-1-ylmethyl)-pyrimidin-4-yloxy]-4-methyl-benzamid 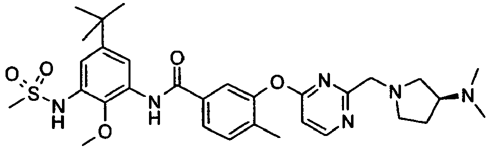
- (R)-N-(5-tert-Butyl-3-methansulfonylamino-2-methoxy-phenyl)-3-[2-(3-dimethylamino-pyrrolidin-1-ylmethyl)-pyrimidin-4-yloxy]-4-methyl-benzamid 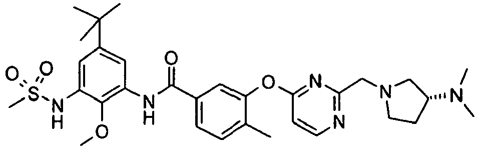
- N-(5-tert-Butyl-3-methansulfonylamino-2-methoxy-phenyl)-4-methyl-3-{2-[(1-methyl-pyrrolidin-3-ylamino)-methyl]-pyrimidin-4-yloxy}-benzamid 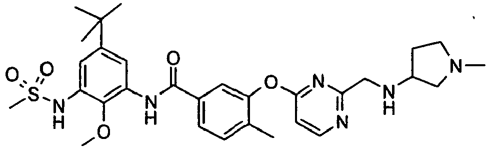
- N-(5-tert-Butyl-3-methansulfonylamino-2-methoxy-phenyl)-3-(2-{[N-(2-dimethylamino-ethyl)-N-methyl-amino]-methyl}-pyrimidin-4-yloxy)-4-methyl-benzamid 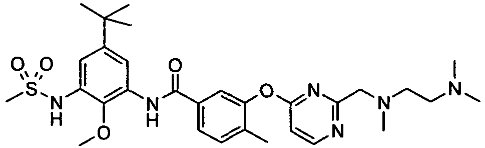
- N-(5-tert-Butyl-3-methansulfonylamino-2-methoxy-phenyl)-3-{2-[(2-dimethylamino-ethylamino)-methyl]-pyrimidin-4-yloxy}-4-methyl-benzamid 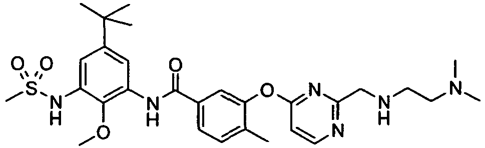
- N-(5-tert-Butyl-3-methansulfonylamino-2-methoxy-phenyl)-3-(2-{[(2-dimethylamino-ethyl)-methyl-amino]-methyl}-pyridin-4-yloxy)-4-methyl-benzamid 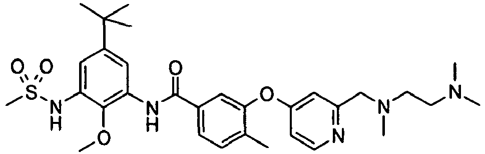
- N-(5-tert-Butyl-3-methansulfonylamino-2-methoxy-phenyl)-3-{2-[(2-dimethylamino-ethylamino)-methyl]-pyridin-4-yloxy}-4-methyl-benzamid 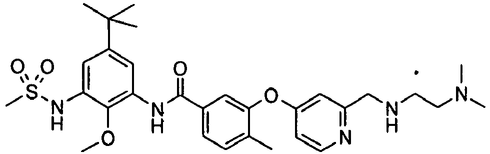
- N-(5-tert-Butyl-3-methansulfonylamino-2-methoxy-phenyl)-3-methyl-5-[2-(4-methyl-piperazin-1-ylmethyl)-pyrimidin-4-yloxy]-benzamid 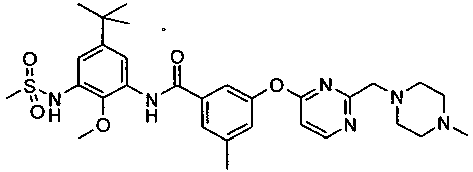
- N-(5-tert-Butyl-3-methansulfonylamino-2-methoxy-phenyl)-3-{6-[(2-dimethylamino-ethylamino)-methyl]-pyrimidin-4-yloxy}-4-methyl-benzamid 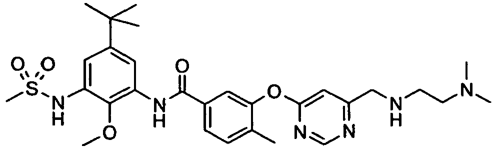
- N-(5-tert-Butyl-3-methansulfonylamino-2-methoxy-phenyl)-3-(6-{[(2-dimethylamino-ethyl)-methyl-amino]-methyl}-pyrimidin-4-yloxy)-4-methyl-benzamid 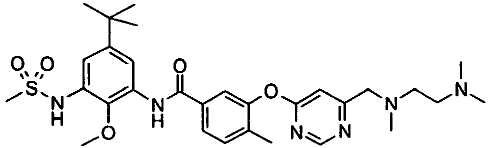
- N-(5-tert-Butyl-3-methansulfonylamino-2-methoxy-phenyl)-4-methyl-3-[6-(1-methyl-piperidin-4-ylmethyl)-pyrimidin-4-yloxy]-benzamid 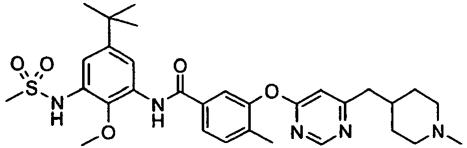
- N-(5-tert-Butyl-3-methansulfonylamino-2-methoxy-phenyl)-4-methyl-3-[2-(1-methyl-piperidin-4-ylmethyl)-pyrimidin-4-yloxy]-benzamid 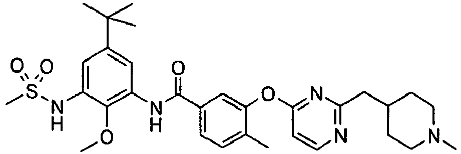
- N-(5-tert-Butyl-3-methansulfonylamino-2-methoxy-phenyl)-4-methyl-3-[2-(1-methyl-piperidin-4-ylmethyl)-pyridin-4-yloxy]-benzamid 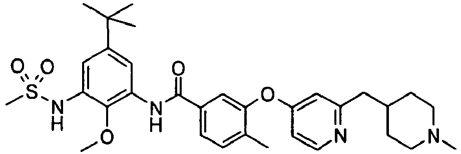
- N-(5-tert-Butyl-3-methansulfonylamino-2-methoxy-phenyl)-4-methyl-3-[2-(pyrrolidin-3-ylamino)-pyrimidin-4-yloxy]-benzamid 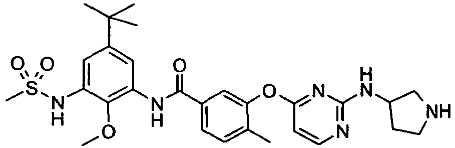
- N-(5-tert-Butyl-3-methansulfonylamino-2-methoxy-phenyl)-4-chlor-3-[2-(4-methyl-piperazin-1-ylmethyl)-pyrimidin-4-yloxy]-benzamid 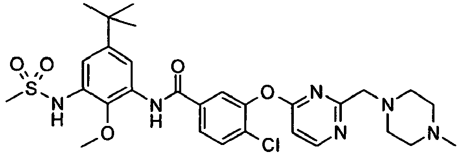
- N-(5-tert-Butyl-3-methansulfonylamino-2-methoxy-phenyl)-3-chlor-4-methyl-5-[2-(4-methyl-piperazin-1-ylmethyl)-pyrimidin-4-yloxy]-benzamid 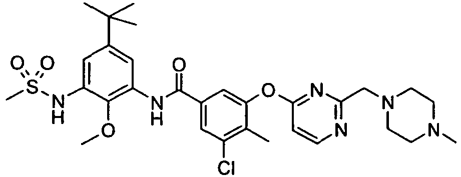
- N-(5-tert-Butyl-3-methansulfonylamino-2-methoxy-phenyl)-4-chlor-3-[6-(1-methyl-piperidin-4-ylamino)-pyrimidin-4-yloxy]-benzamid 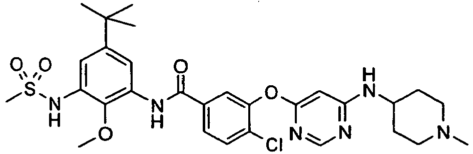
- N-(5-tert-Butyl-3-methansulfonylamino-2-methoxy-phenyl)-4-chloro-3-[2-(1-methyl-piperidin-4-ylamino)-pyridin-4-yloxy]-benzamid 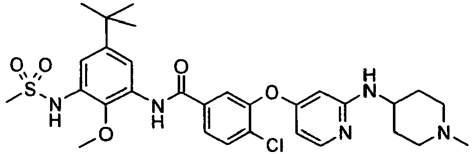
- N-(5-tert-Butyl-3-methansulfonylamino-2-methoxy-phenyl)-4-chlor-3-[6-(piperidin-4-ylamino)-pyrimidin-4-yloxy]-benzamid 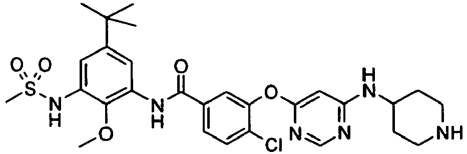
- N-(5-tert-Butyl-3-methansulfonylamino-2-methoxy-phenyl)-4-chlor-3-[2-(1-methyl-piperidin-4-ylamino)-pyrimidin-4-yloxy]-benzamid 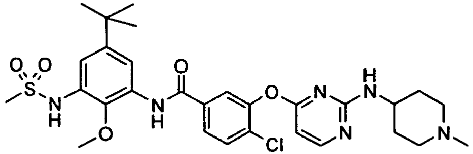
- N-(5-tert-Butyl-3-methansulfonylamino-2-methoxy-phenyl)-4-methyl-3-{2-[methyl-(1-methyl-piperidin-4-yl)-amino]-pyrimidin-4-yloxy}-benzamid 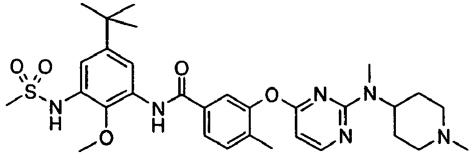
- N-(5-tert-Butyl-3-methanesulfonylamino-2-methoxy-phenyl)-4-methyl-3-{6-[methyl-(1-methyl-piperidin-4-yl)-amino]-pyrimidin-4-yloxy}-benzamid 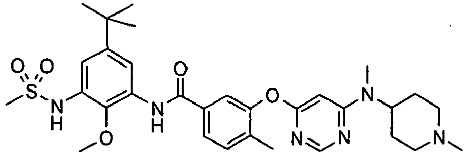
- N-(5-tert-Butyl-3-methansulfonylamino-2-methoxy-phenyl)-4-chlor-3-[2-(piperidin-4-ylamino)-pyrimidin-4-yloxy]-benzamid 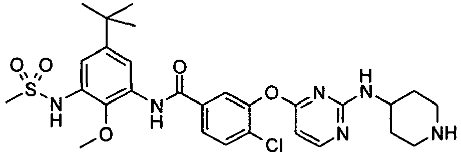
- N-(5-tert-Butyl-3-methansulfonylamino-2-methoxy-phenyl)-4-chlor-3-(2-piperidin-4-ylmethyl-pyridin-4-yloxy)-benzamid 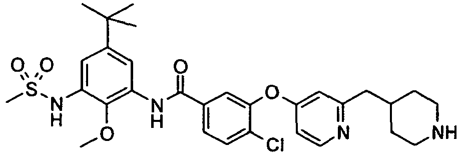
- N-(5-tert-Butyl-3-methansulfonylamino-2-methoxy-phenyl)-4-chlor-3-(2-piperidin-4-ylmethyl-pyrimidin-4-yloxy)-benzamid 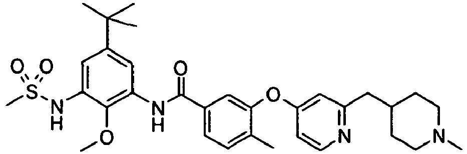
- N-(5-tert-Butyl-3-methansulfonylamino-2-methoxy-phenyl)-4-chlor-3-{6-[(2-dimethylamino-ethylamino)-methyl)-pyrimidin-4-yloxy}-benzamid 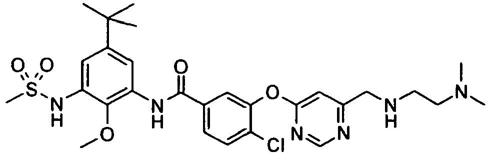
- N-(5-tert-Butyl-3-methansulfonylamino-2-methoxy-phenyl)-4-chlor-3-(6-{[(2-dimethylamino-ethyl)-methyl-amino]-methyl}-pyrimidin-4-yloxy)-benzamid 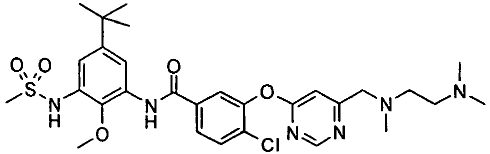
- N-(5-tert-Butyl-3-methansulfonylamino-2-methoxy-phenyl)-4-chlor-3-[2-(piperidin-4-ylamino)-pyridin-4-yloxy]-benzamid 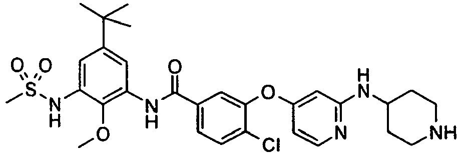
- N-(5-tert-Butyl-3-methansulfonylamino-2-methoxy-phenyl)-4-chlor-3-(6-piperazin-1-ylmethyl-pyrimidin-4-yloxy)-benzamid 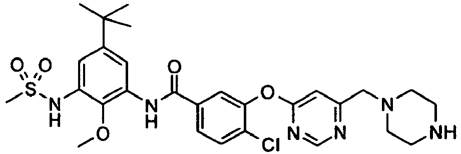
- N-(5-tert-Butyl-3-methansulfonylamino-2-methoxy-phenyl)-4-chlor-3-(6-piperidin-4-ylmethyl-pyrimidin-4-yloxy)-benzamid 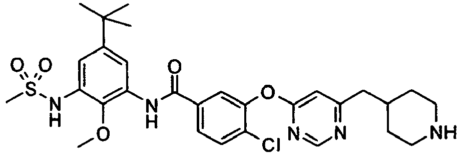
- N-(5-tert-Butyl-3-methansulfonylamino-2-methoxy-phenyl)-4-chlor-3-[6-(4-methyl-piperazin-1-ylmethyl)-pyrimidin-4-yloxy]-benzamid 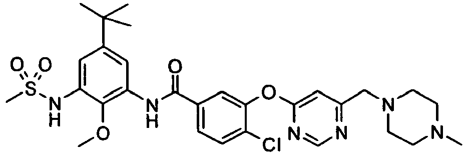
- N-(5-tert-Butyl-3-methansulfonylamino-2-methoxy-phenyl)-4-chlor-3-[6-(1-methyl-piperidin-4-ylmethyl)-pyrimidin-4-yloxy]-benzamid 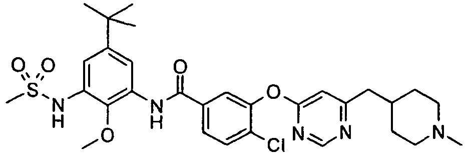
- N-(5-tert-Butyl-3-methansulfonylamino-2-methoxy-phenyl)-4-chlor-3-[2-(1-methyl-piperidin-4-ylmethyl)-pyrimidin-4-yloxy]-benzamid 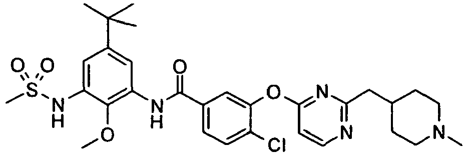
- N-(5-tert-Butyl-3-methansulfonylamino-2-methoxy-phenyl)-4-chlor-3-(2-{[(2-dimethylamino-ethyl)-methyl-amino]-methyl}-pyridin-4-yloxy)-benzamid 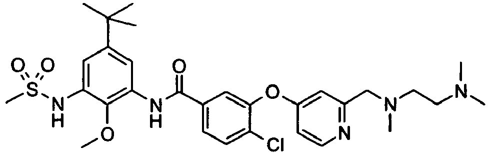
- N-(5-tert-Butyl-3-methansulfonylamino-2-methoxy-phenyl)-3-[2-(trans-4-dimethylamino-cyclohexylamino)-pyrimidin-4-yloxy]-4-methyl-benzamid 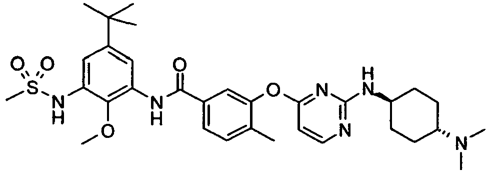
- N-(5-tert-Butyl-3-methansulfonylamino-2-methoxy-phenyl)-4-chlor-3-{2-[(2-dimethylamino-ethylamino)-methyl]-pyridin-4-yloxy}-benzamid 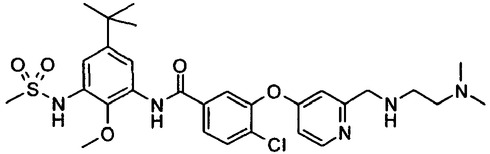
- N-(5-tert-Butyl-3-methansulfonylamino-2-methoxy-phenyl)-4-chlor-3-(2-piperazin-1-ylmethyl-pyridin-4-yloxy)-benzamid 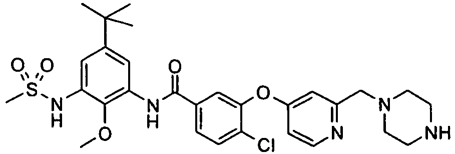
- N-(5-tert-Butyl-3-methansulfonylamino-2-methoxy-phenyl)-4-methyl-3-{2-[methyl-(1-methyl-pyrrolidin-3-yl)-amino]-pyrimidin-4-yloxy}-benzamid 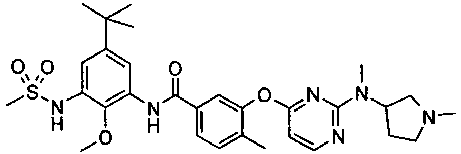
- N-(5-tert-Butyl-3-methansulfonylamino-2-methoxy-phenyl)-4-chlor-3-[2-(4-methyl-piperazin-1-ylmethyl)-pyridin-4-yloxy]-benzamid 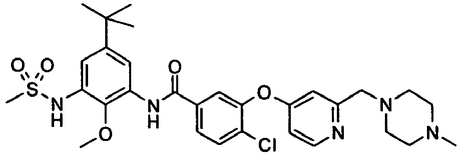
- N-(5-tert-Butyl-3-methansulfonylamino-2-methoxy-phenyl)-4-methyl-3-{6-[methyl-(1-methyl-pyrrolidin-3-yl)-amino]-pyrimidin-4-yloxy}-benzamid 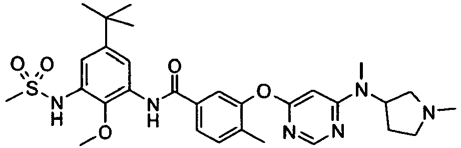
- N-(5-tert-Butyl-3-methansulfonylamino-2-methoxy-phenyl)-4-chlor-3-[2-(4-methyl-[1,4]diazepan-1-ylmethyl)-pyrimidin-4-yloxy]-benzamid 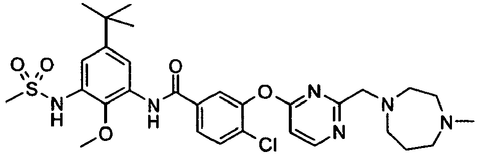
- N-(5-tert-Butyl-3-methansulfonylamino-2-methoxy-phenyl)-4-chlor-3-(2-{[(2-dimethylamino-ethyl)-methyl-amino]-methyl}-pyrimidin-4-yloxy)-benzamid 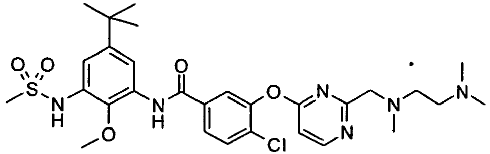
- N-(5-tert-Butyl-3-methansulfonylamino-2-methoxy-phenyl)-4-chlor-3-{2-[(2-dimethylamino-ethylamino)-methyl]-pyrimidin-4-yloxy}-benzamid 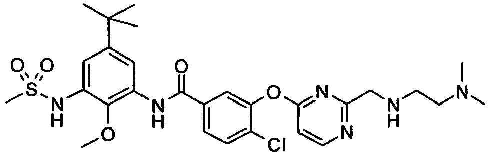
- N-(5-tert-Butyl-3-methansulfonylamino-2-methoxy-phenyl)-4-chlor-3-[6-(4-methyl-[1,4]diazepan-1-ylmethyl)-pyrimidin-4-yloxy]-benzamid 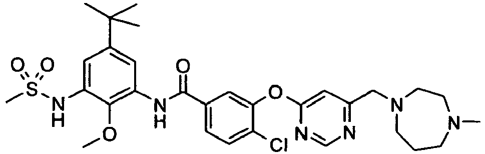
- N-(5-tert-Butyl-3-methansulfonylamino-2-methoxy-phenyl)-4-chlor-3-(2-piperazin-1-ylmethyl-pyrimidin-4-yloxy)-benzamid 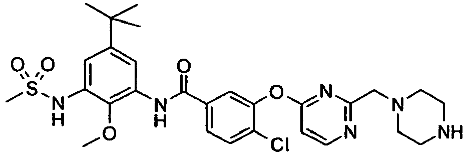
- N-(5-tert-Butyl-3-methansulfonylamino-2-methoxy-phenyl)-4-chlor-3-(6-[1,4]diazepan-1-ylmethyl-pyrimidin-4-yloxy)-benzamid 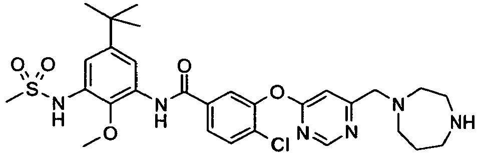
- N-(5-tert-Butyl-3-methansulfonylamino-2-methoxy-phenyl)-4-chlor-3-[2-(4-methyl-[1,4]diazepan-1-ylmethyl)-pyridin-4-yloxy]-benzamid 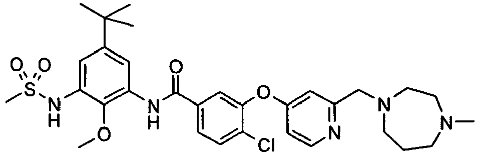
- N-(5-tert-Butyl-3-methansulfonylamino-2-methoxy-phenyl)-4-chlor-3-(2-[1,4]diazepan-1-ylmethyl-pyridin-4-yloxy)-benzamid 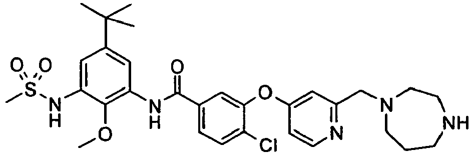
- N-(5-tert-Butyl-3-methansulfonylamino-2-methoxy-phenyl)-4-chlor-3-[2-(1-methyl-piperidin-4-ylmethyl)-pyridin-4-yloxy]-benzamid 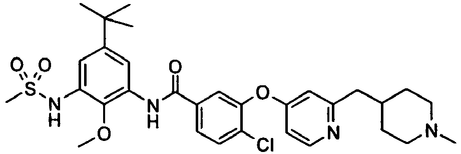
- N-(5-tert-Butyl-3-methansulfonyl-2-methoxy-phenyl)-4-methyl-3-(2-piperidin-4-ylmethyl-pyrimidin-4-yloxy)-benzamid 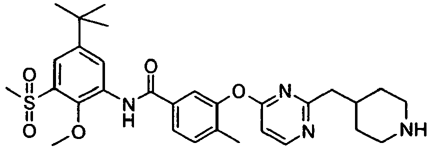
- N-(5-tert-Butyl-3-methansulfonylamino-2-methoxy-phenyl)-3-(2-[1,4]diazepan-1-ylmethyl-pyridin-4-yloxy)-4-methyl-benzamid 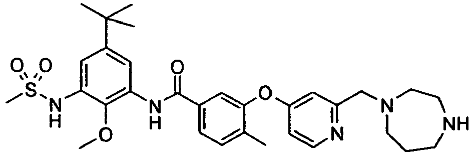
- N-(5-tert-Butyl-2-methoxy-3-methylsulfanylmethyl-phenyl)-4-methyl-3-(2-piperidin-4-ylmethyl-pyridin-4-yloxy)-benzamid 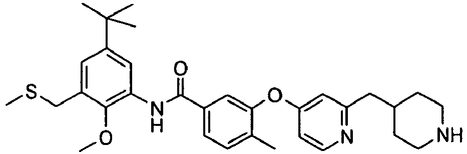
- N-(5-tert-Butyl-3-methansulfonylmethyl-2-methoxy-phenyl)-4-methyl-3-(2-piperidin-4-ylmethyl-pyridin-4-yloxy)-benzamid 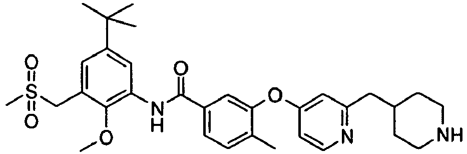
- N-(5-tert-Butyl-3-methansulfinylmethyl-2-methoxy-phenyl)-4-methyl-3-(2-piperidin-4-ylmethyl-pyridin-4-yloxy)-benzamid 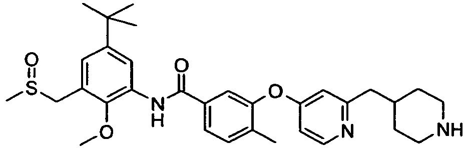
- N-[5-tert-Butyl-3-(cyclopropancarbonyl-amino)-2-methoxy-phenyl]-4-methyl-3-(2-piperidin-4-ylmethyl-pyridin-4-yloxy)-benzamid 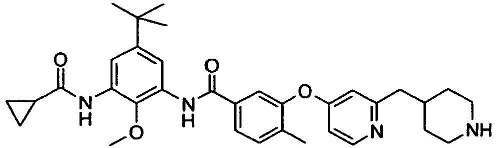
- N-(5-tert-Butyl-2-isopropoxy-3-methansulfonylamino-phenyl)-4-methyl-3-(2-piperidin-4-ylmethyl-pyridin-4-yloxy)-benzamid 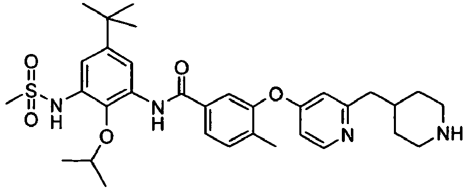
- N-(5-tert-Butyl-2-ethoxy-3-methansulfonylamino-phenyl)-4-methyl-3-(2-piperidin-4-ylmethyl-pyridin-4-yloxy)-benzamid 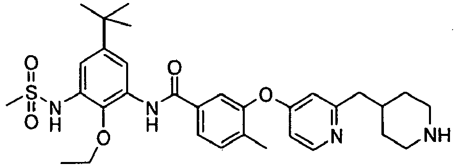
- N-[5-tert-Butyl-2-methoxy-3-(2-methyl-propan-1-sulfinylamino)-phenyl]-4-methyl-3-(2-piperidin-4-ylmethyl-pyridin-4-yloxy)-benzamid 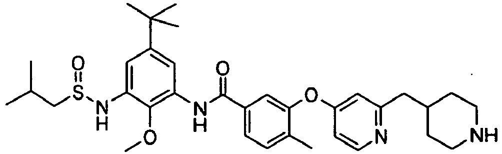
- N-[3-(Butan-1-sulfonylamino)-5-tert-butyl-2-methoxy-phenyl]-4-methyl-3-(2-piperidin-4-ylmethyl-pyridin-4-yloxy)-benzamid 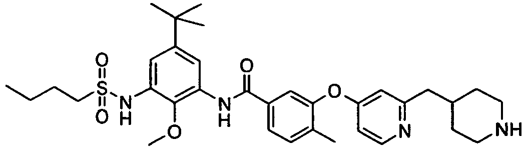
- N-(5-tert-Butyl-2-methoxy-3-pentanoylamino-phenyl)-4-methyl-3-(2-piperidin-4-ylmethyl-pyridin-4-yloxy)-benzamid 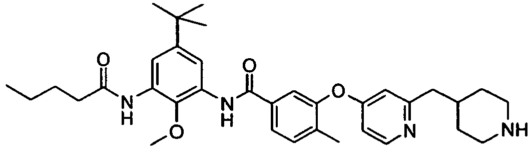
- N-(5-tert-Butyl-3-methansulfonyl-2-methoxy-phenyl)-4-methyl-3-(2-piperidin-4-ylmethyl-pyridin-4-yloxy)-benzamid 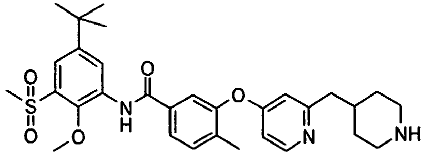
- N-[5-tert-Butyl-2-methoxy-3-(3-methyl-butyrylamino)-phenyl]-4-methyl-3-(2-piperidin-4-ylmethyl-pyridin-4-yloxy)-benzamid 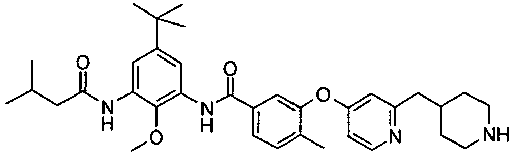
- N-(5-tert-Butyl-3-isobutyrylamino-2-methoxy-phenyl)-4-methyl-3-(2-piperidin-4-ylmethyl-pyridin-4-yloxy)-benzamid 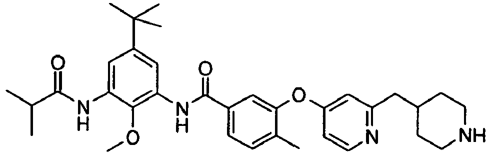
- N-(5-tert-Butyl-3-methansulfinyl-2-methoxy-phenyl)-4-methyl-3-(2-piperidin-4-ylmethyl-pyridin-4-yloxy)-benzamid 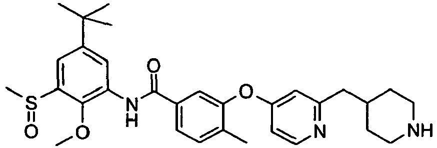
- N-(5-tert-Butyl-3-methansulfinylmethyl-2-methoxy-phenyl)-4-methyl-3-(2-piperidin-4-ylmethyl-pyridin-4-yloxy)-benzamid 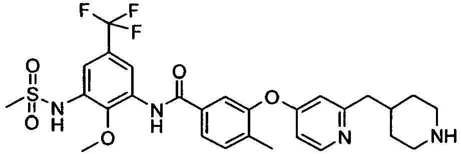
- N-(5-tert-Butyl-3-methansulfinylmethyl-2-methoxy-phenyl)-4-methyl-3-(2-piperidin-4-ylmethyl-pyridin-4-yloxy)-benzamid 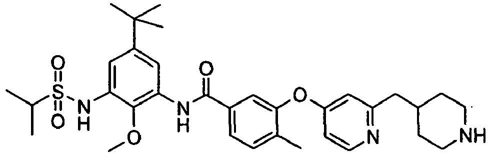
- N-(5-tert-Butyl-3-cyclopropansulfonylamino-2-methoxy-phenyl)-4-methyl-3-(2-piperidin-4-ylmethyl-pyridin-4-yloxy)-benzamid 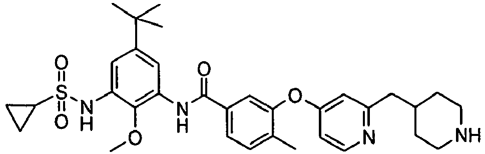
- 3-(2-Azepan-4-ylmethyl-pyridin-4-yloxy)-N-(5-tert-butyl-3-methansulfonylamino-2-methoxy-phenyl)-4-methyl-benzamid 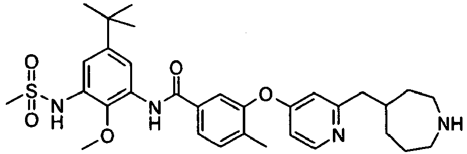
- 3-(2-Azepan-4-ylmethyl-pyrimidin-4-yloxy)-N-(5-tert-butyl-3-methansulfonylamino-2-methoxy-phenyl)-4-methyl-benzamid 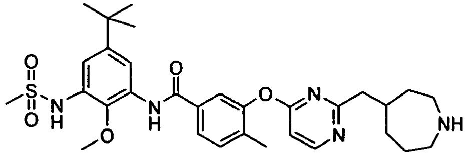
- N-(5-tert-Butyl-3-methansulfonylamino-2-methoxy-phenyl)-4-methyl-3-[2-(1-methyl-azepan-4-ylmethyl)-pyridin-4-yloxy]-benzamid 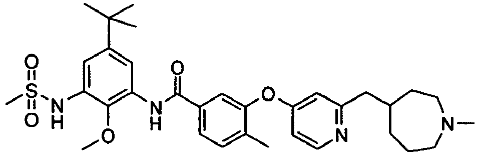
- N-(5-tert-Butyl-3-methansulfonylamino-2-methoxy-phenyl)-4-methyl-3-[2-(1-methyl-azepan-4-ylmethyl)-pyrimidin-4-yloxy]-benzamid 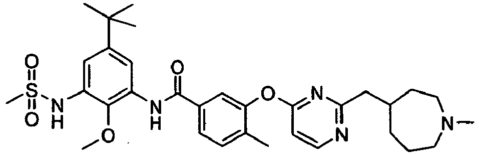
- 3-(6-Azepan-4-ylmethyl-pyrimidin-4-yloxy)-N-(5-tert-butyl-3-methanesulfonylamino-2-methoxy-phenyl)-4-methyl-benzamid 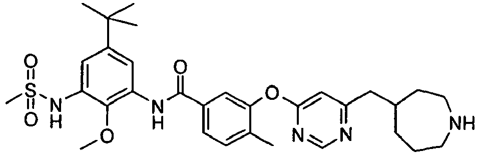
- N-(5-tert-Butyl-3-methansulfonylamino-2-methoxy-phenyl)-4-methyl-3-[6-(1-methyl-azepan-4-ylmethyl)-pyrimidin-4-yloxy]-benzamid 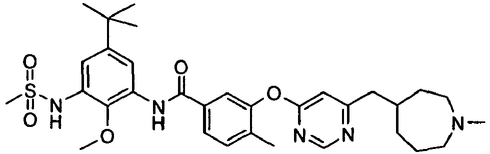
- N-(3-Methanesulfonylamino-2-methoxy-5-pentafluoroethyl-phenyl)-4-methyl-3-(2-piperidin-4-ylmethyl-pyridin-4-yloxy)-benzamide 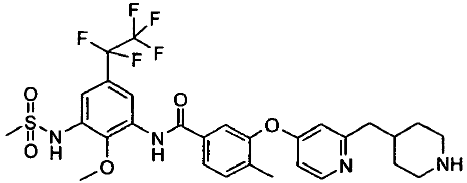
- N-(5-tert-Butyl-3-hydroxymethyl-2-methoxy-phenyl)-4-methyl-3-[2-(4-methylpiperazin-1-ylmethyl)-pyrimidin-4-yloxy]-benzamid 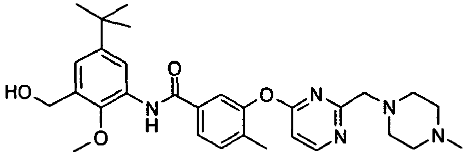
- N-(5-tert-Butyl-3-methansulfinyl-2-methoxy-phenyl)-4-methyl-3-[2-(4-methylpiperazin-1-ylmethyl)-pyrimidin-4-yloxy]-benzamid 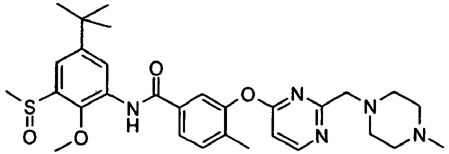
- N-(5-tert-Butyl-3-dimethylaminomethyl-2-methoxy-phenyl)-4-methyl-3-(2-piperidin-4-ylmethyl-pyridin-4-yloxy)-benzamid 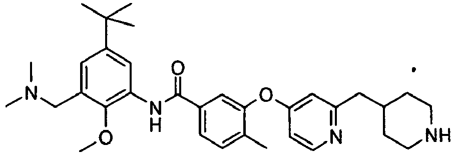
- N-(5-tert-Butyl-3-methansulfonylamino-2-methoxy-phenyl)-4-methyl-3-{2-[4-(2,2,2-trifluor-ethyl)-piperazin-1-ylmethyl]-pyridin-4-yloxy}-benzamid 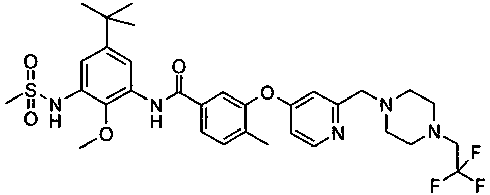
- N-(5-tert-Butyl-3-methansulfonylamino-2-methoxy-phenyl)-3-[2-(4-cyanmethyl-piperazin-1-ylmethyl)-pyridin-4-yloxy]-4-methyl-benzamid 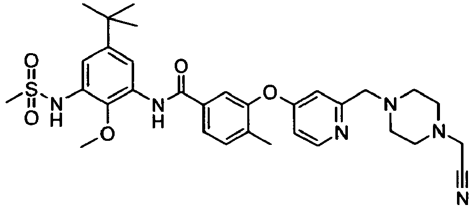
- N-(5-tert-Butyl-2-methoxy-3-pyrrolidin-1-ylmethyl-phenyl)-4-methyl-3-(2-piperidin-4-ylmethyl-pyridin-4-yloxy)-benzamid 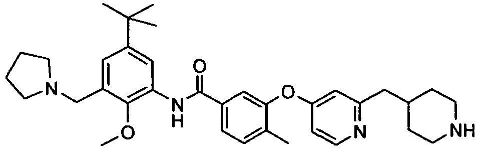
- N-(5-tert-Butyl-3-hydroxymethyl-2-methoxy-phenyl)-4-methyl-3-(2-piperazin-1-ylmethyl-pyridin-4-yloxy)-benzamid 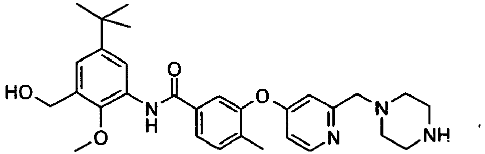
- N-(5-tert-Butyl-3-hydroxymethyl-2-methoxy-phenyl)-4-methyl-3-[2-(4-methylpiperazin-1-ylmethyl)-pyridin-4-yloxy]-benzamid 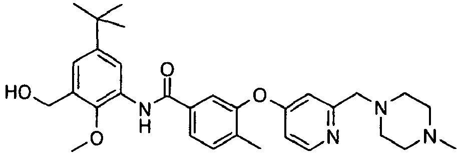
- N-(5-tert-Butyl-3-hydroxymethyl-2-methoxy-phenyl)-3-(2-[1,4]diazepan-1-ylmethyl-pyridin-4-yloxy)-4-methyl-benzamid 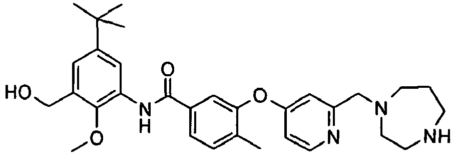
- N-(5-tert-Butyl-3-hydroxymethyl-2-methoxy-phenyl)-4-methyl-3-[2-(4-methyl-[1,4]diazepan-1-ylmethyl)-pyridin-4-yloxy]-benzamid 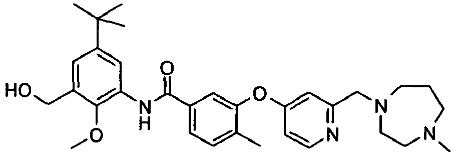
- N-(5-tert-Butyl-3-cyclopropylaminomethyl-2-methoxy-phenyl)-4-methyl-3-(2-piperidin-4-ylmethyl-pyridin-4-yloxy)-benzamid 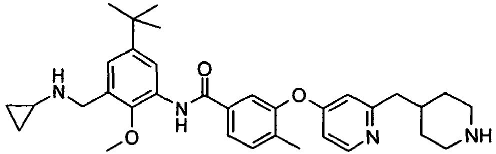
einschließlich deren Tautomere, deren Stereoisomere und deren Gemische.

Im folgenden werden Begriffe, die zuvor und nachfolgend zur Beschreibung der erfindungsgemäßen Verbindungen verwendet werden, näher definiert.

Der Begriff "substituiert", wie er hierin verwendet wird, bedeutet, dass ein oder mehrere Wasserstoffatom(e) an einem bestimmten Atom (einer Gruppe/eines Restes) durch ein Atom (eine Gruppe/ein Rest) ausgewählt aus einer bestimmten Gruppe ersetzt ist/sind, vorausgesetzt die mögliche Valenzzahl des entsprechenden Atoms wird nicht überschritten und die Substitution führt zu einer stabilen Verbindung.

Die Bezeichnung Halogen bezeichnet ein Atom ausgewählt aus der Gruppe bestehend aus F, Cl, Br und I.

Die Bezeichnung C₁₋ₙ-Alkyl, wobei n einen Wert von 2 bis 18 besitzen kann, bedeutet eine gesättigte, verzweigte oder unverzweigte Kohlenwasserstoffgruppe mit 1 bis n C-Atomen. Beispiele solcher Gruppen umfassen Methyl, Ethyl, n-Propyl, iso-Propyl, Butyl, iso-Butyl, sec-Butyl, tert-Butyl, n-Pentyl, iso-Pentyl, neo-Pentyl, tert-Pentyl, n-Hexyl, iso-Hexyl, etc.

Die Bezeichnung C₂₋ₙ-Alkenyl, wobei n einen Wert von 2 bis 18 besitzen kann, bedeutet eine gesättigte, verzweigte oder unverzweigte Kohlenwasserstoffgruppe mit 1 bis n C-Atomen. Beispiele solcher Gruppen umfassen Ethenyl, Propenyl, Butenyl, Pentenyl, etc

Der Begriff C₂₋ₙ-Alkinyl, wobei n einen Wert von 3 bis 6 besitzt, bezeichnet eine verzweigte oder unverzweigte Kohlenwasserstoffgruppe mit 2 bis n C-Atomen und einer C≡C-Dreifachbindung. Beispiele solcher Gruppen umfassen Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl, 3-Methyl-1-butinyl, 1-Hexinyl, 2-Hexinyl, 3-Hexinyl, 4-Hexinyl-, 5-Hexinyl etc..

Der Begriff C₁₋ₙ-Alkoxy oder C₁₋ₙ-Alkyloxy bezeichnet eine C₁₋ₙ-Alkyl-O-Gruppe, worin C₁₋ₙ-Alkyl wie oben definiert ist. Beispiele solcher Gruppen umfassen Methoxy, Ethoxy, n-Propoxy, iso-Propoxy, n-Butoxy, iso-Butoxy, sec-Butoxy, tert-Butoxy, n-Pentoxy, iso-Pentoxy, neo-Pentoxy, tert-Pentoxy, n-Hexoxy, iso-Hexoxy etc..

Der Begriff C₁₋ₙ-Alkylcarbonyl bezeichnet eine C₁₋ₙ-Alkyl-C(=O)-Gruppe, worin C₁₋ₙ-Alkyl wie oben definiert ist. Beispiele solcher Gruppen umfassen Methylcarbonyl, Ethylcarbonyl, n-Propylcarbonyl, iso-Propylcarbonyl, n-Butylcarbonyl, iso-Butylcarbonyl, sec-Butylcarbonyl, tert-Butylcarbonyl, n-Pentylcarbonyl, iso-Pentylcarbonyl, neo-Pentylcarbonyl, tert-Pentylcarbonyl, n-Hexylcarbonyl, iso-Hexylcarbonyl, etc..

Der Begriff C₃₋ₙ-Cycloalkyl bezeichnet eine gesättigte mono-, bi-, tri- oder spirocarbocyclische Gruppe mit 3 bis n C-Atomen, wobei n für 4 bis 15, bevorzugt 8, steht. Beispiele solcher Gruppen umfassen Cyclopropyl, Cyclobutyl, Cyclopentyl, Cyclohexyl, Cycloheptyl, Cyclooctyl, Cyclononyl, Cyclododecyl, Bicyclo[3.2.1.]octyl, Spiro[4.5]decyl, Norpinyl, Norbonyl, Norcaryl, Adamantyl, etc.. Vorzugsweise umfasst der Begriff C₃₋₇-Cycloalkyl gesättigte monocyclische Gruppen. Entsprechend bezeichnet C₃₋ₙ Heterocycloalkyl gesättigte mono-, bi- oder spirocarbocyclische Gruppen mit 3-15, bevorzugt 3-8, C-Atomen und mindestens einem Heteroatom ausgewählt aus N, O oder S im Ring.

Der Begriff C₃₋ₙ-Cycloalkyloxy bezeichnet eine C₃₋ₙ-Cycloalkyl-O-Gruppe, worin C₃₋ₙ-Cycloalkyl wie oben definiert ist. Beispiele solcher Gruppen umfassen Cyclopropyloxy, Cyclobutyloxy, Cyclopentyloxy, Cyclohexyloxy, Cycloheptyloxy, etc..

Der Begriff C₅₋ₙ-Cycloalkenyl bezeichnet eine C₅₋ₙ-Cycloalkyl-Gruppe, die wie oben definiert ist und zusätzlich mindestens eine ungesättigte C=C-Doppelbindung hat.

Der Begriff C₃₋ₙ-Cycloalkylcarbonyl bezeichnet eine C₃₋ₙ-Cycloalkyl-C(=O)-Gruppe, worin C₃₋ₙ-Cycloalkyl wie oben definiert ist. n ist bevorzugt 7.

Der Begriff C₃₋ₙ-Heterocycloalkylcarbonyl bezeichnet eine C₃₋ₙ-Heteroccyloalkyl-C(=O)-Gruppe, worin C₃₋ₙ-Heterocycloalkyl wie oben definiert ist. n ist bevorzugt 7.

Der Begriff C₃₋ₙ-Cycloheteroalkyl bezeichnet eine gesättigte mono-, bi-, tri- oder spirocarbocyclische Gruppe mit 3-m bis n-m C Atomen, wobei n für 4 bis 12 C-Atome und m für 1 bis 3 Heteroatome, unabhängig voneinander ausgewählt aus NR^{N}, O, S, SO und SO₂, steht, welche zusätzlich eine Carbonylgruppe beinhalten kann. Beispiele solcher Gruppen umfassen Aziridinyl, Oxiranyl, Azetidinyl, Oxetanyl, Pyrrolidinyl, Tetrahydrofuranyl, Piperidinyl, Tetrahydropyranyl, Azepanyl, Piperazinyl, Morpholinyl, Tetrahydrofuranonyl, Tetrahydropyranonyl, Pyrrolidinonyl, Piperidinonyl, Piperazinonyl, Morpholinonyl. Vorzugsweise umfasst der Begriff C₃₋₇-Cycloheteroalkyl gesättigte monocyclische Gruppen mit einem oder zwei Heteroatomen.

Der Begriff Aryl bezeichnet aromatische Kohlenstoffringe. Bevorzugt im Rahmen der Erfindung ist C₅₋₁₀ Aryl. Bevorzugte Beispiele sind Phenyl und Naphtyl.

Der Begriff Heteroaryl bezeichnet aromatische Kohlenstoffringe mit mindestens einem Heteroatom im Ring ausgewählt aus N, O uns S. Bevorzugt im Rahmen der Erfindung ist C₅₋₁₀ Heteroaryl. Bevorzugte Beispiele sind Pyridin, Pyrimidin.

Der Begriff Tri-(C₁₋₄-alkyl)silyl umfasst Silyl-Gruppen, die gleiche oder zwei oder drei verschiedene Alkylgruppen aufweisen.

Der Begriff Di-(C₁₋₄-alkyl)amino umfasst Amino-Gruppen, die gleiche oder zwei verschiedene Alkylgruppen aufweisen.

Der Begriff N-Heterocycloalkyl bezeichnet einen gesättigten carbocyclischen Ring, der mindestens ein Stickstoff-Atom im Ring aufweist und zusätzlich weitere Stickstoff-Gruppen, O-und/oder S-Atome im Ring aufweisen kann. Beispiele solcher N-Heterocycloalkyl-Gruppen sind Pyrrolidin, Piperidin, Piperazin, Homopiperazin und Morpholin.

Falls in Gruppen, beispielsweise in R¹, R², R³ oder R⁴, vorkommende Alkyl-Reste substituiert, beispielsweise fluoriert, sein können, so umfasst dies nicht nur Alkyl-Reste in den Gruppen die unmittelbar Alkyl bedeuten, sondern auch andere, Alkyl-Reste/Fragmente aufweisende Bedeutungen, wie beispielsweise Alkyloxy, Alkylcarbonyl, Alkoxyalkyl, etc.. So umfasst beispielsweise R¹, R², R³ oder R⁴ in der Bedeutung Alkyloxy, wobei Alkylreste teilweise oder vollständig fluoriert sein können, auch Difluormethoxy und Trifluormethoxy. Das gleiche gilt für Alkyl-Reste/Fragmente, in denen eine CH₂-Gruppe durch ein Atom, z.B. O oder S, oder eine Gruppe, z.B. NR^{N}, CO oder SO₂, ersetzt sein kann, was beispielsweise in der Bedeutung Hydroxy-C₁₋₃-alkyl, wobei in Alkylresten eine CH₂-Gruppe durch CO ersetzt sein kann, auch Carboxy, Carboxymethyl, Hydroxymethylcarbonyl, Carboxyethyl, Hydroxymethylcarbonylmethyl und Hydroxyethylcarbonyl umfasst.

Die vorstehend und nachfolgend verwendete Schreibweise, bei der in einer Phenylgruppe eine Bindung eines Substituenten zur Mitte des Phenylrings hin dargestellt ist, bedeutet, sofern nicht anders angegeben, dass dieser Substituent an jede freie, ein H-Atom tragende Position des Phenylrings gebunden sein kann.

Alle in der vorliegenden Schrift erwähnten Elemente/Atome, einschließlich der, die Bestandteil einer Gruppe sind, umfassen alle stabilen isotopen Formen des jeweiligen Elements. So umfasst z.B. die Nennung des Atoms/Elements Wasserstoff neben Wasserstoff selbst auch immer das stabile Isotop Deuterium.

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym p38 MAP-Kinase.

Die biologischen Eigenschaften der neuen Verbindungen können wie folgt geprüft werden:
Die Hemmung der p38 MAP-Kinase vermittelten Signalübertragung kann z.B. mittels eines enzymatischen Assays nachgewiesen werden. Dabei wird die Menge eines durch die Kinase phosphorylierten Substrats quantifiziert. Der Test wird wie folgt durchgeführt:

### Enzymatischer Assay:

Die Kinasereaktion wird in Anwesenheit von HEPES (20 mM, pH 7), MgCl₂ (10 mM), DTT (1 mM) und TWEEN20 (0.01%) durchgeführt. Zuerst werden in einem Reaktionsgefäß Dimethylsulfoxid oder Inhibitor (Endkonzentration: 1 µM) gelöst in Dimethylsulfoxid vorgelegt. Danach wird die aktivierte p38 MAP-Kinase (Endkonzentration 1 nM) zugegeben und das Gemisch für 4 h bei Raumtemperatur inkubiert. Danach wird ein Gemisch aus Substrat (GSTtagged ATF2) und ATP zugegeben und alles zusammen für eine weitere Stunde inkubiert (Endkonzentration ATP: 100 µM; Endkonzentration ATF2: 10 nM).

Die Konzentration von phosphoryliertem ATF2 wird durch Chemolumineszenz induzierte Lichtemission quantifiziert. Dazu wird zu dem Reaktionsgemisch ein Glutathion-Donor-Bead (Endkonzentration: 5 µg/ml), welcher an das Gluthathion am ATF2 bindet, und phospho-ATF2 Antikörper hinzugegeben (Endkonzentration: 3 nM; dieser bindet die durch die Kinasereaktion angefügte Phosphatgruppe) an welchen ein Protein A Acceptor Bead (Endkonzentration: 5 µg/ml) bindet. Diese Komponenten sind in einem Puffer gelöst, welcher 20 mM HEPES pH 7.0, 200 mM NaCL, 80 mM EDTA sowie 0.3% BSA enthält. Dieses Reaktionsgemisch wird für 1 Stunde bei Raumtemperatur im Dunkeln inkubiert. Kommen diese beiden Beads in räumliche Nähe wird sichtbares Licht emittiert, welches in einem Photometer bei 520-620nm gemessen wird.

### Ausweitung:

Zur Bestimmung der inhibitorischen Aktivität der erfindungsgemäßen Verbindungen wird berechnet um wieviel Prozent die Kinaseaktivität bei einer festen Inhibitorkonzentration von 1µM inhibiert ist. Die maximale Aktivität wird dabei durch die nicht inhibierte Kinase bestimmt. Die minimale Aktivität bzw. unspezifische Hintergrundaktivität wird durch einen Reaktionsansatz ohne Kinase ermittelt. Die erfindungsgemäßen Verbindungen der allgemeinen Formel I zeigen beispielsweise Inhibitionswerte von >50%, vorzugsweise von >90%.

In Tabelle 2 ist das Maß der Inhibition des Enzyms p38 MAP-Kinase -so wie oben beschrieben bestimmt- der erfindungsgemäßen Verbindungen, die im Abschnitt "Herstellung der Endverbindungen" dargestellt sind, zusammengefasst.

**Tabelle 2. Hemmwirkung von erfindungsgemäßen Verbindungen der allgemeinen Formel I auf das Enzym p38 MAP-Kinase bei 1 µM Inhibitorkonzentration**

| Beispiel | %Hemmwirkung bei 1 µM | Beispiel | %Hemmwirkung bei 1 µM | Beispiel | %Hemmwirkung bei 1 µM | Beispiel | %Hemmwirkung bei 1 µM |
|---|---|---|---|---|---|---|---|
| 1 | 90,8 | 5(11) | 98,4 | 5(47) | 97,3 | 8(23) | 97,8 |
| 2 | 97,7 | 5(12) | 98,6 | 5(48) | 99,4 | 8(24) | 92,4 |
| 3 | 98,1 | 5(13) | 98,2 | 5(49) | 99,2 | 8(25) | 98,6 |
| 3(1) | 97,0 | 5(14) | 98,5 | 5(50) | 99,6 | 8(26) | 97,7 |
| 4 | 97,7 | 5(15) | 96,0 | 5(52) | 99,5 | 8(27) | 98,4 |
| 4(1) | 96,8 | 5(16) | 98,9 | 5(53) | 97,0 | 8(28) | 81,8 |
| 4(2) | 94,8 | 5(17) | 98,2 | 5(54) | 99,7 | 8(29) | 89,0 |
| 4(3) | 96,3 | 5(18) | 98,7 | 5(55) | 99,6 | 8(30) | 91,3 |
| 4(4) | 97,5 | 5(19) | 99,7 | 6 | 94,4 | 8(31) | 86,5 |
| 4(5) | 97,4 | 5(20) | 99,5 | 7 | 95,6 | 8(32) | 92,6 |
| 4(6) | 98,0 | 5(21) | 88,9 | 7(2) | 96,3 | 8(33) | 97,1 |
| 4(7) | 98,6 | 5(22) | 85,7 | 7(3) | 98,8 | 8(34) | 99,3 |
| 4(8) | 98,6 | 5(23) | 98,3 | 7(4) | 98,1 | 8(35) | 98,6 |
| 4(9) | 98,6 | 5(24) | 97,9 | 7(5) | 98,5 | 8(36) | 82,6 |
| 4(10) | 100 | 5(25) | 97,6 | 8 | 95,0 | 8(37) | 91,3 |
| 4(11) | 99,1 | 5(26) | 97,6 | 8(1) | 55,9 | 8(38) | 98,8 |
| 4(12) | 95,8 | 5(27) | 99,2 | 8(2) | 98,7 | 8(39) | 98,9 |
| 4(13) | 96,3 | 5(28) | 99,0 | 8(3) | 95,2 | 9 | 98,4 |
| 4(14) | 99,4 | 5(29) | 99,0 | 8(4) | 66,0 | 9(1) | 99,9 |
| 4(15) | 99,6 | 5(30) | 99,0 | 8(5) | 95,8 | 9(2) | 98,5 |
| 4(16) | 99,4 | 5(31) | 99,4 | 8(6) | 98,7 | 10 | 99,6 |
| 4(17) | 98,7 | 5(32) | 97,9 | 8(7) | 94,1 | 11 | 99,2 |
| 4(18) | 99,0 | 5(33) | 99,2 | 8(9) | 98,4 | 11(1) | 99,4 |
| 4(19) | 98,6 | 5(34) | 99,1 | 8(10) | 98,9 | 11(2) | 95,4 |
| 4(20) | 95,6 | 5(35) | 98,3 | 8(11) | 96,7 | 11(3) | 99,5 |
| 5 | 99,0 | 5(36) | 99,3 | 8(12) | 97,0 | 11(4) | 95,1 |
| 5(1) | 96,8 | 5(37) | 99,3 | 8(13) | 96,8 | 12 | 97,5 |
| 5(2) | 93,2 | 5(38) | 98,8 | 8(14) | 91,1 | 12(1) | 99,4 |
| 5(3) | 95,0 | 5(39) | 99,4 | 8(15) | 95,9 | 12(2) | 98,7 |
| 5(4) | 45,3 | 5(40) | 99,5 | 8(16) | 98,7 | 12(3) | 99,6 |
| 5(5) | 98,3 | 5(41) | 99,6 | 8(17) | 96,7 | 12(4) | 99,3 |
| 5(6) | 98,3 | 5(42) | 99,5 | 8(18) | 98,6 | 12(5) | 99,5 |
| 5(7) | 97,8 | 5(43) | 98,6 | 8(19) | 97,0 | 12(6) | 99,6 |
| 5(8) | 98,9 | 5(44) | 93,2 | 8(20) | 99,4 | 12(7) | 99,8 |
| 5(9) | 98,5 | 5(45) | 99,6 | 8(21) | 98,5 | 13 | 98,2 |
| 5(10) | 94,6 | 5(46) | 99,6 | 8(22) | 98,3 | 13(1) | 97,0 |

### Indikationen

Im Hinblick auf die Fähigkeit die p38 MAP-Kinase-Aktivität zu hemmen, sind die erfindungsgemäßen Verbindungen der allgemeinen Formel I und ihre entsprechenden pharmazeutisch akzeptablen Salze prinzipiell geeignet, alle diejenigen Zustände oder Krankheiten zu behandeln und/oder vorbeugend zu behandeln, die durch eine Hemmung der p38 MAP-Kinase-Aktivität beeinflusst werden. Die erfindungsgemäßen Verbindungen eignen sich z.B. zur Verbesserung eines durch p38 MAP-Kinase vermittelten abnormen Zytokinspiegels, im Besonderen zur Regulierung einer Überproduktion der Zytokine IL-1, IL-4, IL-8 und TNF-α. Daher sind die erfindungsgemäßen Verbindungen zur Prophylaxe oder Behandlung von Krankheiten, insbesondere bei Atemwegserkrankungen, gastrointestinalen Erkrankungen oder Beschwerden, entzündlichen Krankheiten (insbesondere der Atemwege, der Gelenke, der Haut oder der Augen), Autoimmunerkrankungen, destruktiven Störungen der Knochen, Proliferationsstörungen, Störungen der Angiogenese, Neurodegenerativen Erkrankungen, Infektionskrankheiten und Viruserkrankungen sowie Erkrankungen des periphären oder zentralen Nervensystems anwendbar.

Bevorzugt genannt sei die Vorbeugung und Behandlung von Atemwegs- oder Lungenerkrankungen, welche mit einer erhöhten Schleimproduktion, Entzündungen und/oder obstruktiven Erkrankungen der Atemwege einhergehen wie z.B. akute, allergische oder chronische Bronchitis, chronisch obstruktive Bronchitis (COPD), Husten, Lungenemphysem, allergische oder nicht-allergische Rhinitis oder Sinusitis, chronische Rhinitis oder Sinusitis, Asthma, Alveolitis, Farmers Krankheit, hyperreaktive Atemwege, infektiöse Bronchitis oder Pneumonitis, pediatrisches Asthma, Bronchiektasien, Lungenfibrose, ARDS (akutes Atemnotsyndrom des Erwachsenen), Bronchialödem, Lungenödem, Bronchitis, Pneumonie oder interstitielle Pneumonie ausgelöst durch Ursachen wie Aspiration, Inhalation von toxischen Gasen oder Bronchitis, Pneumonie oder interstitielle Pneumonie ausgelöst durch Herzinsuffizienz, Bestrahlung, Chemotherapie, Zystische Fibrose oder Mukoviszidose und alpha1-Antitrypsin-Mangel.

Des Weiteren sei die Behandlung von entzündlichen Erkrankungen des Gastrointestinaltraktes wie z.B. akute oder chronische entzündliche Veränderungen bei Gallenblasenentzündung, Morbus Crohn, Colitis ulcerosa, entzündliche Pseudopolypen, juvenile Polypen, Colitis cystica profunda, Pneumatosis cystoides intestinales, Erkrankungen der Gallengänge und Gallenblase, z.B. Gallensteine und Konglomerate, entzündliche Erkrankungen der Gelenke wie rheumatoide Arthritis oder entzündliche Erkrankungen der Haut (z.B. Psoriasis) und der Augen bevorzugt genannt.

Außerdem sei die Vorbeugung und Behandlung von Erkrankungen des periphären oder zentralen Nervensystems wie z.B. Alzheimersche Erkrankung, Parkinsonsche Erkrankung, akute und chronische Multiple Sklerose, akute und chronische Schmerzzustände sowie Verletzungen des Gehirns hervorgerufen durch Schlaganfall, Hypoxie oder Schädel-Him-Trauma bevorzugt genannt.

Ganz besonders sind die erfindungsgemäßen Verbindungen, einschließlich deren physiologisch verträglichen Salze, zur Prophylaxe oder Behandlung von Atemwegserkrankungen, insbesondere COPD und Asthma geeignet.

### Kombinationen

Auf Grund ihrer biologischen Eigenschaften können die erfindungsgemäßen Verbindungen der allgemeinen Formel I allein oder in Kombination mit anderen erfindungsgemäßen Wirkstoffen der Formel I zur Anwendung gelangen. Gegebenenfalls können die Verbindungen der Formel I auch in Kombination mit einem oder mehreren weiteren pharmakologisch aktiven Wirkstoffen eingesetzt werden. Für die Behandlung von Atemwegserkrankungen können die erfindungsgemäßen Verbindungen der allgemeinen Formel I allein oder in Kombination mit anderen Atemwegstherapeutika, wie z.B. sekretolytisch (z.B. Ambroxol, N-acetylcystein, EGFR-Hemmern), broncholytisch (z.B. Tiotropium oder Ipratropium oder Fenoterol, Salmeterol, Salbutamol) und/oder entzündungshemmend [z.B. Theophylline oder Glucocorticoide (wie z.B., Prednisolon, Prednison, Butixocortpropionat, Beclomethason, Budesonid, Fluticason, Mometason, Ciclesonid, Dexamethason, Betamethason), Leukotrienrezeptorinhibitoren oder Leukotrienbiosyntheseinhibitoren, Antihistaminika, PDE4 Inhibitoren (wie z.B. Enprofyllin, Theophyllin, Roflumilast, Ariflo (Cilomilast), Tofimilast, Pumafentrin, Lirimilast, Arofyllin, Atizoram)] wirksamen Substanzen angewendet werden. Außerdem können diese Verbindungen mit nicht-steroidalen antiinflammatorischen Substanzen ("NSAID"; wie z.B. Ibuprofen, Celecoxib und Rofecoxib), Dopamin-Agonisten, Statinen, antiviralen Wirkstoffen wie Abacavir, P13-Kinase Inhibitoren, MRP4-Inhibitoren, PAF-Antagonisten und antiproliferativen Agentien (z.B. Methotrexat, Leflunomid, FK506 (Tacrolimus, Prograf)) kombiniert werden. Die Kombinationen, die eine oder mehrere der oben genannten Verbindungen enthalten, können zusammen oder nacheinander, zur simultanen, sequentiellen oder separaten Verabreichung eingesetzt werden. Die Anwendung dieser Verbindungen entweder alleine oder in Kombination mit anderen Wirkstoffen kann intravenös, subkutan, intramuskulär, intraperitoneal, intranasal, durch Inhalation oder transdermal oder oral erfolgen, wobei zur Inhalation insbesondere Aerosolformulierungen geeignet sind.

Zur Behandlung von Erkrankungen im Bereich des Magen-Darm-Traktes können die erfindungsgemäßen Verbindungen der allgemeinen Formel I ebenfalls alleine oder in Kombination mit Motilitäts- oder Sekretions-beeinflussenden Substanzen gegeben werden. Diese Kombinationen können entweder simultan oder sequentiell verabreicht werden.

Des Weiteren können die erfindungsgemäßen Verbindungen in der Tumortherapie in Kombination mit Anti-Tumor Therapeutika, beispielsweise in Kombination mit Topoisomerase-Inhibitoren (z.B. Etoposide), Mitoseinhibitoren (z.B. Vinblastin), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Antikörpern etc. angewandt werden. Diese Kombinationen können entweder simultan oder sequentiell verabreicht werden.

### Darreichungsformen

Die zur Erzielung einer entsprechenden Wirkung bei der Behandlung oder Prophylaxe erforderliche Dosierung hängt üblicherweise von der zu verabreichenden Verbindung, vom Patienten, von der Art und Schwere der Krankheit oder des Zustandes und der Art und Häufigkeit der Verabreichung ab und liegt im Ermessen des zu behandelnden Arztes. Zweckmäßigerweise kann die Dosierung bei inhalativer Gabe im Bereich von 0.01 bis 100 mg/kg, vorzugsweise 0.01 bis 10 mg/kg, und bei oraler Gabe im Bereich von 0.01 bis 100 mg/kg, vorzugsweise 0.01 bis 10 mg/kg, jeweils 1 bis 4 x täglich, liegen. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen, Konservierungs- und/oder Verdünnungsmitteln, z.B. mit Glucose, Arabinose, Lactose, Saccharose, Maltose, Dextrane, Maisstärke, Milchzucker, Rohrzucker, mikrokristalliner Zellulose, Sorbit, Mannit, Xylit, Magnesiumstearat, Polyvinylpyrrolidon, Zitronensäure, Weinsäure, Äpfelsäure, Ascorbinsäure, Maleinsäure, Bernsteinsäure, Fumarsäure, Essigsäure, Natriumchlorid, Calciumcarbonat, Wasser, Wasser/Ethanol, Wasser/Glycerin, Wasser/Sorbit, Wasser/Polyethylenglykol, Propylenglykol, Cetylstearylalkohol, Carboxymethylcellulose, fetthaltigen Substanzen wie Hartfett oder deren geeigneten Gemischen, Cetylpyridiniumchlorid, Benzalkoniumchlorid, Benzoesäure, Natriumbenzoat, oberflächenaktive Stoffe wie Sojalecithin, Ölsäure, Polysorbate oder Polyvinylpyrrolidon in üblichen galenischen Zubereitungen wie Tabletten, Dragees, Kapseln, Pulver, Lösungen, Suspensionen, Zäpfchen, Emulsionen, Inhalationspulver oder -aerosole einarbeiten. Zur Herstellung von treibgashaltigen Inhalationsaerosolen werden Treibgase bzw. Gemische von Treibgasen wie z.B. n-Propan, n-Butan, Isobutan, Halogenkohlenwasserstoffe wie fluorierte Derivate des Methans, Ethans [z.B. 1,1,1,2-Tetrafluorethan (TG134a)], Propans [z.B. 1,1,1,2,3,3,3-Heptafluorpropan (TG227)], Butans, Cyclopropans oder Cyclobutans verwendet.

Die Dosis für die zuvor angeführten Kombinationspartner beträgt hierbei zweckmäßigerweise 1/5 der üblicherweise empfohlenen niedrigsten Dosierung bis zu 1/1 der normalerweise empfohlenen Dosierung.

Daher betrifft ein weiterer Gegenstand dieser Erfindung die Verwendung einer erfindungsgemäßen Verbindung oder eines physiologisch verträglichen Salzes solch einer Verbindung in Kombination mit mindestens einem der zuvor als Kombinationspartner beschriebenen Wirkstoffe zur Herstellung eines Arzneimittels, das zur Behandlung oder Prophylaxe von Erkrankungen oder Zuständen geeignet ist, die durch Inhibierung des Enzyms p38 MAP-Kinase beeinflussbar sind. Hierbei handelt es sich vorzugsweise um eine Atemwegserkrankung, insbesondere eine der zuvor angeführten Erkrankungen oder Zustände, ganz besonders COPD oder Asthma.

Die Verwendung der erfindungsgemäßen Verbindung, oder eines physiologisch verträglichen Salzes hiervon, in Kombination mit einem weiteren Wirkstoff kann zeitgleich oder zeitlich versetzt, insbesondere aber zeitnah erfolgen. Bei einer zeitgleichen Verwendung werden beide Wirkstoffe dem Patienten zusammen verabreicht; bei einer zeitlich versetzten Verwendung werden beide Wirkstoffe dem Patienten in einem Zeitraum von kleiner gleich 12, insbesondere kleiner gleich 6 Stunden nacheinander verabreicht.

Folglich betrifft ein weiterer Gegenstand dieser Erfindung ein Arzneimittel, das eine erfindungsgemäße Verbindung oder ein physiologisch verträgliches Salz solch einer Verbindung sowie mindestens einen der zuvor als Kombinationspartner beschriebenen Wirkstoffe neben gegebenenfalls einem oder mehreren inerten Trägerstoffen, Konservierungs-und/oder Verdünnungsmitteln aufweist.

Die erfindungsgemäße Verbindung, oder eines physiologisch verträglichen Salzes, und der damit zu kombinierende weitere Wirkstoff können zusammen in einer Darreichungsform, beispielsweise einer Tablette, Kapsel, eines Inhalationspulvers oder -aerosols, oder getrennt in zwei gleichen oder verschiedenen Darreichungsformen, beispielsweise als sogenanntes kit-ofparts, vorliegen.

### Experimenteller Abschnitt

### Allgemeine Synthese

Die erfindungsgemäßen Verbindungen sind unter Anwendung im Prinzip bekannter Syntheseverfahren erhältlich. Bevorzugt werden die Verbindungen nach den nachfolgend näher erläuterten erfindungsgemäßen Herstellungsverfahren erhalten.

Die Herstellung von Verbindungen der allgemeinen Formel I kann gemäß der in Schemata 1 und 2 dargestellten Strategien durchgeführt werden; R¹ bis R⁴, X und Y haben darin die wie zuvor und nachfolgend definierte Bedeutung, Ar steht für den mit R⁵, R⁶ und R⁷ substituierten Phenylring, PG¹ ist eine Carbonsäureschutzgruppe (s. nachfolgende Schutzgruppenbeschreibung), PG² eine Phenolschutzgruppe und LG steht entweder für eine Austrittsgruppe wie Halogen, C₁₋₄-Alkyloxy, 2,2,2-Trifluorethoxy, Aryloxy, C₁₋₄-Alkylsulfonyloxy, Trifluormethylsulfonyloxy, Arylsulfonyloxy, Nitro, Cyano, C₁₋₄-Alkylsulfanyl, C₁₋₄-Alkylsulfinyl, C₁₋₄-Alkylsulfonyl, Arylsulfanyl, Arylsulfinyl oder Arylsulfonyl oder für eine B(OH)₂-, BF₃K-, B(OC₁₋₄-alkyl)₂- oder B(OCMe₂CMe₂O)-Gruppe. Die beiden Strategien umfassen die gleichen Transformationen an den jeweiligen funktionellen Gruppen, jedoch in veränderter Reihenfolge; nachfolgend sind deshalb die einzelnen Transformationen nur für die in Schema 1 dargestellte Sequenz beschrieben.

Synthesestrategie 1 geht von 3-Hydroxy-benzoesäurederivaten aus, die entweder in der Literatur beschrieben oder in Analogie zu verwandten Strukturen für den Fachmann ohne Weiteres herstellbar sind. Der erste Reaktionsschritt in Synthesestrategie 1 ist die Umwandlung der phenolischen OH-Gruppe in ein geschütztes Derivat, OPG². Geeignete Schutzgruppen für die OH-Gruppe von Phenolen, deren Herstellung und Entschützung sind nachfolgend dargestellt und können hier analog angewendet werden; so können hier z.B. Methyl, Benzyl, Allyl, 4-Methoxybenzyl und Methylsulfonyl als Schutzgruppe zur Anwendung kommen. Nachfolgend wird die Schutzgruppe PG¹ der Carbonsäurefunktionalität entfernt, um die freie Säure mit dem Amin-Baustein, Ar-NH₂, in einer Amidknüpfungsreaktion umzusetzen. Die Freisetzung von Carbonsäuren ist nachfolgend beschrieben und kann hier entsprechend durchgeführt werden. Die Amidknüpfung erfolgt nach Überführung der Carbonsäuregruppe in eine aktivierte Form derselben wie z.B. ein Acylfluorid, -chlorid, -bromid, -cyanid, -imidazolid (N-acyliert), einen Ester, z.B. 2,2,2-Trifluorethylester, Vinylester, Phenylester, Pentafluorphenylester, 4-Nitrophenylester, Succinyl-N-oxy-ester, Triazinylester oder Arylotriazol-1-oxy-ester, oder einen Thioester wie z.B. Methylsulfanylcarbonyl und Phenylsulfanylcarbonyl. Gemischte Anhydride der Carbonsäure mit Kohlensäureestern, z.B. abgeleitet vom Isobutylkohlensäureester, oder andere Abgangsgruppen, wie z.B. Dimethylaminocarbonyloxy, Pyrrol-1-ylcarbonyloxy, Piperidin-1-yl-carbonyloxy, Morpholin-4-ylcarbonyloxy, Trimethylcarbamimidoyloxy, N,N,N',N'-Tetramethyl-uronyl, N,N'-Dicyclohexyl-uronyl, Diisopropoxy-phosphoryloxy, Di-(dimethylamino)phos-phoryloxy und Dipyrrolidin-1-yl-phosphoryloxy, sind ebenfalls geeignete aktivierte Formen der Carbonsäure zur Kupplung mit einem Amin. Die genannten aktivierten Acylverbindungen werden in Abhängigkeit von ihrer Stabilität bevorzugt in einem separaten Reaktionsschritt oder in situ hergestellt. So wird z.B. das Säurechlorid durch Behandeln der Carbonsäure mit Thionylchlorid, Phosphoroxychlorid oder Oxalylchlorid eventuell in Gegenwart von katalytischen Mengen an N,N-Dimethylformamid in Dichlormethan, 1,2-Dichlorethan, Acetonitril, Toluol, Benzol, Tetrahydrofuran, Ether oder ohne Lösungsmittel in einem Überschuss von Chlorierungsreagenz bei Temperaturen zwischen -20 und 120°C oder das Imidazolid durch Reaktion der Carbonsäure mit Carbonyldiimidazol in Dichlormethan, Tetrahydrofuran, Acetontril oder 1,4-Dioxan bei Temperaturen zwischen 20 und 120°C erhalten. Reagenzien, die sich zur in situ-Aktivierung von Carbonsäuren eignen, sind z.B. N,N'-Diisopropylcarbodiimid, N,N'-Dicyclohexylcarbodiimid, N-(3-Dimethylamino-propyl)-N'-ethyl-carbodiimid, O-(Benzotriazo)-1-yl)-N,N,N',N'-tetramethyluroniumtetrafluorborat (TBTU), O-(Benzotriazol-1-yl)-N,N,N',N'-tetramethyluroniumhexafluorphosphat (HBTU), n-Propylphosphonsäureanhydrid, O-(7-Azabenzotriazol-1-yl)-N,N,N',N'-tetramethyluroniumhexafluorphosphat (HATU), (Benzotriazol-1-yloxy)-tris-(dimethylamino)phosphoniumhexafluorphosphat (BOP) oder (Benzotriazol-1-yloxy)-tripyrrolidin-1-yl-phosphoniumhexafluorphosphat (PyBOP). Die Amidknüpfungsreaktion wird bevorzugt in einem Lösungsmittel wie beispielsweise Dichlormethan, 1,2-Dichlorethan, Toluol, Benzol, N,N-Dimethylformamid, N,N-Dimethylacetamid, N-Methylpyrrolidinon, Tetrahydrofuran, Acetonitril, Dimethylsulfoxid oder Gemischen daraus durchgeführt. Alkohole, z.B. Methanol, Ethanol oder Isopropanol, oder Wasser können in einigen Fällen ebenfalls als Lösungsmittel oder Co-Solvens eingesetzt werden. Die Reaktionen werden bevorzugt in Gegenwart einer Base wie z.B. Triethylamin, Pyridin, Imidazol, Diisopropylethylamin oder Kaliumcarbonat und gegebenenfalls eines Additivs, z.B. 4-Dimethylaminopyridin oder 1-Hydroxybenzotriazol, bei Temperaturen zwischen -20 und 120°C, bevorzugt jedoch zwischen 0 und 80°C durchgeführt.

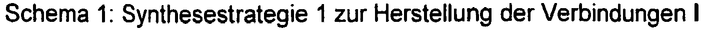

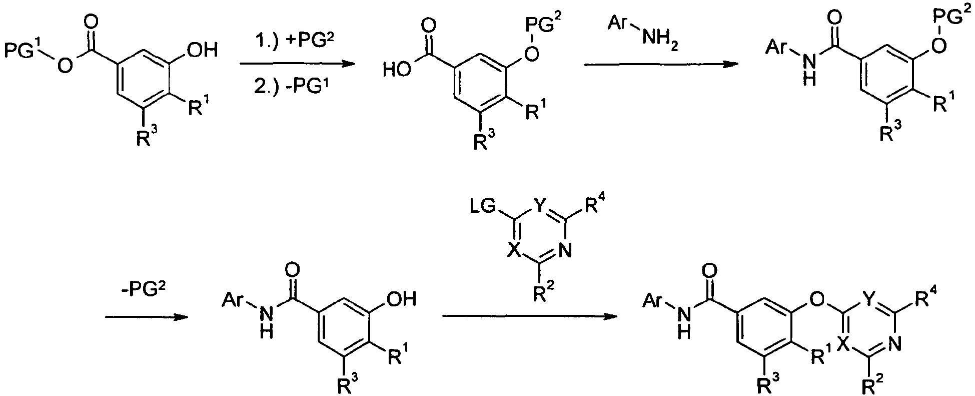

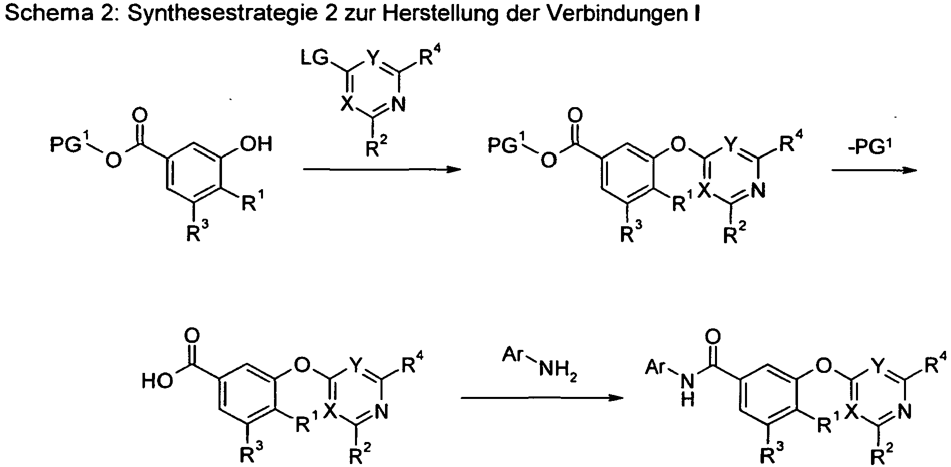

Nach Entfernung der phenolischen O-Schutzgruppe (Vorgehensweise s. nachfolgend) wird der Phenolsauerstoff durch Reaktion mit einem Elektrophil oder Nucleophil des N-Heteroaromaten in den entsprechenden Diarylether überführt. Bei Reaktionen mit einem elektrophilen N-Heteroaromaten (LG = anionische Austrittsgruppe) kann die Reaktion in Gegenwart eines Übergangsmetallkatalysators oder unkatalysiert durchgeführt werden. Unkatalysierte Reaktionen lassen sich besonders gut mit N-Heteroaromaten durchführen, die Fluor, Chlor, Nitro, 2,2,2-Trifluorethoxy, C₁₋₄-Alkylsulfonyloxy, Trifluormethylsulfonyloxy, Arylsulfonyloxy, C₁₋₄-Alkylsulfinyl, C₁₋₄-Alkylsulfonyl, Arylsulfinyl oder Arylsulfonyl als LG tragen. Dazu wird das Phenol mit einer Base, wie z.B. Natriumhydrid, Kaliumhydrid, KO*t*Bu, NaO*t*Bu, NaOMe, NaOEt, NaO*i*Pr, KF, Kaliumcarbonat, Cäsiumcarbonat, Pyridin, 4-Dimethylaminopyridin, NEt₃ oder EtN*i*Pr₂, in einem Lösungsmittel, wie beispielsweise Tetrahydrofuran, 1,4-Dioxan, 1,2-Dimethoxyethan, Aceton, Acetonitril, N,N-Dimethylformamid, N,N-Dimethylacetamid, N-Methylpyrrolidinon, Wasser, Methanol, Ethanol, Isopropanol, Dimethylsulfoxid oder Gemischen daraus, deprotoniert und bei Temperaturen zwischen -20 und 180°C, bevorzugt zwischen 0 und 120°C, mit dem Elektrophil umgesetzt. Die Reaktionen können auch unter Mikrowellenbestrahlung in einem Mikrowellengerät durchgeführt werden. Sollte die Elektrophilie des N-Heteroaromaten zur Substitution durch den Phenolbaustein nicht ausreichend sein, kann durch Oxidation eines Stickstoffatoms, das Bestandteil des aromatischen Rings ist, in beispielsweise Toluol, Dichlormethan oder 1,2-Dichlorethan mit z.B. 3-Chlorperoxybenzoesäure (mCPBA), Wasserstoffperoxid oder tert-Butylhydroperoxid, eventuell in Gegenwart eines Übergangsmetallkatalysators, z.B. Methylrheniumtrioxid, diese durch Bildung des entsprechenden N-Oxids (→Pyridin-N-oxid, Pyrimidin-N-oxid) erhöht werden. Die N-Oxide lassen sich dann nach Einführung des Phenolrestes wieder durch Reduktion, z.B. mit Zn oder Fe in Essingsäure oder mit Wasserstoff in Gegenwart eines Übergangsmetalls wie Pd/C oder Rh/C, in die N-deoxygenierten Heteroaromaten überführen. Katalysierte Reaktionen werden bevorzugt mit Aza-Heteroaromaten durchgeführt, die Iod, Brom, Chlor, Tritluormethylsulfonyloxy, Mesyloxy oder Tosyloxy als LG tragen. Als Katalysatoren eignen sich dabei Komplexe, Salze oder elementare Formen von Cu, Ni, Pd oder Fe. In den Komplexen können Phosphine, z.B. Triphenylphosphin, Tritolylphosphin, Tricyclohexylphosphin, Tri-tert-butylphosphin, 1,1'-Bis(diphenylphosphino)ferrocen, substituierte (2-Phenylphen-1-yl)-di-tert-butylphosphine oder (2-Phenylphen-1-yl)-dicyclohexylphosphine, 2,2'-Bis(diphenylphosphinyl)-1,1'-binaphthyl, 1,3-Diaryl-imidazol- oder imidazolidincarbene, 2,2,6,6-Tetramethylheptan-3,5-dion, Thienyl-2-carboxylat, Phosphite, Nitrile, z.B. Acetonitril oder Benzonitril, oder Alkene, z.B. Dibenzylidenaceton oder Allyl, Verwendung finden. Als Salzformen eignen sich z.B. Fluoride, Chloride, Bromide, Trifluormethansulfonate, Trifluoracetate oder Acetate. Elementare Formen der Übergangsmetalle sind z.B. Palladium auf Kohle oder Nanopartikel von Palladium oder Eisen. Die Reaktionen werden bevorzugt in Gegenwart einer Base, wie z.B. einer der bereits bei der unkatalysierten Variante genannten, bevorzugt mit NaO*t*Bu, Cs₂CO₃ oder K₃PO₄, in einem Lösungsmittel, wie beispielsweise Tetrahydrofuran, Toluol, Benzol, 1,4-Dioxan, 1,2-Dimethoxyethan, Acetonitril, N,N-Dimethylformamid, N,N-Dimethylacetamid oder N-Methylpyrrolidinon, zwischen 0 und 180°C, bevorzugt zwischen 20 und 120°C durchgeführt.

Kupplungen des Phenols mit Borderivaten des N-Heteroaromaten werden bevorzugt unter oxidativen Bedingungen mit Cu(II)-Salzen, z.B. Cu(OCOCH₃)₂, in Gegenwart von Molekularsieb und Triethylamin, Pyridin oder 4-Dimethylaminopyridin in Dichlormethan, 1,2-Dichlorethan, Toluol, Tetrahydrofuran oder Benzol bei 0 bis 120°C oder in der Mikrowelle bei bis zu 160°C durchgeführt. Die Reaktionen können durch Verwendung eines Co-Oxidans, z.B. Sauerstoff oder Pyridin-N-oxid, auch mit katalytischen Mengen an Cu(II) durchgeführt werden. Die Herstellung des Vorläufers des N-Heteroaromaten, R²-Ar^{Het} (s. Schema 3), wird in Abhängigkeit des die Reste Ar^{Het} und R² verknüpfenden Atoms unterschiedlich ausgeführt. Ist R² über eine Aminogruppe mit Ar^{Het} verknüpft, wird bevorzugt von einem Ar^{Het} ausgegangen, das in der zu verknüpfenden Position eine Abgangsgruppe LG² trägt, welche über die Aminogruppe mit dem Rest R² nucleophil substitiuert wird. Diese Reaktion kann durch ein Übergangsmetall, bevorzugt einem Palladium- oder Kupferkomplex, katalysiert werden, wird aber bevorzugt, gilt besonders für LG² = F, Cl, NO₂, SOC₁₋₄-alkyl, SO₂C₁₋₄-alkyl, ohne Katalysator in Gegenwart einer Base, z.B. KO*t*Bu, K₂CO₃, Na₂CO₃, Cs₂CO₃, Pyridin, 4-Dimethylaminopyridin, NEt₃ oder EtN*i*Pr₂, in beispielsweise Tetrahydrofuran, 1,4-Dioxan, 1,2-Dimethoxyethan, Acetonitril, N,N-Dimethylformamid, N,N-Dimethylacetamid, N-Methylpyrrolidinon, Wasser, Methanol, Ethanol, Isopropanol, Dimethylsulfoxid oder Gemischen daraus bei Temperaturen zwischen 0 und 180°C, bevorzugt zwischen 20 und 120°C durchgeführt. Alternativ lässt sich der Rest R² durch ein Übergangsmetall, eines Salzes oder Komplexes davon katalysiert an die Ar^{Het}-Gruppe anbinden; LG² ist in diesem Fall z.B. Cl, Br, I, OSO₂CF₃, OSO₂Me, OSO₂Tol. Von Palladium und Kupfer abgeleitete Katalysatoren, z.B. Pd₂(Dibenzylidenaceton)₃ kombiniert mit (2-Dimethylaminophenylphen-1-yl)-di-tert-butylphosphin, [2-(2,4,6-Triisopropylphenyl)phen-1-yl]-dicyclohexyl-phosphin, 2-Chlor-1,3-bis(2,6-diisopropylphenyl)-1,3,2-diazaphospholidin oder [2-(2,4,6-Triisopropylphenyl)phen-1-yl]-di-tert-butyl-phosphin oder Cul kombiniert mit 2-(Isopropylcarbonyl)-cyclohexanon oder trans-1,2-Di-(methylamino)-cyclohexan, die bevorzugt in Lösungsmitteln wie Tetrahydrofuran, Toluol, Benzol, 1,4-Dioxan, 1,2-Dimethoxyethan, N,N-Dimethylformamid, N,N-Dimethylacetamid, N-Methylpyrrolidinon oder Acetonitril zwischen 0 und 180°C, bevorzugt zwischen 20 und 120°C eingesetzt werden, sind dafür besonders geeignet.

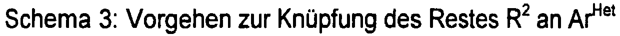

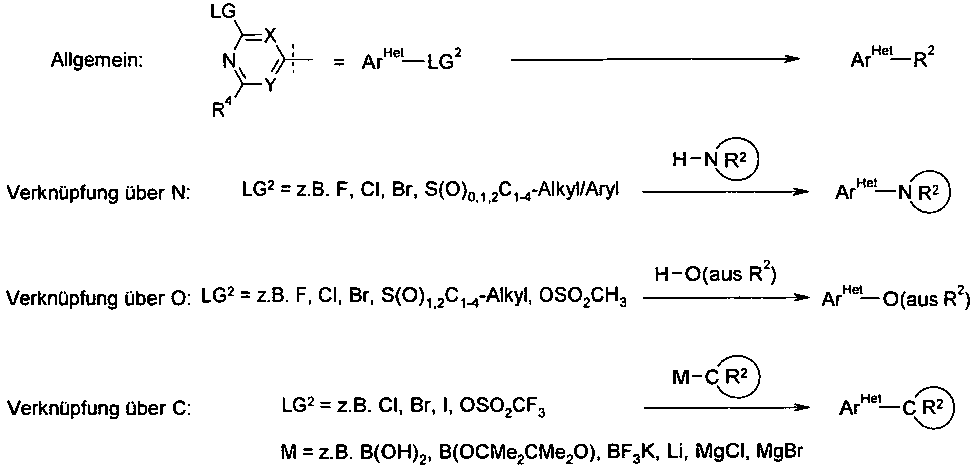

In den Fällen, in denen R² über O an Ar^{Het} angeknüpft ist, kann wie zur Herstellung der Diarylether wie oben dargestellt (s. Schemata 1 und 2) vorgegangen werden. Der Hydroxy-Rest lässt sich auch hier Übergangsmetall-katalysiert oder unkatalysiert einbringen. Die dort als geeignet bezeichneten Reaktionsbedingungen sind hier analog anwendbar (siehe zur O-und N-Knüpfung auch: Angew. Chem. 2003, 115, 5558-5607 und dort zitierte Literaturstellen). Zur Herstellung eines über ein C verknüpftes Ar^{Het}-R bedient man sich bevorzugt der Übergangsmetall-vermittelten Kupplung eines Ar^{Het}-LG², worin LG² für Cl, Br, I, OSO₂CF₃, OSO₂Me oder OSO₂Tol steht, mit einem C-Nucleophil von R². Geeignete Katalysatoren können Komplexe des Übergangsmetalls, bevorzugt Pd, Ni, Fe und Cu, mit Phosphinen [z.B. Tri-tert-butylphosphin, Tricyclohexylphosphin, 2,2'-Bis(diphenylphosphinyl)-1,1'-binaphthyl, substitutierte (2-Phenyl-phen-1-yl)-dicyclohexylphosphine (z.B. Xphos), substituierte (2-Phenyl-phen-1-yl)-di-tert-butylphosphine, Triphenylphosphin, Tritolylphosphin, Trifurylphosphin, 1,1'-Bis(diphenylphosphinyl)ferrocenel, Phosphite, 1,3-disubstituierte Imidazolcarbene oder Imidazolidincarbene, Dibenzylidenaceton, Allyl oder Nitrile sein, außerdem können auch elementare Formen des Übergangsmetalls wie z.B. Palladium auf Kohle oder Nanopartikel von Pd oder Fe geeignet sein. Die aktiven Komplexe werden häufig erst in situ aus Salzformen des Übergangsmetalls hergestellt, wobei u.a. Fluoride, Chloride, Bromide, Acetate, Triflate oder Trifluoroacetate eingesetzt werden können. Die Umsetzung wird bevorzugt mit einem Trifluorborat, einer Boronsäure oder einem Boronsäureester (Suzuki oder Suzuki-artige Kupplung), einem Zinkhalogenid (Negishi oder Negishi-artige Kupplung), einem Stannan (Stille oder Stille-artige Kupplung), einem Silan (Hiyama oder Hiyama-artige Kupplung), einem Magnesiumhalogenid (Kumada oder Kumada-artige Kupplung) des von R² zu kuppelnden C durchgeführt. In Abhängigkeit von der Natur der elektrophilen und nucleophilen Reaktionspartner können Additive wie Halogenide, z.B. LiCl, KF, *n*Bu₄NF, Hydroxide, z.B. KOH, K₂CO₃, Cs₂CO₃, Silbersalze, z.B. Ag₂O oder AgOTf, und Kupfersalze, z.B. CuCl, Cul oder Kupfer(I)thiophencarboxylat, vorteilhaft oder sogar essenziell sein. Die Kupplung wird optional in Dichlormethan, N,N-Dimethylformamid, N-Methylpyrrolidinon, Benzol, Toluol, Tetrahydrofuran, Wasser, Ethylacetat, Alkohol, Ether, Dimethylsulfoxid, 1,2-Dimethoxyethan, 1,4-Dioxan oder Mischungen daraus durchgeführt, wobei in Abhängigkeit vom Nucleophil das ein oder andere Lösungsmittel weniger gut oder überhaupt nicht geeignet ist. Die Reaktionen werden vorzugsweise zwischen -10 und 180°C durchgeführt.

Trägt die Gruppe Ar^{Het} in der gewünschten Position eine Carboxy- oder Acylgruppe kann über diese der Rest R² ebenfalls aufgebaut werden. Mögliche Anknüpfungen sind dabei u.a. über eine Carbonsäureamidfunktion, wobei R² über eine Aminocarbonylgruppe an Ar^{Het} gebunden ist, und ein über eine Aminomethyleinheit angebundenes R². Der Aufbau von Carbonsäureamiden ist zuvor beschrieben und kann hier analog zur Anwendung kommen. Die Anknüpfung über eine Aminomethyleinheit wird bevorzugt durch reduktive Aminierung des acylierten Ar^{Het} mit einem primären oder sekundären Amin bewerkstelligt. Dazu werden die Reaktionspartner in Gegenwart eines Reduktionsmittels, z.B. NaBH(OCOCH₃)₃, NaH₃B(CN) oder NaBH₄, und optional einer Lewis-Säure, z.B. Essigsäure oder ZnCl₂, in Tetrahydrofuran, 1,4-Dioxan, Methanol, Ethanol, Isopropanol, Wasser, N,N-Dimethylformamid, N-Methylpyrrolidinon, Acetonitril, Toluol, Dichlormethan oder 1,2-Dichlorethan bei -10 bis 100°C umgesetzt. Alternativ ist der Zugang zu dieser Aminomethylverknüpfung auch über die Reduktion der oben erwähnten Carbonsäureamidfunktion möglich. Geeignete Reduktionsmittel dafür sind u.a. Lithiumaluminiumhydrid, Diisobutylaluminiumhydrid oder Borane, die bevorzugt in Tetrahydrofuran, 1,4-Dioxan, Ether, Toluol oder Dichlormethan bei -50 bis 100°C angewendet werden.

Im nachfolgenden sind einige Transformationen ausgehend von Verbindungen der allgemeinen Formel I oder naher Verwandter beschrieben, über die weitere Strukturen der allgemeinen Formel I zugänglich sind.

Wird eine Verbindung der allgemeinen Struktur I erhalten, die eine Amino- oder Alkylaminogruppe trägt, so lässt sich diese mittels Acylierung oder Sulfonylierung in die entsprechende Acylamino- bzw. Sulfonylamino-Verbindung überführen.

Wird eine Verbindung der allgemeinen Struktur I erhalten, die eine Hydroxygruppe trägt, so lässt sich diese mittels Acylierung oder Sulfonylierung in die entsprechende Acyloxy- bzw. Sulfonyloxy-Verbindung überführen.

Wird eine Verbindung der allgemeinen Struktur I erhalten, die eine Hydroxygruppe trägt, so lässt sich diese mittels Alkylierung in den entsprechenden Alkylether überführen.

Wird eine Verbindung der allgemeinen Struktur I erhalten, die eine Aminogruppe trägt, so lässt sich diese durch Umsetzung mit einem Isocyanat oder Carbamoylchlorid in das entsprechende Harnstoffderivat überführen.

Wird eine Verbindung der allgemeinen Struktur I erhalten, die eine Nitrogruppe trägt, so lässt sich diese durch Reduktion in die entsprechende Aminoverbindung überführen.

Wird eine Verbindung der allgemeinen Struktur I erhalten, die eine C₁₋₄-Alkyloxycarbonylgruppe trägt, so lässt sich diese zur entsprechenden Carbonsäure spalten.

Wird eine Verbindung der allgemeinen Struktur I erhalten, die eine Carboxygruppe trägt, so lässt sich diese durch Veresterung zum entsprechenden Carbonsäureester umformen.

Wird eine Verbindung der allgemeinen Struktur I erhalten, die eine Carboxy- oder Estergruppe trägt, so lässt sich diese zum entsprechenden Carbonsäureamid umformen.

Wird eine Verbindung der allgemeinen Struktur I erhalten, die eine Carboxygruppe oder aktivierte Form (z.B. Anhydrid, Carbonsäurechlorid) derselben trägt, lässt sich diese durch eine C-Abbaureaktion in das entsprechende Amino-, Harnstoff- oder Urethanderivat überführen.

Wird eine Verbindung der allgemeinen Struktur I erhalten, die eine aromatische/heteroaromatische Aminogruppe enthält, lässt sich diese in das entsprechende aromatische/heteroaromatische Chlorid, Fluorid, Bromid, Cyanid, Hydroxid, Azid, Sulfid oder Thiol durch Überführung der Aminogruppe in eine Diazogruppe und anschließender Umsetzung dieser mit einer Chlorid-, Fluorid-, Bromid-, Cyanid-, Hydroxid-, Azid-, Sulfid- bzw. Thiolquelle umformen.

Wird eine Verbindung der allgemeinen Struktur I erhalten, die ein Amino-, Alkylamino- oder Dialkylaminofragment beinhaltet, lässt sich diese in das entsprechende Alkylamino-, Dialkylamino- oder Trialkylaminoderivat durch Alkylierung mit einem Alkylelektrophil oder durch reduktive Alkylierung mit einem Aldehyd oder Keton überführen.

Wird eine Verbindung der allgemeinen Struktur I erhalten, die einen aromatischen/heteroaromatischen Cl-, Br-, I-, F₃CO₂SO-, MeO₂SO- oder TolO₂SO-Rest trägt, lässt sich diese durch einen Übergangsmetall-vermittelten Austausch der genannten Reste durch Aryl-, Alkenyl-, Alkinyl- oder Alkylgruppen in die entsprechenden Derivate überführen.

Wird eine Verbindung der allgemeinen Struktur I erhalten, die einen aromatischen/heteroaromatischen Cl-, Br-, I-, F₃CO₂SO-, MeO₂SO- oder TolO₂SO-Rest trägt, lässt sich diese durch einen Übergangsmetall-vermittelten Austausch der genannten Reste durch Alkyloxycarbonyl in die entsprechenden Carbonsäureester überführen.

Wird eine Verbindung der allgemeinen Struktur I erhalten, die einen Alkenylrest trägt, lässt sich diese durch Dihydroxylierung in eine eine 1,2-Dihyroxyethyleinheit-tragende Verbindung der allgemeinen Formel I überführen.

Wird eine Verbindung der allgemeinen Struktur I erhalten, die ein 1,2-Dihydroxyethylfragment beinhaltet, lässt sich diese durch Glycolspaltung in den entsprechenden verkürzten Aldehyd umwandeln.

Wird eine Verbindung der allgemeinen Struktur I erhalten, die eine Carbonsäureesterfunktionalität trägt, lässt sich diese durch Reduktion in den entsprechenden Aldehyd umformen.

Die nachfolgende Acylierung oder Suffonylierung wird optional in einem Lösungsmittel oder Lösungsmittelgemisch ausgewählt aus CH₂Cl₂, Benzol, Chlorbenzol, Toluol, Tetrahydrofuran oder 1,4-Dioxan mit einem entsprechenden Acyl- oder Sulfonylelektrophil, optional in Gegenwart eines tetriären Amins, einer anorganischen Base und/oder eines Dehydratisierungsreagenzes, durchgeführt. Gewöhnlich genutzte Reagenzien sind z.B. Thionylchlorid, Isobutylchlorformat, Trimethylchlorsilan, Schwefelsäure, Methansulfonsäure, p-Toluolsulfonsäure, PCl₃, P₂O₅, N,N'-Dicyclohexylcarbodiimid, N,N'-Carbonyldiimidazol, Triphenylphosphin mit CCl₄ oder Kombinationen daraus, die optional in Gegenwart von 4-Dimethylaminopyridin und/oder 1-Hydroxybenzotriazol zwischen 0 and 150°C, bevorzugt zwischen 0 und 80°C eingesetzt werden.

Die nachfolgende Veresterung wird mit dem entsprechenden Alkohol, optional in einem Lösungsmittel oder Lösungsmittelgemisch ausgewählt aus CH₂Cl₂, N,N-Dimethylformamid, Benzol, Chlorbenzol, Toluol, Tetrahydrofuran oder 1,4-Dioxan oder besonders bevorzugt im Alkohol selbst, mit einer Säure, z.B. Salzsäure oder Schwefelsäure, oder einem Dehydratisierungsreagenz durchgeführt. Isobutylchlorformat, Trimethylchlorsilan, Schwefelsäure, Methansulfonsäure, Thionylchlorid, p-Toluolsulfonsäure, N,N'-Dicyclohexylcarbodiimid, N,N'-Carbonyldiimidazol, PCl₃, P₂O₅, Triphenylphosphin/CCl₄ oder Kombinationen daraus, optional in Gegenwart von 4-Dimethylaminopyridin und/oder 1-Hydroxybenzotriazol, gehören zu den häufig genutzten Reagenzien. Die Reaktionen werden bei -20 - 100°C, bevorzugt bei 0 - 80°C durchgeführt.

Die Veresterung kann ebenfalls mit einem Alkylelektrophil wie z.B. Mel, Me₂SO₄, BnBr oder Allylbromid in Gegenwart einer Base, z.B. CsOH, Cs₂CO₃, K₂CO₃, NaOH, NEt₃, ausgeführt werden.

Die nachfolgende Alkylierung/Veretherung wird optional in z.B. Dichlormethan, Benzol, Toluol, N,N-Dimethylformamid, Tetrahydrofuran oder 1,4-Dioxan mit einem Alkylierungsmittel des entsprechenden Halogenids oder Sulfonsäureesters, z.B. Mel, MeBr, EtBr, Me₂SO₄, BnCl, optional in Gegenwart einer tertiären organischen Base oder einer anorganischen Base bei Temperaturen zwischen 0 und 150°C, bervozugt zwischen 0 und 100°C durchgeführt.

Die nachfolgende Bildung eines Harnstoffs ausgehend von einem Amin kann z.B. in einem Lösungsmittel oder Lösungsmittelgemisch ausgewählt aus Dimethylformamid, N-Methylpyrrolidinon, Toluol, Acetonitril, Dichloromethan, 1,2-Dichloroethan, Tetrahydrofuran, Ether, 1,2-Dimethoxyethan oder Dioxan mit einem Isocyanat oder Carbamoylchlorid, optional in der Gegenwart einer tertiären organischen Base, z.B. NEt₃ oder EtN/Pr₂, oder einer anorganischen Base, z.B. K₂CO₃ oder CaO, bei 0 bis 180°C, bevorzugt bei 5 bis 120°C bewerkstelligt werden. Die Zugabe von Additiven wie Pyridin oder 4-Dimethylaminopyridin kann dabei vorteilhaft sein.

Die nachfolgende Reduktion einer Nitro-Gruppe wird beispielsweise mit Wasserstoff in Gegenwart eines Übergangsmetallkatalysators, z.B. Palladium auf Kohle, PtO₂ oder Raney-Nickel, oder mit Eisen oder Zink in Gegenwart einer Säure wie Essigsäure ausgeführt.

Die nachfolgende Spaltung einer C₁₋₄-Alkyloxycarbonylgruppe zur Bildung der freien Carbonsäure wird beispielsweise durch saure oder basische Hydrolyse mit HCl, Schwefelsäure, LiOH, NaOH oder KOH in einem wässrigen oder alkoholischen Lösungsmittel oder -gemisch erreicht.

Der nachfolgende Austausch einer Carboxygruppe, z.B. Carbonsäure- oder aktivierte Carbonsäuregruppe, gegen einen Stickstoffrest wird z.B. durch eine Abbaureaktion über das entsprechende Acylazid verwirklicht (z.B. Curtius-Abbau, Hofmann-Abbau). Dabei erhält man das Acylazid z.B. durch Reaktion der Carbonsäure mit (PhO)₂P(O)N₃ in Gegenwart einer Base, z.B. NEt₃, *i*Pr₂NEt, Pyridin, 4-Dimethylaminopyridin, K₂CO₃ oder Cs₂CO₃, in z.B. Cyclohexan, tert-Butanol, Toluol, Benzol, Dichloromethan, 1,2-Dichloroethan, 1,4-Dioxan, Tetrahydrofuran, N,N-Dimethylformamid oder Gemischen daraus. Ausgehend von einer aktivierten Carbonsäurefunktion, z.B. Acylchlorid, gemischte Anhydride mit z.B. Urethanen, Kohlensäureestern oder Phosphorsäureestern, Arylester wie Pentafluorophenyl- oder 4-Nitrophenylester, Alkyl- oder Arylthioester, kann das Acylazid nach Umsetzung mit einem Azidnucleophil, z.B. NaN₃ oder Me₃SiN₃, optional in Gegenwart eines Additivs, z.B. Bu₄NBr, vorzugsweise in Toluol, Benzol, Tetrahydrofuran, Ether, 1,4-Dioxan, Dichloromethan, N,N-Dimethylformamid, N-Methylpyrrolidinon, Acetonitril, Aceton, Wasser oder Gemischen daraus erhalten werden; in Abhängigkeit von der Azidquelle sind einige der genannten Kombinationen ungeeignet. Das Acylazid lagert -eventuell erst durch Erhöhung der Temperatur auf vorzugweise 60 bis 140°C eingeleitet- zum Isocyanat um, welches im Fall eines inerten Lösungsmittels isoliert werden kann. Durch Zugabe von Wasser wird daraus das freie Amin, von Alkohol das entsprechende Urethan und nach Zugabe eines Amins der entsprechende Harnstoff erhalten.

Die nachfolgende Umwandlung einer reaktiven Carbonsäurefunktionalität in das entsprechende Carbonsäureamid wird durch Reaktion mit dem entsprechenden Amin vorzugsweise in einem Lösungsmittel wie Dichlormethan, N,N-Dimethylformamid, N-Methylpyrrolidinon, Benzol, Toluol, Chlorbenzol, Tetrahydrofuran, 1,4-Dioxan, Gemischen daraus oder im Amin selbst, optional in Gegenwart einer tertiären organischen Aminbase oder einer anorganischen Base und optional mit 4-Dimethylaminopyridin als Additiv, bei Temperaturen von 0 bis 180°C, vorzugsweise zwischen 0 und 80°C, erzielt.

Die nachfolgende Umwandlung einer aromatischen/heteroaromatischen Aminogruppe wird durch Diazotierung der Aminogruppe mit beispielsweise salpetriger Säure, einer Nitrosoniumquelle oder Äquivalenten davon wie z.B. NaNO₂/HCl, NOBF₄, tert-Butylnitrit oder Isoamylnitrit erreicht. Die Diazotierung wird beispielsweise in Dichlormethan, 1,2-Dichloroethan, N,N-Dimethylformamid, N-Methylpyrrolidinon, Benzol, Toluol, Chlorobenzol, Tetrahydrofuran, Wasser, Ethylacetat, Alcohol, Ether, 1,2-Dimethoxyethan, 1,4-Dioxan oder Gemischen daraus bei -10 bis 100°C durchgeführt (Diazotierungen von Aminogruppen sind z.B. in Angew. Chem. Int. Ed. 1976, 15, 251 und dort zitierten Literaturstellen beschrieben). Der anschließende Austausch der Diazogruppe gegen eine Cyanogruppe, ein Chlor- oder Bromatom mittels CuCN, CuCl bzw. CuBr ist in der Literatur als die Sandmeyer-Reaktion bekannt (s. March's Advanced Organic Chemistry, Michael B. Smith and Jerry March, John Wiley & Sons Inc., 6. Ed., New Jersey, 2007 und dort zitierte Literaturstellen). Die Reaktion wird vorzugsweise zwischen -10 und 120°C in einem der oben genannten Lösungsmittel oder Gemischen ausgeführt. Der Austausch einer Diazogruppe gegen Fluor kann durch Umsetzung der Diazoverbindung mit einem Alkali-Tetrafluorborat oder Tetrafluorborsäure bei 20 bis 160°C realisiert werden; diese Reaktion ist unter dem Namen Schiemann-Reaktion bekannt. lod kann durch Behandlung der Diazoverbindung mit einem lodid, z.B. Nal, vorzugsweise in Wasser oder einem wässrigen Lösungsmittelgemisch, bei 0 bis 120°C eingeführt werden. Der Diazo-Hydroxy-Austausch kann durch Umsetzung in Gegenwart von Wasser bei 0 bis 180°C erreicht werden. Diese Reaktion läuft normalerweise ohne weitere Additive ab, jedoch kann die Zugabe von Kupferoxid oder starker Säure vorteilhaft sein. Mercapto- oder Alkylsulfanylreste können durch Umsetzung der Diazoverbindung mit Alkalidisulfiden oder Dialkylsulfiden bei Temperaturen von 0 bis 120 °C eingeführt werden; in Abhängigkeit von der einzuführenden Schwefelspezies sind inerte oder wässrige Lösungsmittelsysteme eher geeignet (siehe z.B. Synth. Commun. 2001, 31, 1857 und darin zitierte Literaturstellen).

Die nachfolgende reduktive Alkylierung eines Amins wird mit der entsprechenden Carbonylverbindung, wie z.B. Formaldehyd, Acetaldehyd, Propionaldehyd, Aceton oder Butyraldehyd, und einem komplexen Metallhydrid, z.B. NaBH₄, LiBH₄, NaHB(OCOCH₃)₃, NaH₃B(CN), vorzugsweise bei einem pH-Wert von 6-7 oder mit Wasserstoff, bei 1 bis 5 bar, in Gegenwart eines Übergangsmetalls, z.B. Pd/C, durchgeführt. Die Methylierung kann auch mit Ameisensäure oder Formiaten als Reduktionsmittel bei 60 bis 120°C erreicht werden.

Der nachfolgende Austausch eines aromatischen/heteroaromatischen Chlor-, Brom-, lodatoms oder einer aromatischen/heteroaromatischen Trifluormethylsulfonyloxy-, Mesyloxy- oder Tosyloxygruppe gegen einen Aryl-, Heteroaryl-, Alkenyl-, Alkinyl- oder Alkylrest wird vorzugsweise durch einen Übergangsmetallkatalysator, z.B. solche die von Pd, Ni, Rh, Cu und Fe abgeleitet sind, vermittelt. Geeignete Katalysatoren oder Vorstufen davon können Komplexe der Übergangsmetalle mit z.B. Phosphinen [z.B. Tri-tert-butylphosphin, Tritolylphosphin, Tricyclohexylphosphin, Triphenylphosphin, substitutierte (2-Phenyl-phen-1-yl)-dicyclohexylphosphine, Trifurylphosphin, substitutierte (2-Phenyl-phen-1-yl)-di-tert-butylphosphine, 1,1'-Bis(diphenylphosphino)ferrocen], 1,3-disubstituierte Imidazolcarbene, 1,3-disubstituierte Imidazolidincarbene, Phosphite, Dibenzylidenaceton, Allyl oder Nitrile, elementare Formen des Übergangsmetalls, z.B. Pd/C oder Nanopartikel von Pd oder Fe, Salze der Übergangsmetalle, wie z.B. Fluorid, Chlorid, Bromid, Acetat, Trifluormethansulfonat oder Trifluoracetat, oder Kombinationen daraus sein. Der Austausch erfolgt vorzugsweise mit dem entsprechenden Alkalimetalltrifluorborat, Boronsäure oder -ester (Suzuki oder Suzukiartig Reaktion), Zinkhalogenid (Negishi oder Negishi-artige Reaktion), Stannan (Stille oder Stille-artige Reaktion), Silan (Hiyama oder Hiyama-artige Reaktion), Magnesiumhalogenid (Kumada oder Kumada-artig Reaktion) des einzuführenden Aryl-, Alkenyl- oder Alkylrestes. Der Austausch gegen ein terminales Alkin wird vorzugsweise mit demselben oder dem entsprechenden Zinkacetylid durchgeführt. In Abhängigkeit der Reaktionsführung und der Natur der Reaktionspartner können Additive wie z.B. Halogenide, z.B. LiCl, KF, nBu₄NF, Hydroxide, z.B. KOH, K₂CO₃, Cs₂CO₃, K₃PO₄, Silbersalze, z.B. Ag₂O oder AgOSO₂CF₃, Kupfersalze, z.B. CuCl oder Kupfer(I)thiophencarboxylat vorteilhaft oder sogar essenziell sein. Cul ist ein bevorzugtes Additiv für die Kupplung mit terminalen Alkinen (Sonogashira-Reaktion). Die Reaktionen werden vorzugsweise in Dichlormethan, N,N-Dimethylformamid, N-Methylpyrrolidinon, Benzol, Toluol, Tetrahydrofuran, Wasser, Ethylacetat, Alkohol, Ether, Dimethylsulfoxid, 1,2-Dimethoxyethan, 1,4-Dioxan oder Gemischen daraus durchgeführt, wobei die richtige Wahl des Lösungsmittels vom Nucleophil abhängig ist und einige nicht in jedem umgesetzt werden können. Die Reaktionen werden bevorzugt bei -10 bis 180°C durchgeführt.

Der nachfolgende Austausch eines aromatischen/heteroaromatischen Chlor-, Brom-, lodatoms oder einer aromatischen/heteroaromatischen Trifluormethylsulfonyloxy-, Mesyloxy- oder Tosyloxygruppe gegen eine Alkyloxycarbonylgruppe wird durch Umsetzung der jeweiligen Verbindung unter CO-Atmosphäre in Gegenwart des entsprechenden Alkohols und eines Übergangsmetallkatalysators erzielt. Geeignete Katalysatorsysteme sind z.B. Pd(OCOCH₃)₂ kombiniert mit PPh₃, Pd(OCOCH₃)₂ kombiniert mit Ph₂P(CH₂)₃PPh₂, PdCl₂[1,1'-Bis(diphenylphosphino)ferrocen], (BINAP)PdCl₂, Pd(P*t*Bu₃)₂, PdCl₂(PhCN)₂/Ph₂P(CH₂)₃PPh₂, Pd(OCOCH₃)₂/Ph₂P(CH₂)₄PPh₂, die vorzugsweise in Gegenwart von Basen, wie z.B. KOCOCH₃, NaOCOCH₃, NEt₃, K₂CO₃, NaHCO₃, in Lösungsmitteln, wie z.B. N,N-Dimethylformamid, N-Methylpyrrolidinon, Toluol, 1,4-Dioxan oder dem Alkohol selbst, eingesetzt werden. Der CO-Druck beträgt dabei vorzugsweise 1 bis 5 bar bei Temperaturen zwischen 20 und 160°C.

Die nachfolgende Dihydroxylierung einer Alkenylgruppe kann mit OsO₄ oder KMnO₄ als Oxidationsmittel ausgeführt werden. Vorzugsweise werden OsO₄ und K₂OsO₄ in Kombination mit einem Co-Oxidans, z.B. K₃Fe(CN)₆, N-Methylmorpholin-N-oxid, NaOCl oder NaClO₂, in katalytischen Mengen eingesetzt. Als Lösungsmittel oder Bestandteil des Lösungsmittel eignen sich z.B. Wasser, Chloroform, Dichlormethan, Ether, Tetrahydrofuran, Aceton, Pyridin, Acetonitril, Toluol und tert-Butanol. Additive wie MeSO₂NH₂, KCI, Basen, z.B. K₂CO₃, Diazabicyclooktan (DABCO), oder Liganden, z.B. 1,4-Bis(dihydrochinidyl)-phthalazin, die in einheitlich chiraler Form auch die enantioselektive Dihydroxylierung erlauben (s. Sharpless-Dihydroxylierung und AD-mix-α, AD-mix-β), sind vorteilhaft oder sogar essenziell. Die Dihydroxylierung kann zwischen -50 und 60°C durchgeführt werden, wird jedoch vorzugsweise zwischen -10 und 40°C durchgeführt.

Alternativ kann diese Transformation auch durch Epoxidierung der Doppelbindung, z.B. mit 3-Chlorperoxybenzoesäure, Dimethyldioxiran oder H₂O₂ oder *t*BuO₂H mit einem Übergangsmetallkatalysator kombiniert, und nachfolgende Öffnung des Oxirans mit einem Hydroxynucleophil, z.B. Wasser, LiOH, NaOH, KOH oder NaOOH mit anschließender Reduktion der O-O-Bindung, realisiert werden.

Die nachfolgende Glycolspaltung eines 1,2-Dihydroxyethylfragments wird entweder mit Pb(OCOCH₃)₄ oder NalO₄ bzw. HlO₄ als Oxidationsmittel durchgeführt. Pb(OCOCH₃)₄ wird vornehmlich in aprotischen Lösungsmitteln, vorzugsweise Benzol oder Dichlormethan, bei Temperaturen zwischen -10 und 80°C eingesetzt. NalO₄ wird dagegen bevorzugt in wässrigen Lösungsmitteln oder Gemischen von organischen Lösungsmitteln, z.B. Dichlormethan, Tetrahydrofuran, 1,4-Dioxan, Methanol, Acetonitril, mit Wasser bei -10 bis 80°C verwendet; NalO₄ in Kombination mit Silicagel oder auf Silicagel aufgezogen erlaubt auch die wasserfreie Reaktionsführung in einem der genannten organischen Lösungsmittel.

Die nachfolgende einstufige Reduktion eines Carbonsäureesters zum entsprechenden Aldehyd wird vorzugsweise mit Diisobutylaluminiumhydrid (DIBAI-H) in Dichlormethan, Toluol, Hexan, Tetrahydrofuran oder Gemischen daraus bei -80 bis 20°C ausgeführt.

Alternativ bietet sich die zweistufige Variante, Reduktion des Esters zum Alkohol, z.B. mit DIBAI-H, LiAlH₄ oder LiBH₄, und nachträgliche Oxidation des Alkohols zum Aldehyd, z.B. mit dem Dess-Martin-Periodinan, Pyridin-SO₃ oder Pyridiniumchlorochromat (PCC), zur Erreichung dieser Umwandlung an.

Bei den vorstehend beschriebenen Umsetzungen können gegebenenfalls vorhandene reaktive Gruppen wie Carboxy-, Carbonyl-, Hydroxy-, Amino- oder Alkylaminogruppen oder terminale Alkine während der Umsetzung durch übliche Schutzgruppen geschützt werden, welche nach der Umsetzung wieder abgespalten werden (siehe z.B.: Protecting Groups, Philip J. Kocienski, 3rd edition, Georg Thieme Verlag, Stuttgart, 2004 und dort zitierte Literaturstellen).

Beispielsweise kommt als Schutzrest für eine Carboxygruppe die Trimethylsilyl-, Methyl-, Ethyl-, tert-Butyl-, Allyl- oder Benzylgruppe in Betracht.

Beispielsweise kommt als Schutzrest für eine Carbonylgruppe eines Ketons oder Aldehyds ein Ketal bzw. Acetal, z.B. abgeleitet von Methanol, Glycol oder Propan-1,3-diol, in Betracht.

Beispielsweise kommen als Schutzrest für eine aliphatische Hydroxygruppe die Trimethylsilyl, tert-Butyldimethylsilyl-, Triisopropylsilyl-, Acetyl-, Pivaloyl, Benzoyl, Methyl, Allyl, Benzyl-, 4-Methoxybenzyl-, Trityl-, Methoxymethyl-, Ethoxymethyl-, 2-Trimethylsilylethoxymethyl- oder Tetrahydropyranylgruppe in Betracht.

Für eine phenolische OH-Gruppe eignen sich, neben den für die aliphatische Hydroxygruppe bereits genannten, Methylsulfonyl, Tosyl und Trifluormethylsulfonyl als Schutzgruppe.

Als Schutzreste für eine Amino- oder Alkylaminogruppe kommen beispielsweise die Formyl-, Acetyl-, Trifluoracetyl-, Ethoxycarbonyl-, tert-Butoxycarbonyl-, Benzyloxycarbonyl-, Benzyl-, 4-Methoxybenzyl- oder 2,4-Dimethoxybenzylgruppe in Betracht; für die NH₂-Gruppe eignet sich noch zusätzlich Phtalyl oder Tetrachlorphthalyl.

Beispielsweise kommt als Schutzrest für eine terminale Alkinylgruppe die Trimethylsilyl-, Triisopropylsilyl- oder 2-Hydroxyprop-2-ylgruppe in Betracht.

Die Abspaltung eines verwendeten Acyl-Schutzrestes erfolgt beispielsweise hydrolytisch in einem wässrigen Lösungsmittel, z.B. Wasser, Isopropanol/Wasser, Essigsäure/Wasser, Tetrahydrofuran/Wasser oder 1,4-Dioxan/Wasser, in Gegenwart einer Säure, z.B. Trifluoressigsäure, Salzsäure oder Schwefelsäure, oder in Gegenwart einer Alkalibase, z.B. Lithiumhydroxid, Natriumhydroxid oder Kaliumhydroxid, bei Temperaturen zwischen 0 und 140°C, vorzugsweise zwischen 10 und 100°C. Eine weitere Methode die sich zur Spaltung eines Alkylesters eignet, ist die Umsetzung mit lodtrimethylsilan, als solches eingesetzt oder in situ hergestellt, in einem inerten Lösungsmittel wie Dichlormethan, 1,2-Dichlorethan, Toluol oder Acetonitril.

Die Abspaltung eines verwendeten Acetal- oder Ketal-Schutzrestes erfolgt bevorzugt in einem wässrigen Lösungsmittel, z.B. Wasser, Isopropanol/Wasser, Tetrahydrofuran/Wasser oder 1,4-Dioxan/Wasser, in Gegenwart einer Säure, z.B. Essigsäure, Trifluoressigsäure, Salzsäure oder Schwefelsäure, bei Temperaturen zwischen 0 und 120°C, vorzugsweise zwischen 10 und 100°C.

Die Abspaltung eines Trimethylsilylrestes erfolgt beispielsweise in Wasser, einem wässrigen Lösemittelgemisch oder einem niederen Alkohol wie Methanol oder Ethanol in Gegenwart einer Base wie Lithiumhydroxid, Natriumhydroxid, Kaliumcarbonat oder Natriummethylat. In wässrigen oder alkoholischen Lösungsmitteln eignen sich ebenfalls Säuren, wie z.B. Salzsäure, Trifluoressigsäure oder Essigsäure. Zur Abspaltung in organischen Lösungsmitteln, wie beispielsweise Diethylether, Tetrahydrofuran oder Dichlormethan, eignen sich auch Fluoridreagenzien, wie z.B. Tetrabutylammoniumfluorid oder HF. Letztere Methode ist, neben der Anwendung von starken Säuren, auch zur Abspaltung von größeren Silylgruppen, wie z.B. tert-Butyldimethylsilyl und Triisopropylsilyl, geeignet.

Die Abspaltung eines Benzyl-, Methoxybenzyl- oder Benzyloxycarbonylrestes erfolgt vorteilhaft hydrogenolytisch, z.B. mit Wasserstoff in Gegenwart eines Katalysators wie Palladium auf Kohle, Palladiumhydroxid oder Platinoxid, in einem geeigneten Lösungsmittel wie Methanol, Ethanol, Essigsäureethylester oder Eisessig, gegebenenfalls unter Zusatz einer Säure wie Salzsäure, bei Temperaturen zwischen 0 und 100°C, vorzugsweise jedoch zwischen 20 und 60°C, und bei einem Wasserstoffdruck von 1 bis 7 bar, vorzugsweise 3 bis 5 bar. Trimethylsilyliodid, Bortrichlorid oder Bortrifluorid in Gegenwart eines Abfangreagenzes, z.B. Anisol, Thioanisol oder Pentamethylbenzol, können ebenfalls zur Spaltung von Benzylethern einschließlich deren substituierten Derivaten verwendet werden. Die Abspaltung von elektronenreichen Benzylresten, wie z.B. 4-Methoxybenzyl, kann auch oxidativ mit z.B. 2,3-Dichlor-5,6-dicyan-1,4-benzochinon (DDQ) oder Cerammoniumnitrat (CAN), bevorzugt in alkoholischen oder wässrigen Lösungen, zwischen 10 und 120°C bewerkstelligt werden. Die 2,4-Dimethoxybenzylgruppe wird vorzugsweise in Trifluoressigsäure in Gegenwart eines Abfangreagenzes, z.B. Anisol, abgespalten.

Die Abspaltung eines tert-Butyl- oder tert-Butyloxycarbonylrestes erfolgt vorzugsweise durch Behandlung mit einer Säure wie Trifluoressigsäure oder Salzsäure gegebenenfalls unter Verwendung eines Lösungsmittels wie Dichlormethan, 1,4-Dioxan, Methanol, Isopropanol oder Diethylether.

Eine Methylgruppe an einem tertiären Amin kann durch Behandlung mit 1-Chlorethylchlorformat abgespalten werden. HBr oder BBr₃ sind besonders zur Spaltung von Methylethern geeignet.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D-und L-Formen von Weinsäure oder Dibenzoylweinsäure, Di-O-Tolylweinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Des Weiteren können die erhaltenen Verbindungen der Formel I in ihre Salze, insbesondere für die pharmazeutische Anwendung in ihre physiologisch verträglichen Salze mit anorganischen oder organischen Säuren, überführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II und III sind teilweise literaturbekannt oder können nach an sich literaturbekannten Verfahren sowie in Analogie zu den in den Beispielen beschriebenen Verfahren, gegebenenfalls unter zusätzlicher Einführung von Schutzresten, erhalten werden.

### Beispiele

Die nachfolgenden Beispiele sollen die vorliegende Erfindung näher erläutern ohne diese zu beschränken.

Die nachfolgend beschriebenen Verbindungen wurden anhand einer charakteristischen Masse nach lonisation in einem Massensprektrometer, eines R_{f}-Wertes auf einer DC-Platte und/oder Retentionszeit auf einer analytischen HPLC charakterisiert.

### Verwandte HPLC Methoden:

Methode 1: Säule: Merck Cromolith Speed ROD, RP18e, 50 x 4,6 mm; 1,5 ml/min; UV-Detektion: 230 nm/254 nm; Laufmittel A: Wasser (0,1% Ameisensäure), Laufmittel B: Acetonitril (0,1 % Ameisensäure)

| Gradient: | Zeit (min.) | % Laufmittel B |
|---|---|---|
| | 0,00 | 10 |
| | 4,50 | 90 |
| | 5,00 | 90 |
| | 5,50 | 10 |

Methode 2: Säule: Agilent Zorbax Bonus RP, 50 x 2,1 mm, 3,5 µm; 1,2 ml/min; UV-Detektion: 230 nm/254 nm; Laufmittel A: Wasser (0,1% Ameisensäure), Laufmittel B: Acetonitril (0,1 % Ameisensäure)

| Gradient: | Zeit (min.) | % Laufmittel B |
|---|---|---|
| | 0,00 | 10 |
| | 4,50 | 99 |
| | 5,00 | 99 |
| | 5,50 | 10 |

Methode 3: Säule: Waters Xbridge C18, 30 x 4,6 mm, 2,5 µm; 1,6 ml/min; UV-Detektion: 230 nm/254 nm; Laufmittel A: Wasser (0, 1 % Ammoniak), Laufmittel B: Methanol

| Gradient: | Zeit (min.) | % Laufmittel B |
|---|---|---|
| | 0,00 | 10 |
| | 0,15 | 10 |
| | 4,00 | 100 |
| | 4,40 | 100 |
| | 4,55 | 10 |
| | 5,00 | 10 |

### Herstellung der Ausgangsverbindungen

### Beispiel I

### N-(5-tert-Butyl-3-methansulfonylamino-2-methoxy-phenyl)-3-hydroxy-4-methyl-benzamid

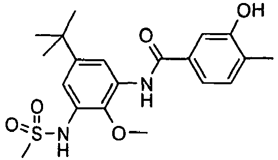

7,10 g 3-Benzyloxy-N-(5-tert-butyl-3-methansulfonylamino-2-methoxy-phenyl)-4-methyl-benzamid werden in einem Gemisch aus 70 ml Methanol und 40 ml Tetrahydrofuran in Gegenwart von 800 mg Palladium auf Aktivkohle (10% Pd) bei Raumtemperatur und 50 psi Wasserstoffpartialdruck hydriert. Anschließend wird der Katalysator abgesaugt und das Filtrat am Rotationsverdampfer eingeengt. Zurück bleibt ein weißer Feststoff, welcher ohne weitere Reinigung weiterumgesetzt wird.
Ausbeute: 5,90 g (100 % der Theorie)
Massenspektrum (ESI⁺): m/z = 407 [M+H]⁺

Analog Beispiel I werden folgende Verbindungen erhalten:
(1) N-(5-tert-Butyl-3-methansulfonylamino-2-methoxy-phenyl)-3-hydroxy-4-chlor-benzamid 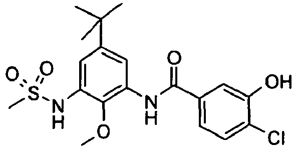 R_{f}-Wert: 0,40 (Kieselgel, Petrolether/Ethylacetat = 1:1)
   Massenspektrum (ESI⁺): m/z = 427/429 (CI) [M+H]⁺
(2) N-(5-tert-Butyl-3-methansulfonylamino-2-methoxy-phenyl)-3-hydroxy-4-trifluormethyl-benzamid 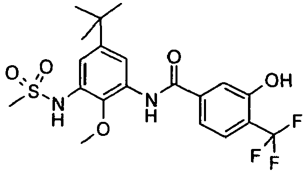 R_{f}-Wert: 0,51 (Kieselgel, Petrolether/Ethylacetat = 1:1)
   Massenspektrum (ESI⁺): m/z = 461 [M+H]⁺
(3) N-(5-tert-Butyl-3-methansulfonylamino-2-methoxy-phenyl)-3-hydroxy-4-methoxy-benzamid 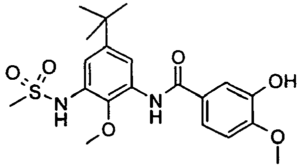 R_{f}-Wert: 0,20 (Kieselgel, Cyclohexan/Ethylacetat = 1:1)
   Massenspektrum (ESI⁺): m/z = 423 [M+H]⁺
(4) N-(5-tert-Butyl-3-methansulfonylamino-2-methoxy-phenyl)-3-hydroxy-4-fluor-benzamid 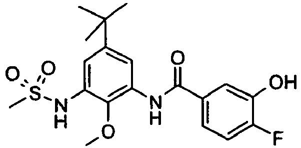 R_{f}-Wert: 0,45 (Kieselgel, Petrolether/Ethylacetat = 1:1)
   Massenspektrum (ESI⁺): m/z = 411 [M+H]⁺
(5) N-(5-tert-Butyl-3-methansulfonylamino-2-methoxy-phenyl)-3-chlor-5-hydroxy-4-methyl-benzamid 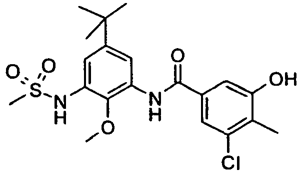 R_{f}-Wert: 0,42 (Kieselgel, Cyclohexan/Ethylacetat = 1:1)
   Massenspektrum (ESI⁺): m/z = 441/443 (CI) [M+H]⁺
(6) N-(5-tert-Butyl-3-methansulfonylamino-2-methoxy-phenyl)-3-fluor-5-hydroxy-4-methyl-benzamid 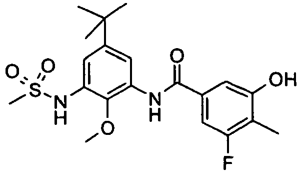 R_{f}-Wert: 0,40 (Kieselgel, Cyclohexan/Ethylacetat = 1:1)
   Massenspektrum (ESI⁺): m/z = 425 [M+H]⁺
(7) N-(5-tert-Butyl-3-methansulfonylamino-2-methoxy-phenyl)-3-hydroxy-5-methyl-benzamid 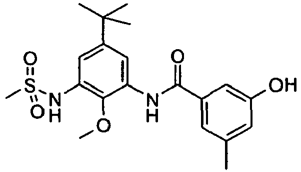 R_{f}-Wert: 0,35 (Kieselgel, Cyclohexan/Ethylacetat = 1:1)
   Massenspektrum (ESI⁺): m/z = 407 [M+H]⁺
(8) N-(5-tert-Butyl-3-methansulfonyl-2-methoxy-phenyl)-3-hydroxy-4-methyl-benzamid 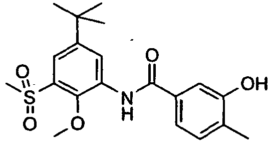 R_{f}-Wert: 0,55 (Kieselgel, Cyclohexan/Ethylacetat = 1:1)

### Beispiel II

### 3-Benzyloxy-N-(5-tert-butyl-3-methansulfonylamino-2-methoxy-phenyl)-4-methyl-benzamid

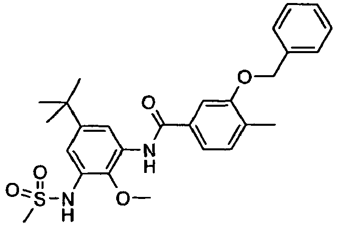

Zu 4,50 g 3-Benzyloxy-4-methyl-benzoesäure in 25 ml N,N-Dimethylformamid werden unter Argonatmosphäre 10,59 g O-(7-Azabenzotriazol-1-yl)-N,N,N',N'-tetramethyl-uronium-hexafluorphosphat, 9,54 ml Diisopropylethylamin und 1,26 g 1-Hydroxy-7-azabenzotriazol gegeben. Das Gemisch wird 15 Minuten bei Raumtemperatur gerührt, bevor 5,74 g N-(3-Amino-5-tert-butyl-2-methoxyphenyl)methansulfonamid-hydrochlorid zugegeben werden. Das Reaktionsgemisch wird über Nacht bei 50°C gerührt. Nach Abkühlung auf Raumtemperatur wird das Reaktionsgemisch mit Ethylacetat verdünnt, mit Wasser, 1 N Salzsäure und gesättigter wässriger Natriumchlorid-Lösung gewaschen, über Magnesiumsulfat getrocknet und eingeengt. Der Kolbenrückstand wird mit Methanol verrührt, und der entstandene weiße Niederschlag wird abgesaugt und getrocknet.
Ausbeute: 7,10 g (77 % der Theorie)
Massenspektrum (ESI⁺): m/z = 497 [M+H]⁺

Analog Beispiel II werden folgende Verbindungen erhalten:
(1) 3-Benzyloxy-N-(5-tert-butyl-3-methansulfonylamino-2-methoxy-phenyl)-4-chlor-benzamid 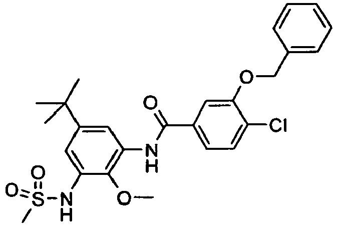 R_{f}-Wert: 0,68 (Kieselgel, Petrolether/Ethylacetat = 1:1)
   Massenspektrum (ESI⁺): m/z = 517/519 (CI) [M+H]⁺
(2) 3-Benzyloxy-N-(5-tert-butyl-3-methansulfonylamino-2-methoxy-phenyl)-4-trifluormethyl-benzamid 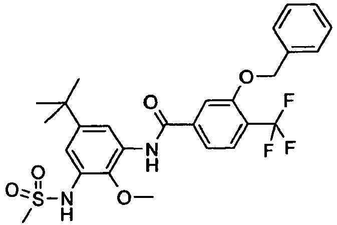 R_{f}-Wert: 0,70 (Kieselgel, Petrolether/Ethylacetat = 1:1)
   Massenspektrum (ESI⁺): m/z = 551 [M+H]⁺
(3) 3-Benzyloxy-N-(5-tert-butyl-3-methansulfonylamino-2-methoxy-phenyl)-4-fluor-benzamid 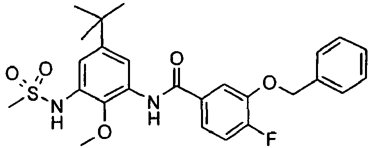 R_{f}-Wert: 0,60 (Kieselgel, Petrolether/Ethylacetat = 1:1)
   Massenspektrum (ESI⁺): m/z = 501 [M+H]⁺
(4) 4-{4-[2-Brom-5-(5-tert-butyl-3-methansulfonylamino-2-methoxy-phenyl-aminocarbonyl)-phenoxy]-pyrimidin-2-yl}-piperazin-1-carbonsäure-tert-butylester 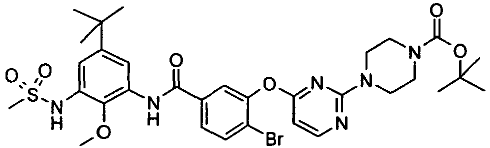 R_{f}-Wert: 0,40 (Kieselgel, Cyclohexan/Ethylacetat = 1:1)
   Massenspektrum (ESI⁺): m/z = 733/735 (Br) [M+H]⁺
(5) 4-{4-[5-(5-tert-Butyl-3-methansulfonylamino-2-methoxy-phenylaminocarbonyl)-2-ethyl-phenoxy]-pyrimidin-2-yl}-piperazin-1-carbonsäure-tert-butylester 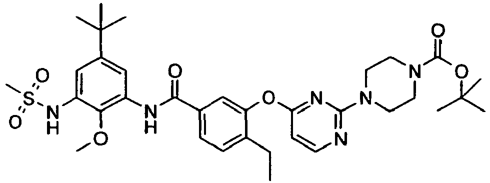 R_{f}-Wert: 0,40 (Kieselgel, Cyclohexan/Ethylacetat = 1:1)
   Massenspektrum (ESI⁺): m/z = 683 [M+H]⁺
(6) 3-Benzyloxy-N-(5-tert-butyl-3-methansulfonylamino-2-methoxy-phenyl)-5-methyl-benzamid 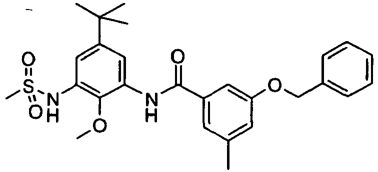 R_{f}-Wert: 0,35 (Kieselgel, Cyclohexan/Ethylacetat = 2:1)
   Massenspektrum (ESI⁺): m/z = 497 [M+H]⁺
(7) 3-Benzyloxy-N-(5-tert-butyl-3-methansulfonyl-2-methoxy-phenyl)-4-methyl-benzamid 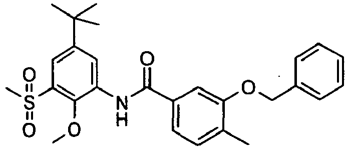 R_{f}-Wert: 0,77 (Kieselgel, Cyclohexan/Ethylacetat = 2:1)
   Massenspektrum (ESI⁺): m/z = 482 [M+H]⁺
(8) 4-{4-[5-(5-tert-Butyl-2-methoxy-3-methylsulfanylmethyl-phenylcarbamoyl)-2-methyl-phenoxy]-pyridin-2-ylmethyl}-piperidin-1-carbonsäure-tert-butyl-ester 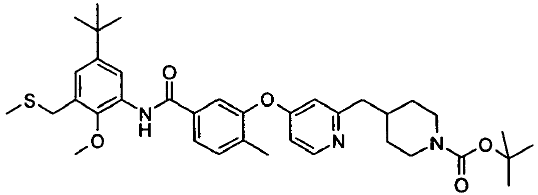 (Die Umsetzung wird mit 2-(Benzotriazol-1-yl)-N,N,N',N'-tetramethyl-uronium-tetrafluorborat als Kupplungsreagenz durchgeführt)
   HPLC (Methode 1): Retentionszeit = 4,00 min
   Massenspektrum (ESI⁺): m/z = 648 [M+H]⁺
(9) 4-{4-[5-(5-tert-Butyl-2-methoxy-3-methylsulfonylmethyl-phenylcarbamoyl)-2-methyl-phenoxy]-pyridin-2-ylmethyl}-piperidin-1-carbonsäure-tert-butyl-ester 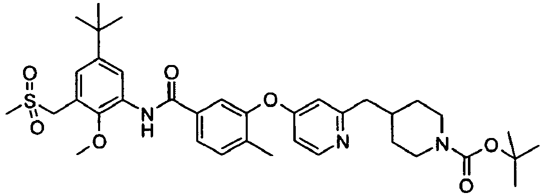 (Die Umsetzung wird mit 2-(Benzotriazol-1-yl)-N,N,N',N'-tetramethyl-uronium-tetrafluorborat als Kupplungsreagenz durchgeführt)
   HPLC (Methode 1): Retentionszeit = 3,37 min
   Massenspektrum (ESI⁺): m/z = 680 [M+H]⁺
(10) 4-{4-[5-(5-tert-Butyl-2-methoxy-3-methylsulfinylmethyl-phenylcarbamoyl)-2-methyl-phenoxy]-pyridin-2-ylmethyl}-piperidin-1-carbonsäure-tert-butyl-ester 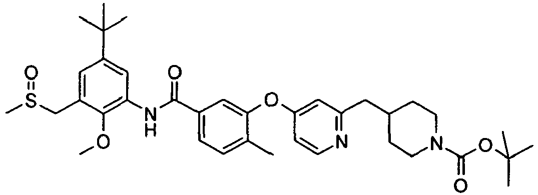 (Die Umsetzung wird mit 2-(Benzotriazol-1-yl)-N,N,N',N'-tetramethyl-uronium-tetrafluor,borat als Kupplungsreagenz durchgeführt)
   HPLC (Methode 1): Retentionszeit = 3,18 min
   Massenspektrum (ESI⁺): m/z = 664 [M+H]⁺
(11) 4-{4-[5-(3-Amino-5-tert-butyl-2-methoxy-phenylcarbamoyl)-2-methyl-phenoxy]-pyridin-2-ylmethyl}-piperidin-1-carbonsäure-tert-butyl-ester 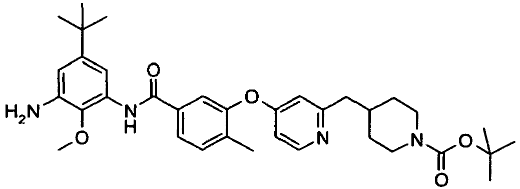 (Die Umsetzung wird mit 2-(Benzotriazol-1-yl)-N,N,N',N'-tetramethyl-uronium-tetrafluorborat als Kupplungsreagenz durchgeführt)
   Massenspektrum (ESI⁺): m/z = 603 [M+H]⁺
(12) 4-{4-[5-(5-tert-Butyl-2-isopropoxy-3-methansulfonylamino-phenylcarbamoyl)-2-methyl-phenoxy]-pyridin-2-ylmethyl}-piperidin-1-carbonsäure-tert-butyl-ester 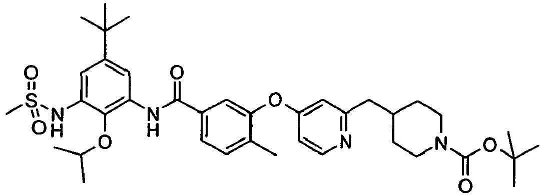 (Die Umsetzung wird mit 2-(Benzotriazol-1-yl)-N,N,N',N'-tetramethyl-urenium-tetrafluorborat als Kupplungsreagenz durchgeführt)
   HPLC (Methode 1): Retentionszeit = 3,60 min
   Massenspektrum (ESI⁺): m/z = 709 [M+H]⁺
(13) 4-{4-[5-(5-tert-Butyl-2-ethoxy-3-methansulfonylamino-phenylcarbamoyl)-2-methyl-phenoxy]-pyridin-2-ylmethyl}-piperidin-1-carbonsäure-tert-butyl-ester 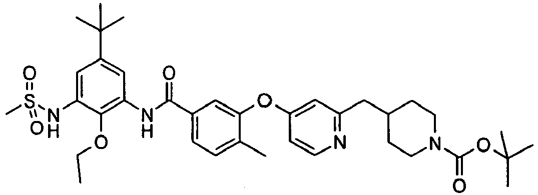 (Die Umsetzung wird mit 2-(Benzotriazol-1-yl)-N,N,N',N'-tetramethyl-uronium-tetrafluorborat als Kupplungsreagenz durchgeführt)
   HPLC (Methode 1): Retentionszeit = 3,54 min
   Massenspektrum (ESI⁺): m/z = 695 [M+H]⁺

### Beispiel III

### N-(5-tert-Butyl-3-methansulfonylamino-2-methoxy-phenyl)-3-(2-chlor-pyrimidin-4-yloxy)-4-methyl-benzamid

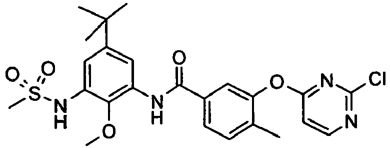

Zu 1,50 g N-(5-tert-Butyl-3-methansulfonylamino-2-methoxy-phenyl)-3-hydroxy-4-methyl-benzamid in 15 ml Acetonitril und 3 ml N,N-Dimethylformamid werden 567 mg 2,4-Dichlorpyrimidin und 663 mg Kaliumcarbonat gegeben, und das Reaktionsgemisch wird über Nacht bei Raumtemperatur gerührt. Anschließend werden nochmals 57 mg 2,4-Dichlorpyrimidin zugegeben, und das Gemisch wird eine weitere Stunde bei Raumtemperatur gerührt. Zur Aufarbeitung wird das Reaktionsgemisch mit Wasser verdünnt und der enstandene weiße Niederschlag abfiltriert, mit Wasser nachgewaschen und im Exsikkator getrocknet.
Ausbeute: 1,69 g (88 % der Theorie)
R_{f}-Wert: 0,30 (Kieselgel, Petrolether/Ethylacetat = 1:1)
Massenspektrum (ESI⁺): m/z = 519/521 (CI) [M+H]⁺

Analog Beispiel III werden folgende Verbindungen erhalten:
(1) N-(5-tert-Butyl-3-methansulfonylamino-2-methoxy-phenyl)-3-(6-chlor-pyrimidin-4-yloxy)-4-methyl-benzamid 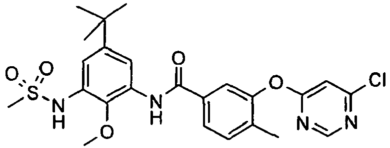 (Umsetzung erfolgt in Gegenwart von Kalium-tert-butylat bei 50°C)
   R_{f}-Wert: 0,66 (Kieselgel, Petrolether/Ethylacetat = 1:2)
   Massenspektrum (ESI⁺): m/z = 519/521 (CI) [M+H]⁺
(2) N-(5-tert-Butyl-3-methansulfonylamino-2-methoxy-phenyl)-3-(2-chlor-pyrimidin-4-yloxy)-4-trifluormethyl-benzamid 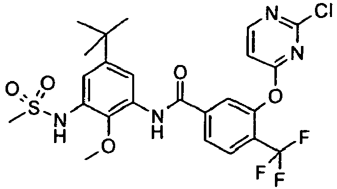 (Umsetzung erfolgt in Dimethylsulfoxid in Gegenwart von Kalium-tert-butylat)
   R_{f}-Wert: 0,44 (Kieselgel, Petrolether/Ethylacetat = 1:1)
   Massenspektrum (ESI⁺): m/z = 573/575 (CI) [M+H]⁺
(3) N-(5-tert-Butyl-3-methansulfonylamino-2-methoxy-phenyl)-3-(2-chlor-pyrimidin-4-yloxy)-4-chlor-benzamid 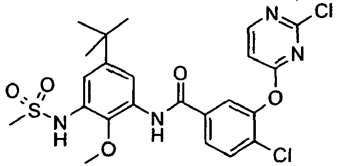 (Umsetzung erfolgt in Dimethylsulfoxid in Gegenwart von Kalium-tert-butylat)
   R_{f}-Wert: 0,34 (Kieselgel, Petrolether/Ethylacetat = 1:1)
   Massenspektrum (ESI⁺): m/z = 539/541/541 (2 CI) [M+H]⁺
(4) 3-(2-Chlor-pyrimidin-4-yloxy)-4-methyl-benzoesäure-methylester 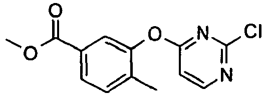 (Umsetzung erfolgt in Dimethylsulfoxid in Gegenwart von Kalium-tert-butylat)
   R_{f}-Wert: 0,87 (Kieselgel, Dichlormethan/Methanol = 95:5)
   Massenspektrum (ESI⁺): m/z = 279/281 (CI) [M+H]⁺
(5) N-(5-tert-Butyl-3-methansulfonylamino-2-methoxy-phenyl)-3-(2-chlor-pyrimidin-4-yloxy)-4-methoxy-benzamid 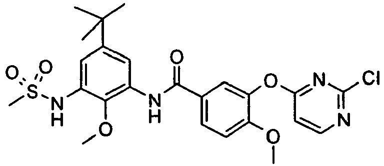 (Umsetzung erfolgt in Dimethylsulfoxid in Gegenwart von Kalium-tert-butylat)
   R_{f}-Wert: 0,15 (Kieselgel, Cyclohexan/Ethylacetat = 1:1)
   Massenspektrum (ESI⁺): m/z = 535/537 (CI) [M+H]⁺
(6) N-(5-tert-Butyl-3-methansulfonylamino-2-methoxy-phenyl)-3-(2-fluor-pyrimidin-4-yloxy)-4-chlor-benzamid 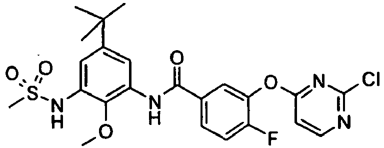 (Umsetzung erfolgt in Dimethylsulfoxid in Gegenwart von Kalium-tert-butylat)
   R_{f}-Wert: 0,41 (Kieselgel, Petrolether/Ethylacetat = 1:1)
   Massenspektrum (ESI⁺): m/z = 523/525 (CI) [M+H]⁺
(7) 4-Brom-3-(2-chlor-pyrimidin-4-yloxy)-benzoesäure-methylester 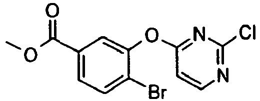 (Umsetzung erfolgt in Dimethylsulfoxid in Gegenwart von Kalium-tert-butylat)
   R_{f}-Wert: 0,45 (Kieselgel, Petrolether/Ethylacetat = 7:3)
   Massenspektrum (ESI⁺): m/z = 343/345/347 (Cl+Br) [M+H]⁺
(8) N-(5-tert-Butyl-3-methansulfonylamino-2-methoxy-phenyl)-3-chlor-5-(2 -chlor-pyrimidin-4-yloxy)-4-methyl-benzamid 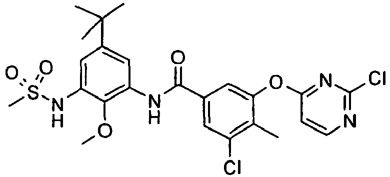 (Umsetzung erfolgt in Dimethylsulfoxid in Gegenwart von Kalium-tert-butylat)
   R_{f}-Wert: 0,30 (Kieselgel, Cyclohexan/Ethylacetat = 1:1)
   Massenspektrum (ESI⁺): m/z = 553/555/557 (2 CI) [M+H]⁺
(9) N-(5-tert-Butyl-3-methansulfonylamino-2-methoxy-phenyl)-3-chlor-5-(2 -fluor-pyrimidin-4-yloxy)-4-methyl-benzamid 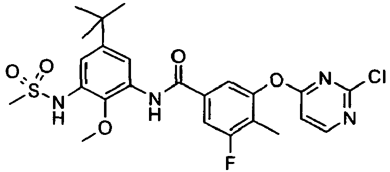 (Umsetzung erfolgt in Dimethylsulfoxid in Gegenwart von Kalium-tert-butylat)
   R_{f}-Wert: 0,28 (Kieselgel, Cyclohexan/Ethylacetat = 1:1)
   Massenspektrum (ESI⁺): m/z = 537/539 (CI) [M+H]⁺
(10) N-(5-tert-Butyl-3-methansulfonylamino-2-methoxy-phenyl)-3-(2-chlor-pyrimidin-4-yloxy)-5-methyl-benzamid 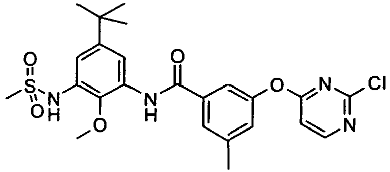 (Umsetzung erfolgt in Dimethylsulfoxid in Gegenwart von Kalium-tert-butylat)
   R_{f}-Wert: 0,30 (Kieselgel, Cyclohexan/Ethylacetat = 1:1)
   Massenspektrum (ESI⁺): m/z = 519/521 (CI) [M+H]⁺
(11) N-(5-tert-Butyl-3-methansulfonylamino-2-methoxy-phenyl)-4-chlor-3-(2-chlorpyridin-4-yloxy)-benzamid 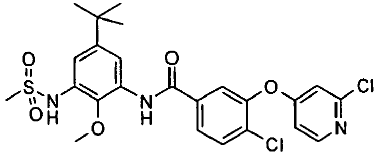 (Umsetzung erfolgt in Dimethylsulfoxid in Gegenwart von Kaliumcarbonat mit 2-Chlor-4-fluorpyridin bei 60°C)
   R_{f}-Wert: 0.30 (Kieselgel, Dichlormethan/Methanol = 98:2)
   Massenspektrum (ESI⁺): m/z = 538/540/540 (2 CI) [M+H]⁺
(12) 6-[5-(5-tert-Butyl-3-methansulfonylamino-2-methoxy-phenylcarbamoyl)-2-chlor-phenoxy]-pyrimidin-4-carbonsäuremethyl ester 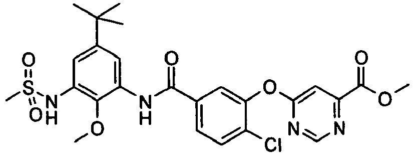 (Umsetzung erfolgt in Dimethylsulfoxid in Gegenwart von Cäsiumcarbonat mit 6-Chlor-pyrimidin-4-carbonsäuremethylester bei 45°C)
   R_{f}-Wert: 0,43 (Kieselgel, Cyclohexan/Ethylacetat = 1:1)
   Massenspektrum (ESI⁺): m/z = 563/565 (CI) [M+H]⁺
(13) N-(5-tert-Butyl-3-methansulfonylamino-2-methoxy-phenyl)-4-chlor-3-(6-chlor-pyrimidin-4-yloxy)-benzamid 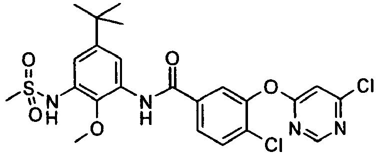 (Umsetzung erfolgt in Dimethylsulfoxid in Gegenwart von Kalium-tert-butylat bei Raumtemperatur)
   R_{f}-Wert: 0,35 (Kieselgel, Cyclohexan/Ethylacetat = 1:1)
   Massenspektrum (ESI⁺): m/z = 539/541/543 (2 CI) [M+H]⁺
(14) N-(5-tert-Butyl-3-methansulfonyl-2-methoxy-phenyl)-3-(2-chlor-pyrimidin-4-yloxy)-4-methyl-benzamid 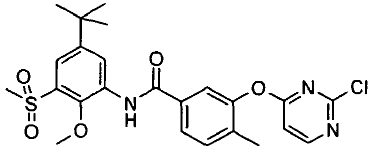 (Umsetzung erfolgt in Dimethylsulfoxid in Gegenwart von Kalium-tert-butylat bei Raumtemperatur)
   R_{f}-Wert: 0,47 (Kieselgel, Cyclohexan/Ethylacetat = 1:1) Massenspektrum (ESI⁺): m/z = 504/506 (CI) [M+H]⁺
(15) 3-(2-Chlor-pyridin-4-yloxy)-4-methyl-benzoesäuremethylester 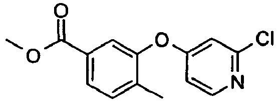 (Umsetzung erfolgt in Dimethylsulfoxid in Gegenwart von Kalium-tert-butylat bei Raumtemperatur)
   HPLC (Methode 1): Retentionszeit = 4,05 min
   Massenspektrum (ESI⁺): m/z = 278/280 (CI) [M+H]⁺

### Beispiel IV

### 4-{4-[5-(5-tert-Butyl-3-methansulfonylamino-2-methoxy-phenylaminocarbonyl)-2-methyl-phenoxy]-pyrimidin-2-yl}-piperazin-1-carbonsäure-tert-butylester

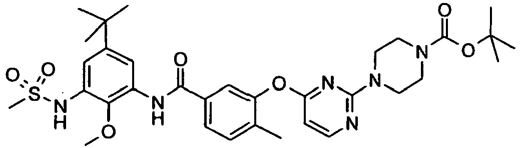

Zu einer Lösung von 350 mg N-(5-tert-Butyl-3-methansulfonylamino-2-methoxyphenyl)-3-(2-chlor-pyrimidin-4-yloxy)-4-methyl-benzamid in 1,4-Dioxan werden 188 mg Piperazin-1-carbonsäure-tert-butylester und 247 µl N,N-Diisopropylethylamin gegeben. Das Reaktionsgemisch wird 4 h bei 70°C gerührt und anschließend über Nacht auf Raumtemperatur abkühlen gelassen. Nun wird das Reaktionsgemisch mit Ethylacetat verdünnt, mit 3M wässriger Kaliumcarbonat-Lösung gewaschen, über Magnesiumsulfat getrocknet und eingeengt. Das Rohprodukt wird chromatographisch über eine Kieselgelsäule mit Ethylacetat/Cyclohexan (40:60→100:0) als Laufmittel gereinigt.
Ausbeute: 433 mg (96 % der Theorie)
R_{f}-Wert: 0,80 (Kieselgel, Dichlormethan/Methanol/konz. Ammoniak = 90:10:1)
Massenspektrum (ESI⁺): m/z = 669 [M+H]⁺

Analog Beispiel IV werden folgende Verbindungen erhalten:
(1) 4-{6-[5-(5-tert-Butyl-3-methansulfonylamino-2-methoxy-phenylcarbamoyl)-2-methyl-phenoxy]-pyrimidin-4-yl}-piperazin-1-carbonsäure-tert-butylester 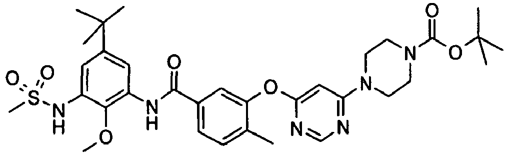 R_{f}-Wert: 0,41 (Kieselgel, Petrolether/Ethylacetat = 1:2)
   Massenspektrum (ESI⁺): m/z = 669 [M+H]⁺
(2) 4-{4-[5-(5-tert-Butyl-3-methansulfonylamino-2-methoxy-phenylaminocarbonyl)-2-trifluormethyl-phenoxy]-pyrimidin-2-yl}-piperazin-1-carbonsäure-tert-butylester 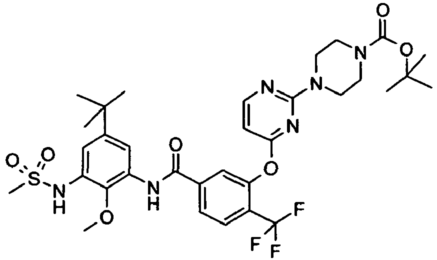 R_{f}-Wert: 0,40 (Kieselgel, Petrolether/Ethylacetat = 1:1)
   Massenspektrum (ESI⁺): m/z = 723 [M+H]⁺
(3) 4-{4-[5-(5-tert-Butyl-3-methansulfonylamino-2-methoxy-phenylaminocarbonyl)-2-chlor-phenoxy]-pyrimidin-2-yl}-piperazin-1-carbonsäure-tert-butylester 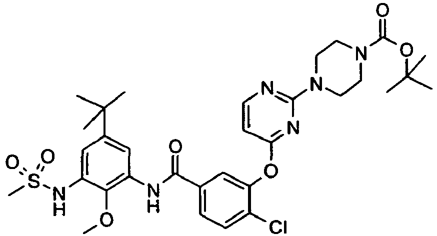 R_{f}-Wert: 0,38 (Kieselgel, Petrolether/Ethylacetat = 1:1)
   Massenspektrum (ESI⁺): m/z = 689/691 (CI) [M+H]⁺
(4) 4-[4-(5-Methoxycarbonyl-2-methyl-phenoxy)-pyrimidin-2-yl]-piperazin-1-carbonsäure-tert-butylester 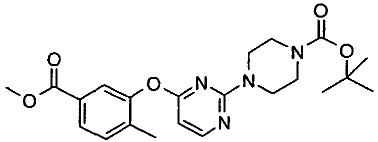 (Umsetzung erfolgt in Tetrahydrofuran in Gegenwart von Triethylamin bei Raumtemperatur)
   R_{f}-Wert: 0,81 (Kieselgel, Dichlormethan/Methanol = 95:5)
   Massenspektrum (ESI⁺): m/z = 429 [M+H]⁺
(5) 4-{4-[5-(5-tert-Butyl-3-methaneulfonylamino-2-methoxy-phenylaminocarbonyl)-2-methoxy-phenoxy]-pyrimidin-2-yl}-piperazin-1-carbonsäure-tert-butylester 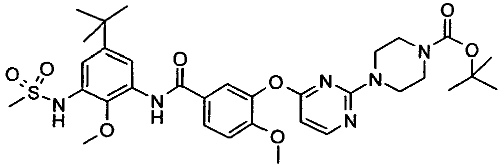 (Umsetzung erfolgt in Acetonitril in Gegenwart von Triethylamin unter Rückfluss)
   R_{f}-Wert: 0,58 (Kieselgel, Dichlormethan/Methanol = 95:5)
   Massenspektrum (ESI⁺): m/z = 685 [M+H]⁺
(6) 4-{4-[5-(5-tert-Butyl-3-methansulfonylamino-2-methoxy-phenylaminocarbonyl)-2-fluor-phenoxy]-pyrimidin-2-yl}-piperazin-1-carbonsäure-tert-butylester 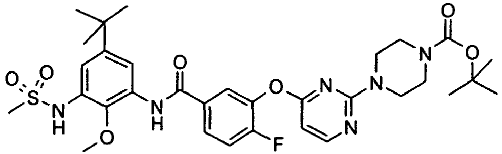 (Umsetzung erfolgt in Tetrahydrofuran in Gegenwart von Triethylamin bei 50°C)
   R_{f}-Wert: 0,48 (Kieselgel, Petrolether/Ethylacetat = 1:1)
   Massenspektrum (ESI⁺): m/z = 673 [M+H]⁺
(7) 4-[4-(2-Brom-5-methoxycarbonyl-phenoxy)-pyrimidin-2-yl]-piperazin-1-carbonsäure-tert-butylester 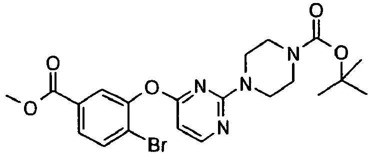 (Umsetzung erfolgt in Acetonitril in Gegenwart von Triethylamin bei 80°C)
   R_{f}-Wert: 0,40 (Kieselgel, Dichlormethan/Methanol = 98:2)
   Massenspektrum (ESI⁺): m/z = 493/495 (Br) [M+H]⁺
(8) 4-{4-[5-(5-tert-Butyl-3-methansulfonylamino-2-methoxy-phenylaminocarbonyl)-3-chlor-2-methyl-phenoxy]-pyrimidin-2-yl}-piperazin-carbonsäure-tert-butylester 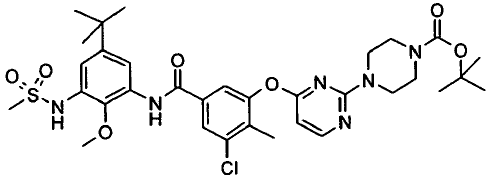 (Umsetzung erfolgt in Acetonitril in Gegenwart von Triethylamin bei 80°C)
   R_{f}-Wert: 0,48 (Kieselgel, Cyclohexan/Ethylacetat = 1:1)
   Massenspektrum (ESI⁺): m/z = 703/705 (CI) [M+H]⁺
(9) 4-{4-[5-(5-tert-Butyl-3-methansulfonylamino-2-methoxy-phenylaminocarbonyl)-3-fluor-2-methyl-phenoxy]-pyrimidin-2-yl}-piperazin-carbonsäure-tert-butylester 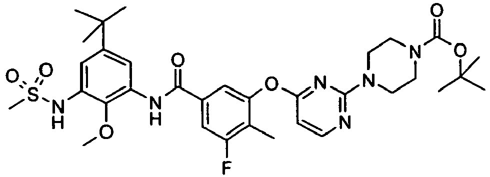 (Umsetzung erfolgt in Acetonitril in Gegenwart von Triethylamin bei 80°C)
   R_{f}-Wert: 0,40 (Kieselgel, Cyclohexan/Ethylacetat = 1:1)
   Massenspektrum (ESI⁺): m/z = 687 [M+H]⁺
(10) 4-({4-[5-(5-tert-Butyl-3-methansulfonylamino-2-methoxy-phenylaminocarbonyl)-2-methyl-phenoxy]-pyrimidin-2-yl}-N-methyl-amino)-piperidin-1-carbonsäure-tert-butylester 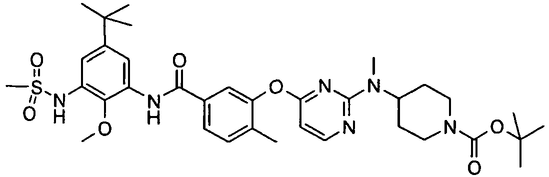 (Umsetzung erfolgt in N,N-Dimethylformamid bei 50°C)
   Massenspektrum (ESI⁺): m/z = 697 [M+H]⁺
(11) 4-({4-[5-(5-tert-Butyl-3-methansulfonylamino-2-methoxy-phenylaminocarbonyl)-2-methyl-phenoxy]-pyrimidin-2-yl}-amino)-piperidin-1-carbonsäure-tert-butylester 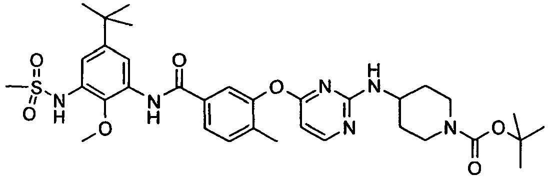 (Umsetzung erfolgt in N,N-Dimethylformamid in Gegenwart von Triethylamin bei 50°C)
   R_{f}-Wert: 0,38 (Kieselgel, Petrolether/Ethylacetat/Essigsäure = 50:50:0,1) Massenspektrum (ESI⁺): m/z = 683 [M+H]⁺
(12) 4-{6-[5-(5-tert-Butyl-3-methansulfonylamino-2-methoxy-phenylaminocarbonyl)-2-methyl-phenoxy]-pyrimidin-4-ylamino}-piperidin-1-carbonsäure-tert-butylester 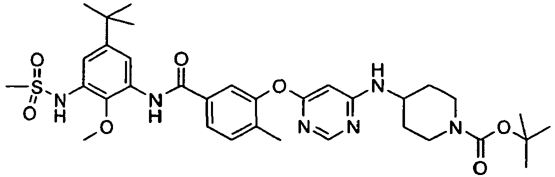 (Umsetzung erfolgt in N,N-Dimethylformamid bei 50°C)
   Massenspektrum (ESI⁺): m/z = 683 [M+H]⁺
(13) 3-{4-[5-(5-tert-Butyl-3-methansulfonylamino-2-methoxy-phenylcarbamoyl)-2-methyl-phenoxy]-pyrimidin-2-ylamino}-pyrrolidin-1-carbonsäure-tert-butyl-ester 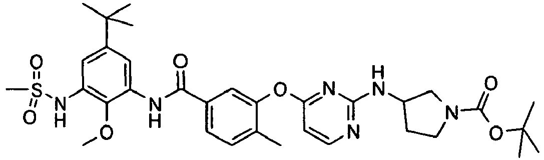 (Umsetzung erfolgt in N,N-Dimethylformamid in Gegenwart von Ethyldiisopropylamin bei 50°C)
   R_{f}-Wert: 0,58 (Kieselgel, Dichloromethan/Methanol/NH₄OH = 90:10:0,1)
   Massenspektrum (ESI⁺): m/z = 669 [M+H]⁺
(14) 3-({4-[5-(5-tert-Butyl-3-methansulfonylamino-2-methoxy-phenylcarbamoyl)-2-methyl-phenoxy]-pyrimidin-2-yl}-methyl-amino)-pyrrolidin-1-carbonsäure-tert-butyl-ester 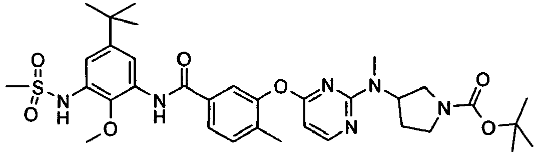 (Umsetzung erfolgt in N,N-Dimethylformamid in Gegenwart von Ethyldiisopropylamin bei 50°C)
   R_{f}-Wert: 0,75 (Kieselgel, Dichloromethan/MethanolNH₄OH = 90:10:0,1)
   Massenspektrum (ESI⁺): m/z = 683 [M+H]⁺
(15) 4-({6-[5-(5-tert-Butyl-3-methansulfonylamino-2-methoxy-phenylcarbamoyl)-2-methyl-phenoxy]-pyrimidin-4-yl}-methyl-amino)-piperidin-1-carbonsäure-tert-butyl ester 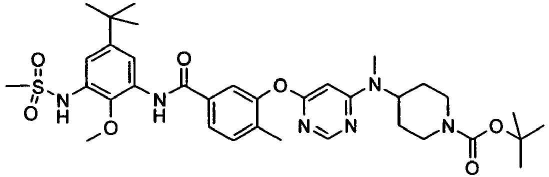 (Umsetzung erfolgt in N,N-Dimethylformamid in Gegenwart von Ethyldiisopropylamin bei 40°C)
   Massenspektrum (ESI⁺): m/z = 697 [M+H]⁺
(16) 3-{6-[5-(5-tert-Butyl-3-methansulfonylamino-2-methoxy-phenylcarbamoyl)-2-methyl-phenoxy]-pyrimidin-4-ylamino}-pyrrolidin-1-carbonsäure-tert-butyl-ester 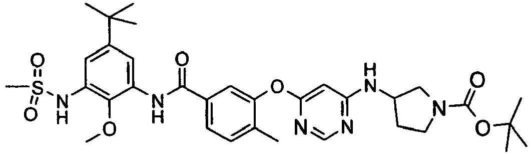 (Umsetzung erfolgt in Dimethylsulfoxid in Gegenwart von Ethyldiisopropylamin bei 40°C)
   HPLC (Methode 1): Retentionszeit = 4,16 min
   Massenspektrum (ESI⁺): m/z = 669 [M+H]⁺
(17) 4-{6-[5-(5-tert-Butyl-3-methansulfonylamino-2-methoxy-phenylcarbamoyl)-2-chlor-phenoxy]-pyrimidin-4-ylamino}-piperidin-1-carbonsäure-tert-butyl-ester 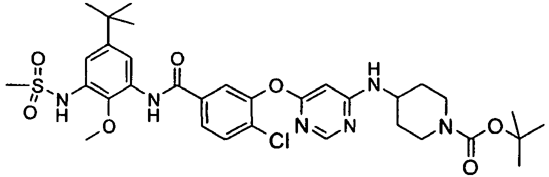 (Umsetzung erfolgt in Acetonitril in Gegenwart von Triethylamin bei 70°C)
   R_{f}-Wert: 0,45 (Kieselgel, Dichloromethan/Methanol = 95:5)
   Massenspektrum (ESI⁺): m/z = 703/705 (CI) [M+H]⁺
(18) 4-{4-[5-(5-tert-Butyl-3-methansulfonylamino-2-methoxy-phenylcarbamoyl)-2-chlor-phenoxy]-pyrimidin-2-ylamino}-piperidin-1-carbonsäure-tert-butyl-ester 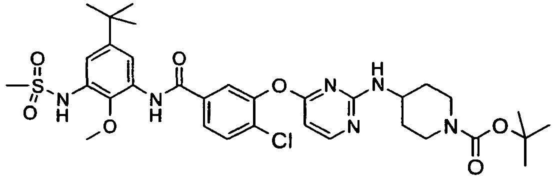 (Umsetzung erfolgt in Acetonitril in Gegenwart von Triethylamin bei 90°C)
   R_{f}-Wert: 0,40 (Kieselgel, Dichloromethan/Methanol = 95:5)
   Massenspektrum (ESI⁺): m/z = 703/705 (CI) [M+H]⁺

### Beispiel V

### 6-Chlor-pyrimidin-4-carbonsäure-methylamid

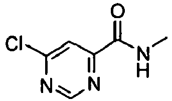

Zu 177 mg 6-Chlor-pyrimidin-4-carbonsäurechlorid in 5 ml Dichlormethan werden unter Argonatmosphäre und Eis/Acetonbad-Kühlung 0.5 ml einer 2 M Methylamid-Lösung in Tetrahydrofuran und 1 ml 1 N Natronlauge gegeben. Das Reaktionsgemisch wird über Nacht unter Rühren auf Raumtemperatur erwärmt und anschließend mit etwas gesättigter wässriger Natriumhydrogencarbonat-Lösung versetzt und mit Dichlormethan extrahiert. Die vereinigten organischen Phasen werden mit 0,1 N Salzsäure und gesättigter Natriumchlorid-Lösung gewaschen, über Magnesiumsulfat getrocknet und eingeengt.
Ausbeute: 110 mg (64 % der Theorie)
R_{f}-Wert: 0,52 (Kieselgel, Petrolether/Ethylacetat = 1:1)

Analog Beispiel V wird folgende Verbindung erhalten:
(1) 4-(6-Chlor-pyrimidin-4-carbonyl)-piperazin-1-carbonsäure-tert-butylester 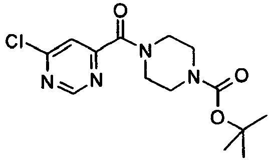 R_{f}-Wert: 0,68 (Kieselgel, Petrolether/Ethylacetat = 1:2)

### Beispiel VI

### 4-{6-[5-(5-tert-Butyl-3-methansulfonylamino-2-methoxy-phenylaminocarbonyl)-2-methyl-phenoxy]-pyrimidin-4-carbonyl}-piperazin-1-carbonsäure-tert-butylester

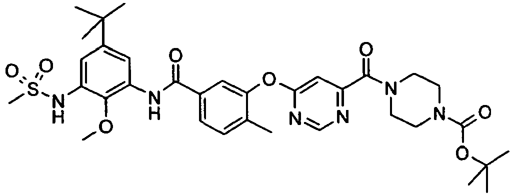

Zu 225 mg N-(5-tert-Butyl-3-methansulfonylamino-2-methoxy-phenyl)-3-hydroxy-4-methyl-benzamid in 3 ml N,N-Dimethylformamid werden bei Raumtemperatur unter Argonatmosphäre 75 mg Kalium-tert-butylat gegeben. Nach zehn Minuten werden 210 mg 4-(6-Chlor-pyrimidin-4-carbonyl)-piperazin-1-carbonsäure-tert-butylester zugegeben, und das Reaktionsgemisch wird sechs Tage bei Raumtemperatur gerührt. Zur Aufarbeitung wird das Reaktionsgemisch mit Wasser versetzt und mit Ethylacetat extrahiert. Die vereinigten Ethylacetat-Extrakte werden mit gesättigter Natriumchlorid-Lösung gewaschen, über Magnesiumsulfat getrocknet und eingeengt. Der Kolbenrückstand wird über eine Kieselgelsäule mit Cyclohexan/Ethylacetat (50:50→20:80) chromatographiert.
Ausbeute: 292 mg (76% der Theorie)
R_{f}-Wert: 0,20 (Kieselgel, Petrolether/Ethylacetat = 1:2)
Massenspektrum (ESI⁻): m/z = 695 [M-H]⁻

Analog Beispiel VI werden folgende Verbindungen erhalten:
(1) 4-{6-[5-(5-tert-Butyl-3-methansulfonylamino-2-methoxy-phenylaminocarbonyl)-2-chlor-phenoxy]-pyrimidin-4-carbonyl}-piperazin-1-carbonsäure-tert-butylester 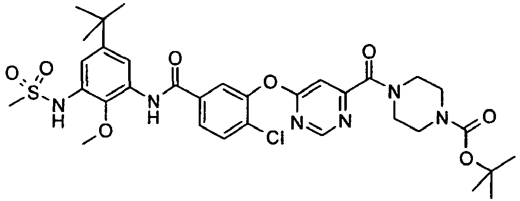 R_{f}-Wert: 0,45 (Kieselgel, Petrolether/Ethylacetat = 1:2)
   Massenspektrum (ESI⁻): m/z = 715/717 (CI) [M-H]⁻
(2) 4-[4-(2-Ethyl-5-methoxycarbonyl-phenoxy)-pyrimidin-2-yl]-piperazin-1-carbonsäure-tert-butylester 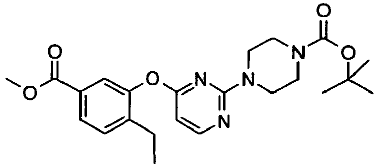 (Umsetzung erfolgt in Dimethylsulfoxid)
   R_{f}-Wert: 0,45 (Kieselgel, Petrolether/Ethylacetat = 7:3)
   Massenspektrum (ESI⁺): m/z = 443 [M+H]⁺

### Beispiel VII

### N-(5-tert-Butyl-3-methansulfonylamino-2-methoxy-phenyl)-3-(6-formyl-pyrimidin-4-yloxy)-4-methyl-benzamid

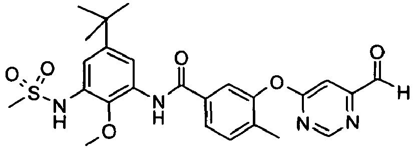

Bei -65°C unter Argonatmosphäre werden zu 1,00 g 6-[5-(5-tert-Butyl-3-methansulfonyl-amino-2-methoxy-phenylaminocarbonyl)-2-methyl-phenoxy]-pyrimidin-4-carbonsäure-methylester in 15 ml Tetrahydrofuran 7.40 ml Diisobutylaluminiumhydrid (1 M in Tetrahydrofuran) getropft, wobei die Temperatur auf -50°C ansteigt. Nach 2 h Rühren werden bei -50°C erneut 7.40 ml Diisobutylaluminiumhydrid (1 M in Tetrahydrofuran) zugegeben. Das Reaktionsgemisch wird über Nacht unter Rühren im Kühlbad auf Raumtemperatur erwärmt und anschließend mit 8 ml 1 M wässriger Kalium-Natriumtartrat-Lösung neutralisiert und mehrmals mit Ethylacetat und Dichlormethan extrahiert. Die vereinigten organischen Phasen werden mit gesättigter wässriger Natriumchlorid-Lösung gewaschen, über Magnesiumsulfat getrocknet und eingeengt. Das Rohprodukt wird ohne weitere Reinigung weiterumgesetzt.
Ausbeute: 640 mg (68 % der Theorie)
Massenspektrum (ESI⁻): m/z = 511 [M-H]⁻

Analog Beispiel VII werden folgende Verbindungen erhalten:
(1) N-(5-tert-Butyl-3-methansulfonylamino-2-methoxy-phenyl)-3-(2-formyl-pyrimidin-4-yloxy)-4-methyl-benzamid 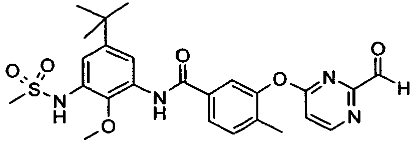 R_{f}-Wert: 0,40 (Kieselgel, Dichlormethan/Methanol = 95:5)
   Massenspektrum (ESI⁻): m/z = 511 [M-H]⁻
(2) N-(5-tert-Butyl-3-methansulfonylamino-2-methoxy-phenyl)-3-(2-formyl-pyridin-4-yloxy)-4-methyl-benzamid 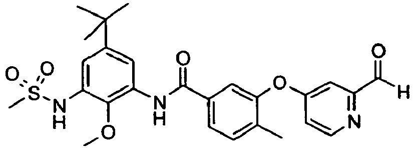 R_{f}-Wert: 0,63 (Kieselgel, Dichlormethan/Methanol/konz. Ammoniak = 90:10:1)
   Massenspektrum (ESI⁺): m/z = 512 [M+H]⁺
(3) 4-[3-(5-tert-Butyl-3-methansulfonylamino-2-methoxy-phenylaminocarbonyl)-5-methyl-phenoxy]-pyrimidin-2-carbonsäure 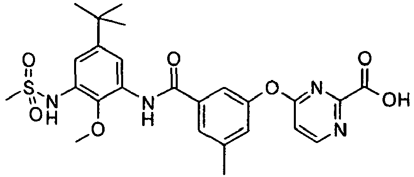 (Wird anstelle des gewünschten N-(5-tert-Butyl-3-methansulfonylamino-2-methoxyphenyl)-3-(2-formyl-pyrimidin-4-yloxy)-5-methyl-benzamids erhalten)
   Massenspektrum (ESI⁺): m/z = 529 [M+H]⁺
(4) N-(5-tert-Butyl-3-methansulfonylamino-2-methoxy-phenyl)-4-chlor-3-(6-formyl-pyrimidin-4-yloxy)-benzamid 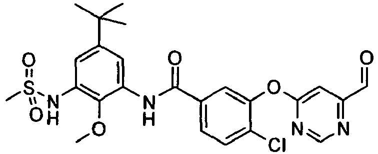 R_{f}-Wert: 0,33 (Kieselgel, Dichlormethan/Methanol = 95:5)
(5) N-(5-tert-Butyl-3-methansulfonylamino-2-methoxy-phenyl)-4-chlor-3-(2-formyl-pyridin-4-yloxy)-benzamid 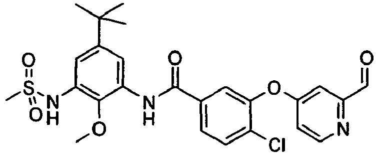 R_{f}-Wert: 0,35 (Kieselgel, Dichlormethan/Methanol = 95:5)
   Massenspektrum (ESI⁺): m/z = 532/534 (CI) [M+H]⁺
(6) N-(5-tert-Butyl-3-methansulfonylamino-2-methoxy-phenyl)-4-chlor-3-(2-formyl-pyrimidin-4-yloxy)-benzamid 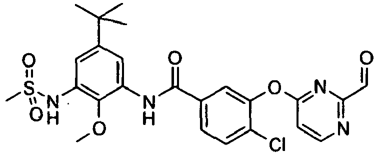 R_{f}-Wert: 0,43 (Kieselgel, Dichlormethan/Methanol = 95:5)

### Beispiel VIII

### 6-[5-(5-tert-Butyl-3-methansulfonylamino-2-methoxy-phenylaminocarbonyl)-2-methyl-phenoxy]-pyrimidin-4-carbonsäure-methylester

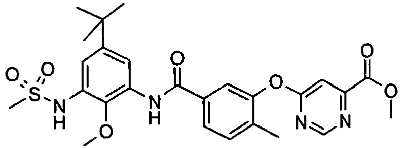

Zu 2,05 g N-(5-tert-Butyl-3-methansulfonylamino-2-methoxy-phenyl)-3-(2-chlor-pyrimidin-4-yloxy)-4-methyl-benzamid in einem Gemisch aus 5 ml Acetonitril und 50 ml Methanol unter Argonatmosphäre werden 30 ml N,N-Dimethylformamid gegeben. Anschließend werden 0.81 ml Triethylamin und 242 mg Dichlor-[1,1-bis(diphenyl-phosphino)ferrocen]-palladium(II)-Dichlormethan-Komplex zugegeben. Das Gemisch wird in einem Druckgefäß mit Kohlenmonoxid versetzt (5 bar) und ca. 15 h auf 70°C erhitzt. Nach Abkühlung auf Raumtemperatur wird der Katalysator abfiltriert und das Filtrat eingeengt. Der Kolbenrückstand wird mit Diethylether verrührt, abgesaugt und getrocknet.
Ausbeute: 2,15 g (100 % der Theorie)
R_{f}-Wert: 0,61 (Kieselgel, Dichlormethan/Methanol = 95:5)
Massenspektrum (ESI⁺): m/z = 543 [M+H]⁺

Analog Beispiel VIII werden folgende Verbindungen erhalten:
(1) 4-[5-(5-tert-Butyl-3-methansulfonylamino-2-methoxy-phenylaminocarbonyl)-2-methyl-phenoxy]-pyrimidin-2-carbonsäure-methylester 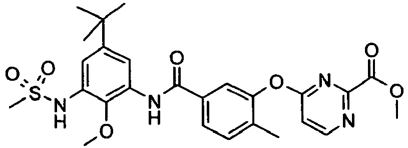 R_{f}-Wert: 0,18 (Kieselgel, Petrolether/Ethylacetat = 3:7)
   Massenspektrum (ESI⁺): m/z = 543 [M+H]⁺
(2) 4-[5-(5-tert-Butyl-3-methansulfonylamino-2-methoxy- phenylaminocarbonyl)-2-methyl-phenoxy]-pyridin-2-carbonsäure-methylester 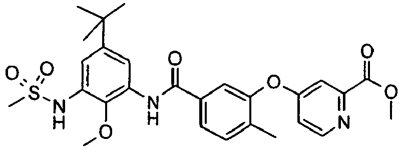 R_{f}-Wert: 0,75 (Kieselgel, Dichlormethan/Methanol/konz. Ammoniak = 90:10:1)
   Massenspektrum (ESI⁺): m/z = 542 [M+H]⁺
(3) 4-[3-(5-tert-Butyl-3-methansulfonylamino-2-methoxy-phenylaminocarbonyl)-5-methyl-phenoxy]-pyrimidin-2-carbonsäure-methylester 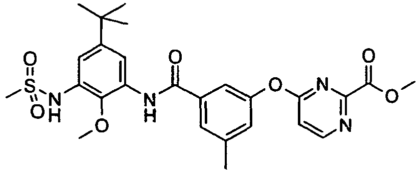 R_{f}-Wert: 0,40 (Reversed Phase DC-Fertigplatte (E. Merck),
   Acetonitril/Wasser/Trifluoressigsäure = 50:50:1)
   Massenspektrum (ESI⁺): m/z = 543 [M+H]⁺
(4) 4-[5-(5-tert-Butyl-3-methansulfonylamino-2-methoxy-phenylcarbamoyl)-2-chlor-phenoxy]-pyridin-2-carbonsäuremethylester 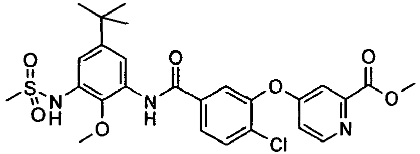 R_{f}-Wert: 0,50 (Kieselgel, Dichlormethan/Methanol = 95:5)
   Massenspektrum (ESI⁺): m/z = 562/564 (CI) [M+H]⁺
(5) 4-[5-(5-tert-Butyl-3-methansulfonylamino-2-methoxy-phenylcarbamoyl)-2-chlor-phenoxy]-pyrimidin-2-carbonsäuremethylester 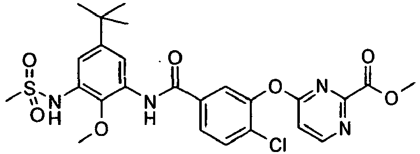 R_{f}-Wert: 0,50 (Kieselgel, Dichlormethan/Methanol = 95:5)
   Massenspektrum (ESI⁺): m/z = 563/565 (CI) [M+H]⁺

### Beispiel IX

### 3-Benzyloxy-4-chlor-benzoesäure

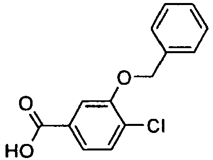

Zu 1.73 g 3-Hydroxy-4-chlor-benzoesäure und 3.04 g Kaliumcarbonat in 10 ml N,N-Dimethylformamid werden 2.67 ml Benzylbromid getropft, und das Reaktionsgemisch wird über Nacht bei Raumtemperatur gerührt. Anschließend wird das Reaktionsgemisch mit Wasser versetzt und mit Ethylacetat extrahiert. Die vereinigten organischen Phasen werden über Magnesiumsulfat getrocknet und eingeengt. Der Kolbenrückstand wird in 5 ml Methanol aufgenommen, mit 3 ml 10 M wässriger Kalilauge versetzt und 4 h bei 50°C gerührt. Die rötliche Lösung wird mit Wasser verdünnt und mit 3 N wässriger Salzsäure angesäuert. Der entstandene Niederschlag wird abgesaugt, mit Wasser gewaschen und getrocknet.
Ausbeute: 2,50g (95 % der Theorie)
Massenspektrum (ESI⁻): m/z = 261/263 (CI) [M-H]⁻
R_{f}-Wert: 0,60 (Kieselgel, Petrolether/Ethylacetat = 1:1)

Analog Beispiel IX werden folgende Verbindungen erhalten:
(1) 3-Benzyloxy-4-trifluormethyl-benzoesäure 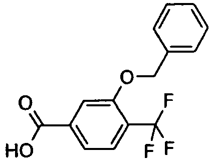 R_{f}-Wert: 0,60 (Kieselgel, Petrolether/Ethylacetat = 1:1)
   Massenspektrum (ESI⁻): m/z = 295 [M-H]⁻
(2) 3-Benzyloxy-5-chlor-4-methyl-benzoesäure 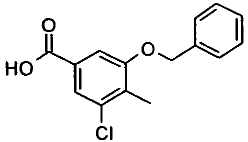 R_{f}-Wert: 0,75 (Kieselgel, Cyclohexan/Ethylacetat/Essigsäure = 50:50:0,1)
   Massenspektrum (ESI⁻): m/z = 275/277 (CI) [M-H]⁻
(3) 3-Benzyloxy-5-fluor-4-methyl-benzoesäure 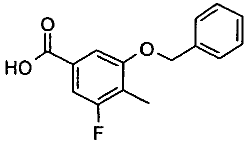 R_{f}-Wert: 0,75 (Kieselgel, Cyclohexan/Ethylacetat/Essigsäure = 50:50:0,1)
   Massenspektrum (ESI⁻): m/z = 259 [M-H]⁻
(4) 3-Benzyloxy-4-methyl-benzoesäure 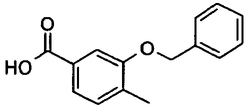 HPLC (Methode 2): Retentionszeit = 3,41 min
   Massenspektrum (ESI⁻): m/z = 241 [M-H]⁻

### Beispiel X

### 4-[4-(5-Carboxy-2-methyl-phenoxy)-pyrimidin-2-yl]-piperazin-1-carbonsäure-tert-butylester

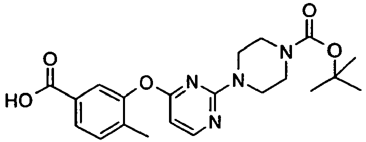

Zu 1.45 g 4-[4-(5-Methoxycarbonyl-2-methyl-phenoxy)-pyrimidin-2-yl]-piperazin-1-carbonsäure-tert-butylester in 15 ml Tetrahydrofuran werden 6.77 ml 1 N Natronlauge gegeben, und das Reaktionsgemisch wird über Nacht bei Raumtemperatur gerührt. Anschließend wird es noch 3 h auf 50°C erwärmt, bis die Umsetzung vollständig ist. Das Reaktionsgemisch wird eingeengt, mit etwas Wasser versetzt und mit 1 N Salzsäure angesäuert. Der ausgefallene Niederschlag wird abgesaugt und getrocknet.
Ausbeute: 1,26 g (90 % der Theorie)
R_{f}-Wert: 0,35 (Kieselgel, Dichlormethan/Methanol = 95:5)
Massenspektrum (ESI⁺): m/z = 415 [M+H]⁺

Analog Beispiel X werden folgende Verbindungen erhalten:
(1) 4-[4-(2-Brom-5-carboxy-phenoxy)-pyrimidin-2-yl]-piperazin-1-carbonsäure-tert-butylester 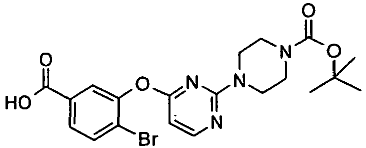 (Esterspaltung erfolgt mit Lithiumhydroxid in einem Tetrahydrofuran/Methanol-Gemisch)
   R_{f}-Wert: 0,13 (Kieselgel, Dichlormethan/Methanol = 98:2)
   Massenspektrum (ESI⁺): m/z = 479/481 (Br) [M+H]⁺
(2) 4-[4-(5-Carboxy-2-ethyl-phenoxy)-pyrimidin-2-yl]-piperazin-1-carbonsäure-tert-butylester 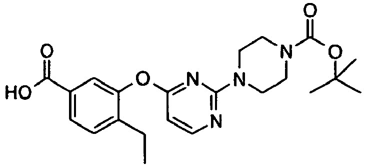 (Esterspaltung erfolgt mit Lithiumhydroxid in einem Tetrahydrofuran/Methanol-Gemisch)
   R_{f}-Wert: 0, 58 (Kieselgel, Cyclohexan/Ethylacetat/Essigsäure = 50:50:0,1)
   Massenspektrum (ESI⁺): m/z = 429 [M+H]⁺
(3) 4-Methyl-3-[2-(4-methyl-piperazin-1-ylmethyl)-pyrimidin-4-yloxy]-benzoesäure 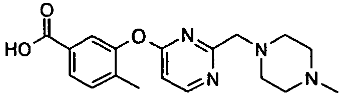 R_{f}-Wert: 0,35 (Kieselgel, Dichlormethan/Methanol/konz. Ammoniak = 95:5:0,1)
   Massenspektrum (ESI⁺): m/z = 343 [M+H]⁺
(4) 4-[4-(5-Carboxy-2-methyl-phenoxy)-pyridin-2-ylmethyl]-piperidin-1-carbonsäure-tert-butyl-ester 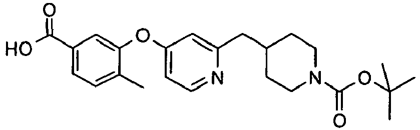 (Die Umsetzung erfolgt in Ethanol statt Tetrahydrofuran)
   HPLC (Methode 1): Retentionszeit = 2,70 min
   Massenspektrum (ESI⁺): m/z = 427 [M+H]⁺
(5) 3-(2-Chlor-pyridin-4-yloxy)-4-methyl-benzoesäure 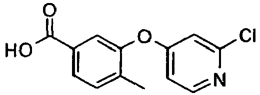 (Die Umsetzung erfolgt in Ethanol statt Tetrahydrofuran)

### Beispiel XI

### 4-{4-[5-(5-tert-Butyl-3-methansulfonylamino-2-methoxy-phenylaminocarbonyl)-2-methyl-phenoxy]-pyrimidin-2-ylmethyl}-piperazin-1-carbonsäure-tert-butylester

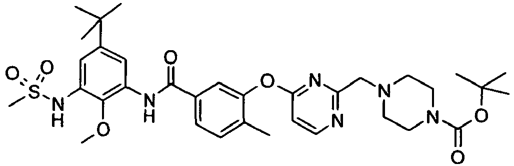

Zu 180 mg N-(5-tert-Butyl-3-methansulfonylamino-2-methoxy-phenyl)-3-(2-formyl-pyrimidin-4-yloxy)-4-methyl-benzamid in 8 ml ClCH₂CH₂Cl werden 262 mg Piperazin-1-carbonsäure-tert-butylester, 89 µl Essigsäure und 89 mg Natriumtriacetoxyborhydrid gegeben, und das Reaktionsgemisch wird 3,5 h bei Raumtemperatur gerührt. Zur Aufarbeitung wird das Reaktionsgemisch mit etwas Dichlormethan verdünnt und mit gesättigter wässriger Natriumhydrogencarbonat-Lösung verrührt. Die organische Phase wird mit Wasser gewaschen, über Magnesiumsulfat getrocknet und eingeengt. Der Kolbenrückstand wird über eine Kieselgelsäule mit Dichlormethan/Methanol (98:2→96:4) chromatographiert. Das harzartige Produkt wird mit wenig Diethylether verrührt und stehen gelassen, bis es vollständig kristallisiert ist. Dann wird der Feststoff abgesaugt und getrocknet.
Ausbeute: 112 mg (58 % der Theorie)
R_{f}-Wert: 0,53 (Kieselgel, Dichlormethan/Methanol = 95:5)
Massenspektrum (ESI⁻): m/z = 681 [M-H]⁻

Analog Beispiel XI werden folgende Verbindungen erhalten:
(1) 4-{6-[5-(5-tert-Butyl-3-methansulfonylamino-2-methoxy-phenylaminocarbonyl)-2-methyl-phenoxy]-pyrimidin-4-ylmethyl}-piperazin-1-carbonsäure-tert-butylester 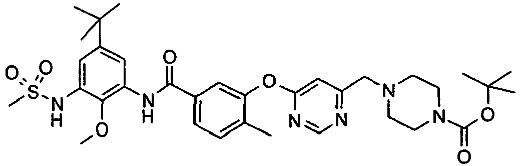 R_{f}-Wert: 0,84 (Kieselgel, Dichlormethan/Methanol/konz. Ammoniak = 90:10:1)
   Massenspektrum (ESI⁺): m/z = 683 [M+H]⁺
(2) 4-{4-[5-(5-tert-Butyl-3-methansulfonylamino-2-methoxy-phenylaminocarbonyl)-2-methyl-phenoxy]-pyridin-2-ylmethyl}-piperazin-1-carbonsäure-tert-butylester 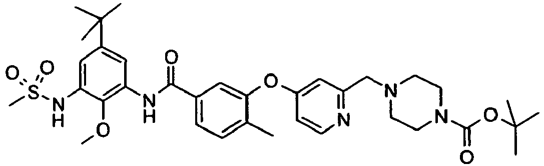 R_{f}-Wert: 0,72 (Kieselgel, Dichlormethan/Methanol/konz. Ammoniak = 90:10:1)
   Massenspektrum (ESI⁺): m/z = 682 [M+H]⁺
(3) 4-{4-[3-(5-tert-Butyl-3-methansulfonylamino-2-methoxy-phenylaminocarbonyl)-5-methyl-phenoxy]-pyrimidin-2-ylmethyl}-piperazin-1-carbonsäure-tert-butylester 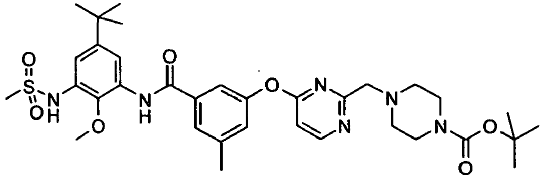 R_{f}-Wert: 0,65 (Kieselgel, Dichlormethan/Methanol = 90:10)
   Massenspektrum (ESI⁺): m/z = 683 [M+H]⁺
(4) 4-{6-[5-(5-tert-Butyl-3-methansulfonylamino-2-methoxy-phenylcarbamoyl)-2-chlor-phenoxy]-pyrimidin-4-ylmethyl}-piperazin-1-carbonsäure-tert-butyl-ester 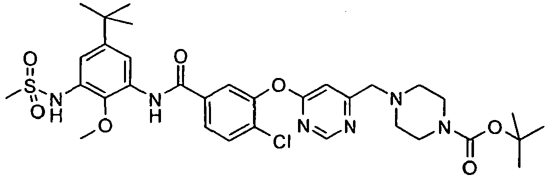 R_{f}-Wert: 0,40 (Kieselgel, Dichlormethan/Methanol = 95:5)
   Massenspektrum (ESI⁺): m/z = 703/705 (CI) [M+H]⁺
(5) 4-{4-[5-(5-tert-Butyl-3-methansulfonylamino-2-methoxy-phenylcarbamoyl)-2-chlor-phenoxy]-pyridin-2-ylmethyl}-piperazin-1-carbonsäure-tert-butyl-ester 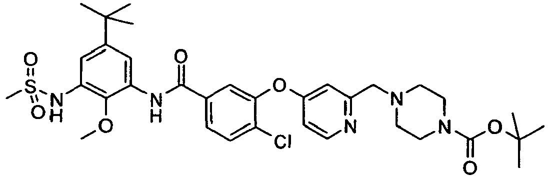 R_{f}-Wert: 0,45 (Kieselgel, Dichlormethan/Methanol = 95:5)
   Massenspektrum (ESI⁺): m/z = 702/704 (CI) [M+H]⁺
(6) 4-{4-[5-(5-tert-Butyl-3-methansulfonylamino-2-methoxy-phenylcarbamoyl)-2-chlor-phenoxy]-pyrimidin-2-ylmethyl}-piperazin-1-carbonsäure-tert-butyl-ester 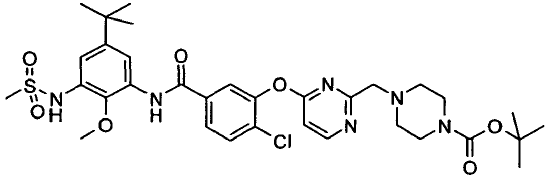 R_{f}-Wert: 0,39 (Kieselgel, Dichlormethan/Methanol = 95:5)
   Massenspektrum (ESI⁻): m/z = 701/703 (CI) [M-H]⁻
(7) 4-{6-[5-(5-tert-Butyl-3-methansulfonylamino-2-methoxy-phenylcarbamoyl)-2-chlor-phenoxy]-pyrimidin-4-ylmethyl}-[1,4]diazepan-1-carbonsäure-tert-butyl-ester 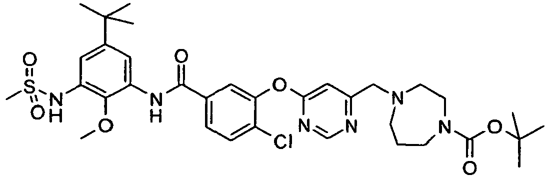 R_{f}-Wert: 0,40 (Kieselgel, Dichlormethan/Methanol = 95:5)
   Massenspektrum (ESI⁺): m/z = 717/719 (CI) [M+H]⁺
(8) 4-{4-[5-(5-tert-Butyl-3-methansulfonylamino-2-methoxy-phenylcarbamoyl)-2-chlor-phenoxy]-pyridin-2-ylmethyl}-[1,4]diazepan-1-carbonsäure-tert-butyl-ester 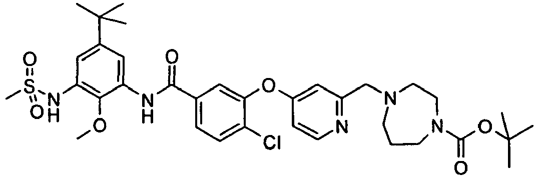 R_{f}-Wert: 0,30 (Kieselgel, Dichlormethan/Methanol = 95:5) Massenspektrum (ESI⁺): m/z = 716/718 (CI) [M+H]⁺
(9) 4-{4-[5-(5-tert-Butyl-3-methansulfonylamino-2-methoxy-phenylcarbamoyl)-2-methyl-phenoxy]-pyridin-2-ylmethyl}-[1,4]diazepan-1-carbonsäure-tert-butyl-ester 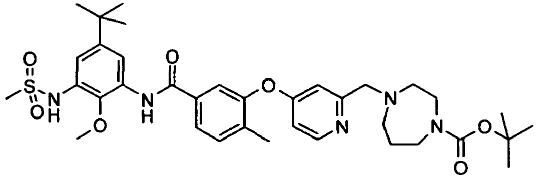 R_{f}-Wert: 0,6 (Kieselgel, Dichlormethan/Methanol = 90:10)
   Massenspektrum (ESI⁺): m/z = 696 [M+H]⁺
(10) (5-tert-Butyl-2-methoxy-3-nitro-benzyl)-dimethyl-amin 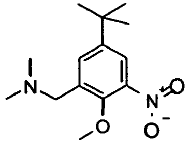 (5-tert-Butyl-2-methoxy-3-nitro-benzaldehyd und Dimethylamin werden miteinander umgesetzt)
   R_{f}-Wert: 0,69 (Kieselgel, Dichlormethan/Methanol/konz. Ammoniak = 90:10:0,1)
   Massenspektrum (ESI⁺): m/z = 267 [M+H]⁺
(11) 4-{4-[5-(5-tert-Butyl-2-methoxy-3-pyrrolidin-1-ylmethyl-phenylcarbamoyl)-2-methyl-phenoxy]-pyridin-2-ylmethyl}-piperidin-1-carbonsäure-tert-butyl-ester 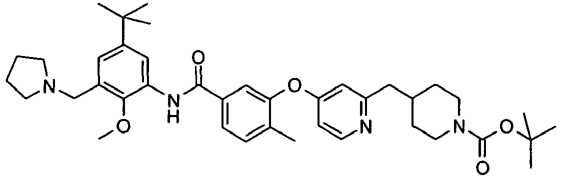 (4-{4-[5-(5-tert-Butyl-3-formyl-2-methoxy-phenylcarbamoyl)-2-methyl-phenoxy]-pyridin-2-ylmethyl}-piperidin-1-carbonsäure-tert-butyl-ester und Pyrrolidin werden miteinander umgesetzt)
   Massenspektrum (ESI⁺): m/z = 671 [M+H]⁺
(12) (5-tert-Butyl-2-methoxy-3-nitro-benzyl)-cyclopropyl-amin 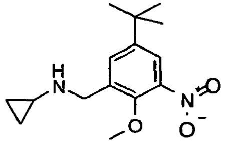 (5-tert-Butyl-2-methoxy-3-nitro-benzaldehyd und Cyclopropylamin werden eingesetzt)
   Massenspektrum (ESI⁺): m/z = 279 [M+H]⁺

### Beispiel XII

### N-(5-tert-Butyl-3-methansulfonylamino-2-methoxy-phenyl)-3-(2-chlor-pyridin-4-yloxy)-4-methyl-benzamid

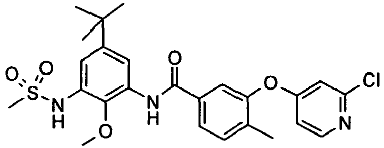

Zu 2,77 g N-(5-tert-Butyl-3-methansulfonylamino-2-methoxy-phenyl)-3-hydroxy-4-methyl-benzamid in 15 ml N,N-Dimethylformamid werden unter Argonatmosphäre und Eisbad-Kühlung 545 mg Natriumhydrid (60%ig in Mineralöl) gegeben, und das Reaktionsgemisch wird bei Raumtemperatur gerührt, bis keine weitere Wasserstoffentwicklung mehr zu erkennen ist. Nun werden 1,63 g 2-Chlor-4-iodpyridin zugegeben, und das Reaktionsgemisch wird über Nacht bei 110°C gerührt. Nach Abkühlung auf Raumtemperatur wird das Lösungsmittel am Rotationsverdampfer abdestilliert. Der Kolbenrückstand wird in Ethylacetat aufgenommen, mit gesättigter wässriger Natriumhydrocencarbonat-Lösung und gesättigter wässriger Natriumchlorid-Lösung gewaschen, über Magnesiumsulfat getrocknet und eingeengt. Der Kolbenrückstand wird über eine Kieselgelsäule mit Petrolether/Ethylacetat (80:20→30:70) als Laufmittel chromatographiert. Das Produkt wird mit Diethylether verrührt, abgesaugt und getrocknet.
Ausbeute: 1,77 g (50 % der Theorie)
R_{f}-Wert: 0,30 (Kieselgel, Petrolether/Ethylacetat = 1:1)
Massenspektrum (ESI⁺): m/z = 518/520 (CI) [M+H]⁺

### Beispiel XIII

### 3-Benzyloxy-N-(5-tert-butyl-3-methansulfonylamino-2-methoxy-phenyl)-4-methoxy-benzamid

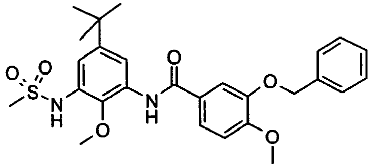

Zu 526 mg N-(3-Amino-5-tert-butyl-2methoxyphenyl)methansulfonamid-hydrochlorid in 7 ml Acetonitril werden 0,86 ml Triethylamin gegeben. Dann wird eine Lösung von 3-Benzyloxy-4-methoxy-benzoylchlorid in 3 ml Acetonitril portionsweise zugegeben, und das Reaktionsgemisch wird 30 Minuten bei Raumtemperatur gerührt, wobei ein heller Niederschlag ausfällt. Die helle Suspension wird teilweise eingeengt, der Rückstand mit Wasser versetzt und angesäuert. Der Niederschlag wird abgesaugt, mit Wasser und wenig Methanol gewaschen und getrocknet.
Ausbeute: 679 mg (86 % der Theorie)
R_{f}-Wert: 0,35 (Kieselgel, Cyclohexan/Ethylacetat = 1:1)
Massenspektrum (ESI⁺): m/z = 513 [M+H]⁺

Analog Beispiel XIII werden folgende Verbindungen erhalten:
(1) 3-Benzyloxy-N-(5-tert-butyl-3-methansulfonylamino-2-methoxy-phenyl)-5-chlor-4-methyl-benzamid 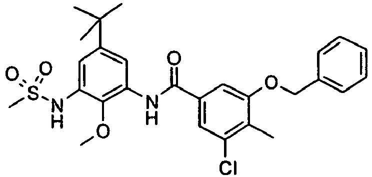 R_{f}-Wert: 0,67 (Kieselgel, Cyclohexan/Ethylacetat = 1:1)
   Massenspektrum (ESI⁺): m/z = 531/533 (CI) [M+H]⁺
(2) 3-Benzyloxy-N-(5-tert-butyl-3-methansulfonylamino-2-methoxy-phenyl)-5-fluor-4-methyl-benzamid 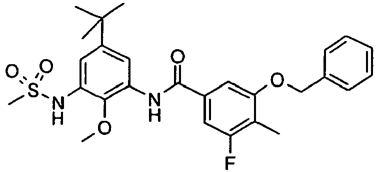 R_{f}-Wert: 0,68 (Kieselgel, Cyclohexan/Ethylacetat = 1:1)
   Massenspektrum (ESI⁺): m/z = 515 [M+H]⁺

### Beispiel XIV

### 3-Benzyloxy-5-chlor-4-methyl-benzoylchlorid

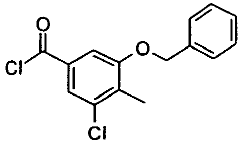

350 mg 3-Benzyloxy-5-chlor-4-methyl-benzoesäure in 10 ml Acetonitril werden mit 0,60 ml Thionychlorid versetzt und 15 Minuten unter leichtem Rückfluss erhitzt. Anschließend wird das Reaktionsgemisch eingeengt und mit Toluol nachgedampft. Das Säurechlorid wird ohne weitere Reinigung weiterumgesetzt.

Analog Beispiel XIV wird folgende Verbindung erhalten:
(1) 3-Benzyloxy-5-fluor-4-methyl-benzoylchlorid

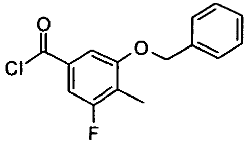

### Beispiel XV

### 3-Chlor-5-hydroxy-4-methyl-benzoesäure

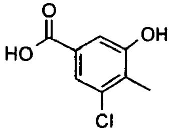

840 mg 3-Amino-5-chlor-4-methyl-benzoesäure werden in 20 ml 30%iger wässriger Schwefelsäure suspendiert und 10 Minuten bei 90°C gerührt. Die feine Suspension wird mit einem Eis-Kochsalzbad abgekühlt und bei 0-5°C Innentemperatur tropfenweise mit einer Lösung von 325 mg Natriumnitrit in 4.5 ml Wasser versetzt. Das Reaktionsgemisch wird noch 30 Minuten bei 0°C Innentemperatur gerührt und anschließend zu 40 ml einer bei 135°C gerührten 20%igen wässrigen Schwefelsäure-Lösung gegossen. Nach 1.5 h wird das Reaktionsgemisch im Eisbad abgekühlt, mit Eiswasser verdünnt und mit tert-Butylmethylether extrahiert. Die vereinigten Extrakte werden mit Wasser und gesättigter wässriger Natriumchlorid-Lösung gewaschen, über Magnesiumsulfat getrocknet und eingeengt.
Ausbeute: 790 mg (94 % der Theorie)
R_{f}-Wert: 0,55 (Kieselgel, Cyclohexan/Ethylacetat/Essigsäure = 50:50:0,1)
Massenspektrum (ESI⁻): m/z = 185/187 (CI) [M-H]⁻

Analog Beispiel XV wird folgende Verbindung erhalten:
(1) 3-Fluor-5-hydroxy-4-methyl-benzoesäure 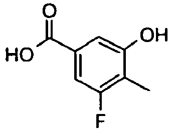 (3-Fluor-5-hydroxy-4-methyl-benzoesäuremethylester wird als Startmaterial eingesetzt; die Estergruppe wird unter den Reaktionsbedingungen zur Säure hydrolysiert)
   R_{f}-Wert: 0,55 (Kieselgel, Cyclohexan/Ethylacetat/Essigsäure = 50:50:0,1) Massenspektrum (ESI⁻): m/z = 169 [M-H]⁻

### Beispiel XVI

### 3-Amino-5-chlor-4-methyl-benzoesäure

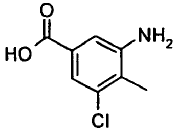

Hergestellt durch Schütteln einer Lösung von 1,00 g 3-Chlor-4-methyl-5-nitrobenzoesäure in 20 ml Tetrahydrofuran mit 100 mg Raney-Nickel in einer Atmosphäre von 50 psi Wasserstoffpartialdruck bei Raumtemperatur. Nach 9 h Reaktionszeit wird der Katalysator abfiltriert und das Filtrat eingeengt.
Ausbeute: 0,85 g (99 % der Theorie)
R_{f}-Wert: 0,60 (Kieselgel, Cyclohexan/Ethylacetat/Essigsäure = 50:50:0,1)
Massenspektrum (ESI⁻): m/z = 184/186 (CI) [M-H]⁻

Analog Beispiel XVI wird folgende Verbindung erhalten:
(1) 3-Amino-5-fluor-4-methyl-benzoesäure-methylester 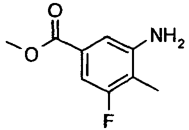 R_{f}-Wert: 0,35 (Kieselgel, Petrolether/Ethylacetat = 7:3)
   Massenspektrum (ESI⁺): m/z = 184 [M+H]⁺

### Beispiel XVII

### 3-Fluor-4-methyl-5-nitro-benzoesäure-methylester

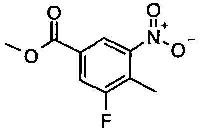

Zu 3,89 g Nitrosyltetrafluorborat in 15 ml 1,4-Dioxan wird unter Argonatmosphäre eine Lösung von 1,00 g 3-Amino-4-methyl-5-nitro-benzoesäure-methylester in 15 ml 1,4-Dioxan unter Rühren rasch zugetropft. Das Reaktionsgemisch wird eine Stunde bei Raumtemperatur, eine weitere Stunde bei 55°C und anschließend weitere acht Stunden bei 90-95°C gerührt. Nach Stehen über Nacht bei Raumtemperatur wird das Reaktionsgemisch eingeengt und der Rückstand zwischen tert-Butylmethylether und Wasser verteilt. Die organische Phase wird mit wässriger Natriumhydrogencarbonat-Lösung, Wasser und gesättigter wässriger Natriumchlorid-Lösung gewaschen, über Magnesiumsulfat getrocknet und eingeengt. Das Rohprodukt wird über eine Kieselgelsäule mit Petrolether/Ethylacetat (95:5) chromatographiert.
Ausbeute: 310 mg (31 % der Theorie)
R_{f}-Wert: 0,60 (Kieselgel, Petrolether/Ethylacetat = 7:3)

### Beispiel XVIII

### 4-{6-[5-(5-tert-Butyl-3-methansulfonylamino-2-methoxy-phenylaminocarbonyl)-2-methyl-phenoxyl-pyrimidin-4-ylmethyl}-piperidin-1-carbonsäure-tert-butylester

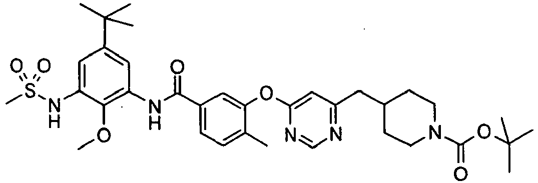

Zu 400 mg N-(5-tert-Butyl-3-methansulfonylamino-2-methoxy-phenyl)-3-(6-chlor-pyrimidin-4-yloxy)-4-methyl-benzamid in 3,5 ml N,N-Dimethylformamid und 350 µl Wasser werden unter Argonatmosphäre 750 mg Kaliumcarbonat und 55 mg Palladium-dichlor[1,1'-bis(diphenylphosphino)ferrocen]*CH₂Cl₂ zugegeben. Dann werden 1,35 ml der Reaktionslösung des Hydroborierungsprodukts aus Beispiel XIX zugegeben, und das Reaktionsgemisch wird 3 h bei 60°C gerührt. Zur Aufarbeitung wird das Reaktionsgemisch eingeengt, der Rückstand in Ethylacetat aufgenommen und filtriert. Das Filtrat wird mehrmals mit Wasser und einmal mit gesättigter wässriger Natriumchlorid-Lösung gewaschen, über Magnesiumsulfat getrocknet und eingeengt. Das Rohprodukt wird über eine Kieselgelsäule mit Dichlormethan/Ethylacetat (40:60→30:70) gereinigt.
Ausbeute: 480 mg (91 % der Theorie)
HPLC (Methode 1): Retentionszeit = 4,59 min
Massenspektrum (ESI⁺): m/z = 682 [M+H]⁺

Analog Beispiel XVIII werden folgende Verbindungen erhalten:
(1) 4-{4-[5-(5-tert-Butyl-3-methansulfonylamino-2-methoxy-phenylaminocarbonyl)-2-methyl-phenoxy]-pyrimidin-2-ylmethyl}-piperidin-1-carbonsäure-tert-butylester 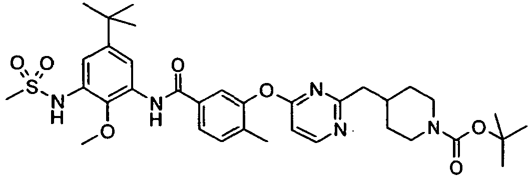 HPLC (Methode 1): Retentionszeit = 4,59 min
   Massenspektrum (ESI⁺): m/z = 682 [M+H]⁺
(2) 4-{4-[5-(5-tert-Butyl-3-methansulfonylamino-2-methoxy-phenylcarbamoyl)-2-methyl-phenoxy]-pyridin-2-ylmethyl}-piperidin-1-carbonsäure-tert-butylester 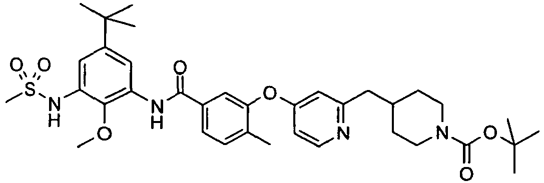 HPLC (Methode 1): Retentionszeit = 3,39 min
   Massenspektrum (ESI⁺): m/z = 681 [M+H]⁺
(3) 4-{4-[5-(5-tert-Butyl-3-methansulfonylamino-2-methoxy-phenylcarbamoyl)-2-chlor-phenoxy]-pyridin-2-ylmethyl}-piperidin-1-carbonsäure-tert-butyl-ester 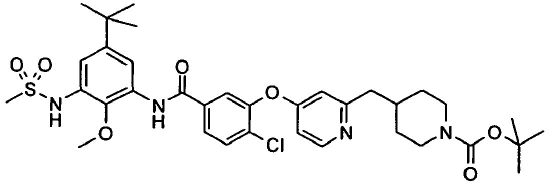 R_{f}-Wert: 0,35 (Kieselgel, Dichlormethan/Methanol = 95:5)
   Massenspektrum (ESI⁺): m/z = 701/703 (CI) [M+H]⁺
(4) 4-{4-[5-(5-tert-Butyl-3-methansulfonylamino-2-methoxy-phenylcarbamoyl)-2-chlor-phenoxy]-pyrimidin-2-ylmethyl}-piperidin-1-carbonsäure-tert-butyl-ester 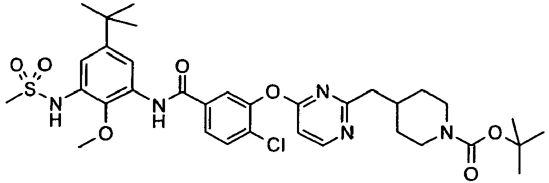 R_{f}-Wert: 0,15 (Kieselgel, Cyclohexan/Ethylacetat = 1:1)
   Massenspektrum (ESI⁺): m/z = 702/704 (CI) [M+H]⁺
(5) 4-{4-[5-(5-tert-Butyl-3-methansülfonylamino-2-methoxy-phenylcarbamoyl)-2-chlor-phenoxy]-pyrimidin-2-ylmethyl}piperidin-1-carbonsäure-tert-butyl-ester 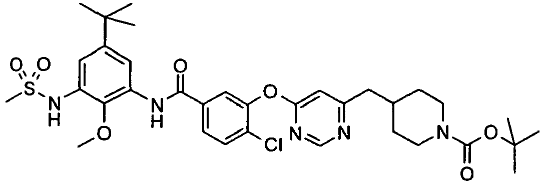 R_{f}-Wert: 0,40 (Kieselgel, Dichlormethan/Methanol = 95:5)
   Massenspektrum (ESI⁻): m/z = 700/702 (CI) [M-H]⁻
(6) 4-{4-[5-(5-tert-Butyl-3-methansulfonyl-2-methoxy-phenylcarbamoyl)-2-methyl-phenoxy]-pyrimidin-2-ylmethyl}-piperidin-1-carbonsäure-tert-butyl-ester 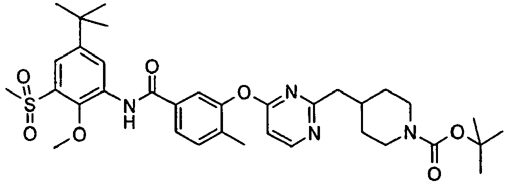 R_{f}-Wert: 0,15 (Kieselgel, Cyclohexan/Ethylacetat = 1:1)
   Massenspektrum (ESI⁺): m/z = 667 [M+H]⁺
(7) 4-[4-(5-Methoxycarbonyl-2-methyl-phenoxy)-pyridin-2-ylmethyl]-piperidin-1-carbonsäure-tert-butyl-ester 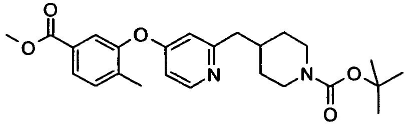 HPLC (Methode 1): Retentionszeit = 3,05 min
   Massenspektrum (ESI⁺): m/z = 441 [M+H]⁺
(8) 4-{4-[5-(5-tert-Butyl-3-methansulfonylamino-2-methoxy-phenylcarbamoyl)-2-methyl-phenoxy]-pyridin-2-ylmethyl}-azepan-1-carbonsäure-tert-butyl-ester 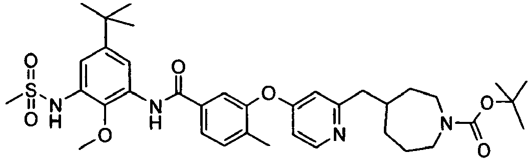 R_{f}-Wert: 0,35 (Kieselgel, Dichlormethan/Methanol = 95:5)
   Massenspektrum (ESI⁺): m/z = 695 [M+H]⁺
(9) 4-{4-[5-(5-tert-Butyl-3-methansulfonylamino-2-methoxy-phenylcarbamoyl)-2-methyl-phenoxy]-pyrimidin-2-ylmethyl}-azepan-1-carbonsäure-tert-butyl-ester 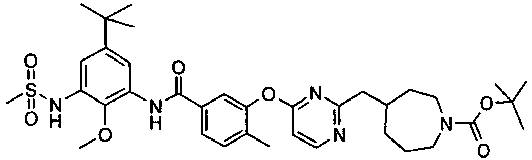 R_{f}-Wert: 0,20 (Kieselgel, Ethylacetat/Petrolether = 7:3)
   Massenspektrum (ESI⁺): m/z = 696 [M+H]⁺
(10) 4-{6-[5-(5-tert-Butyl-3-methansulfonylamino-2-methoxy-phenylcarbamoyl)-2-methyl-phenoxy]-pyrimidin-4-ylmethyl}-azepan-1-carbonsäure-tert-butyl-ester 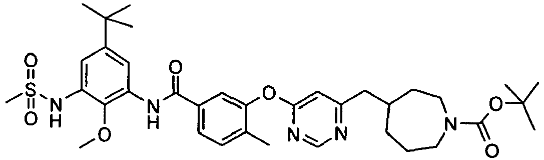 Massenspektrum (ESI⁺): m/z = 696 [M+H]⁺

### Beispiel XIX

### 4-(9-Bora-bicyclo[3.3.1]non-9-ylmethyl)-piperidin-1-carbonsäure-tert-butylester

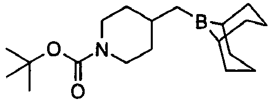

Zu 620 mg 4-Methylen-piperidin-1-carbonsäure-tert-butylester werden unter Argonatmosphäre 6,29 ml einer 0.5 M Lösung von 9-Borabicyclo[3.3.1]nonan in Tetrahydrofuran gegeben, und das Reaktionsgemisch wird 1 h unter Rückfluss erhitzt. Nach Abkühlung auf Raumtemperatur wird die Reaktionslösung ohne weitere Aufarbeitung weiterumgesetzt.

Analog Beispiel XIX wird folgende Verbindung erhalten:
(1) 4-(9-Bora-bicyclo[3.3.1]non-9-ylmethyl)-azepan-1-carbonsäure-tert-butyl ester

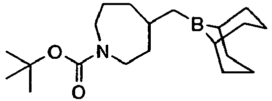

### Beispiel XX

### 4{4-[3-(5-tert-Butyl-3-methansulfonylamino-2-methoxy-phenylaminocarbonyl)-5-methyl-phenoxy]-pyrimidin-2-carbonyl}-piperazin-1-carbonsäure-tert-butylester

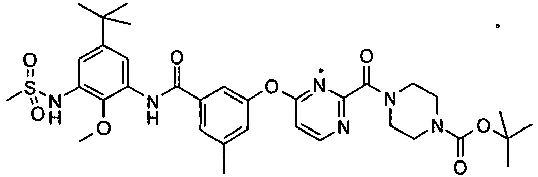

Ein Gemisch aus 39 mg 4-[3-(5-tert-Butyl-3-methansulfonylamino-2-methoxy-phenyl-aminocarbonyl)-5-methyl-phenoxy]-pyrimidin-2-carbonsäure, 25 mg O-(Benzotriazol-1-yl)-1,1,3,3-tetramethyluronium-tetrafluorborat, 25 µl Diisopropylethylamin und 15 mg Piperazin-1-carbonsäure-tert-butylester in 3 ml N,N-Dimethylformamid wird über Nacht bei Raumtemperatur gerührt. Anschließend wird das Reaktionsgemisch eingeengt und der Kolbenrückstand über eine Kieselgelsäule mit Dichlormethan/Methanol (100:0→90:10) als Laufmittel chromatographiert.
Ausbeute: 37 mg (72 % der Theorie)
R_{f}-Wert: 0,15 (Reversed Phase DC-Fertigplatte (E. Merck), Acetonitril/Wasser/Trifluoressigsäure = 50:50:1)

### Beispiel XXI

### N-(5-tert-Butyl-3-methansulfonylamino-2-methoxy-phenyl)-3-(2-formyl-pyrimidin-4-yloxy)-5-methyl-benzamid

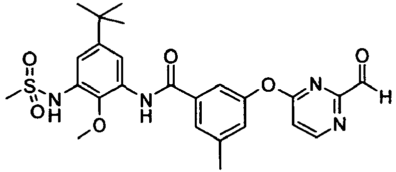

Zu einer Suspension aus 3,00 g Kieselgel in 12 ml Dichlormethan wird eine Lösung aus 400 mg Natriumperiodat in 3 ml Wasser getropft. Dann wird eine Lösung von 247 mg N-(5-tert-Butyl-3-methansulfonylamino-2-methoxy-phenyl)-3-[2-(1,2-dihydroxy-ethyl)-pyrimidin-4-yloxy]-5-methyl-benzamid in 3 ml Dichlormethan zugegeben, und das Reaktionsgemisch wird zwei Stunden bei Raumtemperatur gerührt. Anschließend wird das Reaktionsgemisch abgesaugt und der Filterkuchen mit Dichlormethan/ Ethylacetat gewaschen. Das Filtrat wird über Magnesiumsulfat getrocknet und einrotiert.
Ausbeute: 215 (93 % der Theorie)
R_{f}-Wert: 0,40 (Reversed Phase DC-Fertigplatte (E. Merck), Acetonitril/Wasser/ Trifluoressigsäure = 50:50:1)
Massenspektrum (ESI⁺): m/z = 513 [M+H]⁺

Analog Beispiel XXI wird folgende Verbindung erhalten:
(1) N-(5-tert-Butyl-3-methansulfonylamino-2-methoxy-phenyl)-3-(6-formyl-pyrimidin-4-yloxy)-4-methyl-benzamid 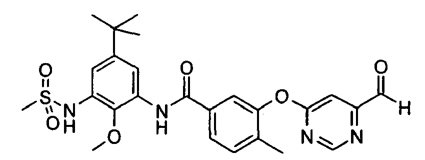 HPLC (Methode 1): Retentionszeit = 3,46 min
   Massenspektrum (ESI⁺): m/z = 513 [M+H]⁺

### Beispiel XXII

### N-(5-tert-Butyl-3-methansulfonylamino-2-methoxy-phenyl)-3-[2-(1,2-dihydroxy-ethyl)-pyrimidin-4-yloxy]-5-methyl-benzamid

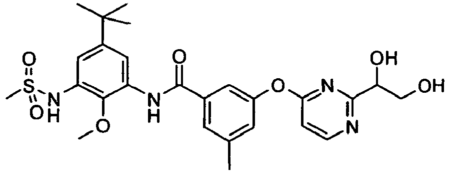

Zu 298 mg N-(5-tert-Butyl-3-methansuffonylamino-2-methoxy-phenyl)-3-methyl-5-(2-vinyl-pyrimidin-4-yloxy)-benzamid in 3 ml Aceton werden 360 mg N-Methyl-morpholin-N-oxid, 110 µl einer 4 %igen wässrigen Osmiumtetroxid-Lösung und 300 µl Wasser gegeben, und das Reaktionsgemisch wird über Nacht bei Raumtemperatur gerührt. Dann wird eine Lösung aus 400 mg Natriumsulfit in 10 ml Wasser zugegeben. Das Gemisch wird eine halbe Stunde gerührt und anschließend im Vakuum eingeengt. Der Rückstand wird mit Ethylacetat extrahiert, die wässrige Phase wird mit gesättigter wässriger Natriumchlorid-Lösung versetzt und erneut mit Ethylacetat extrahiert. Die vereinigten organischen Phasen werden über Magnesiumsulfat getrocknet und eingeengt.
Ausbeute: 247 mg (78 % der Theorie)
R_{f}-Wert: 0,45 (Reversed Phase DC-Fertigplatte (E. Merck), Acetonitril/Wasser/ Trifluoressigsäure = 50:50:1)
Massenspektrum (ESI⁺): m/z = 545 [M+H]⁺

Analog Beispiel XXII wird folgende Verbindung erhalten:
(1) N-(5-tert-Butyl-3-methansulfonylamino-2-methoxy-phenyl)-3-[6-(1,2-dihydroxy-ethyl)-pyrimidin-4-yloxy]-4-methyl-benzamid 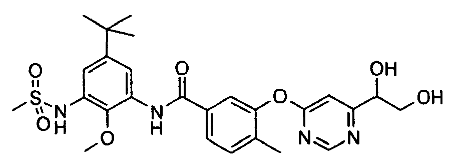 HPLC (Methode 1): Retentionszeit = 3,15 min
   Massenspektrum (ESI⁺): m/z = 545 [M+H]⁺

### Beispiel XXIII

### N-(5-tert-Butyl-3-methansulfonylamino-2-methoxy-phenyl)-3-methyl-5-(2-vinyl-pyrimidin-4-yloxy)-benzamid

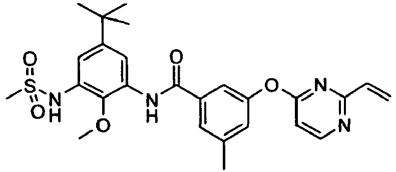

Zu 529 mg N-(5-tert-Butyl-3-methansulfonylamino-2-methoxy-phenyl)-3-(2-chlor-pyrimidin-4-yloxy)-5-methyl-benzamid und 600 mg Kalium-vinyltrifluorborat in 8 ml Tetrahydrofuran und 2 ml Toluol unter Argonatmosphäre werden 120 µl Wasser und 240 mg Tetrakistriphenylphosphinpalladium(0) gegeben, und das Reaktionsgemisch wird über Nacht bei 100 °C gerührt. Zur Aufarbeitung wird das Reaktionsgemisch mit Ethylacetat verdünnt, mit Wasser, 10%iger wässriger Ammoniumchlorid-Lösung und gesättigter wässriger Natriumchlorid-Lösung gewaschen, über Magnesiumsulfat getrocknet und eingeengt. Der Kolbenrückstand wird über eine Kieselgelsäule mit Cylohexan/Ethylacetat (80:20→0:100) als Laufmittel chromatographiert.
Ausbeute: 298 mg (57 % der Theorie)
R_{f}-Wert: 0,20 (Kieselgel, Cyclohexan/Ethylacetat = 1:1)
Massenspektrum (ESI⁺): m/z = 511 [M+H]⁺

Analog Beispiel XXIII wird folgende Verbindung erhalten:
(1) N-(5-tert-Butyl-3-methansulfonylamino-2-methoxy-phenyl)-4-methyl-3-(6-vinyl-pyrimidin-4-yloxy)-benzamid 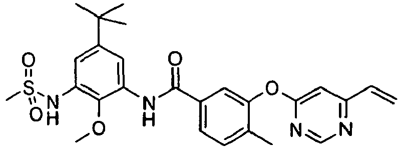 HPLC (Methode 1): Retentionszeit = 4,08 min
   Massenspektrum (ESI⁺): m/z = 511 [M+H]⁺

### Beispiel XXIV

### 4-Methyl-3-[2-(4-methyl-piperazin-1-ylmethyl)-pyrimidin-4-yloxy]-benzoesäure-methylester

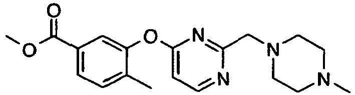

Zu 264 mg 3-Hydroxy-4-methyl-benzoesäure-methylester in 4 ml Dimethylsulfoxid werden unter Argonatmosphäre 225 mg Kalium-tert-butanolat gegeben. Nach einigen Minuten werden 360 mg 4-Chlor-2-(4-methyl-piperazin-1-ylmethyl)-pyrimidin in 2 ml Dimethylsulfoxid gelöst zugegeben, und das Reaktionsgemisch wird bei Raumtemperatur über Nacht gerührt. Zur Aufarbeitung wird das Reaktionsgemisch mit Wasser versetzt und mit Ethylacetat extrahiert. Die vereinigten Ethylacetat-Extrakte werden mit gesättigter wässriger Natriumchlorid-Lösung gewaschen, über Magnesiumsulfat getrocknet und eingeengt. Das Rohprodukt wird chromatographisch über eine Kieselgelsäule mit Dichlormethan/Methanol/konz. Ammoniak (96:4:0→95:4:1) gereinigt.
Ausbeute: 313 mg (55 % der Theorie)
R_{f}-Wert: 0,28 (Kieselgel, Dichlormethan/Methanol/konz. Ammoniak = 95:5:0,1)
Massenspektrum (ESI⁺): m/z = 357 [M+H]⁺

### Beispiel XXV

### 4-Chlor-2-(4-methyl-piperazin-1-ylmethyl)-pyrimidin

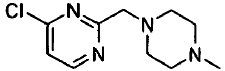

700 mg 2-(4-Methyl-piperazin-1-ylmethyl)-pyrimidin-4-ol in 4 ml Acetonitril werden zum Sieden erhitzt und mit 2 ml Phosphoroxychlorid versetzt. Nach 10 Minuten wird das Reaktionsgemisch im Wasserstrahlvakuum eingeengt, mit Eis und Dichlormethan versetzt und unter Eisbad-Kühlung mit gesättigter wässriger Natriumcarbonat-Lösung alkalisch gestellt. Nach beendeter Hydrolyse wird die wässrige Phase mit Dichlormethan extrahiert und die vereinigten Ethylacetat-Extrakte werden mit verdünnter Natriumcarbonat-Lösung gewaschen und über Magnesiumsulfat getrocknet. Die Lösung wird mit 2 g Kieselgel verrührt und abgesaugt. Der Filterkuchen wird mit Aceton gewaschen und das Filtrat eingeengt. Das gelbliche, harzartige Rohprodukt wird ohne weitere Reinigung weiterumgesetzt.
Ausbeute: 385 mg (51 % der Theorie)
R_{f}-Wert: 0,40 (Kieselgel, Dichlormethan/Methanol/konz. Ammoniak = 95:5:0,1)
Massenspektrum (ESI⁺): m/z = 227/229 (CI) [M+H]⁺

### Beispiel XXVI

### 2-(4-Methyl-piperazin-1-ylmethyl)-pyrimidin-4-ol

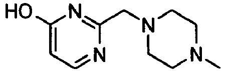

Ein Gemisch aus 1,74 g 2-Chlormethyl-pyrimidin-4-ol und 1,60 ml N-Methylpiperazin in 65 ml n-Propanol wird 4,5 h unter Rückfluss erhitzt. Anschließend wird das Reaktionsgemisch mit 12 ml 1 N wässriger Natronlauge versetzt und eingeengt. Der Kolbenrückstand wird mit Toluol nachgedampft und erneut zur Trockene eingeengt. Der feste Eindampfrückstand wird mit 150 ml Aceton bei Raumtemperatur verrührt, abgesaugt und mit 100 ml Aceton gewaschen. Das Filtrat wird eingeengt und über Nacht im Exsikkator getrocknet.
Ausbeute: 1,54 g (62 % der Theorie)
R_{f}-Wert: 0,20 (Kieselgel, Dichlormethan/Methanol/konz. Ammoniak = 95:5:0,1)
Massenspektrum (ESI⁺): m/z = 209 [M+H]⁺

### Beispiel XXVII

### 2-Chlormethyl-pyrimidin-4-ol

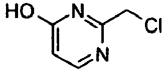

30.50 g Natrium-2-ethoxycarbonyl-ethenolat und 18.06 g Chloracetamidinhydrochlorid werden mit 300 ml Wasser versetzt, und das Reaktionsgemisch wird drei Tage bei Raumtemperatur stehen gelassen. Anschließend wird der entstandene dunkle Niederschlag abgesaugt, das Filtrat mit 3 N wäsriger Salzsäure auf pH 5-6 eingestellt und teilweise eingeengt. Der Rückstand wird mit insgesamt 500 ml Methylethylketon extrahiert. Die vereinigten Extrakte werden mit gesättigter wässriger Natriumchlorid-Lösung gewaschen, über Magnesiumsulfat getrocknet und eingeengt. Das Rohprodukt wird mit 30 ml Methanol kurz zum Sieden erhitzt, im Eisbad abgekühlt, abfiltriert, mit wenig kaltem Methanol gewaschen und getrocknet.
Ausbeute: 5,98 g (30 % der Theorie)
R_{f}-Wert: 0,25 (Kieselgel, Ethylacetat)
Massenspektrum (ESI⁺): m/z = 145/147 (CI) [M+H]⁺

### Beispiel XXXVIII

### 5-tert-Butyl-2-methoxy-3-nitro-anilin

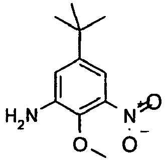

2.0 g 5-tert-Butyl-2-methoxy-1,3-dinitro-benzol werden in 20 ml Ethanol gelöst, mit 100 µl Wasser und 100 mg 10 % Palladium auf Aktivkohle versetzt und dann zum Rückfluss erhitzt. Man tropft 1,9 ml 4-Methyl-cyclohexen zu und erhitzt für weitere 2 Stunden zum Rückfluss. Anschließend werden weitere 950 µl 4-Methyl-cyclohexen zugetropft und dann 16 Stunden zum Rückfluss erhitzt. Die Lösungsmittel werden dann im Vakuum entfernt, der Rückstand in Ethylacetat aufgenommen und nacheinander mit gesättigter wässriger Natriumhydrogencarbonatlösung und gesättigter wässriger Natriumchloridlösung gewaschen. Nach Trocknen mit Natriumsulfat werden die Lösungsmittel im Vakuum entfernt und der Rückstand im Vakuum getrocknet.
Ausbeute: 1.7 g (96% der Theorie)
R_{f}-Wert: 0.20 (Kieselgel, Petrolether/Ethylacetat = 9:1)

### Beispiel XXXIX

### N-(5-tert-Butyl-3-methansulfonylamino-2-methoxy-phenyl)-4-chlor-3-(2-chlor-pyridin-4-yloxy)-benzamid

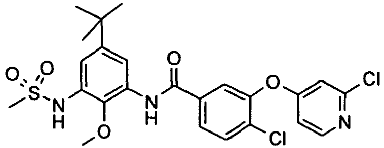

Ein Gemisch aus 500 mg N-(5-tert-Butyl-3-methansulfonylamino-2-methoxy-phenyl)-4-Chlor-3-hydroxy-benzamid, 170 mg 2-Chlor-4-fluor-pyridin, 240 mg K₂CO₃ und 3 ml Dimethylsufloxid wird auf 50°C erwärmt und über Nacht bei dieser Temperatur gerührt. Das Gemisch wird dann auf Raumtemperatur abgekühlt und mit Wasser verdünnt. Der ausgefallene Niederschlag wird abfiltriert, mit Wasser gewaschen und getrocknet.
Ausbeute: 485 mg (77% der Theorie)
R_{f}-Wert: 0,65 (Kieselgel, Dichlormethan/Methanol = 95:5)
Massenspektrum (ESI⁺): m/z = 538/540/542 (2 CI) [M+H]⁺

### Beispiel XL

### 4-{4-[5-(5-tert-Butyl-3-methansulfonylamino-2-methoxy-phenylcarbamoyl)-2-chlor-Phenoxy]-pyridin-2-ylamino}-piperidin-1-carbonsäure-tert-butyl-ester

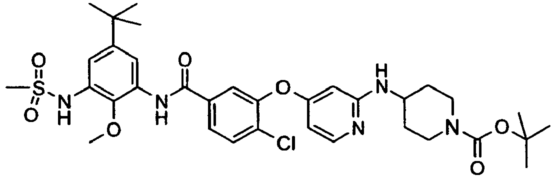

55 µl 2,8,9-Triisobutyl-2,5,8,9-tetraaza-1-phosphabicyclo[3.3.3]undekan und 36 mg Pd₂(Dibenzylidenaceton)₃ werden zu einer unter Argonatmosphäre gehaltenen Mischung aus 350 mg N-(5-tert-Butyl-3-methansulfonylamino-2-methoxy-phenyl)-4-chlor-3-(2-chlor-pyridin-4-yloxy)-benzamid, 110 mg 4-Amino-piperidine-1-carbonsäure-tert-butyl-ester und 290 mg KOBu in 6 ml Toluol gegeben. Das Gemisch wird auf 105°C erwärmt und über 40 h bei dieser Temperatur gerührt. Danach wird das Gemisch auf Raumtemperatur abgekühlt und Ethylacetat zugegeben. Das Gemisch wird dann jeweils einmal mit verdünnter Zitronensäure, Wasser und gesättigter Kochsalzlösung gewaschen. Die organische Phase wird getrocknet (Na₂SO₄), und dann wird das Lösungsmittel entfernt. Der Kolbenrückstand wird über Kieselgel mit Dichlormethan/Methanol (100:0→95:5) als Laufmittel chromatographiert.
Ausbeute: 35 mg (8% der Theorie)
R_{f}-Wert: 0,35 (Kieselgel, Dichlormethan/Methanol = 95:5)
Massenspektrum (ESI⁺): m/z = 702/704 (CI) [M+H]⁺

### Beispiel XLI

### 5-tert-Butyl-3-methansulfonyl-2-methoxy-anilin

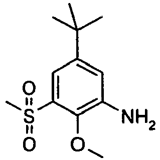

Ein Gemisch aus 14,5 g 5-tert-Butyl-1-methansulfonyl-2-methoxy-3-nitro-benzol, 3,0 g 10% Palladium auf Kohle und 250 ml Methanol wird 6 h bei Raumtemperatur unter Wasserstoff-Atmosphäre (50 psi) geschüttelt. Danach wird der Katalysator abfiltriert und das Filtrat bis zur Trockene eingeengt.
Ausbeute: 1,7 g (96% der Theorie)
R_{f}-Wert: 0,3 (Kieselgel, Petrolether/Ethylacetat = 4:1)

Analog Beispiel XLI werden folgende Verbindungen erhalten:
(1) 5-tert-Butyl-3-methansulfonylmethyl-2-methoxy-anilin 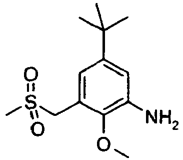 HPLC (Methode 1): Retentionszeit = 2,81 min
   Massenspektrum (ESI⁺): m/z = 272 [M+H]⁺
(2) 5-tert-Butyl-3-methansulfinylmethyl-2-methoxy-anilin 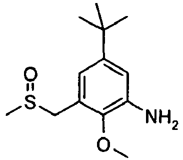 HPLC (Methode 1): Retentionszeit = 2,39 min
   Massenspektrum (ESI⁺): m/z = 256 [M+H]⁺
(3) 1,3-Diamino-5-tert-butyl-2-isopropoxy-benzol 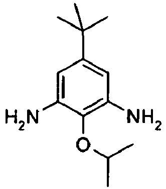 (5-tert-Butyl-2-isopropoxy-1,3-dinitro-benzol wird als Ausgangsverbindung eingesetzt)
   Massenspektrum (ESI⁺): m/z = 223 [M+H]⁺
(4) 1,3-Diamino-5-tert-butyl-2-ethoxy-benzol 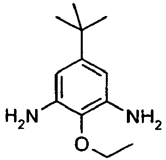 (5-tert-Butyl-2-ethoxy-1,3-dinitro-benzol wird als Ausgangsverbindung eingesetzt)
   HPLC (Methode 1): Retentionszeit = 1,94 min
   Massenspektrum (ESI⁺): m/z = 209 [M+H]⁺
(5) 5-tert-Butyl-3-methansulfinyl-2-methoxy-anilin 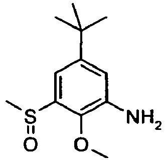 (Die Umsetzung wird mit Palladiumhydroxid in Ethylacetat durchgeführt)
   R_{f}-Wert: 0,3 (Kieselgel, Petrolether/Ethylacetat = 1:1)
(6) 1,3-Diamino-2-methoxy-5-trifluoromethyl-benzol 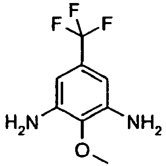 (2-Methoxy-1,3-dinitro-5-trifluormethyl-benzol wird als Ausgangsverbindung und Raney-Nickel als Katalysator eingesetzt)
   HPLC (Methode 1): Retentionszeit = 1,94 min
   Massenspektrum (ESI⁺): m/z = 209 [M+H]⁺
(7) Propan-2-sulfon-säure-(3-amino-5-tert-butyl-2-methoxy-phenyl)-amid 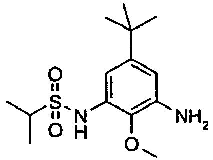 (Propan-2-sulfonsäure-(5-tert-butyl-2-methoxy-3-nitro-phenyl)-amid wird als Ausgangsverbindung eingesetzt)
   HPLC (Methode 1): Retentionszeit = 3,42 min
(8) Cyclopropansulfonsäure-(3-amino-5-tert-butyl-2-methoxy-phenyl)-amid 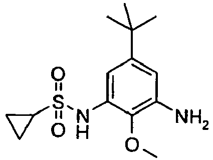 (Cyclopropansulfonsäure-(5-tert-butyl-2-methoxy-3-nitro-phenyl)-amid wird als Ausgangsverbindung eingesetzt)
   HPLC (Methode 1): Retentionszeit = 3,24 min
   Massenspektrum (ESI⁺): m/z = 299 [M+H]⁺
(9) 1,3-Diamino-2-methoxy-5-pentafluorethyl-benzol 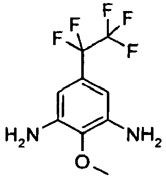 (2-Methoxy-1,3-dinitro-5-pentafluorethyl-benzol wird als Ausgangsverbindung und Raney-Nickel als Katalysator eingesetzt)
   HPLC (Methode 1): Retentionszeit = 3,19 min
   Massenspektrum (ESI⁺): m/z = 257 [M+H]⁺
(10) (3-Amino-5-tert-butyl-2-methoxy-phenyl)-methanol 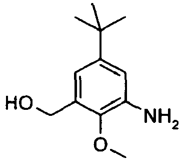 [Die Umsetzung erfolgt in Ethylacetat und Methanol (5:1)]
   HPLC (Methode 1): Retentionszeit = 2,08 min
   Massenspektrum (ESI⁺): m/z = 210 [M+H]⁺
(11) 5-tert-Butyl-3-dimethylaminomethyl-2-methoxy-anilin 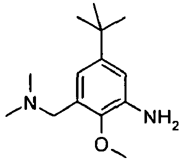 R_{f}-Wert: 0,28 (Kieselgel, Dichlormethan/Methanol/konz. Ammoniak = 90:10:0,1)
   Massenspektrum (ESI⁺): m/z = 237 [M+H]⁺
(12) 3-Amino-5-tert-butyl-2-methoxy-benzesäuremethylester 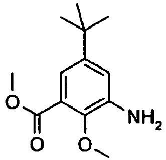 HPLC (Methode 1): Retentionszeit = 2,08 min
   Massenspektrum (ESI⁺): m/z = 238 [M+H]⁺
(13) (3-Amino-5-tert-butyl-2-methoxy-benzyl)-cyclopropyl-carbaminsäure-tert-butyl-ester 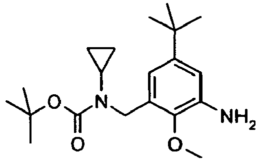 Massenspektrum (ESI⁺): m/z = 349 [M+H]⁺

### Beispiel XLII

### 5-tert-Butyl-1-methansulfonyl-2-methoxy-3-nitro-benzol

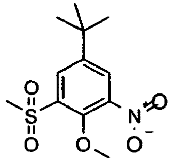

850 mg 77%ige m-Chlorperoxybenzoesäure werden zu einer eisgekühlten Lösung von 500 mg 5-tert-Butyl-2-methoxy-1-methylsulfanyl-3-nitro-benzol (kann ausgehend von 5-tert-Butyl-2-methoxy-3-nitro-anilin analog zu dem in Syn. Commun. 1984, 14, 215-8 oder in Syn. Commun. 2001, 31, 1857-62 beschriebenen Vorgehen erhalten werden) in 10 ml Dichlormethan gegeben. Die Lösung wird 6 h bei Raumtemeperatur gerührt und dann mit Dichlormethan verdünnt. Die verdünnte Reaktionslösung wird mit gesättigter wässriger Na₂CO₃-Lösung und gesättigter wässriger Kochsalzlösung gewaschen und getrocknet (Na₂SO₄). Danach wird das Lösungsmittel entfernt und der Rückstand auf Kieselgel mit Petrolether/Ethylacetat (9:1) als Laufmittel chromatographiert.
Ausbeute: 350 mg (62% der Theorie)
R_{f}-Wert: 0,3 (Kieselgel, Petrolether/Ethylacetat = 9:1)

Analog Beispiel XLII wird folgende Verbindung erhalten:
(1) 5-tert-Butyl-1-methansulfonylmethyl-2-methoxy-3-nitro-benzol 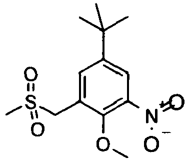 HPLC (Methode 1): Retentionszeit = 3,69 min

### Beispiel XLIII

### 5-tert-Butyl-1-methansulfinyl-2-methoxy-3-nitro-benzol

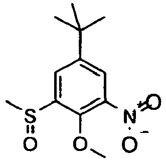

Bei Raumtemperatur werden 1,1 ml einer 35%igen wässrigen WasserstoffperoxidLösung zu einer Lösung von 500 mg 5-tert-Butyl-2-methoxy-1-methylsulfanyl-3-nitrobenzol (kann ausgehend von 5-tert-Butyl-2-methoxy-3-nitro-anilin analog zu dem in Syn. Commun. 1984, 14, 215-8 oder in Syn. Commun. 2001, 31, 1857-62 beschriebenen Vorgehen erhalten werden) in 15 ml 1,1,1,3,3,3-Hexafluorisopropanol gegeben. Die Lösung wird 5 h bei Raumtemperatur gerührt und dann mit 10%iger wässriger Na₂S₂O₃-Lösung gequencht. Das resultierende Gemisch wird mit Ethylacetat extrahiert, und die vereinigten Extrakte werden mit gesättigter wässriger NaHCO₃-Lösung und gesättigter wässriger Kochsalzlösung gewaschen. Die organische Phase wird getrocknet (Na₂SO₄) und das Lösungsmittel abdestilliert. Der Rückstand wird auf Kieselgel mit Petrolether/Ethylacetat (20:1) als Laufmittel chromatographiert.
Ausbeute: 350 mg (66% der Theorie)
R_{f}-Wert: 0,2 (Kieselgel, Petrolether/Ethylacetat = 9:1)

Analog Beispiel XLIII wird folgende Verbindung erhalten:
(1) 5-tert-Butyl-1-methansulfinylmethyl-2-methoxy-3-nitro-benzol 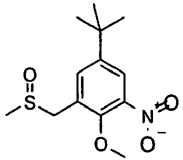 HPLC (Methode 1): Retentionszeit = 3,28 min
   Massenspektrum (ESI⁺): m/z = 286 [M+H]⁺

### Beispiel XLIV

### 3-(2-Chlor-pyridin-4-yloxy)-4-methyl-benzoesäuremethylester

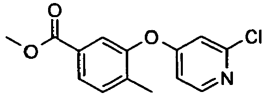

Bei Raumtemperatur werden 1,6 ml Methyliodid zu einem Gemisch aus 6,21 g 3-(2-Chlor-pyridin-4-yloxy)-4-methyl-benzoesäure, 4,88 g Kaliumcarbonat und 30 ml N,N-Dimethylformamid gegeben. Das Gemisch wird über Nacht bei Raumtemperatur gerührt. Dann wird das Gemisch in eiskaltes Wasser gegeben, und der Niederschlag wird abfiltriert und bei 45°C getrocknet.
Ausbeute: 6,26 g (96% der Theorie)
Massenspektrum (ESI⁺): m/z = 278/280 (Cl) [M+H]⁺

Analog Beispiel XLIV werden folgende Verbindungen erhalten:
(1) 5-tert-Butyl-2-methoxy-3-nitro-benzaldehyd 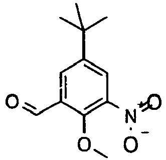 HPLC (Methode 1): Retentionszeit = 4,21 min
   Massenspektrum (ESI⁺): m/z = 238 [M+H]⁺
(2) 5-tert-Butyl-2-isopropoxy-1,3-dinitro-benzol 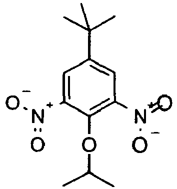 (4-tert-Butyl-2,6-dinitro-phenol und Isopropyliodid werden miteinander umgesetzt)
   HPLC (Methode 1): Retentionszeit = 4,68 min
(3) 5-tert-Butyl-2-ethoxy-1,3-dinitro-benzol 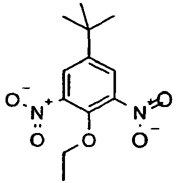 (4-tert-Butyl-2,6-dinitro-phenol und Ethyliodid werden miteinander umgesetzt)
   HPLC (Methode 1): Retentionszeit = 4,58 min

### Beispiel XLV

### 5-tert-Butyl-2-methoxy-3-methylsulfanylmethyl-anilin

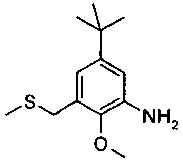

Ein Gemisch aus 1,00 g 5-tert-Butyl-2-methoxy-1-methylsulfanylmethyl-3-nitro-benzol, 4,20 g Zinndichloriddihydrat und 15 ml Ethanol wird 2 h unter Rückfluss gekocht. Danach wird das Lösungsmittel entfernt, und Wasser und Dichlormethan werden zum Rückstand gegeben. Das Gemisch wird über Celite filtriert, und der wässrige Teil des Filtrats wird abgetrennt und zweimal mit Dichlormethan extrahiert. Die vereinigten organischen Phasen werden getrocknet (Na₂SO₄), und das Lösungsmittel wird entfernt. Der Rückstand wird auf Aluminiumoxid mit Cyclohexan/Ethylacetat (4:1→1:4) als Fließmittel chromatographiert.
Ausbeute: 0,62 g (70% der Theorie)
HPLC (Methode 1): Retentionszeit = 3,85 min
Massenspektrum (ESI⁺): m/z = 240 [M+H]⁺

### Beispiel XLVI

### 5-tert-Butyl-2-methoxy-1-methylsulfanylmethyl-3-nitro-benzol

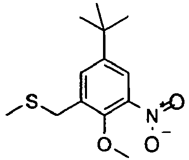

Bei Raumtemperatur werden 1,50 g Natriumthiomethylat zu einer Lösung von 4,64 g Methansulfonsäure-5-tert-butyl-2-methoxy-3-nitro-benzylester in 50 ml 1,4-Dioxan gegeben. Die Lösung wird über Nacht bei 40°C gerührt. Danach wird das Lösungsmittel entfernt und der Rückstand auf Kieselgel mit Cyclohexan/Ethylacetat (98:2→60:40) als Fließmittel chromatographiert.
Ausbeute: 2,91 g (87% der Theorie)
HPLC (Methode 1): Retentionszeit = 4,74 min

### Beispiel XLVII

### Methansulfonsäure-5-tert-butyl-2-methoxy-3-nitro-benzylester

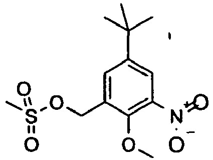

1,20 ml Methansulfonsäurechlorid werden zu einer eisgekühlten Lösung von 3,43 g (5-tert-Butyl-2-methoxy-3-nitro-phenyl)-methanol und 2,40 ml Triethylamin in 30 ml Dichlormethan gegeben. Die Lösung wird unter Eiskühlung 2 h gerührt und dann mit Dichlormethan verdünnt. Die verdünnte Lösung wird drei Mal mit Wasser und einmal mit gesättigter wässriger Kochsalzlösung gewaschen, getrocknet (MgSO₄) und bis zur Trockene eingeengt.
Ausbeute: 3,97 g (87% der Theorie)
Massenspektrum (ESI⁺): m/z = 335 [M+NH₄]⁺

### Beispiel XLVIII

### (5-tert-Butyl-2-methoxy-3-nitro-phenyl)-methanol

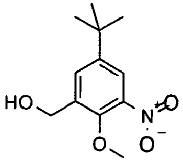

Bei ca. 10°C werden 0,65 g Natriumborhydrid zu einer Lösung von 3,75 g 5-tert-Butyl-2-methoxy-3-nitro-benzaldehyd in 15 ml Dichlormethan und 15 ml Methanol gegeben. Die Lösung wird 2 h bei Raumtemperatur gerührt und dann eingeengt. Der Rückstand wird mit wässriger Essigsäure versetzt und mit Ethylacetat extrahiert. Die vereinigten Extrakte werden mit Wasser und mit gesättigter wässriger Kochsalzlösung gewaschen, getrocknet (MgSO₄) und bis zur Trockene eingeengt.
Ausbeute: 3,63 g (96% der Theorie)
HPLC (Methode 1): Retentionszeit = 3,64 min
Massenspektrum (ESI⁻): m/z = 238 [M-H]⁻

### Beispiel IL

### 5-tert-Butyl-2-hydroxy-3-nitro-benzaldehyd

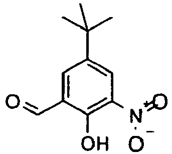

Bei -30°C werden 4,84 g Nitroniumtetrafluorborat zu einer Lösung von 5,00 g 5-tert-Butyl-2-hydroxy-benzaldehyd in 200 ml Acetonitril gegeben. Die Lösung wird innerhalb 1 h auf -15°C erwärmt und dann mit Ethylacetat und gesättigter wässriger NaHCO₃-Lösung versetzt. Das resultierende Gemisch wird mit Ethylacetat extrahiert, und die vereinigten Extrakte werden mit gesättigter wässriger Kochsalzlösung gewaschen, getrocknet (MgSO₄) und eingeengt. Der Rückstand wird in 2 ml Wasser und 40 ml konzentrierter Essigsäure aufgenommen, und das resultierende Gemisch wird 2 h unter Rückfluss gekocht. Die erkaltete Lösung wird in eiskaltes Wasser gegossen, und der sich bildende Niederschlag wird abfiltriert und in Ethylacetat gelöst. Die organische Lösung wird getrocknet (MgSO₄) und eingeengt. Das zurückbleibende Öl verfestigt sich rasch.
Ausbeute: 5,89 g (94% der Theorie)
HPLC (Methode 1): Retentionszeit = 4,04 min
Massenspektrum (ESI⁻): m/z = 222 [M-H]⁻

Analog Beispiel IL werden folgende Verbindungen erhalten:
(1) 4-tert-Butyl-2,6-dinitro-phenol 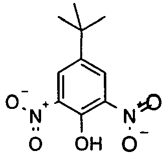 (4-tert-Butyl-phenol wird mit 2,7 Äquivalenten Nitroniumtetrafluorborat umgesetzt)
   HPLC (Methode 1): Retentionszeit = 4,09 min
   Massenspektrum (ESI⁻): m/z = 239 [M-H]⁻
(2) 2-Methoxy-1,3-dinitro-5-trifluormethyl-benzol 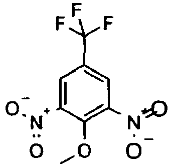 (1-Methoxy-2-nitro-4-trifluormethyl-benzol wird als Ausgangsverbindung eingesetzt)
   HPLC (Methode 1): Retentionszeit = 3,93 min
(3) 2-Methoxy-1,3-dinitro-5-pentafluorethyl-benzol 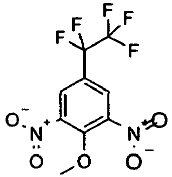 (1-Methoxy-4-pentafluoroethyl-benzol wird mit 2,7 Äquivalenten Nitroniumtetrafluorborat umgesetzt)
   HPLC (Methode 1): Retentionszeit = 4,23 min

### Beispiel L

### 4-(4-{5-[5-tert-Butyl-3-(cyclopropancarbonyl-amino)-2-methoxy-phenylcarbamoyl]-2-methyl-phenoxy}-pyridin-2-ylmethyl)-piperidin-1-carbonsäure-tert-butyl-ester

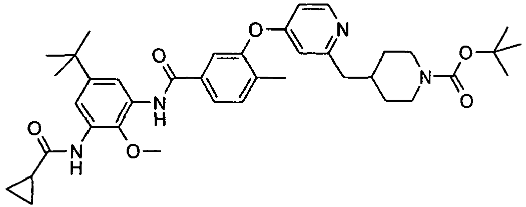

Bei Raumtemperatur werden 36 µl Cyclopropancarbonsäurechlorid zu einer Lösung von 160 mg 4-{4-[5-(3-Amino-5-tert-butyl-2-methoxy-phenylcarbamoyl)-2-methyl-phenoxy]-pyridin-2-ylmethyl}-piperidin-1-carbonsäure-tert-butyl-ester und 70 µl Triethylamin in 2 ml Dichlormethan gegeben. Die Lösung wird über Nacht bei Raumtemperatur gerührt und dann mit Wasser versetzt. Das Gemisch wird mit Dichlormethan extrahiert, und die vereinigten Extrakte werden mit Wasser und gesättigter wässriger Kochsalzlösung gewaschen, getrocknet (MgSO₄) und eingeengt. Der Rückstand wird auf Kieselgel mit Cyclohexan/Ethylacetat (50:50→0:100) als Fließmittel chromatographiert.
Ausbeute: 5,89 g (94% der Theorie)
HPLC (Methode 1): Retentionszeit = 3,56 min
Massenspektrum (ESI⁺): m/z = 671 [M+H]⁺

Analog Beispiel L werden folgende Verbindungen erhalten:
(1) 4-{4-[5-(5-tert-Butyl-2-methoxy-3-pentanoylamino-phenylcarbamoyl)-2-methyl-phenoxy]-pyridin-2-ylmethyl}-piperidin-1-carbonsäure-tert-butyl-ester 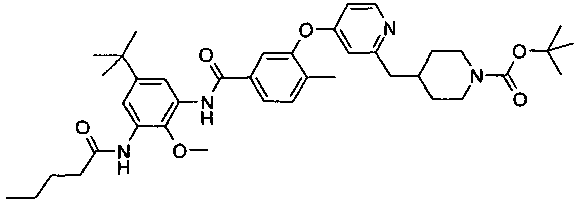 (Valeriansäurechlorid wird als Acylierungsreagenz eingesetzt)
   HPLC (Methode 1): Retentionszeit = 3,80 min
   Massenspektrum (ESI⁺): m/z = 687 [M+H]⁺
(2) 4-(4-{5-[5-tert-Butyl-2-methoxy-3-(3-methyl-butyrylamino)-phenylcarbamoyl]-2-methyl-phenoxy}-pyridin-2-ylmethyl)-piperidin-1-carbonsäure-tert-butyl-ester 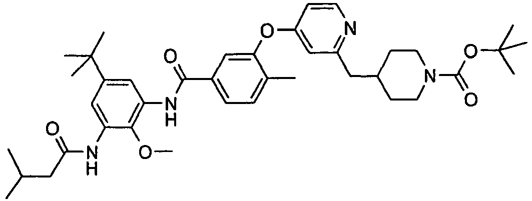 (Isovaleriansäurechlorid wird als Acylierungsreagenz eingesetzt)
   HPLC (Methode 1): Retentionszeit = 3,77 min
   Massenspektrum (ESI⁺): m/z = 687 [M+H]⁺
(3) 4-{4-[5-(5-tert-Butyl-3-isobutyrylamino-2-methoxy-phenylcarbamoyl)-2-methyl-phenoxy]-pyridin-2-ylmethyl}-piperidin-1-carbonsäure-tert-butyl-ester 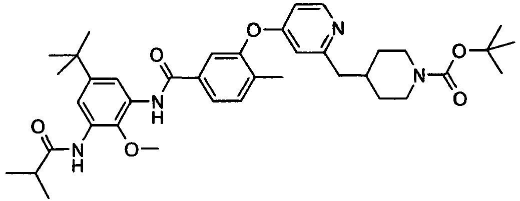 (Isobuttersäurechlorid wird als Acylierungsreagenz eingesetzt)
   HPLC (Methode 1): Retentionszeit = 3,62 min
   Massenspektrum (ESI⁺): m/z = 673 [M+H]⁺

### Beispiel LI

### N-(3-Amino-5-tert-butyl-2-isopropoxy-phenyl)-methansulfonamid

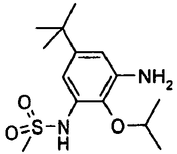

Bei Raumtemperatur werden 0,20 ml Methansulfonsäurechlorid tropfenweise zu einer Lösung von 0,48 g 1,3-Diamino-5-tert-butyl-2-isopropoxy-benzol und 0,35 ml Pyridin in 5 ml Dichlormethan gegeben. Die Lösung wird 2 h bei Raumtemperatur gerührt und dann eingeengt. Der Rückstand wird auf Kieselgel mit Cyclohexan/Ethylacetat (90:10→40:60) als Fließmittel chromatographiert.
Ausbeute: 0,35 g (54% der Theorie)
HPLC (Methode 1): Retentionszeit = 3,42 min
Massenspektrum (ESI⁺): m/z = 301 [M+H]⁺

Analog Beispiel LI werden folgende Verbindungen erhalten:
(1) N-(3-Amino-5-tert-butyl-2-ethoxy-phenyl)-methansulfonamid 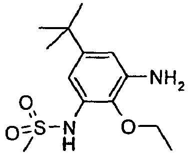 HPLC (Methode 1): Retentionszeit = 3,16 min
   Massenspektrum (ESI⁺): m/z = 287 [M+H]⁺
(2) 4-(4-{5-[5-tert-Butyl-2-methoxy-3-(2-methyl-propan-1-sulfonylamino)-phenylcarbamoyl]-2-methyl-phenoxy}-pyridin-2-ytmethyl)-piperidine-1-carbonsäure-tert-butyl-ester 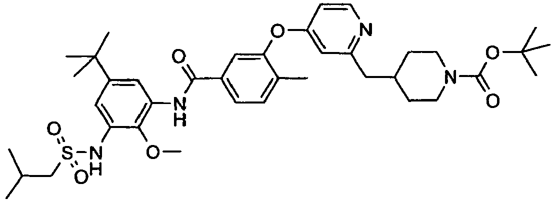 (Isobutansulfonsäurechlorid wird als Sulfonylierungsreagenz eingesetzt)
   HPLC (Methode 1): Retentionszeit = 3,86 min
   Massenspektrum (ESI⁺): m/z = 723 [M+H]⁺
(3) 4-(4-{5-[3-(Butan-1-sulfonylamino)-5-tert-butyl-2-methoxy-phenylcarbamoyl]-2-methyl-phenoxy}-pyridin-2-ylmethyl)-piperidin-1-carbonsäure-tert-butyl-ester 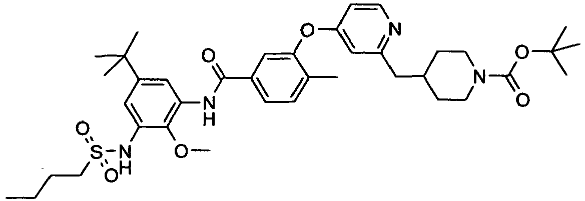 (n-Butansulfonsäurechlorid wird als Sulfonylierungsreagenz eingesetzt)
   HPLC (Methode 1): Retentionszeit = 3,85 min
   Massenspektrum (ESI⁺): m/z = 723 [M+H]⁺
(4) N-(3-Amino-2-methoxy-5-trifluormethyl-phenyl)-methansulfonamid 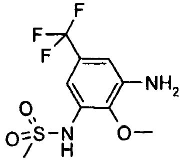 HPLC (Methode 1): Retentionszeit = 2,93 min
   Massenspektrum (ESI⁺): m/z = 285 [M+H]⁺
(5) Propan-2-sulfonsäure-(5-tert-butyl-2-methoxy-3-nftro-phenyl)-amid 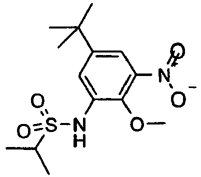 (Isopropylsulfonsäurechlorid wird als Sulfonylierungsreagenz eingesetzt)
   HPLC (Methode 1): Retentionszeit = 4,18 min
   Massenspektrum (ESI⁺): m/z = 331 [M+H]⁺
(6) Cyclopropansulfonsäure-(5-tert-butyl-2-methoxy-3-nitro-phenyl)-amid 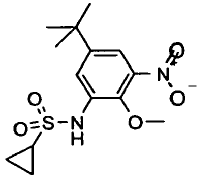 (Cyclopropylsulfonsäurechlorid wird als Sulfonylierungsreagenz eingesetzt)
   HPLC (Methode 1): Retentionszeit = 4,09 min
   Massenspektrum (ESI⁺): m/z = 329 [M+H]⁺
(7) N-(3-Amino-2-methoxy-5-pentafluorethyl-phenyl)-methansulfonamid 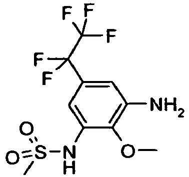 Massenspektrum (ESI⁺): m/z = 335 [M+H]⁺

### Beispiel LII

### 4-{4-[5-(5-tert-Butyl-3-methansulfonyl-2-methoxy-phenylcarbamoyl)-2-methyl-phenoxyl-pyridin-2-ylmethyl}-piperidin-1-carbonsäure-tert-butyl-ester

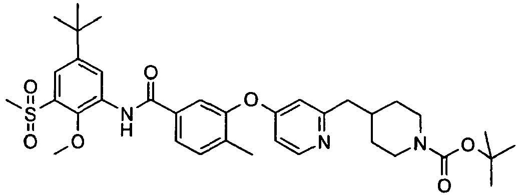

Bei Raumtemperatur werden nacheinander 10 µl N,N-Dimethylformamid und 100 µl Oxalylchlorid zu einer Lösung von 140 mg 4-[4-(5-Carboxy-2-methyl-phenoxy)-pyridin-2-ylmethyl]-piperidin-1-carbonsäure-tert-butyl-ester in 2 ml Acetonitril getropft. Die Lösung wird 2 h bei Raumtemperatur gerührt und dann bis zur Trockene eingeengt. Der Rückstand wird in 2 ml 1,2-Dichlorethan aufgenommen und bei Raumtemperatur mit 85 mg 5-tert-Butyl-3-methansulfonyl-2-methoxy-anilin und 180 µl Triethylamin versetzt. Die Lösung wird über Nacht bei Raumtemperatur gerührt und danach mit Wasser und gesättigter wässriger Kochsalzlösung gewaschen und getrocknet (Na₂SO₄). Das Lösungsmittel wird abgedampft, und der Rückstand wird auf Kieselgel mit Cyclohexan/Ethylacetat (50:40→0:100) als Fließmittel chromatographiert.
Ausbeute: 0,35 g (54% der Theorie)
Massenspektrum (ESI⁺): m/z = 666 [M+H]⁺

### Beispiel LIII

### 4-{4-[5-(5-tert-Butyl-3-methansulfinyl-2-methoxy-phenylcarbamoyl)-2-methyl-phenoxy]-pyridin-2-ylmethyl}-piperidin-1-carbonsäure-tert-butyl ester

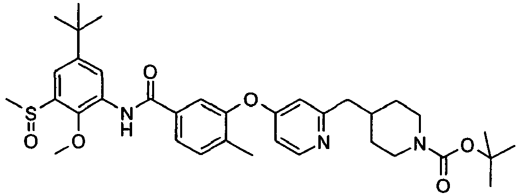

Bei Raumtemperatur werden 560 µl einer 50%igen Lösung von Propanphosphorsäurecycloanhydrid in Ethylacetat zu einer Lösung von 120 mg 4-[4-(5-Carboxy-2-methyl-phenoxy)-pyridin-2-ylmethyl]-piperidin-1-carbonsäure-tert-butyl-ester, 68 mg 5-tert-Butyl-3-methansulfinyl-2-methoxy-anilin und 145 µl Triethylamin in 4 ml Tetrahydrofuran getropft. Die Lösung wird auf 80°C erwärmt und bei dieser Temperatur über Nacht gerührt. Danach wird die Lösung bis zur Trockene eingeengt und der Rückstand mit gesättigter wässriger NaHCO₃-Lösung und Ethylacetat versetzt. Die organische Phase wird abgetrennt und mit Wasser und gesättigter wässriger Kochsalzlösung gewaschen und getrocknet (Na₂SO₄). Das Lösungsmittel wird abgedampft, und der Rückstand wird auf Kieselgel mit Dichlormethan/10% methanolischer Ammoniaklösung (99:1→90:10) als Fließmittel chromatographiert.
Ausbeute: 105 mg (57% der Theorie)
HPLC (Methode 1): Retentionszeit = 3,21 min
Massenspektrum (ESI⁺): m/z = 650 [M+H]⁺

Analog Beispiel LIII werden folgende Verbindungen erhalten:
(1) 4-{4-[5-(3-Methansulfonylamino-2-methoxy-5-trifluormethyl-phenylcarbamoyl)-2-methyl-phenoxy]-pyridin-2-ylmethyl}-piperidin-1-carbonsäure-tert-butyl-ester 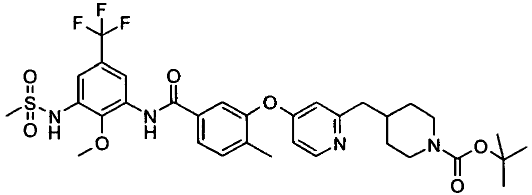 HPLC (Methode 1): Retentionszeit = 3,35 min
   Massenspektrum (ESI⁺): m/z = 693 [M+H]⁺
(2) 4-(4-{5-[5-tert-Butyl-2-methoxy-3-(propan-2-sulfonylamino)-phenylcarbamoyl]-2-methyl-phenoxy}-pyridin-2-ylmethyl)-piperidin-1-carbonsäure-tert-butyl-ester 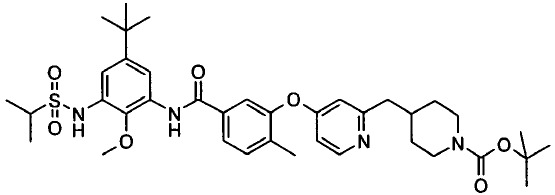 HPLC (Methode 1): Retentionszeit = 3,67 min
   Massenspektrum (ESI⁺): m/z = 709 [M+H]⁺
(3) 4-{4-[5-(5-tert-Butyl-3-cyclopropansulfonylamino-2-methoxy-phenylcarbamoyl)-2-methyl-phenoxy]-pyridin-2-ylmethyl}-piperidin-1-carbonsäure-tert-butyl-ester 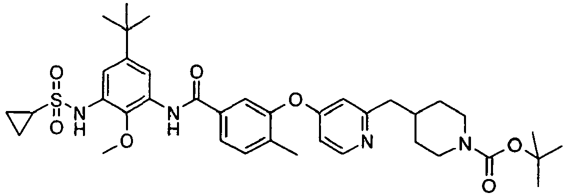 HPLC (Methode 1): Retentionszeit = 3,63 min
   Massenspektrum (ESI⁺): m/z = 707 [M+H]⁺
(4) 4-{4-[5-(3-Methansulfonylamino-2-methoxy-5-pentafluorethyl-phenylcarbamoyl)-2-methyl-phenoxy]-pyridin-2-ylmethyl}-piperidin-1-carbonsäure-tert-butyl-ester 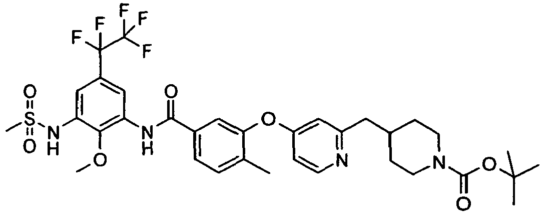 HPLC (Methode 1): Retentionszeit = 3,55 min
   Massenspektrum (EI): m/z = 316 [M*]⁺
(5) 4-{4-[5-(5-tert-Butyl-3-dimethylaminomethyl-2-methoxy-phenylcarbamoyl)-2-methyl-phenoxy]-pyridin-2-ylmethyl}-piperidin-1-carbonsäure-tert-butyl-ester 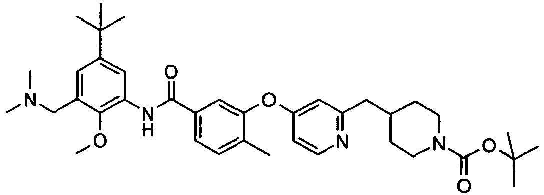 HPLC (Methode 1): Retentionszeit = 2,71 min
   Massenspektrum (ESI⁺): m/z = 645 [M+H]⁺
(6) 4-{4-[5-(5-tert-Butyl-2-methoxy-3-trimethylsilyloxymethyl-phenylcarbamoyl)-2-methyl-phenoxy]-pyridin-2-ylmethyl}-piperidin-1-carbonsäure-tert-butyl-ester 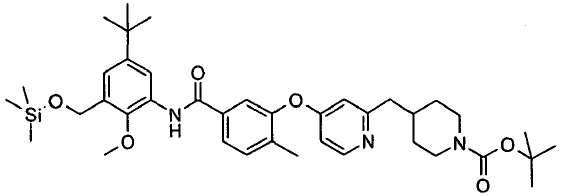 HPLC (Methode 1): Retentionszeit = 3,33 min
(7) 5-tert-Butyl-3-[3-(2-chlor-pyridin-4-yloxy)-4-methyl-benzoylamino]-2-methoxybenzoesäuremethylester 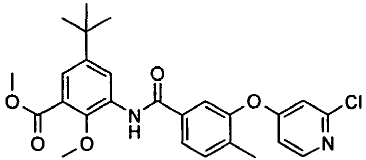 HPLC (Methode 3): Retentionszeit = 4,21 min
   Massenspektrum (ESI⁺): m/z = 483/485 (CI) [M+H]⁺
(8) 4-[4-(5-{3-[(tert-Butoxycarbonyl-cyclopropyl-amino)-methyl]-5-tert-butyl-2-methoxy-phenylcarbamoyl}-2-methyl-phenoxy)-pyridin-2-ylmethyl]-piperidin-1-carbonsäure-tert-butyl-ester

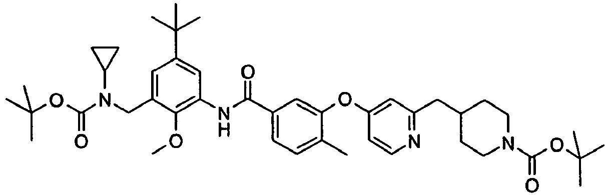

### Beispiel LIV

### 1-Methoxy-4-pentafluorethyl-benzol

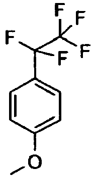

Eine Mischung aus 2,00 g 4-lod-anisol, 2,00 g Pentafluorethyl-trimethylsilan, 0,60 g Kaliumfluorid, 2,43 g Kupferiodid und 15 ml N,N-Dimethylformamid wird über Nacht bei 80°C gerührt. Danach wird das Gemisch noch weitere 36 h bei 80°C gerührt, wobei nach 4 h, 12 h und 24 h jeweils noch einmal 2,00 g Pentafluorethyl-trimethylsilan zugegeben werden. Nach Abkühlen auf Raumtemperatur wird 2 M wässrige Ammoniak-Lösung zugegeben und das Gemisch filtriert. Das Filtrat wird mit Ethylacetat extrahiert, und die vereinigten organischen Extrakte werden getrocknet (Na₂SO₄) und eingedampft. Der Rückstand wird über Kieselgel mit Petrolether/Ethylacetat (9:1) als Fließmittel chromatographiert.
Ausbeute: 1,14 g (59% der Theorie)
HPLC (Methode 1): Retentionszeit = 4,40 min
Massenspektrum (EI): m/z = 226 [M*]⁺

### Beispiel LV

### 4-{4-[5-(5-tert-Butyl-3-formyl-2-methoxy-phenylcarbamoyl)-2-methyl-phenoxy]-pyridin-2-ylmethyl}-piperidin-1-carbonsäure-tert-butyl-ester

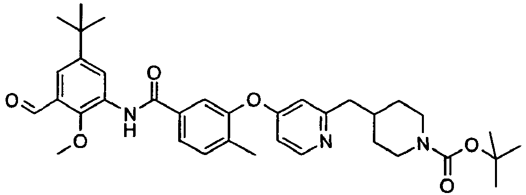

120 mg 1,1,1-Triacetoxy-1,1-dihydro-1,2-benziodoxol-3(1H)-on ("Dess-Martin-Periodinan") werden zu einer eisgekühlten Lösung von 200 mg 4-{4-[5-(5-tert-Butyl-3-hydroxymethyl-2-methoxy-phenylcarbamoyl)-2-methyl-phenoxy]-pyridin-2-ylmethyl}-piperidin-1-carbonsäure-tert-butyl-ester in 6 ml Dichlormethan gegeben. Das Kühlbad wird entfernt, und die Lösung wird über Nacht bei Raumtemperatur gerührt. Dann wird die Lösung mit Dichlormethan verdünnt und mit wässriger K₂CO₃-Lösung und wässriger Na₂S₂O₃-Lösung gewaschen. Die organische Phase wird getrocknet (Na₂SO₄) und das Lösungsmittel entfernt. Der Rückstand wird über Kieselgel mit Dichlormethan/Methanol (99:1→90:10) als Fließmittel chromatographiert.
Ausbeute: 55 mg (46% der Theorie)
HPLC (Methode 3): Retentionszeit = 4,34 min
Massenspektrum (ESI⁺): m/z = 616 [M+H]⁺

### Beispiel LVI

### 5-tert-Butyl-2-methoxy-3-trimethylsilyloxymethyl-phenylamin

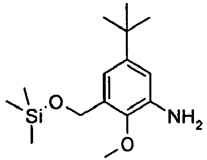

Bei Raumtemperatur werden 0,45 ml Trimethylsilylchlorid zu einer Lösung von 0,50 g (3-Amino-5-tert-butyl-2-methoxy-phenyl)-methanol und 0,39 g Imidazol in 6 ml Tetrahydrofuran getropft. Die Lösung wird 2 h bei Raumtemperatur gerührt und dann bis zur Trockene eingeengt. Der Rückstand wird in Ethylacetat aufgenommen und mit wässriger 10% K₂CO₃-Lösung und gesättigter wässriger Kochsalzlösung gewaschen. Danach wird die organische Phase getrocknet (Na₂SO₄) und eingeengt. Der Rückstand wird über Kieselgel mit Cyclohexan/Ethylacetat (95:5→50:50) als Fließmittel chromatographiert.
Ausbeute: 0,51 g (76% der Theorie)
R_{f}-Wert: 0,5 (Kieselgel, Cyclohexan/Ethylacetat = 3:2)

### Beispiel LVII

### 5-tert-Butyl-2-methoxy-3-[4-methyl-3-(2-piperazin-1-ylmethyl-pyridin-4-yloxy)-benzoylamino]-benzoesäuremethylester

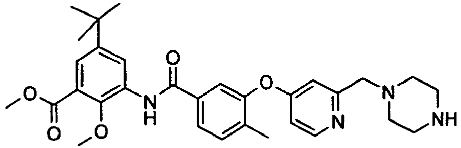

Bei Raumtemperatur werden 1,4 mL einer 5-6 M isopropanolischen Salzsäure-Lösung zu einer Lösung von 270 mg 4-{4-[5-(5-tert-Butyl-2-methoxy-3-methoxycarbonyl-phenylcarbamoyl)-2-methyl-phenoxy]-pyridin-2-ylmethyl}-piperazin-1-carbonsäure-tert-butyl-ester in 6 ml Dichlormethan gegeben. Die Lösung wird über Nacht bei Raumtemperatur gerührt und dann mit Dichlormethan verdünnt. Die Lösung wird mit gesättigter wässriger NaHCO₃-Lösung gewaschen und die wässrige Phase einmal mit Dichlormethan extrahiert. Die vereinigten organischen Phasen werden getrocknet (Na₂SO₄), und das Lösungsmittel wird vollständig entfernt.
Ausbeute: 215 mg (94 % der Theorie)
HPLC (Methode 1): Retentionszeit = 2,61 min
Massenspektrum (ESI⁺): m/z = 547 [M+H]⁺

Analog Beispiel LVII wird folgende Verbindung erhalten:
(1) 5-tert-Butyl-3-[3-(2-[1,4]diazepan-1-ylmethyl-pyridin-4-yloxy)-4-methyl-benzoylamino]-2-methoxy-benzoesäuremethylester 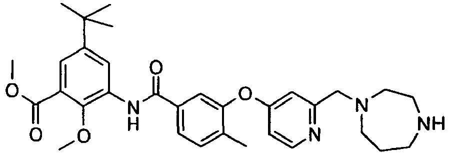 HPLC (Methode 1): Retentionszeit = 2,73 min
   Massenspektrum (ESI⁺): m/z = 561 [M+H]⁺

### Beispiel LVIII

### 4-{4-[5-(5-tert-Butyl-2-methoxy-3-methoxycarbonyl-phenylcarbamoyl)-2-methyl-phenoxy]-pyridin-2-ylmethyl}-piperazin-1-carbonsäure-tert-butyl-ester

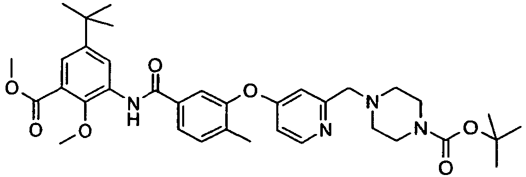

Eine Mischung aus 414 mg 5-tert-Butyl-3-[3-(2-chlor-pyridin-4-yloxy)-4-methyl-benzoylamino]-2-methoxy-benzoesäuremethylester, 370 mg Kalium-(4-tert-butyloxycarbonyl-piperazin-1-ylmethyl)-trifluorborat, 0,84 g Cs₂CO₃, 0,80 ml Wasser und 8 ml Tetrahydrofuran wird 10 min mit Argon gespült. Danach werden 200 mg 2-Dicyclohexylphosphinyl-2',4',6'-triisopropyl-1,1'-biphenyl (XPhos) und 20 mg Palladium(II)acetat zugegeben, und das Gemisch wird auf 80°C erwärmt. Das Reaktionsgemisch wird in Argonatmosphäre über Nacht bei 80°C gerührt. Nach dem Abkühlen auf Raumtemperatur wird das Gemisch eingeengt und der Rückstand in Ethylacetat und Wasser aufgenommen. Die organische Phase wird abgetrennt, mit Wasser und wässriger gesättigter Kochsalzlösung gewaschen und getrocknet (MgSO₄). Das Lösungsmittel wird entfernt, und der Rückstand wird über Kieselgel mit Dichlormethan/Methanol (99:1→90:10) und anschließend auf reversed phase (HPLC, Xbridge C18) mit Methanol/Wasser/Ammoniak chromatographiert.
Ausbeute: 270 mg (49 % der Theorie)
HPLC (Methode 1): Retentionszeit = 3,54 min
Massenspektrum (ESI⁺): m/z = 647 [M+H]⁺

Analog Beispiel LVIII wird folgende Verbindung erhalten:
(1) 4-{4-[5-(5-tert-Butyl-2-methoxy-3-methoxycarbonyl-phenylcarbamoyl)-2-methyl-phenoxy]-pyridin-2-ylmethyl}-[1,4]diazepan-1-carbonsäure-tert-butyl-ester 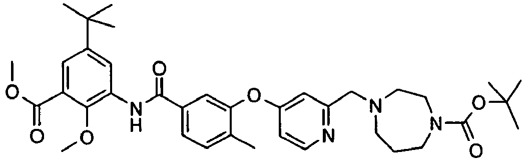 HPLC (Methode 3): Retentionszeit = 4,39 min
   Massenspektrum (ESI⁺): m/z = 661 [M+H]⁺

### Beispiel LIX

### Kalium-(4-tert-butyloxycarbonyl-piperazin-1-ylmethyl)-trifluorborat

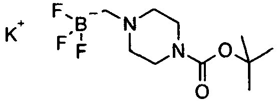

5,00 g Kalium-brommethyl-trifluorborat werden zu einer Lösung von 4,87 g Piperazin-1-carbonsäure-tert-butyl-ester in 25 ml Tetrahydrofuran gegeben. Das Gemisch wird 3 h bei 80°C gerührt und dann auf Raumtemperatur abgekühlt. Das Lösungsmittel wird entfernt, und der Rückstand wird in 50 ml Aceton suspendiert und mit 3,44 g Kaliumcarbonat versetzt. Das Gemisch wird über Nacht bei Raumtemperatur gerührt. Dann wird das Gemisch filtriert und das Filtrat eingeengt. Das zurückbleibende Öl wird mit Diisopropylether versetzt und der nach einer Weile entstehende farblose Niederschlag abgetrennt und getrocknet.
Ausbeute: 4,20 g (55% der Theorie)
Massenspektrum (ESI⁻): m/z = 267 [M]⁻[4-tert-butyloxycarbonyl-piperazin-1-ylmethyl)-trifluorborat]

Analog Beispiel LIX wird folgende Verbindung erhalten:
(1) Kalium-(4-tert-butyloxycarbonyl-homopiperazin-1-ylmethyl)-trifluorborat 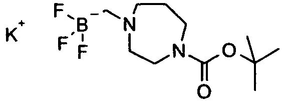 Massenspektrum (ESI⁻): m/z = 281 [M]⁻ [4-tert-butyloxycarbonyl-homopiperazin-1-ylmethyl)-trifluorborat]

### Beispiel LX

### 5-tert-Butyl-2-methoxy-3-nitro-benzoesäure

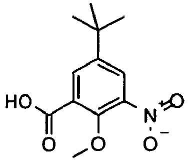

Eine Mischung aus 2,3 ml 65% Salpetersäure und 2,6 ml 96% Schwefelsäure wird zu einer eisgekühlten Lösung von 5,00 g 5-tert-Butyl-2-methoxy-benzoesäure in 15 ml 96% Schwefelsäure getropft. Die Lösung wird 1,5 h im Kühlbad und dann 1 h bei Raumtemperatur gerührt. Danach wird die Lösung in Eiswasser gegeben, und der sich bildende Niederschlag wird abfiltriert und in Dichlormethan aufgenommen. Die Dichlormethanphase wird getrocknet (Na₂SO₄), und das Lösungsmittel wird vollständig entfernt.
Ausbeute: 5,40 g (89% der Theorie)

### Beispiel LXI

### 5-tert-Butyl-2-methoxy-3-nitro-benzoesäuremethylester

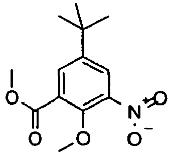

2,3 ml Thionylchlorid werden zu einer eisgekühlten Lösung von 5,40 g 5-tert-Butyl-2-methoxy-3-nitro-benzoesäure in 50 ml Methanol getropft. Das Kühlbad wird entfernt und die Lösung auf 60°C erwärmt. Die Lösung wird über Nacht bei 60°C gerührt und dann auf Raumtemperatur abgekühlt. Die Lösung wird vollständig eingeengt und der Rückstand mit Wasser versetzt. Das wässrige Gemisch wird mit Ethylacetat extrahiert, und die vereinigten Extrakte werden getrocknet (MgSO₄). Das Lösungsmittel wird entfernt, und der Rückstand wird über Kieselgel mit Cyclohexan/Ethylacetat (98:2→60:40) chromatographiert.
Ausbeute: 2,69 g (47% der Theorie)
HPLC (Methode 1): Retentionszeit = 4,30 min
Massenspektrum (ESI⁺): m/z = 268 [M+H]⁺

### Beispiel LXII

### (5-tert-Butyl-2-methoxy-3-nitro-benzyl)-cyclopropyl-carbaminsäure-tert-butyl-ester

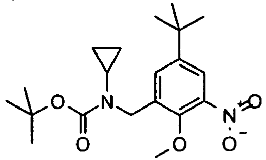

Bei Raumtemperatur werden 0,44 g (*t*BuOCO)₂O zu einer Lösung von 0,53 g (5-tert-Butyl-2-methoxy-3-nitro-benzyl)-cyclopropylamin, 0,3 ml Triethylamin und einer Spatelspitze 4-Dimethylaminopyridin in 6 ml Tetrahydrofuran gegeben. Die Lösung wird über Nacht bei Raumtemperatur gerührt und dann eingeengt. Der Rückstand wird in Ethylacetat aufgenommen und mit Wasser und gesättigter wässriger Kochsalzlösung gewaschen und dann getrocknet (MgSO₄). Das Lösungsmittel wird entfernt, und der Rückstand wird ohne weitere Reinigung zur Reduktion der Nitrogruppe eingesetzt.

### Herstellung der Endverbindungen

### Beispiel 1

### N-(5-tert-Butyl-3-methansulfonylamino-2-methoxy-phenyl)-4-methyl-3-(1-oxy-pyridin-4-yloxy)-benzamid

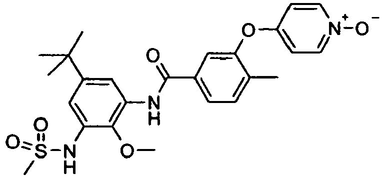

Zu 800 mg N-(5-tert-Butyl-3-methansulfonylamino-2-methoxy-phenyl)-3-hydroxy-4-methyl-benzamid in 5 ml N-Methylpyrrolidinon werden unter Argonatmosphäre 962 mg Cäsiumcarbonat gegeben. Nach 30 Minuten werden 306 mg 4-Chlorpyridin-N-oxid zugegeben, und das Reaktionsgemisch wird 1 h bei 80°C gerührt. Anschließend wird das Reaktionsgemisch auf 100°C erwärmt und über Nacht bei dieser Temperatur gerührt. Dann werden nochmals 255 mg 4-Chlorpyridin-N-oxid und 641 mg Cäsiumcarbonat zugesetzt, und das Reaktionsgemisch wird weitere 24 h bei 100°C gerührt. Zur Aufarbeitung wird das abgekühlte Reaktionsgemisch mit Ethylacetat verdünnt, mit Wasser und gesättigter wässriger Natriumchlorid-Lösung gewaschen, über Magnesiumsulfat getrocknet und eingeengt. Der Kolbenrückstand wird chromatographisch über eine Kieselgelsäule mit Dichlormethan/Methanol (93:7) als Laufmittel gereinigt.
Ausbeute: 781 mg (80 % der Theorie)
Massenspektrum (ESI⁺): m/z = 500 [M+H]⁺

### Beispiel 2

### N-(5-tert-Butyl-3-methansulfonylamino-2-methoxy-phenyl)-4-methyl-3-(pyridin-4-yloxy)-benzamid

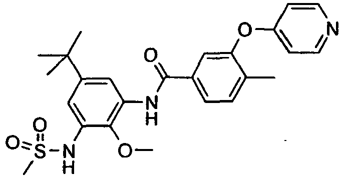

150 mg N-(5-tert-Butyl-3-methansulfonylamino-2-methoxy-phenyl)-4-methyl-3-(1-oxy-pyridin-4-yloxy)-benzamid in 10 ml Tetrahydrofuran werden in Gegenwart von 20 mg Rhodium auf Aktivkohle (5% Rh) bei Raumtemperatur und 50 psi Wasserstoffpartialdruck hydriert. Nach 2 h werden weitere 20 mg Katalysator zugegeben. Da die Umsetzung noch nicht vollständig ist, werden anschließend nochmals 50 mg und dann weitere 40 mg Katalysator zugegeben. Da keine weitere Umsetzung mehr zu beobachten ist, wird die Reaktion abgebrochen. Der Katalysator wird abgesaugt und das Filtrat eingeengt. Der Kolbenrückstand wird chromatographisch über eine Reversed-Phase-Säule mit Acetonitril/Wasser/konz. Ammoniak als Laufmittel gereinigt.
Ausbeute: 65 mg (45 % der Theorie)
Massenspektrum (ESI⁺): m/z = 484 [M+H]⁺

### Beispiel 3

### N-(5-tert-Butyl-3-methansulfonylamino-2-methoxy-phenyl)-4-methyl-3-(2-methylamino-pyrimidin-4-yloxy)-benzamid

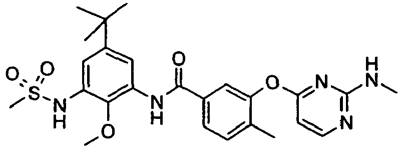

Ein Gemisch aus 200 mg N-(5-tert-Butyl-3-methansulfonylamino-2-methoxy-phenyl)-3-(2-chlor-pyrimidin-4-yloxy)-4-methyl-benzamid und 2 ml einer 2 M MethylaminLösung in Tetrahydrofuran wird bei Raumtemperatur über Nacht in einem verschlossenen Mikrowellengläschen gerührt. Zur Aufarbeitung wird das Reaktionsgemisch mit Ethylacetat verdünnt, mit Wasser und gesättigter wässriger Natriumchlorid-Lösung gewaschen, über Magnesiumsulfat getrocknet und eingeengt. Das Rohprodukt wird über eine Kieselgelsäule mit Ethylacetat/Cyclohexan (50:50→100:0) als Laufmittel chromatographiert.
Ausbeute: 146 mg (74 % der Theorie)
R_{f}-Wert: 0,09 (Kieselgel, Petrolether/Ethylacetat = 1:1)
Massenspektrum (ESI⁺): m/z = 514 [M+H]⁺

Analog Beispiel 3 wird folgende Verbindung erhalten:
(1) N-(5-tert-Butyl-3-methansulfonylamino-2-methoxy-phenyl)-4-methyl-3-(6-methylamino-pyrimidin-4-yloxy)-benzamid 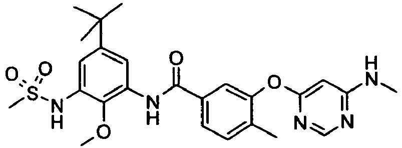 R_{f}-Wert: 0,11 (Kieselgel, Petrolether/Ethylacetat = 1:2)
   Massenspektrum (ESI⁺): m/z = 514 [M+H]⁺

### Beispiel 4

### N-(5-tert-Butyl-3-methansulfonylamino-2-methoxy-phenyl)-4-methyl-3-[2-(2-pyrrolidin-1-yl-ethylamino)-pyrimidin-4-yloxy]-benzamid

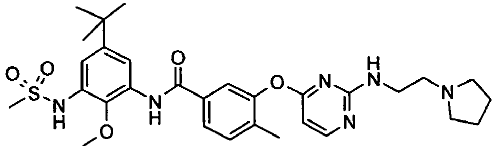

Zu 300 mg N-(5-tert-Butyl-3-methansulfonylamino-2-methoxy-phenyl)-3-(2-chlor-pyrimidin-4-yloxy)-4-methyl-benzamid in 3 ml 1,4-Dioxan werden 147 µl 1-(2-Aminoethyl)pyrrolidin und 161 µl Triethylamin gegeben, und das Reaktionsgemisch wird 5 h auf 70°C erhitzt. Anschließend wird die Heizung ausgeschaltet, und das Gemisch wird über Nacht bei Raumtemperatur gerührt. Zur Aufarbeitung wird das Reaktionsgemisch mit Ethylacetat verdünnt, mit 3 M wässriger Kaliumcarbonat-Lösung gewaschen, über Magnesiumsulfat getrocknet und eingeengt. Das Rohprodukt wird über eine Kieselgelsäule mit Dichlormethan/Methanol (75:25→0:100) als Laufmittel chromatographiert.
Ausbeute: 183 mg (53 % der Theorie)
R_{f}-Wert: 0,2 (Kieselgel, Dichlormethan/Methanol/konz. Ammoniak = 90:10:1)
Massenspektrum (ESI⁺): m/z = 597 [M+H]⁺

Analog Beispiel 4 werden folgende Verbindungen erhalten:
(1) N-(5-tert-Butyl-3-methansulfonylamino-2-methoxy-phenyl)-3-[6-(2-hydroxy-ethylamino)-pyrimidin-4-yloxy]-4-methyl-benzamid 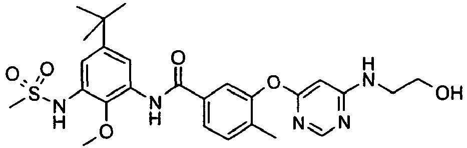 (Umsetzung erfolgt in Tetrahydrofuran bei 50°C)
   R_{f}-Wert: 0,4 (Kieselgel, Dichlormethan/Methanol/konz. Ammoniak = 90:10:1)
   Massenspektrum (ESI⁺): m/z = 544 [M+H]⁺
(2) N-(5-tert-Butyl-3-methansulfonylamino-2-methoxy-phenyl)-3-[6-(2-dimethylamino-ethylamino)-pyrimidin-4-yloxy]-4-methyl-benzamid 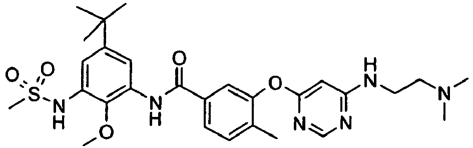 (Umsetzung erfolgt in Tetrahydrofuran bei 50°C)
   R_{f}-Wert: 0,3 (Kieselgel, Dichlormethan/Methanol/konz. Ammoniak = 90:10:1)
   Massenspektrum (ESI⁺): m/z = 571 [M+H]⁺
(3) N-(5-tert-Butyl-3-methansulfonylamino-2-methoxy-phenyl)-4-methyl-3-[6-(3-oxo-piperazin-1-yl)-pyrimidin-4-yloxy]-benzamid 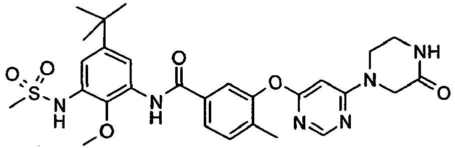 (Umsetzung erfolgt in Tetrahydrofuran bei 50°C)
   R_{f}-Wert: 0,5 (Kieselgel, Dichlormethan/Methanol/konz. Ammoniak = 90:10:1)
   Massenspektrum (ESI⁺): m/z = 583 [M+H]⁺
(4) N-(5-tert-Butyl-3-methansulfonylamino-2-methoxy-phenyl)-4-methyl-3-(6-pyrrolidin-1-yl-pyrimidin-4-yloxy)-benzamid 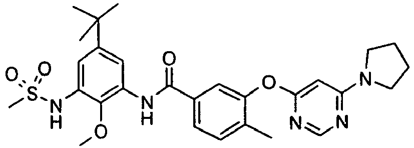 (Umsetzung erfolgt in Tetrahydrofuran bei Raumtemperatur)
   R_{f}-Wert: 0,3 (Kieselgel, Dichlormethan/Methanol/konz. Ammoniak = 90:10:1)
   Massenspektrum (ESI⁺): m/z = 554 [M+H]⁺
(5) N-(5-tert-Butyl-3-methansulfonylamino-2-methoxy-phenyl)-4-methyl-3-(6-morpholin-4-yl-pyrimidin-4-yloxy)-benzamid 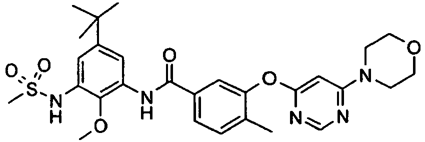 (Umsetzung erfolgt in Tetrahydrofuran bei Raumtemperatur)
   R_{f}-Wert: 0,5 (Kieselgel, Dichlormethan/Methanol/konz. Ammoniak = 90:10:1)
   Massenspektrum (ESI⁺): m/z = 570 [M+H]⁺
(6) (*R*)-N-(5-tert-Butyl-3-methansulfonylamino-2-methoxy-phenyl)-3-[2-(3-dimethylamino-pyrrolidin-1-yl)-pyrimidin-4-yloxy]-4-methyl-benzamid 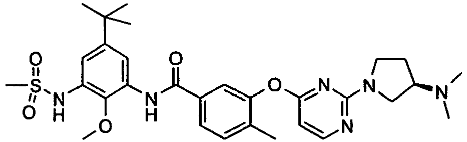 (Umsetzung erfolgt in N,N-Dimethylformamid in Gegenwart von Diisopropylethylamin bei 50°C)
   R_{f}-Wert: 0,3 (Kieselgel, Dichlormethan/Methanol/konz. Ammoniak = 90:10:1)
   Massenspektrum (ESI⁺): m/z = 597 [M+H]⁺
(7) (S)-N-(5-tert-Butyl-3-methansulfonylamino-2-methoxy-phenyl)-3-[2-(3-dimethylamino-pyrrolidin-1-yl)-pyrimidin-4-yloxy]-4-methyl-benzamid 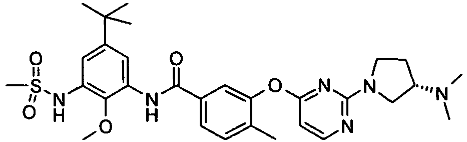 (Umsetzung erfolgt in N,N-Dimethylformamid in Gegenwart von Diisopropylethylamin bei 50°C)
   R_{f}-Wert: 0,3 (Kieselgel, Dichlormethan/Methanol/konz. Ammoniak = 90:10:1)
   Massenspektrum (ESI⁺): m/z = 597 [M+H]⁺
(8) N-(5-tert-Butyl-3-methansulfonylamino-2-methoxy-phenyl)-3-[2-(4-dimethylamino-piperidin-1-yl)-pyrimidin-4-yloxy]-4-methyl-benzamid 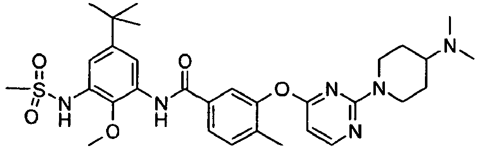 (Umsetzung erfolgt in N,N-Dimethylformamid in Gegenwart von Diisopropylethylamin bei 50°C)
   Massenspektrum (ESI⁺): m/z = 611 [M+H]⁺
(9) N-(5-tert-Butyl-3-methansulfonylamino-2-methoxy-phenyl)-4-methyl-3-[2-(1-methylpiperidin-4-ylamino)-pyrimidin-4-yloxy]-benzamid 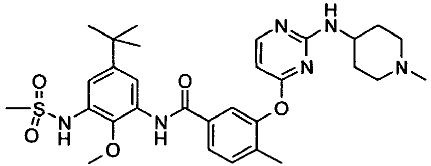 (Umsetzung erfolgt in Dimethylformamid bei 50°C)
   Massenspektrum (ESI⁺): m/z = 597 [M+H]⁺
(10) N-(5-tert-Butyl-3-methansuffonylamino-2-methoxy-phenyl)-4-methyl-3-[6-(1-methyl-piperidin-4-ylamino)-pyrimidin-4-yloxy]-benzamid 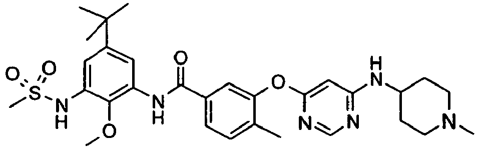 (Umsetzung erfolgt in Tetrahydrofuran bei Raumtemperatur)
   HPLC (Methode 1): Retentionszeit = 2,70 min
   Massenspektrum (ESI⁺): m/z = 597 [M+H]⁺
(11) N-(5-tert-Butyl-3-methansulfonylamino-2-methoxy-phenyl)-3-[6-(4-dimethylamino-piperidin-1-yl)-pyrimidin-4-yloxy]-4-methyl-benzamid 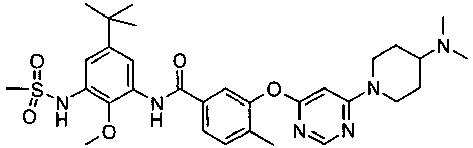 (Umsetzung erfolgt in N,N-Dimethylformamid in Gegenwart von Diisopropylethylamin bei Raumtemperatur)
   HPLC (Methode 1): Retentionszeit = 2,81 min
   Massenspektrum (ESI⁺): m/z = 611 [M+H]⁺
(12) N-(5-tert-Butyl-3-methansulfonylamino-2-methoxy-phenyl)-3-[6-((*R*)-3-dimethylamino-pyrrolidin-1-yl)-pyrimidin-4-yloxy]-4-methyl-benzamid 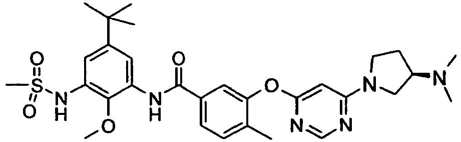 (Umsetzung erfolgt in einem Gemisch aus Acetonitril und N-Methylpyrrolidinon in Gegenwart von Diisopropylethylamin bei Raumtemperatur)
   R_{f}-Wert: 0,4 (Kieselgel, Dichlormethan/Methanol/konz. Ammoniak = 90:10:0,1)
   Massenspektrum (ESI⁺): m/z = 597 [M+H]⁺
(13) N-(5-tert-Butyl-3-methansulfonylamino-2-methoxy-phenyl)-3-[6-((*S*)-3-dimethylamino-pyrrolidin-1-yl)-pyrimidin-4-yloxy]-4-methyl-benzamid 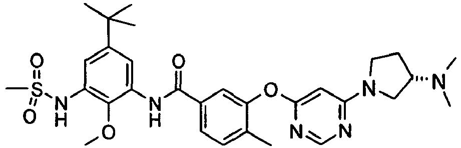 (Umsetzung erfolgt in einem Gemisch aus Acetonitril und N-Methylpyrrolidinon in Gegenwart von Diisopropylethylamin bei Raumtemperatur)
   R_{f}-Wert: 0,4 (Kieselgel, Dichlormethan/Methanol/konz. Ammoniak = 90:10:0,1)
   Massenspektrum (ESI⁺): m/z = 597 [M+H]⁺
(14) N-(5-tert-Butyl-3-methansulfonylamino-2-methoxy-phenyl)-4-chloro-3-[6-(1-methyl-piperidin-4-ylamino)-pyrimidin-4-yloxy]-benzamid 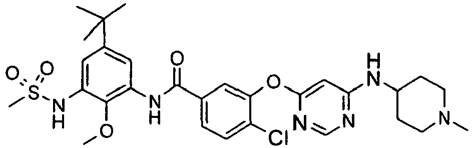 (Umsetzung erfolgt ohne zusätzliche Base mit 2,5 Äquivalenten 1-Methyl-piperidin-4-ylamin in Acetonitril bei 60°C)
   R_{f}-Wert: 0,3 (Kieselgel, Dichlormethan/Methanol/methanolischer Ammoniak = 95:5:0,1)
   Massenspektrum (ESI⁺): m/z = 617/619 (CI) [M+H]⁺
(15) N-(5-tert-Butyl-3-methansulfonylamino-2-methoxy-phenyl)-4-chlor-3-[2-(1-methylpiperidin-4-ylamino)-pyrimidin-4-yloxy]-benzamid 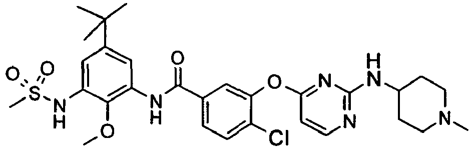 (Umsetzung erfolgt ohne zusätzliche Base mit 2,5 Äquivalenten 1-Methyl-piperidin-4-ylamin in Acetonitril bei 90°C)
   R_{f}-Wert: 0,4 (Kieselgel, Dichlormethan/Methanol/methanolischer Ammoniak = 95:5:0,1)
   Massenspektrum (ESI⁺): m/z = 617/619 (CI) [M+H]⁺
(16) N-(5-tert-Butyl-3-methansulfonylamino-2-methoxy-phenyl)-4-methyl-3-{2-[methyl-(1-methyl-piperidin-4-yl)-amino]-pyrimidin-4-yloxy}-benzamid 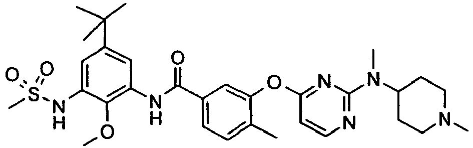 (Umsetzung erfolgt in N,N-Dimethylformamid mit Diisopropylethylamin bei Raumtemperatur)
   R_{f}-Wert: 0,3 (Kieselgel, Dichlormethan/Methanol/konz. Ammoniak = 90:10:0,1)
   Massenspektrum (ESI⁺): m/z = 611 [M+H]⁺
(17) N-(5-tert-Butyl-3-methansulfonylamino-2-methoxy-phenyl)-4-methyl-3-{6-[methyl-(1-methyl-piperidin-4-yl)-amino]-pyrimidin-4-yloxy}-benzamid 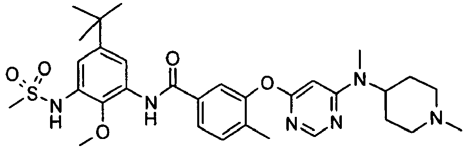 (Umsetzung erfolgt in N,N-Dimethylformamid mit Diisopropylethylamin bei Raumtemperatur)
   R_{f}-Wert: 0,3 (Kieselgel, Dichlormethan/Methanol/konz. Ammoniak = 90:10:0,1)
   Massenspektrum (ESI⁺): m/z = 611 [M+H]⁺
(18) N-(5-tert-Butyl-3-methansulfonylamino-2-methoxy-phenyl)-3-[2-(trans-4-dimethylamino-cyclohexylamino)-pyrimidin-4-yloxy]-4-methyl-benzamid 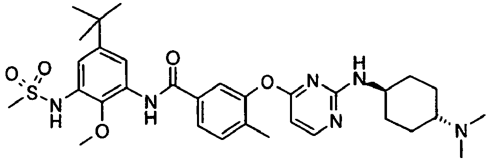 (Umsetzung erfolgt in N,N-Dimethylformamid mit Diisopropylethylamin bei 40°C)
   Massenspektrum (ESI⁺): m/z = 625 [M+H]⁺
(19) N-(5-tert-Butyl-3-methansulfonylamino-2-methoxy-phenyl)-4-methyl-3-{2-[methyl-(1-methyl-pyrrolidin-3-yl)-amino]-pyrimidin-4-yloxy}-benzamid 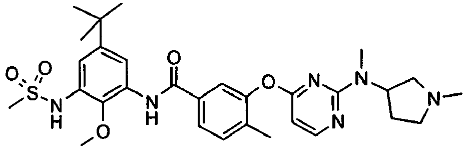 (Umsetzung erfolgt in N,N-Dimethylformamid mit Diisopropylethylamin bei 40°C)
   R_{f}-Wert: 0,3 (Kieselgel, Dichlormethan/Methanol/konz. Ammoniak = 90:10:0,1)
   Massenspektrum (ESI⁺): m/z = 597 [M+H]⁺
(20) N-(5-tert-Butyl-3-methansulfonylamino-2-methoxy-phenyl)-4-methyl-3-{6-[methyl-(1-methyl-pyrrolidin-3-yl)-amino]-pyrimidin-4-yloxy}-benzamid 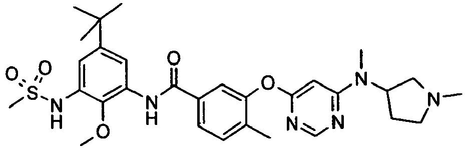 (Umsetzung erfolgt in N,N-Dimethylformamid mit Diisopropylethylamin bei 40°C)
   R_{f}-Wert: 0,3 (Kieselgel, Dichlormethan/Methanol/konz. Ammoniak = 90:10:0,1)
   Massenspektrum (ESI⁺): m/z = 597 [M+H]⁺

### Beispiel 5

### N-(5-tert-Butyl-3-methansulfonylamino-2-methoxy-phenyl)-4-methyl-3-(2-piperazin-1-yl-pyrimidin-4-yloxy)-benzamid x Trifluoressigsäure

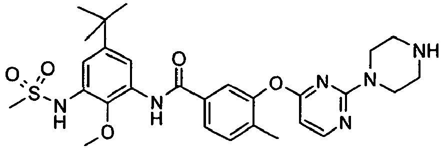

Zu 433 mg 4-{4-[5-(5-tert-Butyl-3-methansulfonylamino-2-methoxy-phenylcarbamoyl)-2-methyl-phenoxy]-pyrimidin-2-yl}-piperazin-1-carbonsäure-tert-butylester in 5 ml Dichlormethan werden 1,5 ml Trifluoressigsäure gegeben, und das Reaktionsgemisch wird 3 h bei Raumtemperatur gerührt. Anschließend wird die farblose Lösung eingeengt, mit Diethylether versetzt und über Nacht bei Raumtemperatur gerührt. Der entstandene Niederschlag wird abgesaugt und im Exsikkator getrocknet.
Ausbeute: 299 mg (68 % der Theorie)
Massenspektrum (ESI⁺): m/z = 569 [M+H]⁺

Analog Beispiel 5 werden folgende Verbindungen erhalten; abweichend von dem beschriebenen Vorgehen wurden einige der nachfolgenden Verbindungen nach wässriger Aufarbeitung und Chromatographie auf Kieselgel als Basen isoliert:
(1) N-(5-tert-Butyl-3-methansulfonylamino-2-methoxy-phenyl)-4-methyl-3-(6-piperazin-1-yl-pyrimidin-4-yloxy)-benzamid 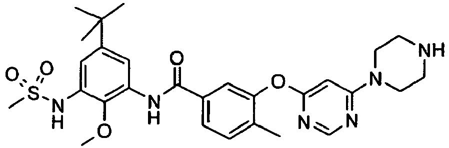 R_{f}-Wert: 0,2 (Kieselgel, Dichlormethan/Methanol/konz. Ammoniak = 90:10:1)
   Massenspektrum (ESI⁺): m/z = 569 [M+H]⁺
(2) N-(5-tert-Butyl-3-methansulfonylamino-2-methoxy-phenyl)-4-methyl-3-[6-(piperazin-1-carbonyl)-pyrimidin-4-yloxy]-benzamid x Trifluoressigsäure 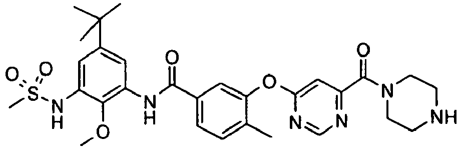 R_{f}-Wert: 0,2 (Kieselgel, Dichlormethan/Methanol/konz. Ammoniak = 90:10:1)
   Massenspektrum (ESI⁺): m/z = 597 [M+H]⁺
(3) N-(5-tert-Butyl-3-methansulfonylamino-2-methoxy-phenyl)-4-chlor-3-[6-(piperazin-1-carbonyl)-pyrimidin-4-yloxy]-benzamid 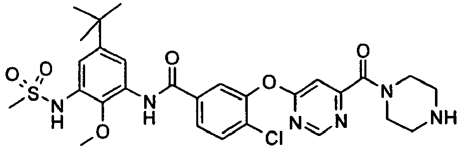 R_{f}-Wert: 0,2 (Kieselgel, Dichlormethan/Methanol/konz. Ammoniak = 90:10:1)
   Massenspektrum (ESI⁺): m/z = 617/619 (CI) [M+H]⁺
(4) N-(5-tert-Butyl-3-methansulfonylamino-2-methoxy-phenyl)-3-(2-piperazin-1-yl-pyrimidin-4-yloxy)-4-trifluormethyl-benzamid x Trifluoressigsäure 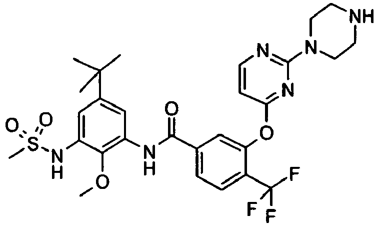 R_{f}-Wert: 0,5 (Kieselgel, Dichlormethan/Methanol/konz. Ammoniak = 90:10:1)
   Massenspektrum (ESI⁺): m/z = 623 [M+H]⁺
(5) N-(5-tert-Butyl-3-methansulfonylamino-2-methoxy-phenyl)-4-chlor-3-(2-piperazin-1-yl-pyrimidin-4-yloxy)-benzamid 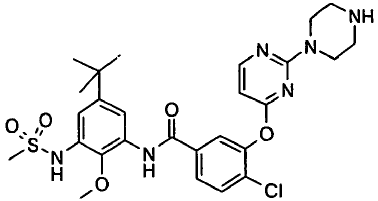 R_{f}-Wert: 0,3 (Kieselgel, Dichlormethan/Methanol/konz. Ammoniak = 90:10:1)
   Massenspektrum (ESI⁺): m/z = 589/591 (Cl) [M+H]⁺
(6) N-(5-tert-Butyl-3-methansulfonylamino-2-methoxy-phenyl)-4-methyl-3-(2-piperazin-1-ylmethyl-pyrimidin-4-yloxy)-benzamid 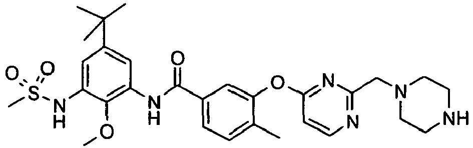 R_{f}-Wert: 0,2 (Kieselgel, Dichlormethan/Methanol/konz. Ammoniak = 90:10:0,1)
   Massenspektrum (ESI⁺): m/z = 583 [M+H]⁺
(7) N-(5-tert-Butyl-3-methansulfonylamino-2-methoxy-phenyl)-4-methyl-3-(6-piperazin-1-ylmethyl-pyrimidin-4-yloxy)-benzamid 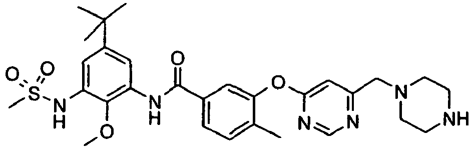 R_{f}-Wert: 0,3 (Kieselgel, Dichlormethan/Methanol/konz. Ammoniak = 90:10:1)
   Massenspektrum (ESI⁺): m/z = 583 [M+H]⁺
(8) N-(5-tert-Butyl-3-methansulfonylamino-2-methoxy-phenyl)-4-methoxy-3-(2-piperazin-1-yl-pyrimidin-4-yloxy)-benzamid 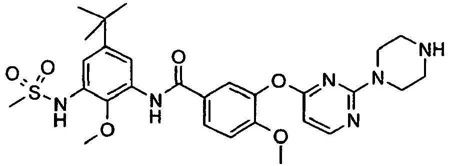 R_{f}-Wert: 0,6 (Kieselgel, Dichlormethan/Methanol/konz. Ammoniak = 90:10:0,1)
   Massenspektrum (ESI⁺): m/z = 585 [M+H]⁺
(9) N-(5-tert-Butyl-3-methansulfonylamino-2-methoxy-phenyl)-4-methyl-3-(2-piperazin-i-ylmethyl-pyridin-4-yloxy)-benzamid x Trifluoressigsäure 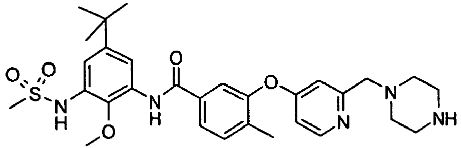 Massenspektrum (ESI⁺): m/z = 582 [M+H]⁺
(10) N-(5-tert-Butyl-3-methansulfonylamino-2-methoxy-phenyl)-4-fluor-3-(2-piperazin-1-yl-pyrimidin-4-yloxy)-benzamid x Trifluoressigsäure 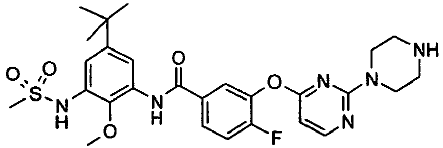 R_{f}-Wert: 0,1 (Kieselgel, Dichlormethan/Methanol/konz. Ammoniak = 95:5:0,1)
   Massenspektrum (ESI⁺): m/z = 573 [M+H]⁺
(11) N-(5-tert-Butyl-3-methansulfonylamino-2-methoxy-phenyl)-4-brom-3-(2-piperazin-1-yl-pyrimidin-4-yloxy)-benzamid 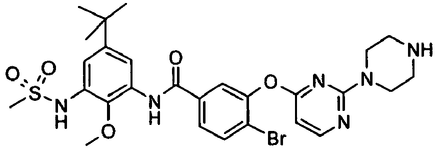 R_{f}-Wert: 0,35 (Kieselgel, Dichlormethan/Methanol/konz. Ammoniak = 95:5:0,1)
   Massenspektrum (ESI⁺): m/z = 633/635 (Br) [M+H]⁺
(12) N-(5-tert-Butyl-3-methansulfonylamino-2-methoxy-phenyl)-3-chlor-4-methyl-5-(2-piperazin-1-yl-pyrimidin-4-yloxy)-benzamid 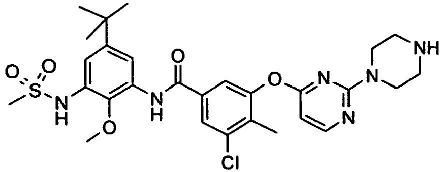 R_{f}-Wert: 0,3 (Kieselgel, Dichlormethan/Methanol/konz. Ammoniak = 95:5:0,1)
   Massenspektrum (ESI⁺): m/z = 603/605 (Cl) [M+H]⁺
(13) N-(5-tert-Butyl-3-methansulfonylamino-2-methoxy-phenyl)-3-fluor-4-methyl-5-(2-piperazin-1-yl-pyrimidin-4-yloxy)-benzamid 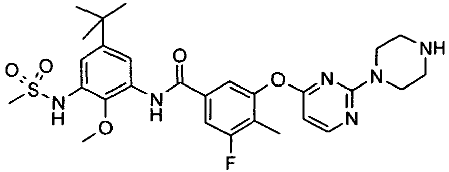 R_{f}-Wert: 0,3 (Kieselgel, Dichlormethan/Methanol/konz. Ammoniak = 95:5:0,1)
   Massenspektrum (ESI⁺): m/z = 587 [M+H]⁺
(14) N-(5-tert-Butyl-3-methansulfonylamino-2-methoxy-phenyl)-4-methyl-3-[2-(N-methyl-N-piperidin-4-yl-amino)-pyrimidin-4-yloxy]-benzamid 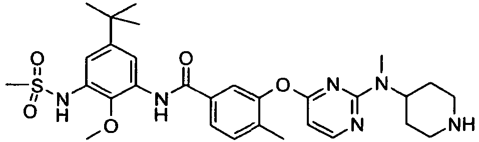 R_{f}-Wert: 0,1 (Kieselgel, Dichlormethan/Methano/konz. Ammoniak =90:10:0,1)
   Massenspektrum (ESI⁺): m/z = 597 [M+H]⁺
(15) N-(5-tert-Butyl-3-methansulfonylamino-2-methoxy-phenyl)-4-ethyl-5-(2-piperazin-1-yl-pyrimidin-4-yloxy)-benzamid 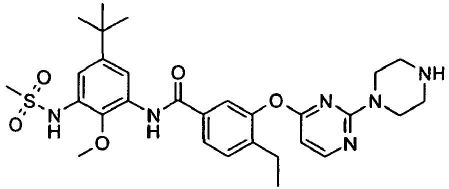 R_{f}-Wert: 0,4 (Kieselgel, Dichlormethan/Methanol/konz. Ammoniak = 95:5:0,1)
   Massenspektrum (ESI⁺): m/z = 583 [M+H]⁺
(16) N-(5-tert-Butyl-3-methansulfonylamino-2-methoxy-phenyl)-4-methyl-3-[2-(piperidin-4-ylamino)-pyrimidin-4-yloxy]-benzamid 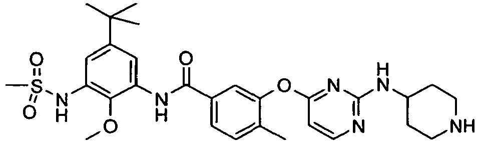 Massenspektrum (ESI⁺): m/z = 583 [M+H]⁺
(17) N-(5-tert-Butyl-3-methansulfonylamino-2-methoxy-phenyl)-4-methyl-3-[6-(piperidin-4-ylamino)-pyrimidin-4-yloxy]-benzamid x Trifluoressigsäure 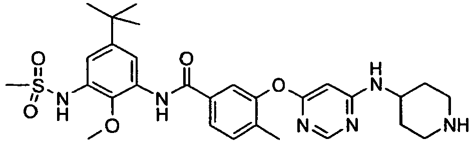 Massenspektrum (ESI⁺): m/z = 583 [M+H]⁺
(18) N-(5-tert-Butyl-3-methansulfonylamino-2-methoxy-phenyl)-4-methyl-3-(6-piperidin-4-ylmethyl-pyrimidin-4-yloxy)-benzamid 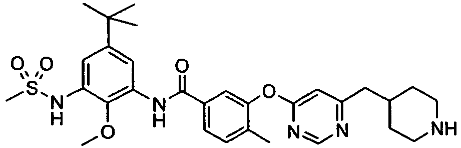 Massenspektrum (ESI⁺): m/z = 582 [M+H]⁺
(19) N-(5-tert-Butyl-3-methansulfonylamino-2-methoxy-phenyl)-4-methyl-3-(2-piperidin-4-ylmethyl-pyrimidin-4-yloxy)-benzamid 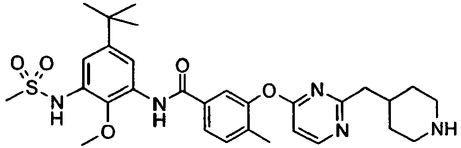 HPLC (Methode 1): Retentionszeit = 2,72 min
   Massenspektrum (ESI⁺): m/z = 582 [M+H]⁺
(20) N-(5-tert-Butyl-3-methansulfonylamino-2-methoxy-phenyl)-4-methyl-3-(2-piperidin-4-ylmethyl-pyridin-4-yloxy)-benzamid 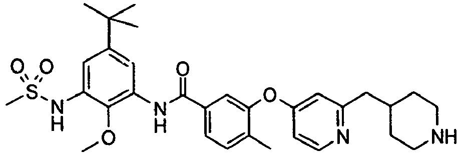 HPLC (Methode 1): Retentionszeit = 2,31 min
   Massenspektrum (ESI⁺): m/z = 581 [M+H]⁺
(21) N-(5-tert-Butyl-3-methansulfonylamino-2-methoxy-phenyl)-3-methyl-5-[2-(piperazin-1-carbonyl)-pyrimidin-4-yloxy]-benzamid x Trifluoressigsäure 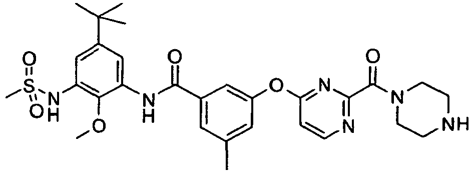 R_{f}-Wert: 0,45 (Reversed Phase DC-Fertigplatte (E. Merck), Acetonitril/Wasser/ Trifluoressigsäure = 50:50:1)
   Massenspektrum (ESI⁺): m/z = 597 [M+H]⁺
(22) N-(5-tert-Butyl-3-methansulfonylamino-2-methoxy-phenyl)-3-methyl-5-(2-piperazin-1-ylmethyl-pyrimidin-4-yloxy)-benzamid 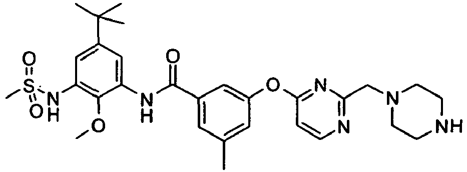 R_{f}-Wert: 0,5 (Reversed Phase DC-Fertigplatte (E. Merck), AcetonitrillWasser/Trifluoressigsäure = 50:50:1)
   Massenspektrum (ESI⁺): m/z = 583 [M+H]⁺
(23) N-(5-tert-Butyl-3-methansulfonylamino-2-methoxy-phenyl)-4-methyl-3-[2-(pyrrolidin-3-ylamino)-pyrimidin-4-yloxy]-benzamid 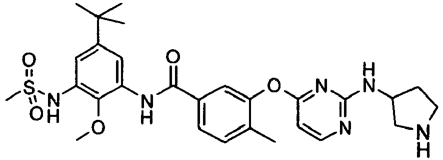 HPLC (Methode 1): Retentionszeit = 2,73 min
   Massenspektrum (ESI⁺): m/z = 569 [M+H]⁺
(24) N-(5-tert-Butyl-3-methanesulfonylamino-2-methoxy-phenyl)-4-methyl-3-[2-(methyl-pyrrolidin-3-yl-amino)-pyrimidin-4-yloxy]-benzamid 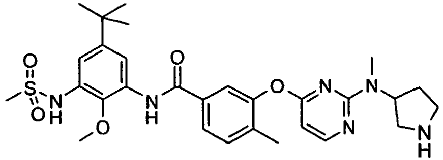 R_{f}-Wert: 0,3 (Kieselgel, Dichlormethan/Methanol/konz. Ammoniak = 90:10:0,1) Massenspektrum (ESI⁺): m/z = 583 [M+H]⁺
(25) N-(5-tert-Butyl-3-methansulfonylamino-2-methoxy-phenyl)-4-methyl-3-[2-(methylpiperidin-4-yl-amino)-pyrimidin-4-yloxy]-benzamid 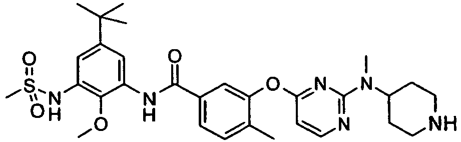 R_{f}-Wert: 0,2 (Kieselgel, Dichtormethan/Methanot/konz. Ammoniak = 90:10:0,1) Massenspektrum (ESI⁺): m/z = 597 [M+H]⁺
(26) N-(5-tert-Butyl-3-methanesulfonylamino-2-methoxy-phenyl)-4-methyl-3-[6-(pyrrolidin-3-ylamino)-pyrimidin-4-yloxy]-benzamide 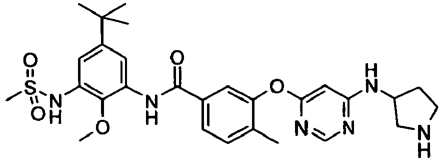 R_{f}-Wert: 0,2 (Kieselgel, Dichtormethan/Methanot/konz. Ammoniak = 90:10:0,1) Massenspektrum (ESI⁺): m/z = 569 [M+H]⁺
(27) N-(5-tert-Butyl-3-methansulfonylamino-2-methoxy-phenyl)-4-chlor-3-[6-(piperidin-4-ylamino)-pyrimidin-4-yloxy]-benzamid 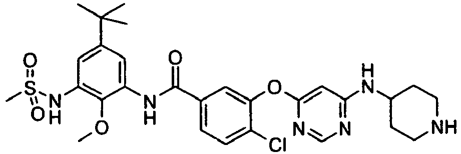 R_{f}-Wert: 0,1 (Kieselgel, Dichlormethan/Methanol/methanolischer Ammoniak = 95:5:0,1)
   Massenspektrum (ESI⁺): m/z = 603/605 (Cl) [M+H]⁺
(28) N-(5-tert-Butyl-3-methansulfonylamino-2-methoxy-phenyl)-4-chlor-3-[2-(piperidin-4-ylamino)-pyrimidin-4-yloxy]-benzamid 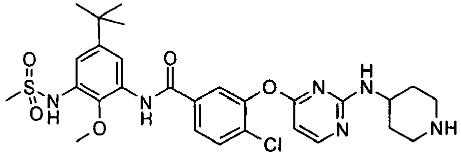 R_{f}-Wert: 0,2 (Kieselgel, Dichlormethan/Methanol/methanolischer Ammoniak = 90:10:0,1)
   Massenspektrum (ESI⁺): m/z = 603/605 (Cl) [M+H]⁺
(29) N-(5-tert-Butyl-3-methansulfonylamino-2-methoxy-phenyl)-4-chlor-3-(2-piperidin-4-ylmethyl-pyridin-4-yloxy)-benzamid 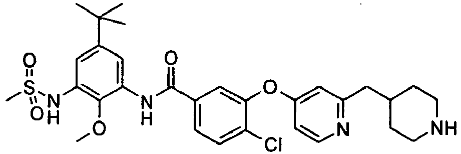 R_{f}-Wert: 0,05 (Kieselgel, Dichlormethan/Methanol/methanolischer Ammoniak = 95:5:0,1)
   Massenspektrum (ESI⁺): m/z = 601/603 (Cl) [M+H]⁺
(30) N-(5-tert-Butyl-3-methansulfonylamino-2-methoxy-phenyl)-4-chlor-3-(2-piperidin-4-ylmethyl-pyrimidin-4-yloxy)-benzamid 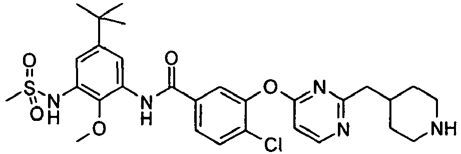 R_{f}-Wert: 0,05 (Kieselgel, Dichlormethan/Methanol/methanolischer Ammoniak = 95:5:0,1)
   Massenspektrum (ESI⁺): m/z = 602/604 (Cl) [M+H]⁺
(31) N-(5-tert-Butyl-3-methansutfonylamino-2-methoxy-phenyl)-4-chlor-3-[2-(piperidin-4-ylamino)-pyridin-4-yloxy]-benzamid 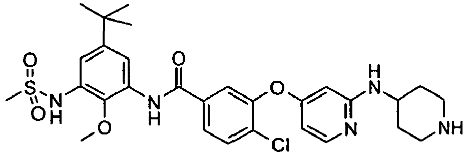 R_{f}-Wert: 0,1 (Kieselgel, Dichlormethan/Methanol/methanolischer Ammoniak = 90:10:0,1)
   Massenspektrum (ESI⁺): m/z = 602/604 (Cl) [M+H]⁺
(32) N-(5-tert-Butyl-3-methansulfonylamino-2-methoxy-phenyl)-4-chlor-3-(6-piperazin-1-ylmethyl-pyrimidin-4-yloxy)-benzamid 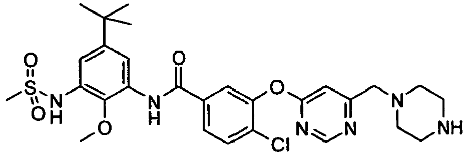 R_{f}-Wert: 0,1 (Kieselgel, Dichlormethan/Methanol/methanolischer Ammoniak = 95:5:0,1)
   Massenspektrum (ESI⁺): m/z = 603/605 (Cl) [M+H]⁺
(33) N-(5-tert-Butyl-3-methansulfonylamino-2-methoxy-phenyl)-4-chlor-3-(6-piperidin-4-ylmethyl-pyrimidin-4-ytoxy)-benzamid 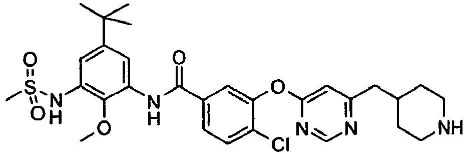 R_{f}-Wert: 0,1 (Kieselgel, Dichlormethan/Methanol/methanolischer Ammoniak = 95:5:0,1)
   Massenspektrum (ESI⁺): m/z = 602/604 (Cl) [M+H]⁺
(34) N-(5-tert-Butyl-3-methansulfonylamino-2-methoxy-phenyl)-4-chlor-3-(2-piperazin-1-ylmethyl-pyridin-4-yloxy)-benzamid 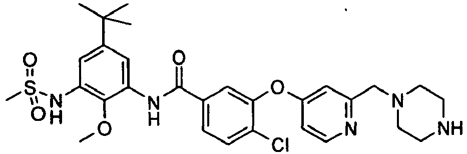 R_{f}-Wert: 0,15 (Kieselgel, Dichlormethan/Methanol/methanolischer Ammoniak = 95:5:0,1)
   Massenspektrum (ESI⁺): m/z = 602/604 (Cl) [M+H]⁺
(35) N-(5-tert-Butyl-3-methansulfonylamino-2-methoxy-phenyl)-4-chlor-3-(2-piperazin-1-ylmethyl-pyrimidin-4-yloxy)-benzamid 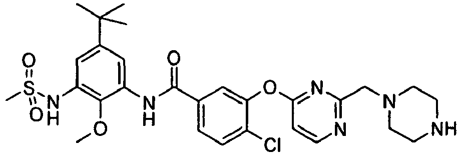 R_{f}-Wert: 0,1 (Kieselgel, Dichlormethan/Methanol/methanolischer Ammoniak = 95:5:0,1)
   Massenspektrum (ESI⁺): m/z = 603/605 (Cl) [M+H]⁺
(36) N-(5-tert-Butyl-3-methansulfonylamino-2-methoxy-phenyl)-4-chlor-3-(6-[1,4]diazepan-1-ylmethyl-pyrimidin-4-yloxy)-benzamid 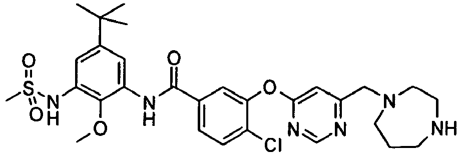 R_{f}-Wert: 0,1 (Kieselgel, Dichlormethan/Methanol/methanolischer Ammoniak = 95:5:0,1)
   Massenspektrum (ESI⁺): m/z = 617/619 (Cl) [M+H]⁺
(37) N-(5-tert-Butyl-3-methansulfonylamino-2-methoxy-phenyl)-4-chlor-3-(2-[1,4]diazepan-1-ylmethyl-pyridin-4-yloxy)-benzamid 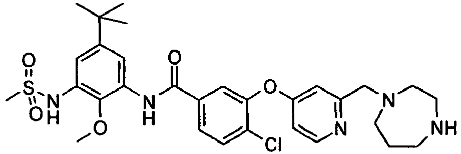 R_{f}-Wert: 0,05 (Kieselgel, Dichlormethan/Methanol/methanolischer Ammoniak = 95:5:0,1)
   Massenspektrum (ESI⁺): m/z = 616/618 (Cl) [M+H]⁺
(38) N-(5-tert-Butyl-3-methansulfonyl-2-methoxy-phenyl)-4-methyl-3-(2-piperidin-4-ylmethyl-pyrimidin-4-yloxy)-benzamid 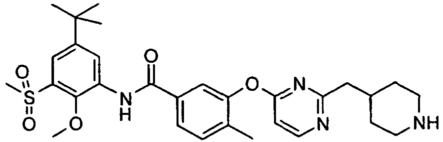 R_{f}-Wert: 0,05 (Kieselgel, Dichlormethan/Methanol/methanolischer Ammoniak = 95:5:0,1)
   Massenspektrum (ESI⁺): m/z = 567 [M+H]⁺
(39) N-(5-tert-Butyl-3-methansulfonyl-2-methoxy-phenyl)-3-(2-[1,4]diazepan-1-ylmethyl-pyridin-4-yloxy)-4-methyl-benzamid 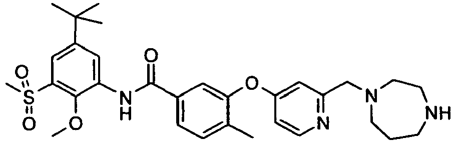 R_{f}-Wert: 0,4 (Reversed Phase DC-Fertigplatte (E. Merck), Acetonitril/Wasser/ Trifluoressigsäure = 50:50:1)
   Massenspektrum (ESI⁺): m/z = 596 [M+H]⁺
(40) N-(5-tert-Butyl-2-methoxy-3-methylsulfanylmethyl-phenyl)-4-methyl-3-(2-piperidin-4-ylmethyl-pyridin-4-yloxy)-benzamid 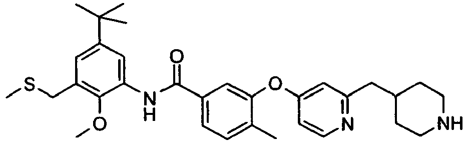 HPLC (Methode 1): Retentionszeit = 2,81 min
   Massenspektrum (ESI⁺): m/z = 548 [M+H]⁺
(41) N-(5-tert-Butyl-3-methansulfonylmethyl-2-methoxy-phenyl)-4-methyl-3-(2-piperidin-4-ylmethyl-pyridin-4-yloxy)-benzamid 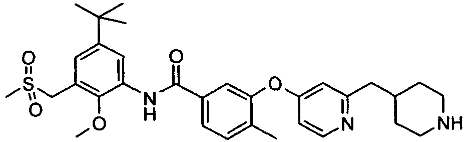 Massenspektrum (ESI⁺): m/z = 580 [M+H]⁺
(42) N-(5-tert-Butyl-2-methoxy-3-methylsulfinylmethyl-phenyl)-4-methyl-3-(2-piperidin-4-ylmethyl-pyridin-4-yloxy)-benzamid 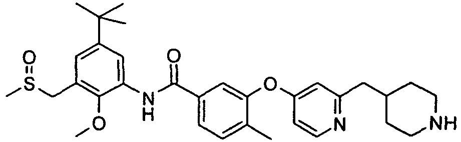 Massenspektrum (ESI⁺): m/z = 564 [M+H]⁺
(43) N-[5-tert-Butyl-3-(cyclopropancarbonyl-amino)-2-methoxy-phenyl]-4-methyl-3-(2-piperidin-4-ylmethyl-pyridin-4-yloxy)-benzamid 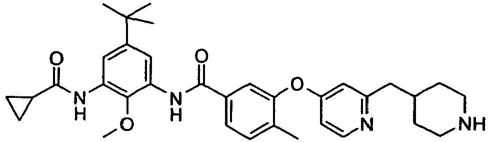 HPLC (Methode 1): Retentionszeit = 2,46 min
   Massenspektrum (ESI⁺): m/z = 571 [M+H]⁺
(44) N-(5-tert-Butyl-2-isopropoxy-3-methansulfonylamino-phenyl)-4-methyl-3-(2-piperidin-4-ylmethyl-pyridin-4-yloxy)-benzamid 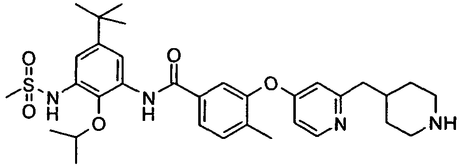 HPLC (Methode 1): Retentionszeit = 2,55 min
   Massenspektrum (ESI⁺): m/z = 609 [M+H]⁺
(45) N-(5-tert-Butyl-2-ethoxy-3-methansulfonylamino-phenyl)-4-methyl-3-(2-piperidin-4-ylmethyl-pyridin-4-yloxy)-benzamid 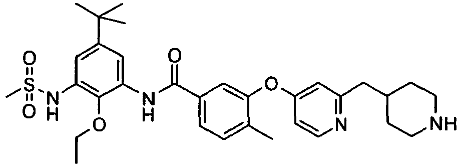 HPLC (Methode 1): Retentionszeit = 2,45 min
   Massenspektrum (ESI⁺): m/z = 595 [M+H]⁺
(46) N-[5-tert-Butyl-2-methoxy-3-(2-methyl-propan-1-sulfonylamino)-phenyl]-4-methyl-3-(2-piperidin-4-ylmethyl-pyridin-4-yloxy)-benzamid 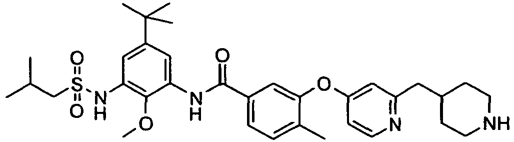 HPLC (Methode 1): Retentionszeit = 2,78 min
   Massenspektrum (ESI⁺): m/z = 623 [M+H]⁺
(47) N-(5-tert-Butyl-3-isobutyrylamino-2-methoxy-phenyl)-4-methyl-3-(2-piperidin-4-ylmethyl-pyridin-4-yloxy)-benzamid 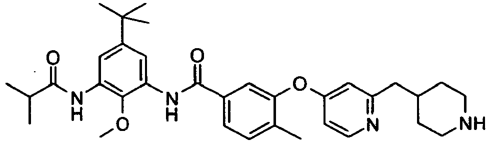 Massenspektrum (ESI⁺): m/z = 573 [M+H]⁺
(48) N-(5-tert-Butyl-3-methansulfinyl-2-methoxy-phenyl)-4-methyl-3-(2-piperidin-4-ylmethyl-pyridin-4-yloxy)-benzamid 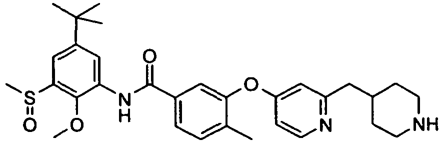 HPLC (Methode 1): Retentionszeit = 2,18 min
   Massenspektrum (ESI⁺): m/z = 550 [M+H]⁺
(49) N-(3-Methansulfonylamino-2-methoxy-5-trifluormethyl-phenyl)-4-methyl-3-(2-piperidin-4-ylmethyl-pyridin-4-yloxy)-benzamid 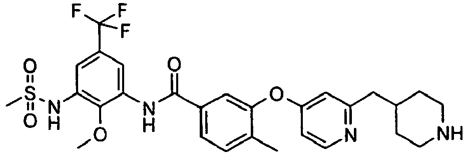 HPLC (Methode 1): Retentionszeit = 2,27 min
   Massenspektrum (ESI⁺): m/z = 593 [M+H]⁺
(50) N-[5-tert-Butyl-2-methoxy-3-(propan-2-sulfonylamino)-phenyl]-4-methyl-3-(2-piperidin-4-ylmethyl-pyridin-4-yloxy)-benzamid 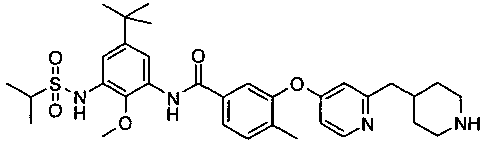 HPLC (Methode 1): Retentionszeit = 2,56 min
   Massenspektrum (ESI⁺): m/z = 609 [M+H]⁺
(51) N-(5-tert-Butyl-3-cyclopropansulfonylamino-2-methoxy-phenyl)-4-methyl-3-(2-piperidin-4-ylmethyl-pyridin-4-yloxy)-benzamid 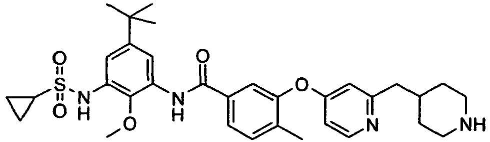 HPLC (Methode 1): Retentionszeit = 2,51 min
   Massenspektrum (ESI⁺): m/z = 607 [M+H]⁺
(52) 3-(2-Azepan-4-ylmethyl-pyridin-4-yloxy)-N-(5-tert-butyl-3-methansulfonylamino-2-methoxy-phenyl)-4-methyl-benzamid 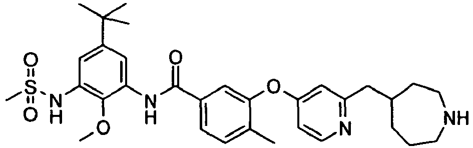 R_{f}-Wert: 0,05 (Kieselgel, Dichlormethan/Methanol/methanolischer Ammoniak = 90:10:0,1)
   Massenspektrum (ESI⁺): m/z = 595 [M+H]⁺
(53) 3-(2-Azepan-4-ylmethyl-pyrimidin-4-yloxy)-N-(5-tert-butyl-3-methansulfonylamino-2-methoxy-phenyl)-4-methyl-benzamid 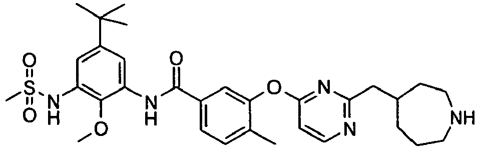 R_{f}-Wert: 0,1 (Kieselgel, Dichlormethan/Methanol/methanolischer Ammoniak = 90:10:0,1)
   Massenspektrum (ESI⁺): m/z = 596 [M+H]⁺
(54) 3-(6-Azepan-4-ylmethyl-pyrimidin-4-yloxy)-N-(5-tert-butyl-3-methansulfonylamino-2-methoxy-phenyl)-4-methyl-benzamid 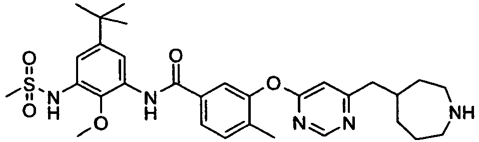 R_{f}-Wert: 0,1 (Kieselgel, Dichlormethan/Methanol/methanolischer Ammoniak = 90:10:0,1)
   Massenspektrum (ESI⁺): m/z = 596 [M+H]⁺
(55) N-(3-Methansulfonylamino-2-methoxy-5-pentafluorethyl-phenyl)-4-methyl-3-(2-piperidin-4-ylmethyl-pyridin-4-yloxy)-benzamid 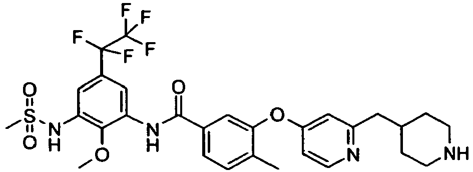 Massenspektrum (ESI⁺): m/z = 643 [M+H]⁺

### Beispiel 6

### N-(5-tert-Butyl-3-methansulfonylamino-2-methoxy-phenyl)-3-methyl-5-(pyridin-4-yloxy)-benzamid

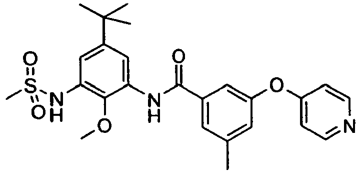

Zu 200 mg N-(5-tert-Butyl-3-methansulfonylamino-2-methoxy-phenyl)-3-hydroxy-5-methyl-benzamid und 325 mg Cäsiumcarbonat in 4 ml N,N-Dimethylformamid werden unter Argon 200 mg 4-lodpyridin, 20 µl 2,2,6,6-Tetramethylheptan-3,5-dion und 35 mg Kupfer(I)chlorid gegeben. Das Reaktionsgemisch wird auf 160°C erwärmt und 1 h bei dieser Temperatur gerührt. Nach Abkühlung auf Raumtemperatur wird das dunkle Reaktionsgemisch mit Wasser versetzt und mit Ethylacetat extrahiert. Die vereinigten Ethylacetat-Extrakte werden mit gesättigter wässriger Natriumchlorid-Lösung gewaschen, über Magnesiumsulfat getrocknet und eingeengt. Der Kolbenrückstand wird über eine Kieselgelsäule mit Dichlormethan/Methanol (99:1→90:10) chromatographiert.
Ausbeute: 112 mg (47% der Theorie)
R_{f}-Wert: 0,75 (Kieselgel, Dichlormethan/Methanol = 90:10)
Massenspektrum (ESI⁺): m/z = 484 [M+H]⁺

### Beispiel 7

### 6-[5-(5-tert-Butyl-3-methansulfonylamino-2-methoxy-phenylaminocarbonyl)-2 -methyl-phenoxy]-pyrimidin-4-carbonsäure-methylamid

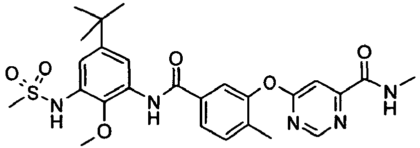

Zu 140 mg N-(5-tert-Butyl-3-methansuffonylamino-2-methoxy-phenyl)-3-hydroxy-4-methyl-benzamid und 46 mg Kalium-tert-butylat in 2 ml N,N-Dimethylformamid werden unter Argon 65 mg 6-Chlor-pyrimidin-4-carbonsäure-methylamid gegeben, und das Reaktionsgemisch wird 20 h bei Raumtemperatur gerührt. Dann wird es mit Wasser versetzt und mit Ethylacetat extrahiert. Die vereinigten Ethylacetat-Extrakte werden mit gesättigter wässriger Natriumchlorid-Lösung gewaschen, über Magnesiumsulfat getrocknet und eingeengt. Der Kolbenrückstand wird über eine Kieselgelsäule mit Cyclohexan/Ethylacetat (40:60→20:80) als Laufmittel chromatographiert. Das Chromatographieprodukt wird mit tert-Butylmethylether verrührt, abgesaugt und getrocknet.
Ausbeute: 104 mg (56 % der Theorie)
R_{f}-Wert: 0,22 (Kieselgel, Petrolether/Ethylacetat = 1:2)
Massenspektrum (ESI⁺): m/z = 542 [M+H]⁺

Analog Beispiel 7 werden folgende Verbindungen erhalten:
(1) N-(5-tert-Butyl-3-methansulfonylamino-2-methoxy-phenyl)-4-methyl-3-[6-(morpholin-4-carbonyl)-pyrimidin-4-yloxy]-benzamid 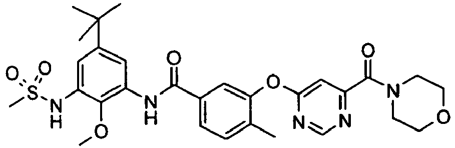 R_{f}-Wert: 0,55 (Kieselgel, Dichlormethan/Methanol = 95:5)
   Massenspektrum (ESI⁺): m/z = 598 [M+H]⁺
(2) N-(5-tert-Butyl-3-methansulfonylamino-2-methoxy-phenyl)-4-methyl-3-[6-(4-methylpiperazin-1-carbonyl)-pyrimidin-4-yloxy]-benzamid 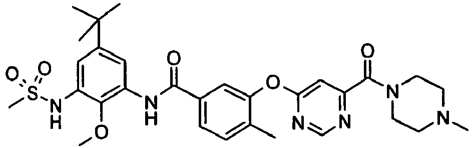 R_{f}-Wert: 0,45 (Kieselgel, Dichlormethan/Methanol = 90:10)
   Massenspektrum (ESI⁺): m/z = 611 [M+H]⁺
(3) N-(5-tert-Butyl-3-methansulfonylamino-2-methoxy-phenyl)-3-fluor-4-methyl-5-[2-(4-methyl-piperazin-1-ylmethyl)-pyrimidin-4-yloxy]-benzamid 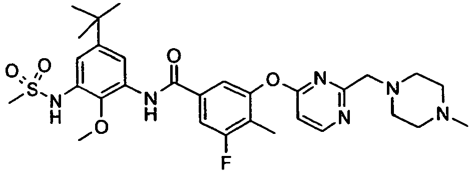 (Umsetzung erfolgt in Dimethylsulfoxid bei Raumtemperatur)
   R_{f}-Wert: 0,4 (Kieselgel, Dichlormethan/Methanol/konz. Ammoniak = 95:5:0,1) Massenspektrum (ESI⁺): m/z = 615 [M+H]⁺
(4) N-(5-tert-Butyl-3-methansulfonylamino-2-methoxy-phenyl)-4-chlor-3-[2-(4-methylpiperazin-1-ylmethyl)-pyrimidin-4-yloxy]-benzamid 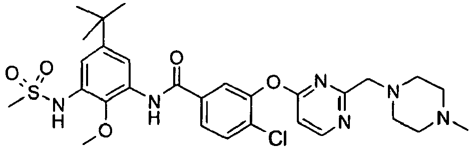 (Umsetzung erfolgt in Dimethylsulfoxid bei Raumtemperatur)
   R_{f}-Wert: 0,4 (Kieselgel, Dichlormethan/Methanol/konz. Ammoniak = 95:5:0,1)
   Massenspektrum (ESI⁺): m/z = 617/619 (Cl) [M+H]⁺
(5) N-(5-tert-Butyl-3-methansulfonylamino-2-methoxy-phenyl)-3-chlor-4-methyl-5-[2-(4-methyl-piperazin-1-ylmethyl)-pyrimidin-4-yloxy]-benzamid 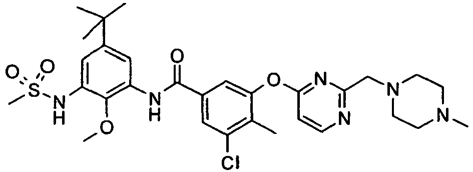 (Umsetzung erfolgt in Dimethylsulfoxid bei Raumtemperatur)
   R_{f}-Wert: 0,3 (Kieselgel, Dichlormethan/Methanol/konz. Ammoniak = 95:5:0,1)
   Massenspektrum (ESI⁺): m/z = 631/633 (Cl) [M+H]⁺

### Beispiel 8

### N-(5-tert-Butyl-3-methansulfonylamino-2-methoxy-phenyl)-4-methyl-3-(6-morpholin-4-ylmethyl-pyrimidin-4-yloxy)-benzamid

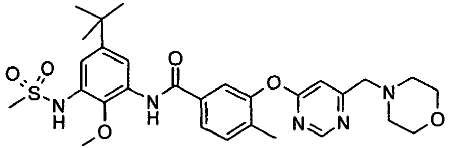

Zu 200 mg N-(5-tert-Butyl-3-methansuffonylamino-2-methoxy-phenyl)-3-(6-formyl-pyrimidin-4-yloxy)-4-methyl-benzamid in 4 ml 1,2-Dichlorethan werden 114 µl Eisessig und 171 µl Morpholin gegeben, und das Reaktionsgemisch wird 20 Minuten bei Raumtemperatur gerührt. Nun werden 107 mg Natriumtriacetoxyborhydrid zugegeben, und das Gemisch wird über Nacht bei Raumtemperatur gerührt. Dann wird das Reaktionsgemisch mit gesättigter wässriger Natriumhydrogencarbonat-Lösung versetzt, fünf Minuten gerührt und anschließend mit Dichlormethan extrahiert. Die vereinigten organischen Phasen werden eingeengt und der Kolbenrückstand wird über eine Kieselgelsäule mit Cyclohexan/Ethylacetat (50:50→5:95) gefolgt von Ethylacetat/Methanol (95:5→80:20) als Laufmittel chromatographiert.
Ausbeute: 20 mg (11 % der Theorie)
R_{f}-Wert: 0,8 (Kieselgel, Dichlormethan/Methanol/konz. Ammoniak = 90:10:1)
Massenspektrum (ESI⁺): m/z = 584 [M+H]⁺

Analog Beispiel 8 werden folgende Verbindungen erhalten:
(1) N-(5-tert-Butyl-3-methansulfonylamino-2-methoxy-phenyl)-4-methyl-3-(6-methylaminomethyl-pyrimidin-4-yloxy)-benzamid 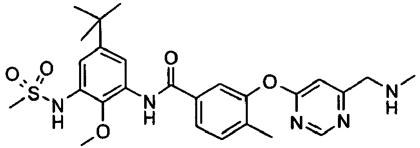 R_{f}-Wert: 0,5 (Kieselgel, Dichlormethan/Methanol/konz. Ammoniak = 90:10:1)
   Massenspektrum (ESI⁺): m/z = 528 [M+H]⁺
(2) N-(5-tert-Butyl-3-methansulfonylamino-2-methoxy-phenyl)-4-methyl-3-[6-(4-methylpiperazin-1-ylmethyl)-pyrimidin-4-yloxy]-benzamid 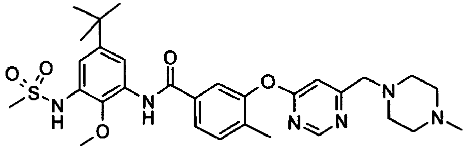 R_{f}-Wert: 0,5 (Kieselgel, Dichlormethan/Methanol/konz. Ammoniak = 90:10:1)
   Massenspektrum (ESI⁺): m/z = 597 [M+H]⁺
(3) N-(5-tert-Butyl-3-methansulfonylamino-2-methoxy-phenyl)-4-methyl-3-(2-morpholin-4-ylmethyl-pyrimidin-4-yloxy)-benzamid 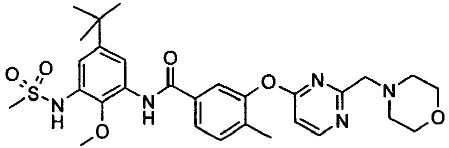 R_{f}-Wert: 0,4 (Kieselgel, Dichlormethan/Methanol = 95:5)
   Massenspektrum (ESI⁺): m/z = 584 [M+H]⁺
(4) N-(5-tert-Butyl-3-methansulfonylamino-2-methoxy-phenyl)-4-methyl-3-(2-methylaminomethyl-pyrimidin-4-yloxy)-benzamid 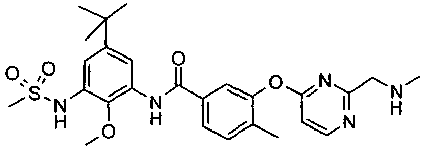 R_{f}-Wert: 0,25 (Kieselgel, Dichlormethan/Methanol/konz. Ammoniak = 95:5:0,1)
   Massenspektrum (ESI⁺): m/z = 528 [M+H]⁺
(5) N-(5-tert-Butyl-3-methansulfonylamino-2-methoxy-phenyl)-4-methyl-3-[2-(3-oxo-piperazin-1-ylmethyl)-pyrimidin-4-yloxy]-benzamid 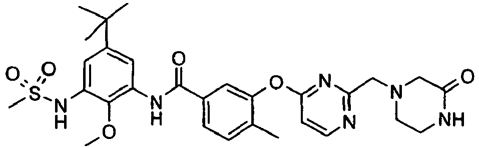 R_{f}-Wert: 0,10 (Kieselgel, Dichlormethan/Methanol = 95:5)
   Massenspektrum (ESI⁺): m/z = 597 [M+H]⁺
(6) N-(5-tert-Butyl-3-methansulfonylamino-2-methoxy-phenyl)-4-methyl-3-[2-(4-methylpiperazin-1-ylmethyl)-pyrimidin-4-yloxy]-benzamid 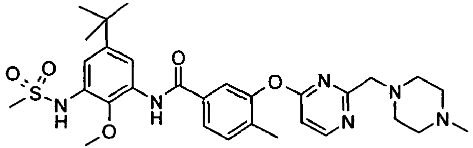 R_{f}-Wert: 0,25 (Kieselgel, Dichtormethan/Methanot/konz. Ammoniak = 95:5:0,1)
   Massenspektrum (ESI⁻): m/z = 595 [M-H]⁻
(7) N-(5-tert-Butyl-3-methansulfonylamino-2-methoxy-phenyl)-4-methyl-3-[6-(3-oxo-piperazin-1-ylmethyl)-pyrimidin-4-yloxy]-benzamid 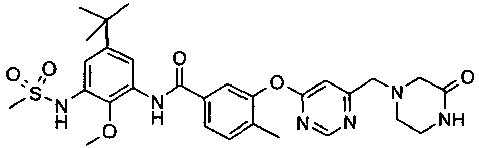 R_{f}-Wert: 0,5 (Kieselgel, Dichlormethan/Methanol/konz. Ammoniak = 90:10:1)
   Massenspektrum (ESI⁺): m/z = 597 [M+H]⁺
(9) N-(5-tert-Butyl-3-methansulfonylamino-2-methoxy-phenyl)-4-methyl-3-(2-morpholin-4-ylmethyl-pyridin-4-yloxy)-benzamid 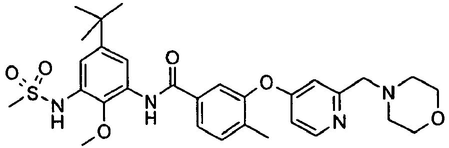 R_{f}-Wert: 0,5 (Kieselgel, Dichlormethan/Methanol/konz. Ammoniak = 90:10:1)
   Massenspektrum (ESI⁺): m/z = 583 [M+H]⁺
(10) N-(5-tert-Butyl-3-methansulfonylamino-2-methoxy-phenyl)-4-methyl-3-[2-(4-methyl-piperazin-1-ylmethyl)-pyridin-4-yloxy]-benzamid 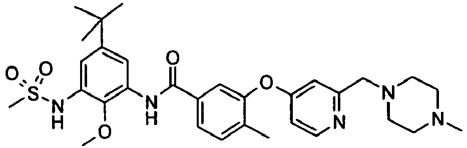 R_{f}-Wert: 0,3 (Kieselgel, Dichlormethan/Methanol/konz. Ammoniak = 90:10:1)
   Massenspektrum (ESI⁺): m/z = 596 [M+H]⁺
(11) (S)-N-(5-tert-Butyl-3-methansulfonylamino-2-methoxy-phenyl)-4-methyl-3-{2-[(tetrahydro-furan-3-ylamino)-methyl]-pyridin-4-yloxy}-benzamid 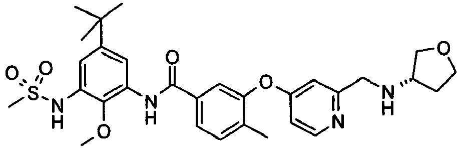 R_{f}-Wert: 0,4 (Kieselgel, Dichlormethan/methanol/konz. Ammoniak = 90:10:1)
   Massenspektrum (ESI⁺): m/z = 583 [M+H]⁺
(12) (R)-N-(5-tert-Butyl-3-methansulfonylamino-2-methoxy-phenyl)-3-[2-(3-hydroxy-pyrrolidin-1-ylmethyl)-pyridin-4-yloxy]-4-methyl-benzamid 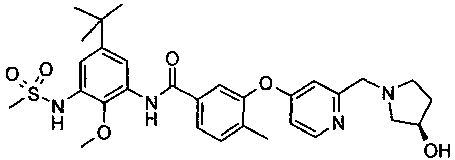 R_{f}-Wert: 0,4 (Kieselgel, Dichlormethan/Methanol/konz. Ammoniak = 90:10:1)
   Massenspektrum (ESI⁺): m/z = 583 [M+H]⁺
(13) (S)-N-(5-tert-Butyl-3-methansulfonylamino-2-methoxy-phenyl)-3-[2-(3-hydroxy-pyrrolidin-1-ylmethyl)-pyridin-4-yloxy]-4-methyl-benzamid 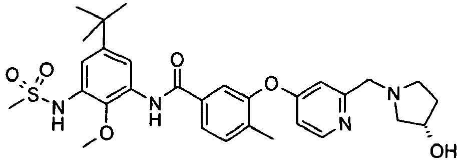 R_{f}-Wert: 0,4 (Kieselgel, Dichlormethan/Methanol/konz. Ammoniak = 90:10:1)
   Massenspektrum (ESI⁺): m/z = 583 [M+H]⁺
(14) N-(5-tert-Butyl-3-methansulfonylamino-2-methoxy-phenyl)-4-methyl-3-(2-methylaminomethyl-pyridin-4-yloxy)-benzamid 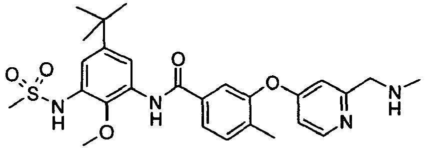 R_{f}-Wert: 0,2 (Kieselgel, Dichlormethan/Methanol/konz. Ammoniak = 90:10:1)
   Massenspektrum (ESI⁺): m/z = 527 [M+H]⁺
(15) (R)-N-(5-tert-Butyl-3-methansuffonylamino-2-methoxy-phenyl)-4-methyl-3-{2-[(tetrahydro-furan-3-ylamino)-methyl]-pyridin-4-yloxy}-benzamid 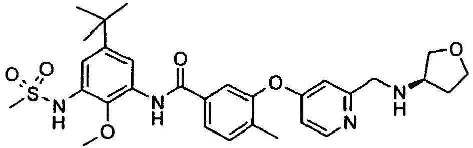 Massenspektrum (ESI⁺): m/z = 583 [M+H]⁺
(16) N-(5-tert-Butyl-3-methansulfonylamino-2-methoxy-phenyl)-4-methyl-3-[2-(4-methyl-homopiperazin-1-ylmethyl)-pyrimidin-4-yloxy]-benzamid 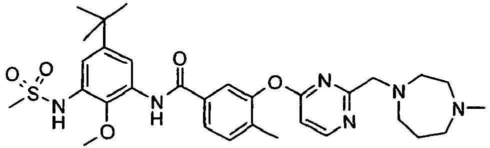 R_{f}-Wert: 0,2 (Kieselgel, Dichlormethan/Methanol/konz. Ammoniak = 95:5:0,1)
   Massenspektrum (ESI⁺): m/z = 611 [M+H]⁺
(17) N-(5-tert-Butyl-3-methansulfonylamino-2-methoxy-phenyl)-4-methyl-3-{2-[(1-methyl-piperidin-4-ylamino)-methyl]-pyrimidin-4-yloxy}-benzamid 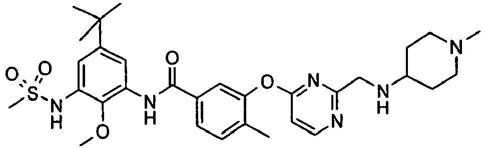 R_{f}-Wert: 0,2 (Kieselgel, Dichlormethan/Methanol/konz. Ammoniak = 95:5:0,1)
   Massenspektrum (ESI⁺): m/z = 611 [M+H]⁺
(18) N-(5-tert-Butyl-3-methansulfonylamino-2-methoxy-phenyl)-4-methyl-3-{2-[(1-methyl-piperidin-3-ylamino)-methyl]-pyrimidin-4-yloxy}-benzamid 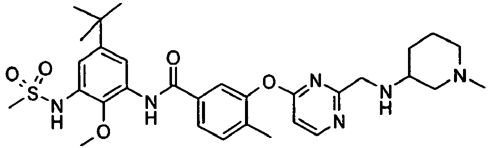 R_{f}-Wert: 0,45 (Kieselgel, Dichlormethan/Methanol/konz. Ammoniak = 95:5:0,1)
   Massenspektrum (ESI⁺): m/z = 611 [M+H]⁺
(19) N-(5-tert-Butyl-3-methansulfonylamino-2-methoxy-phenyl)-4-methyl-3-(2-{[methyl-(1-methyl-pyrrolidin-3-yl)-amino]-methyl}-pyrimidin-4-yloxy)-benzamid 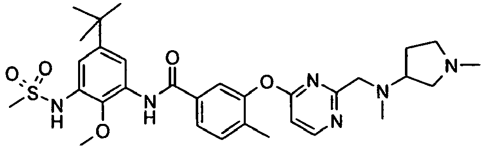 R_{f}-Wert: 0,3 (Kieselgel, Dichlormethan/Methanol/konz. Ammoniak = 95:5:0,1)
   Massenspektrum (ESI⁺): m/z = 611 [M+H]⁺
(20) N-(5-tert-Butyl-3-methansuffonylamino-2-methoxy-phenyl)-4-methyl-3-[2-(4-methyl-homopiperazin-1-ylmethyl)-pyridin-4-yloxy]-benzamid 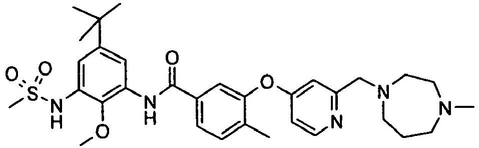 R_{f}-Wert: 0,4 (Kieselgel, Dichlormethan/Methanol/konz. Ammoniak = 90:10:1)
   Massenspektrum (ESI⁺): m/z = 610 [M+H]⁺
(21) (S)-N-(5-tert-Butyl-3-methansulfonylamino-2-methoxy-phenyl)-3-[2-(3-dimethylamino-pyrrolidin-1-ylmethyl)-pyrimidin-4-yloxy]-4-methyl-benzamid 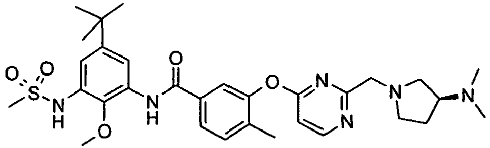 Massenspektrum (ESI⁺): m/z = 611 [M+H]⁺
(22) (R)-N-(5-tert-Butyl-3-methansulfonylamino-2-methoxy-phenyl)-3-[2-(3-dimethylamino-pyrrolidin-1-ylmethyl)-pyrimidin-4-yloxy]-4-methyl-benzamid 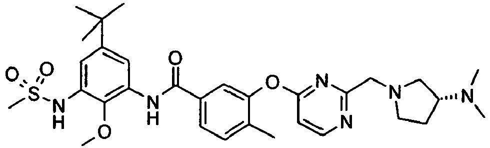 Massenspektrum (ESI⁺): m/z = 611 [M+H]⁺
(23) N-(5-tert-Butyl-3-methansulfonylamino-2-methoxy-phenyl)-4-methyl-3-{2-[(1-methyl-pyrrolidin-3-ylamino)-methyl]-pyrimidin-4-yloxy}-benzamid 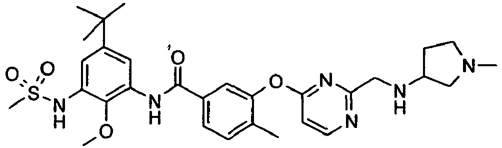 R_{f}-Wert: 0,25 (Kieselgel, Dichlormetha/Methanol/konz. Ammoniak = 90:10:0,1)
   Massenspektrum (ESI⁺): m/z = 597 [M+H]⁺
(24) N-(5-tert-Butyl-3-methansulfonylamino-2-methoxy-phenyl)-3-(2-{[N-(2-dimethylamino-ethyl)-N-methyl-amino]-methyl}-pyrimidin-4-yloxy)-4-methyl-benzamid 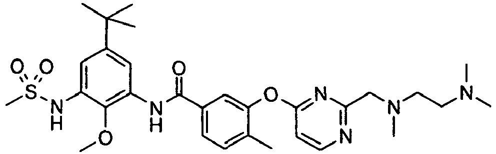 HPLC (Methode 1): Retentionszeit = 2,63 min
   Massenspektrum (ESI⁺): m/z = 599 [M+H]⁺
(25) N-(5-tert-Butyl-3-methansulfonylamino-2-methoxy-phenyl)-3-{2-[(2-dimethylamino-ethylamino)-methyl]-pyrimidin-4-yloxy}-4-methyl-benzamid 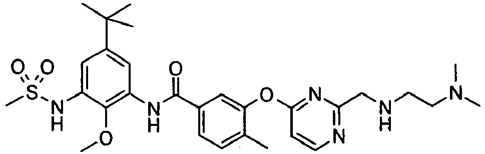 HPLC (Methode 1): Retentionszeit = 2,32 min
   Massenspektrum (ESI⁺): m/z = 585 [M+H]⁺
(26) N-(5-tert-Butyl-3-methansulfonylamino-2-methoxy-phenyl)-3-(2-{[(2-dimethylamino-ethyl)-methyl-amino]-methyl}-pyridin-4-yloxy)-4-methyl-benzamid 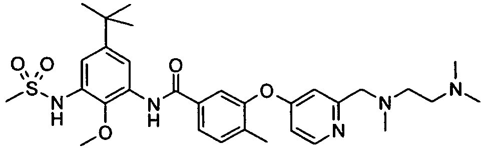 HPLC (Methode 1): Retentionszeit = 2,41 min
   Massenspektrum (ESI⁺): m/z = 598 [M+H]⁺
(27) N-(5-tert-Butyl-3-methansulfonylamino-2-methoxy-phenyl)-3-{2-[(2-dimethylamino-ethylamino)-methyl]-pyridin-4-yloxy}-4-methyl-benzamid 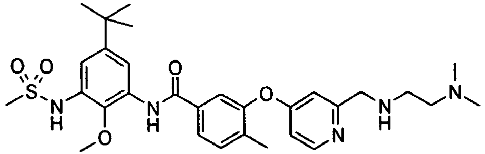 HPLC (Methode 1): Retentionszeit = 2,39 min
   Massenspektrum (ESI⁺): m/z = 584 [M+H]⁺
(28) N-(5-tert-Butyl-3-methansulfonylamino-2-methoxy-phenyl)-3-methyl-5-[2-(4-methyl-piperazin-1-ylmethyl)-pyrimidin-4-yloxy]-benzamid 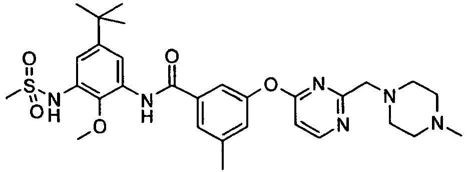 R_{f}-Wert: 0,55 (Kieselgel, Dichlormethan/Methanol = 90:10)
   Massenspektrum (ESI⁺): m/z = 597 [M+H]⁺
(29) N-(5-tert-Butyl-3-methansulfonylamino-2-methoxy-phenyl)-3-{6-[(2-dimethylamino-ethylamino)-methyl]-pyrimidin-4-yloxy}-4-methyl-benzamid 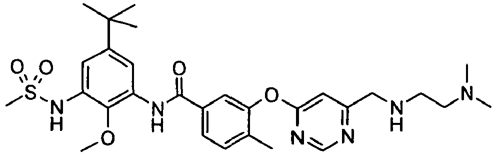 HPLC (Methode 1): Retentionszeit = 2,38 min
   Massenspektrum (ESI⁺): m/z = 585 [M+H]⁺
(30) N-(5-tert-Butyl-3-methansulfonylamino-2-methoxy-phenyl)-3-(6-{[(2-dimethylamino-ethyl)-methyl-amino]-methyl}pyrimidin-4-yloxy)-4-methyl-benzamid 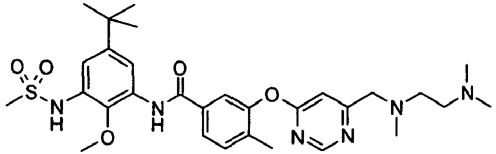 HPLC (Methode 1): Retentionszeit = 2,73 min
   Massenspektrum (ESI⁺): m/z = 599 [M+H]⁺
(31) N-(5-tert-Butyl-3-methansuffonylamino-2-methoxy-phenyl)-4-chlor-3-{6-[(2-dimethylamino-ethylamino)-methyl]-pyrimidin-4-yloxy}-benzamid 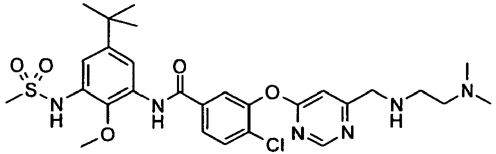 R_{f}-Wert: 0,4 (Kieselgel, Dichlormethan/Methanol/methanolischer Ammoniak = 95:5:0,1)
   Massenspektrum (ESI⁺): m/z = 605/607 (Cl) [M+H]⁺
(32) N-(5-tert-Butyl-3-methansulfonylamino-2-methoxy-phenyl)-4-chlor-3-(6-{[(2-dimethylamino-ethyl)-methyl-amino]-methyl}-pyrimidin-4-yloxy)-benzamid 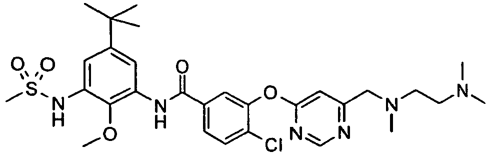 R_{f}-Wert: 0.4 (Kieselgel, Dichlormethan/Methanol/methanolischer Ammoniak = 95:5:0,1)
   Massenspektrum (ESI⁺): m/z = 619/621 (Cl) [M+H]⁺
(33) N-(5-tert-Butyl-3-methansulfonylamino-2-methoxy-phenyl)-4-chlor-3-(2-{[(2-dimethylamino-ethyl)-methyl-amino]-methyl}-pyridin-4-yloxy)-benzamid 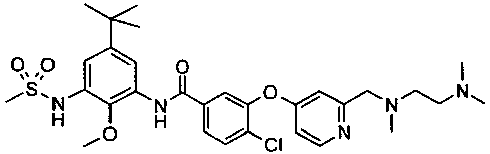 R_{f}-Wert: 0,35 (Kieselgel, Dichlormethan/Methanol/methanolischer Ammoniak = 95:5:0,1)
   Massenspektrum (ESI⁺): m/z = 618/620 (Cl) [M+H]⁺
(34) N-(5-tert-Butyl-3-methansulfonylamino-2-methoxy-phenyl)-4-chlor-3-{2-[(2-dimethylamino-ethylamino)-methyl]-pyridin-4-yloxy}-benzamid 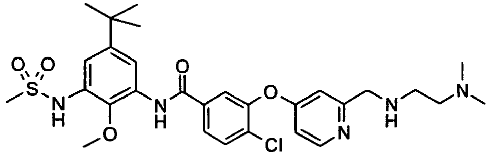 R_{f}-Wert: 0,3 (Kieselgel, Dichlormethan/Methanol/methanolischer Ammoniak = 95:5:0,1) .
   Massenspektrum (ESI⁺): m/z = 604/606 (Cl) [M+H]⁺
(35) N-(5-tert-Butyl-3-methansuffonylamino-2-methoxy-phenyl)-4-chlor-3-[2-(4-methyl-[1,4]diazepan-1-ylmethyl)-pyrimidin-4-yloxy]-benzamid 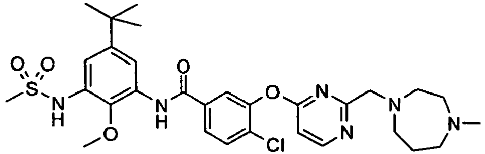 R_{f}-Wert: 0,2 (Kieselgel, Dichlormethan/Methanol/methanolischer Ammoniak = 95:5:0,1)
   Massenspektrum (ESI⁺): m/z = 631/633 (Cl) [M+H]⁺
(36) N-(5-tert-Butyl-3-methansulfonylamino-2-methoxy-phenyl)-4-chlor-3-(2-{[(2-dimethylamino-ethyl)-methyl-amino]-methyl}-pyrimidin-4-yloxy)-benzamid 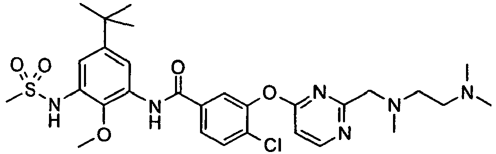 R_{f}-Wert: 0,25 (Kieselgel, Dichlormethan/Methanol/methanolischer Ammoniak = 95:5:0,1)
   Massenspektrum (ESI⁺): m/z = 619/621 (Cl) [M+H]⁺
(37) N-(5-tert-Butyl-3-methansulfonylamino-2-methoxy-phenyl)-4-chlor-3-{2-[(2-dimethylamino-ethylamino)-methyl]-pyrimidin-4-yloxy}-benzamid 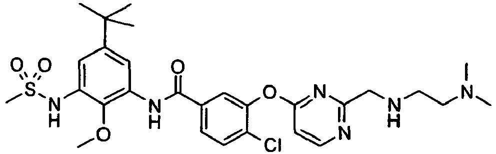 R_{f}-Wert: 0,2 (Kieselgel, Dichlormethan/Methanol/methanolischer Ammoniak = 95:5:0,1)
   Massenspektrum (ESI⁺): m/z = 605/607 (Cl) [M+H]⁺
(38) N-(5-tert-Butyl-3-methansulfonylamino-2-methoxy-phenyl)-4-chlor-3-[6-(4-methyl-[1,4]diazepan-1-ylmethyl)-pyrimidin-4-yloxy]-benzamid 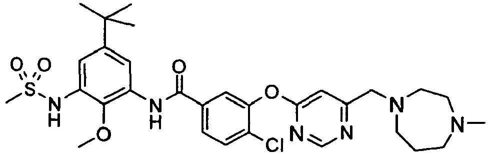 R_{f}-Wert: 0,3 (Kieselgel, Dichlormethan/Methanol/methanolischer Ammoniak = 95:5:0,1)
   Massenspektrum (ESI⁺): m/z = 631/633 (Cl) [M+H]⁺
(39) N-(5-tert-Butyl-3-methansulfonylamino-2-methoxy-phenyl)-4-chlor-3-[2-(4-methyl-[1,4]diazepan-1-ylmethyl)-pyridin-4-yloxy]-benzamid 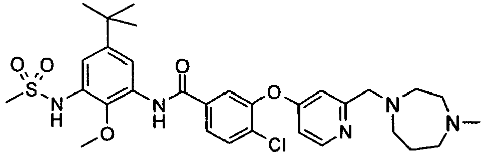 R_{f}-Wert: 0,1 (Kieselgel, Dichlormethan/Methanol/methanolischer Ammoniak = 95:5:0,1)
   Massenspektrum (ESI⁺): m/z = 630/632 (Cl) [M+H]⁺

### Beispiel 9

### N-(5-tert-Butyl-3-methansulfonylamino-2-methoxy-phenyl)-4-methyl-3-[6-(1-methylpiperidin-4-ylmethyl)-pyrimidin-4-yloxy]-benzamid

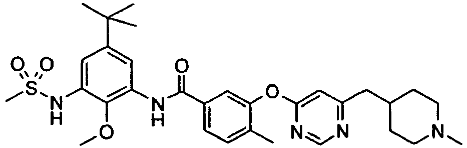

Zu 260 mg N-(5-tert-Butyl-3-methansutfonylamino-2-methoxy-phenyl)-4-methyl-3-(6-piperidin-4-ylmethyl-pyrimidin-4-yloxy)-benzamid in 10 ml Methanol werden 40 µl Paraformaldehyd (37%ig in Wasser) und 30 mg Palladium auf Aktivkohle (10%ig) gegeben, und das Reaktionsgemisch wird bei Raumtemperatur und einem Wasserstoffpartialdruck von 4 bar hydriert. Nach beendeter Wasserstoffaufnahme wird der Katalysator abgesaugt und das Filtrat eingeengt. Der Kolbenrückstand wird über Aluminiumoxid mit Dichlormethan/Methanol (99:1→95:5) als Laufmittel chromatographiert.
Ausbeute: 172 mg (65 % der Theorie)
HPLC (Methode 1): Retentionszeit = 2,69 min
Massenspektrum (ESI⁺): m/z = 596 [M+H]⁺

Analog Beispiel 9 werden folgende Verbindungen erhalten:
(1) N-(5-tert-Butyl-3-methansulfonylamino-2-methoxy-phenyl)-4-methyl-3-[2-(1-methylpiperidin-4-ylmethyl)-pyrimidin-4-yloxy]-benzamid 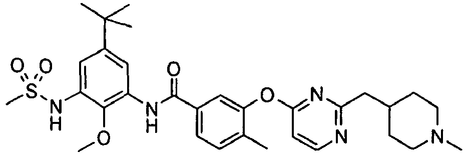 HPLC (Methode 1): Retentionszeit = 2,78 min
   Massenspektrum (ESI⁺): m/z = 596 [M+H]⁺
(2) N-(5-tert-Butyl-3-methansulfonylamino-2-methoxy-phenyl)-4-methyl-3-[2-(1-methylpiperidin-4-ylmethyl)-pyridin-4-yloxy]-benzamid 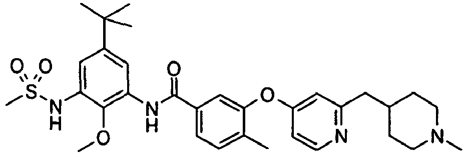 HPLC (Methode 1): Retentionszeit = 2,27 min
   Massenspektrum (ESI⁺): m/z = 595 [M+H]⁺
(3) N-(5-tert-Butyl-3-hydroxymethyl-2-methoxy-phenyl)-4-methyl-3-[2-(4-methylpiperazin-1-ylmethyl)-pyridin-4-yloxy]-benzamid 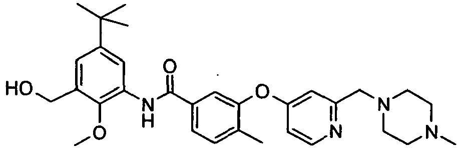 Massenspektrum (ESI⁺): m/z = 533 [M+H]⁺
(4) N-(5-tert-Butyl-3-hydroxymethyl-2-methoxy-phenyl)-4-methyl-3-[2-(4-methyl-[1,4]diazepan-1-ylmethyl)-pyridin-4-yloxy]-benzamid 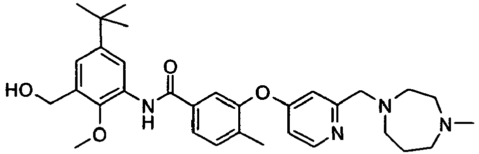 Massenspektrum (ESI⁺): m/z = 547 [M+H]⁺

### Beispiel 10

### N-(5-tert-Butyl-3-methansulfonylamino-2-methoxy-phenyl)-4-chlor-3-12-(1-methylpiperidin-4-ylamino)-pyridin-4-yloxyl-benzamid

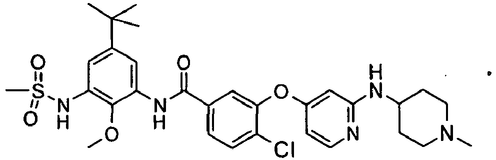

48 µl 2,8,9-Triisobutyl-2,5,8,9-tetraaza-l-phosphabicyclo[3.3.3]undekan und 31 mg Pd₂(Dibenzylidenaceton)₃ werden zu einer unter Argonatmosphäre gehaltenen Mischung aus 300 mg N-(5-tert-Butyl-3-methansulfonylamino-2-methoxy-phenyl)-4-chlor-3-(2-chlor-pyridin-4-yloxy)-benzamid, 110 mg 1-Methyl-piperidin-4-ylamin und 250 mg KO'Bu in 5 ml Toluol gegeben. Das Gemisch wird auf 105°C erwärmt und über Nacht bei dieser Temperatur gerührt. Danach wird auf Raumtemperatur abgekühlt und Ethylacetat zugegeben. Das Gemisch wird dann mit wenig Wasser gewaschen und dann zweimal mit 1 M wässriger Salzsäure extrahiert. Die vereinigten wässrigen, sauren Extrakte werden mit wässriger K₂CO₃-Lösung alkalisch eingestellt und dann mit Ethylacetat extrahiert. Die vereinigten organischen Extrakte werden mit gesättigter Kochsalzlösung gewaschen, getrocknet (Na₂SO₄) und dann vom Lösungsmittel befreit. Der Kolbenrückstand wird über Kieselgel mit Dichlormethan/ Methanol/methanol. Ammoniak (97:2:1→96:3:1) als Laufmittel chromatographiert.
Ausbeute: 18 mg (5 % der Theorie)
R_{f}-Wert: 0,3 (Kieselgel, Dichtormethan/Methanot/konz. Ammoniak = 95:5:0,1)
Massenspektrum (ESI⁺): m/z = 616/618 (Cl) [M+H]⁺

### Beispiel 11

### N-(5-tert-Butyl-3-methansulfonylamino-2-methoxy-phenyl)-4-chlor-3-[6-(piperidin-4-ylamino)-pyrimidin-4-yloxy]-benzamid

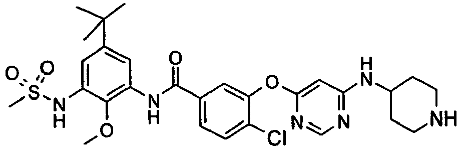

1 mL 5-6 M isopropanolische Salzsäure wird bei Raumtemperatur zu einer Lösung von 138 mg 4-{6-[5-(5-tert-Butyl-3-methansulfonylamino-2-methoxy-phenylcarbamoyl)-2-chlor-phenoxy]-pyrimidin-4-ylamino}-piperidin-1-carbonsäure-tert-butyl-ester in 2 ml Dichlormethan gegeben. Die Lösung wird über Nacht bei Raumtemperatur gerührt und dann eingeengt. Der Rückstand wird in Wasser aufgenommen und die Lösung mit wässriger K₂CO₃-Lösung alkalisch eingestellt. Die alkalische Lösung wird mit einem ca. 20:1 Dichlormethan/Methanol-Gemisch extrahiert, und die vereinigten Extrakte werden getrocknet (MgO₄) und eingeengt. Der Rückstand wird mit Diethylether verrrührt und dann getrocknet.
Ausbeute: 105 mg (89 % der Theorie)
R_{f}-Wert: 0,1 (Kieselgel, Dichlormethan/Methanol/methanolischer Ammoniak = 95:5:0.1)
Massenspektrum (ESI⁺): m/z = 603/605 (Cl) [M+H]⁺

Analog Beispiel 11 werden folgende Verbindungen erhalten:
(1) N-[3-(Butan-1-sulfonylamino)-5-tert-butyl-2-methoxy-phenyl]-4-methyl-3-(2-piperidin-4-ylmethyl-pyridin-4-yloxy)-benzamid 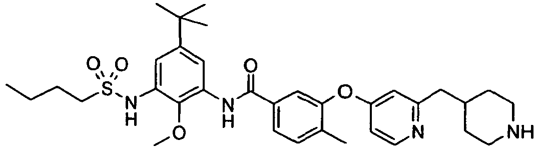 HPLC (Methode 1): Retentionszeit = 2,81 min
   Massenspektrum (ESI⁺): m/z = 623 [M+H]⁺
(2) N-(5-tert-Butyl-2-methoxy-3-pentanoylamino-phenyl)-4-methyl-3-(2-piperidin-4-ylmethyl-pyridin-4-yloxy)-benzamid 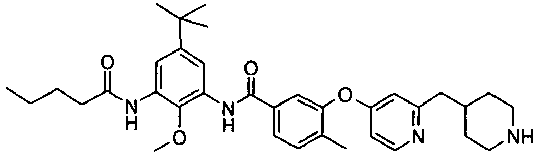 HPLC (Methode 1): Retentionszeit = 2,69 min
   Massenspektrum (ESI⁺): m/z = 587 [M+H]⁺
(3) N-(5-tert-Butyl-3-methansulfonyl-2-methoxy-phenyl)-4-methyl-3-(2-piperidin-4-ylmethyl-pyridin-4-yloxy)-benzamid 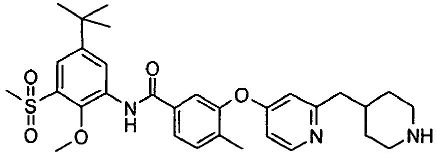 HPLC (Methode 1): Retentionszeit = 2,46 min
   Massenspektrum (ESI⁺): m/z = 566 [M+H]⁺
(4) N-[5-tert-Butyl-2-methoxy-3-(3-methyl-butyrylamino)-phenyl]-4-methyl-3-(2-piperidin-4-ylmethyl-pyridin-4-yloxy)-benzamid 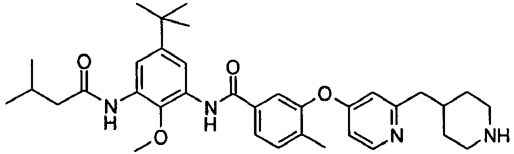 HPLC (Methode 1): Retentionszeit = 2,65 min
   Massenspektrum (ESI⁺): m/z = 587 [M+H]⁺
(5) N-(5-tert-Butyl-3-dimethylaminomethyl-2-methoxy-phenyl)-4-methyl-3-(2-piperidin-4-ylmethyl-pyridin-4-yloxy)-benzamid 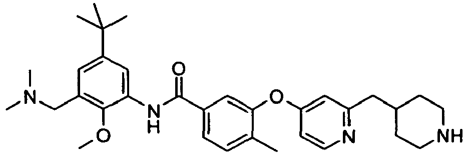 HPLC (Methode 3): Retentionszeit = 4,40 min
   Massenspektrum (ESI⁺): m/z = 545 [M+H]⁺
(6) N-(5-tert-Butyl-2-methoxy-3-pyrrolidin-1-ylmethyl-phenyl)-4-methyl-3-(2-piperidin-4-ylmethyl-pyridin-4-yloxy)-benzamid 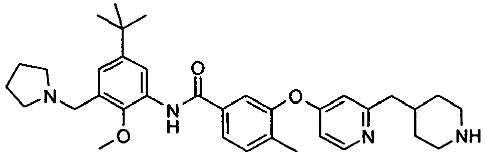 HPLC (Methode 1): Retentionszeit = 1,94 min
   Massenspektrum (ESI⁺): m/z = 571 [M+H]⁺
(7) N-(5-tert-Butyl-3-cyclopropylaminomethyl-2-methoxy-phenyl)-4-methyl-3-(2-piperidin-4-ylmethyl-pyridin-4-yloxy)-benzamid 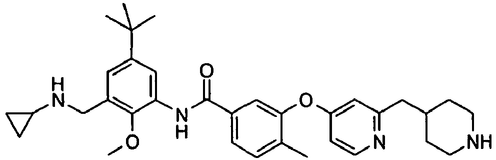 Massenspektrum (ESI⁺): m/z = 557 [M+H]⁺

### Beispiel 12

### N-(5-tert-Butyl-3-methansulfonylamino-2-methoxy-phenyl)-4-methyl-3-[2-(1-methyl-azepan-4-ylmethyl)-pyridin-4-yloxy]-benzamid

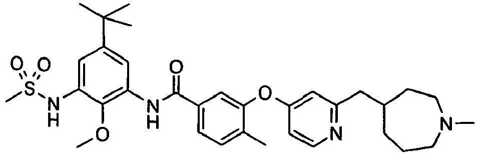

Bei Raumtemperatur werden 141 mg Natrium-triacetoxyborhydrid zu einer Lösung von 330 mg 3-(2-Azepan-4-ylmethyl-pyridin-4-yloxy)-N-(5-tert-butyl-3-methansulfonyl-amino-2-methoxy-phenyl)-4-methyl-benzamid und 50 µl einer 37%igen wässrigen Formaldehyd-Lösung in 5 ml Methanol gegeben. Dann wird die Lösung eingeengt und der Rückstand in Ethylacetat aufgenommen. Dir organische Phase wird mit wässriger Natriumcarbonatlösung und gesättigter wässriger Kochsalzlösung gewaschen und danach getrocknet (Na₂SO₄). Das Lösungsmittel wird entfernt und der Kolbenrückstand wird über Kieselgel mit Dichlormethan/Methanol/methanolischer Ammoniak-Lösung (94:5:1→90:9:1) als Laufmittel chromatographiert.
Ausbeute: 226 mg (67 % der Theorie)
R_{f}-Wert: 0,25 (Kieselgel, Dichlormethan/Methanol/methanolischer Ammoniak = 90:10:0,1)
Massenspektrum (ESI⁺): m/z = 609 [M+H]⁺

Analog Beispiel 12 werden folgende Verbindungen erhalten:
(1) N-(5-tert-Butyl-3-methansulfonylamino-2-methoxy-phenyl)-4-chlor-3-[6-(4-methylpiperazin-1-ylmethyl)-pyrimidin-4-yloxy]-benzamid 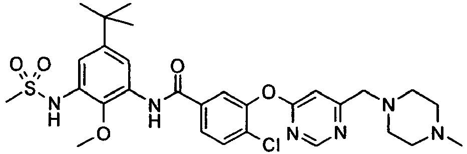 R_{f}-Wert: 0,4 (Kieselgel, Dichlormethan/Methanol/methanolischer Ammoniak = 95:5:0,1)
   Massenspektrum (ESI⁺): m/z = 617/619 (Cl) [M+H]⁺
(2) N-(5-tert-Butyl-3-methansulfonylamino-2-methoxy-phenyl)-4-chlor-3-[6-(1-methylpiperidin-4-ylmethyl)-pyrimidin-4-yloxy]-benzamid 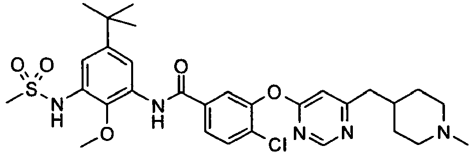 R_{f}-Wert: 0,35 (Kieselgel, Dichlormethan/Methanol/methanolischer Ammoniak = 95:5:0,1)
   Massenspektrum (ESI⁺): m/z = 616/618 (Cl) [M+H]⁺
(3) N-(5-tert-Butyl-3-methansulfonylamino-2-methoxy-phenyl)-4-chlor-3-[2-(1-methylpiperidin-4-ylmethyl)-pyrimidin-4-yloxy]-benzamid 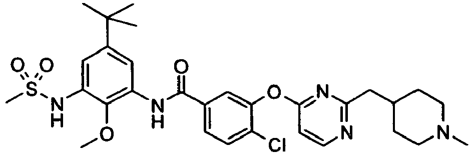 R_{f}-Wert: 0,3 (Kieselgel, Dichlormethan/Methanol/methanolischer Ammoniak = 95:5:0,1)
   Massenspektrum (ESI⁺): m/z = 616/618 (Cl) [M+H]⁺
(4) N-(5-tert-Butyl-3-methansulfonylamino-2-methoxy-phenyl)-4-chlor-3-[2-(4-methylpiperazin-1-ylmethyl)-pyridin-4-yloxy]-benzamid 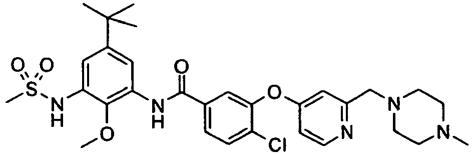 R_{f}-Wert: 0,4 (Kieselgel, Dichlormethan/Methanol/methanolischer Ammoniak = 95:5:0,1)
   Massenspektrum (ESI⁺): m/z = 616/618 (Cl) [M+H]⁺
(5) N-(5-tert-Butyl-3-methansulfonylamino-2-methoxy-phenyl)-4-chlor-3-[2-(1-methylpiperidin-4-ylmethyl)-pyridin-4-yloxy]-benzamid 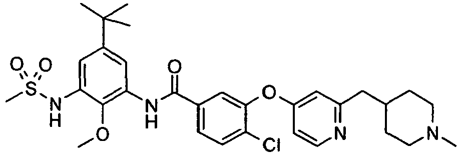 R_{f}-Wert: 0,15 (Kieselgel, Dichlormethan/Methanol/methanolischer Ammoniak = 95:5:0,1)
   Massenspektrum (ESI⁺): m/z = 615/617 (Cl) [M+H]⁺
(6) N-(5-tert-Butyl-3-methansulfonylamino-2-methoxy-phenyl)-4-methyl-3-[2-(1-methyl-azepan-4-ylmethyl)-pyrimidin-4-yloxy]-benzamid 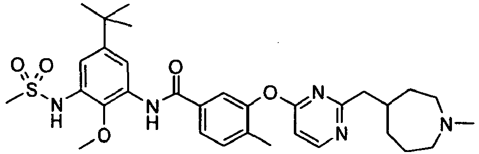 R_{f}-Wert: 0,2 (Kieselgel, Dichlormethan/Methanol/methanolischer Ammoniak = 90:10:0,1)
   Massenspektrum (ESI⁺): m/z = 610 [M+H]⁺
(7) N-(5-tert-Butyl-3-methansulfonylamino-2-methoxy-phenyl)-4-methyl-3-[6-(1-methyl-azepan-4-ylmethyl)-pyrimidin-4-yloxy]-benzamid 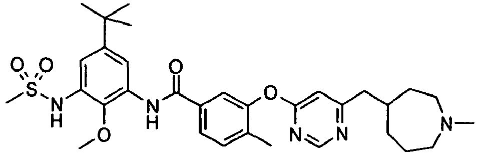 R_{f}-Wert: 0,4 (Kieselgel, Dichlormethan/Methanol/methanolischer Ammoniak = 90:10:0,1)
   Massenspektrum (ESI⁺): m/z = 610 [M+H]⁺

### Beispiel 13

### N-(5-tert-Butyl-3-hydroxymethyl-2-methoxy-phenyl)-4-methyl-3-[2-(4-methyl-piperazin-1-ylmethyl)-pyrimidin-4-yloxy]-benzamid

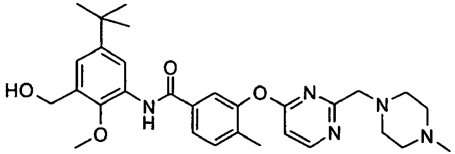

Zu 120 mg 4-Methyl-3-[2-(4-methyl-piperazin-1-ylmethyl)-pyrimidin-4-yloxy]-benzoesäure in 2 ml N,N-Dimethylformamid werden unter Argonatmosphäre 144 mg O-(7-Azabenzotriazol-1-yl)-N,N,N',N'-tetramethyl-uronium-hexafluorphosphat, 110 µl Diisopropylethylamin und 14 mg 1-Hydroxy-7-azabenzotriazol gegeben. Das Gemisch wird 15 Minuten bei Raumtemperatur gerührt, bevor 66 mg (3-Amino-5-tert-butyl-2-methoxy-phenyl)-methanol zugegeben werden. Das Reaktionsgemisch wird 3 h bei Raumtemperatur gerührt und dann mit Wasser und wässriger NaHCO₃-Lösung verdünnt. Das resultierende Gemisch wird mit Ethylacetat extrahiert, und die vereinten Extrakte werden mit gesättigter wässriger Natriumchlorid-Lösung gewaschen, über Magnesiumsulfat getrocknet und eingeengt. Der Kolbenrückstand wird über Kieselgel mit Dichlormethan/Methanol/methanolischer Ammoniak-Lösung (97:3:0→96:3:1) als Laufmittel chromatographiert.
Ausbeute: 20 mg (18% der Theorie)
R_{f}-Wert: 0,3 (Kieselgel, Dichlormethan/Methanol/methanolischer Ammoniak = 95:5:0,1)
Massenspektrum (ESI⁺): m/z = 534 [M+H]⁺

Analog Beispiel 13 wird folgende Verbindung erhalten:
(1) N-(5-tert-Butyl-3-methansulfinyl-2-methoxy-phenyl)-4-methyl-3-[2-(4-methylpiperazin-1-ylmethyl)-pyrimidin-4-yloxy]-benzamid 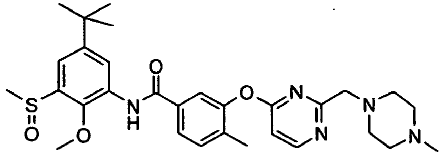 R_{f}-Wert: 0,5 (Kieselgel, Dichlormethan/Methanol/methanolischer Ammoniak = 95:5:0,1)
   Massenspektrum (ESI⁺): m/z = 566 [M+H]⁺

### Beispiel 14

### N-(5-tert-Butyl-3-methansulfonylamino-2-methoxy-phenyl)-4-methyl-3-{2-[4-(2,2,2-trifluor-ethyl)-piperazin-1-ylmethyl]-pyridin-4-yloxy}-benzamid

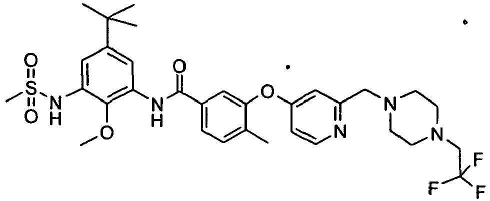

Bei Raumtemperatur werden 40 µl Trifluormethansulfonsäure-2,2,2-trifluorethylester zu einer Lösung von 149 mg N-(5-tert-Butyl-3-methansulfonylamino-2-methoxyphenyl)-4-methyl-3-(2-piperazin-1-ylmethyl-pyridin-4-yloxy)-benzamid und 100 µl Ethyldiisopropylamin in 2 ml Tetrahydrofuran gegeben. Die Lösung wird über Nacht bei Raumtemperatur gerührt, bevor 100 µl Trifluormethansulfonsäure-2,2,2-trifluorethylester zugegeben werden. Nach weiteren 24 h Rührens bei Raumtemperatur wird die Lösung mit Ethylacetat verdünnt und mit Wasser und gesättigter wässriger Kochsalzlösung gewaschen. Die organische Phase wird über Magnesiumsulfat getrocknet und eingeengt. Der Kolbenrückstand wird über Kieselgel mit Dichlormethan/Methanol (99:1→80:20) als Laufmittel chromatographiert.
Ausbeute: 89 mg (52% der Theorie)
R_{f}-Wert: 0,49 (Kieselgel, Dichlormethan/Methanol = 90:10)
Massenspektrum (ESI⁺): m/z = 664 [M+H]⁺

### Beispiel 15

### N-(5-tert-Butyl-3-methansulfonylamino-2-methoxy-phenyl)-3-[2-(4-cyanmethyl-piperazin-1-ylmethyl)-pyridin-4-yloxy]-4-methyl-benzamid

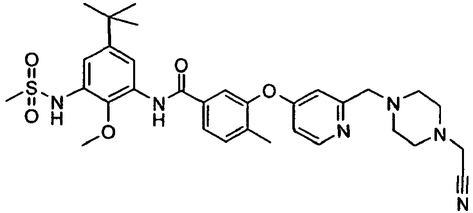

Bei Raumtemperatur werden 18 µl Bromacetonitril zu einer Mischung von 149 mg N-(5-tert-Butyl-3-methansulfonylamino-2-methoxy-phenyl)-4-methyl-3-(2-piperazin-1-ylmethyl-pyridin-4-yloxy)-benzamid, 71 mg Kaliumcarbonat und 2 ml Acetonitril gegeben. Das Gemisch wird über Nacht bei Raumtemperatur gerührt und dann mit Ethylacetat verdünnt. Das resultierende Gemisch wird mit Wasser und gesättigter wässriger Kochsalzlösung gewaschen, getrocknet (Magnesiumsulfat) und eingeengt. Der Kolbenrückstand wird über HPLC (reversed phase, XBridge C18) mit Wasser/Methanol (90:10→0:100) als Laufmittel chromatographiert.
Ausbeute: 48 mg (30% der Theorie)
R_{f}-Wert: 0,82 (Kieselgel, Dichlormethan/Methanol = 80:20)
Massenspektrum (ESI⁺): m/z = 621 [M+H]⁺

### Beispiel 16

### N-(5-tert-Butyl-3-hydroxymethyl-2-methoxy-phenyl)-4-methyl-3-(2-piperazin-1-ylmethyl-pyridin-4-yloxy)-benzamid

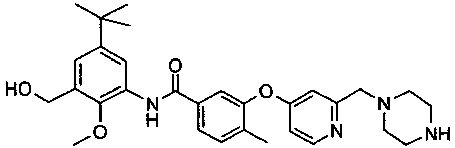

0,78 ml einer 1 M Lösung von LiAlH₄ in Tetrahydrofuran werden zu einer auf 0°C gekühlten Lösung von 210 mg 5-tert-Butyl-2-methoxy-3-[4-methyl-3-(2-piperazin-1-ylmethyl-pyridin-4-yloxy)-benzoylamino]-benzoesäuremethylester in 4 ml Tetrahydrofuran gegeben. Die Lösung wird 2 h bei 0°C gerührt und dann mit 0,80 ml einer wässrigen 2 N NaOH-Lösung versetzt. Das Gemisch wird mit Ethylacetat verdünnt und über Celite filtriert. Das Filtrat wird über MgSO₄ getrocknet und bis zur Trockene eingeengt.
Ausbeute: 195 mg (98% der Theorie)
Massenspektrum (ESI⁺): m/z = 519 [M+H]⁺

Analog Beispiel 16 wird folgende Verbindung erhalten:
(1) N-(5-tert-Butyl-3-hydroxymethyl-2-methoxy-phenyl)-3-(2-[1,4]diazepan-1-ylmethyl-pyridin-4-yloxy)-4-methyl-benzamid 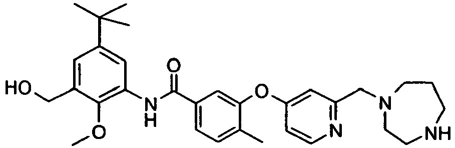 Massenspektrum (ESI⁺): m/z = 533 [M+H]⁺

Nachfolgend werden Beispiele zu Darreichungsformen beschrieben, worin die Angabe "Wirksubstanz" eine oder mehrere erfindungsgemäße Verbindungen, einschließlich deren Salze bedeutet. Im Falle einer der beschriebenen Kombinationen mit einem oder mehreren weiteren Wirksubstanzen umfasst der Begriff "Wirkstoff" auch die weiteren Wirksubstanzen.

### Beispiel A

### Dragees mit 75 mg Wirksubstanz

### Zusammensetzung:

### 1 Dragéekern enthält:

| | |
|---|---|
| Wirksubstanz | 75.0 mg |
| Calciumphosphat | 93.0 mg |
| Maisstärke | 35.5 mg |
| Polyvinylpyrrolidon | 10.0 mg |
| Hydroxypropylmethylcellulose | 15.0 mg |
| Magnesiumstearat | 1.5 mg |
| | 230.0 mg |

### Verstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1.5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.

| | |
|---|---|
| Dragéegewicht: | 245 mg. |

### Beispiel B

### Tabletten mit 100 mg Wirksubstanz

### Zusammensetzung:

### 1 Tablette enthält:

| | |
|---|---|
| Wirksubstanz | 100.0 mg |
| Milchzucker | 80.0 mg |
| Maisstärke | 34.0 mg |
| Polyvinylpyrrolidon | 4.0 mg |
| Magnesiumstearat | 2.0 mg |
| | 220.0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2.0 mm-Maschenweite) und Trocknen im Hordentrockenschrank bei 50°C wird erneut gesiebt (1.5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel C

### Tabletten mit 150 mg Wirksubstanz

### Zusammensetzung:

### 1 Tablette enthält:

| | |
|---|---|
| Wirksubstanz | 150.0 mg |
| Milchzucker pulv. | 89.0 mg |
| Maisstärke | 40.0 mg |
| Kolloide Kieselgelsäure | 10.0 mg |
| Polyvinylpyrrolidon | 10.0 mg |
| Magnesiumstearat | 1.0 mg |
| | 300.0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1.5 mm-Maschenweite geschlagen.

Das bei 45°C getrocknete Granulat wird nochmals durch dasselbe Sieb gerieben und mit der angegebenen Menge Magnesiumstearat gemischt. Aus der Mischung werden Tabletten gepreßt..

| | |
|---|---|
| Tablettengewicht: | 300 mg |
| Stempel: | 10 mm, flach |

### Beispiel D

### Hartgelatine-Kapseln mit 150 mg Wirksubstanz

### Zusammensetzung:

### 1 Kapsel enthält:

| | |
|---|---|
| Wirkstoff | 150.0 mg |
| Maisstärke getr. ca. | 180.0 mg |
| Milchzucker pulv. ca. | 87.0 mg |
| Magnesiumstearat | 3.0 mg |
| ca. | 420.0 mg |

### Herstellung:

Der Wirkstoff wird mit den Hilfsstoffen vermengt, durch ein Sieb von 0.75 mm-Maschenweite gegeben und in einem geeigneten Gerät homogen gemischt.
Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel E

### Suppositorien mit 150 mg Wirksubstanz

### Zusammensetzung:

### 1 Zäpfchen enthält:

| | |
|---|---|
| Wirkstoff | 150.0 mg |
| Polyäthylenglykol 1500 | 550.0 mg |
| Polyäthylenglykol 6000 | 460.0 mg |
| Polyoxyäthylensorbitanmonostearat | 840.0 mg |
| | 2000.0 mg |

### Herstellung:

Nach dem Aufschmelzen der Suppositorienmasse wird der Wirkstoff darin homogen verteilt und die Schmelze in vorgekühlte Formen gegossen.

### Beispiel F

### Suspension mit 50 mg Wirksubstanz

### Zusammensetzung:

### 100 ml Suspension enthalten:

| | |
|---|---|
| Wirkstoff | 1.00 g |
| Carboxymethylcellulose-Na-Salz | 0.10 g |
| p-Hydroxybenzoesäuremethylester | 0.05 g |
| p-Hydroxybenzoesäurepropylester | 0.01 g |
| Rohrzucker | 10.00 g |
| Glycerin | 5.00 g |
| Sorbitlösung 70%ig | 20.00 g |
| Aroma | 0.30 g |

Wasser dest. ad 100.00 ml

### Herstellung:

Destilliertes Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

5 ml Suspension enthalten 50 mg Wirkstoff.

### Beispiel G

### Ampullen mit 10 mg Wirksubstanz

### Zusammensetzung:

| | |
|---|---|
| Wirkstoff | 10.0 mg |
| 0.01 n Salzsäure s.q. | |
| Aqua bidest ad | 2.0 ml |

### Herstellung:

Die Wirksubstanz wird in der erforderlichen Menge 0.01 n HCl gelöst, mit Kochsalz isotonisch gestellt, sterilfiltriert und in 2 ml Ampullen abgefüllt.

### Beispiel H

### Ampullen mit 50 mg Wirksubstanz

### Zusammensetzung:

| | |
|---|---|
| Wirkstoff | 50.0 mg |
| 0.01 n Salzsäure s.q. | |
| Aqua bidest | ad 10.0 ml |

### Herstellung:

Die Wirksubstanz wird in der erforderlichen Menge 0.01 n HCl gelöst, mit Kochsalz isotonisch gestellt, sterilfiltriert und in 10 ml Ampullen abgefüllt.

### Beispiel I

### Kapseln zur Pulverinhalation mit 5 mg Wirksubstanz

### 1 Kapsel enthält:

| | |
|---|---|
| Wirksubstanz | 5.0 mg |
| Lactose für Inhalationszwecke | 15.0 mg |
| | 20.0 mg |

### Herstellung:

Die Wirksubstanz wird mit Lactose für Inhalationszwecke gemischt. Die Mischung wird auf einer Kapselmaschine in Kapseln (Gewicht der Leerkapsel ca. 50 mg) abgefüllt.
Kapselgewicht: 70.0 mg
Kapselgröße: 3

### Beispiel J

Inhalationslösung für Handvernebler mit 2.5 mg Wirksubstanz

### 1 Hub enthält:

| | |
|---|---|
| Wirksubstanz | 2.500 mg |
| Benzalkoniumchlorid | 0.001 mg |
| 1N-Salzsäure q.s. | |
| Ethanol/Wasser (50/50) | ad 15.000 mg |

### Herstellung:

Die Wirksubstanz und Benzalkoniumchlorid werden in Ethanol/Wasser (50/50) gelöst. Der pH-Wert der Lösung wird mit 1 N-Salzsäure eingestellt. Die eingestellte Lösung wird filtriert und in für den Handvernebler geeignete Behälter (Kartuschen) abgefüllt.
Füllmasse des Behälters: 4.5 g

## Patentansprüche

1. Heteroarytoxy-substituierte Benzoesäureamide der allgemeinen Formel I 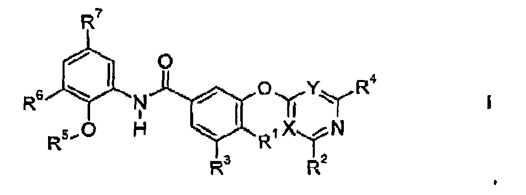 in der
R¹ L₁-R₈ ist,
wobei L₁ ausgewählt ist aus einer Bindung, O, CO, NH, CONH, NHCO, SO, SO₂, SO₂NH, NHSO₂ und C₁-C₄-Alkylen,
wobei R₈ ausgewählt ist aus Wasserstoff, Halogen, C₁₋₆-Alkyl, C₂₋₆-Alkinyl, C₂₋₆-Alkenyl, C₃₋₈-Cycloalkyl, C₃₋₈-Heterocycloalkyl, C₀₋₁₀-Aryl, C₅₋₁₀-Heteroaryl, Cyan, Carboxy, C₁₋₄-Alkyloxycarbonyl Nitro, Amino, Di-(C₁₋₄-alkyl)-amino, Hydroxy, C₁₋₆-Alkylsulfanyl, wobei R₈ teilweise oder vollständig fluoriert und/oder mit einem oder mehreren Substituenten L substituiert sein kann, und wobei In Heterocycloalkyl-Resten eine Methylengruppe durch CO, SO oder SO₂ substituiert sein können, und
wobei R¹ nicht Wasserstoff ist, wenn R³ die Bedeutung Wasserstoff hat, und
R² L₂-R₉ ist,
wobei L₂ ausgewählt ist aus einer Bindung, O, CO, NH, CONH, NHCO, SO, SO₂, SO₂NH, NHSO₂ und C₁-C₄-Alkylen wobei R₉ ausgewählt ist aus Wasserstoff. Halogen, C₁₋₆-Alkyl, C₂₋₆-Alkinyl, C₂₋₆-Alkenyl, C₃₋₈-Cycloalkyl, C₄₋₈-Cycloalkenyl, C₃₋₈-Haterocycloalkyl, C₆₋₁₀₋Aryl, C₅₋₁₀-Heteroaryl, Cyan, Carboxy, C₁₋₄-Alkyloxycarbonyl, Nitro, Amino, Di-(C₁₋₄-alkyl)-amino, Hydroxy, C₁₋₆-Alkylsulfanyl,
wobei R₉ teilweise oder vollständig fluoriert und/oder mit einem oder mehreren Substituenten L substituiert sein kann, und
wobei in den genannten Alkyl-Resten optional eine oder zwei CH₂-Gruppe gegen O und/oder NR^{N} ausgetauscht sind,
wobei in den genannten Cycloalkyl-Resten eine oder zwei CH₂-Gruppen unabhängig voneinander durch NR^{N}, O, S, CO, SO oder SO₂ und eine oder zwei CH-Gruppen durch N ersetzt sein können, und
R³ L₃-R₁₀ ist
wobei L₃ ausgewählt ist aus einer Bindung, O, CO, NH, CONH, NHCO, SO, SO₂, SO₂NH, NHSO₂ und C₁-C₄-Alkylen
wobei R₁₀ ausgewählt ist aus Wasserstoff, Halogen, C₁₋₆Alkyl, C₂₋₆-Alkinyl, C₂₋₆-Alkenyl, C₃₋₈-Cycloalkyl, C₄₋₈-Cycloalkenyl, C₃₋₈-Heterocycloalkyl, C₅₋₁₀-Aryl, C₅-₁₀-Heteroaryl, Cyan, Carboxy, C₁₋₄-Alkyloxycarbonyl, Nitro, Amino, Di-(C₁₋₄-alkyl)-amino, Hydroxy, C₁₋₆-Alkylsulfanyl
wobei R₁₀ teilweise oder vollständig fluoriert und/oder mit einem oder mehreren Substituenten L substituiert sein kann, und
wobei R³ nicht Wasserstoff ist, wenn R¹ die Bedeutung Wasserstoff hat,
R⁴ L₄-R₁₁ ist
wobei L₄ ausgewählt ist aus einer Bindung, O, CO, NH, CONH, NHCO,
wobei R₁₁ ausgewählt ist aus Wasserstoff, Halogen, Amino, C₁₋₆-Alkyl, C₃₋₃-Cycloalkyl, C₃₋₈-Heterocycloalkyl, Trifluoromethyl, Difluormethyl, Cyan, Carboxy, C₁₋₄-Alkyloxycarbonyl, Di-(C₁₋₄-alkyl)-amino, C₁₋₄-Alkylsulfanyl,
R⁵ C₁₋₆-Alkyl,
R⁶ ausgewählt ist aus Dimethylamino-C₁₋₃-alkyl, Cyclopropylamino-C₁₋₃-alkyl, Pyrrolidinyl-C₁₋₃-alkyl, Hydroxy-C₁₋₃-alkyl, Methylsulfanyl-C₁₋₃-alkyl, Methylsulfinyl-C₁₋₃-alkyl, Methylsulfonyl-C₁₋₃-alkyl, C₁₋₄-Alkylcarbonylamino, C₃₋₅-Cycloalkyl-carbonylamino, C₁₋₄-Alkylsulfonylamino, C₃₋₅-Cycloalkylsulfonylamino, C₁₋₃-Alkylsulfinyl und C₁₋₃-Alkylsulfonyl,
R⁷ C₁₋₆-Alkyl, Trifluormethyl, Pentafluorethyl,
X,Y unabhängig voneinander N oder C-R₁₃, wobei X und Y nicht beide N sein können und R₁₃ ausgewählt ist aus Wasserstoff, Fluor, Chlor, C₁₋₄-Alkyl, Trifluormethyl, OH, C₁₋₄-Alkyloxy, Trifluormethoxy, Difluormethoxy und Cyan,
R^{N} ausgewählt ist aus H, C₁₋₄-Alkyl, 1,1,1-Trifluorethyl, Cyanmethyl, Acetyl, Methylsulfonyl, C₆₋₁₀-Arylmethyl,
L ausgewählt ist aus der Gruppe bestehend aus Halogen, Cyan, C₁₋₆-Alkyl, C₁₋₆-Alkyloxy, Amino, C₁₋₃Alkylamino, Di-(C₁₋₃-Alkyl)-amino, C₁₋₄-Alkylcarbonylamino, Difluormethyl, C₁₋₄-Alkylsulfonylamino Trifluormethyl, Hydroxy, C₁₋₃-Alkoxy, Difluormethoxy, Trifluormethoxy, Acetylamino, C₆₋₁₀-Aryl und C₅₋₁₀-Heteroaryl.

2. Heteroaryloxy-substituierte Benzoesäureamide nach Anspruch 1 in denen
R¹ ausgewählt ist aus Wasserstoff, Halogen, C₁₋₆-Alkyl, C₂₋₆-Alkinyl, C₂₋₆-Alkenyl, C₃₋₇-Cycloalkyl, C₃₋₇-Cycloalkyl-C₁₋₃-alkyl, C₆₋₁₀-Aryl, C₅₋₁₀-Heteroaryl, C₆₋₁₀-Aryl-C₁₋₃-alkyl, C₅₋₁₀-Heteroaryl-C₁₋₃-alkyl, C₁₋₄-Alkylcarbonyl, Cyan, Aminocarbonyl, C₁₋₄-Alkyl-aminocarbonyl, Di-(C₁₋₃-Alkyl)-aminocarbonyl, Pyrrolidin-1-ylcarbonyl, Piperidin-1-ylcarbonyl, Morpholin-4-ylcarbonyl, Piperazin1-ylcarbonyl, 4-(C₁₋₄-Alkyl)-piperazin-1-ylcarbonyl, Carboxy, C₁₋₄-Alkyloxycarbonyl, Nitro, Amino, C₁₋₄-Alkylamino, Di-(C₁₋₃-alkyl)-amino, Pyrrolidin-1-yl, Piperidin-1-yl, Morpholin-4-yl, Piperazin-1-yl, 4-(C₁₋₄-Alkyl)piperazin-1-yl, C₁₋₄-Alkylcarbonylamino, C₁₋₄-Alkylsulfonylamino, Hydroxy, C₁₋₆-Alkyloxy, C₃₋₇-Cycloalkyloxy, C₆₋₁₀-Aryloxy, C₁₋₄-Alkylsulfanyl, C₁₋₄-Alkylsulfinyl und C₁₋₄-Alkylsulfonyl,
wobei Alkyl-, Alkenyl-, Alkinyl-, Cycloalkyl- und Heterocycloalkyl-Reste teilweise oder vollständig fluoriert und/oder ein- oder zweifach mit gleichen oder verschiedenen Substituenten ausgewählt aus Cyan, Hydroxy, C₁₋₃-Alkyloxy, Acetylamino, Methylsulfonylamino und C₁₋₃-Alkyl substituiert sein können, und
wobei in Cycloalkyl-Resten ein oder zwei Methylengruppen unabhängig voneinander durch O, S, CO, SO oder SO₂ substituiert sein können, und
wobei in N-Heterocycloalkyl-Resten eine Methylengruppe durch CO oder SO₂ substituiert sein kann,
wobei R¹ nicht Wasserstoff ist, wenn R³ die Bedeutung Wasserstoff hat, und
R² ausgewählt ist aus Wasserstoff, C₁₋₈-Alkyl, C₂₋₆-Alkenyl, C₂₋₆-Alkinyl, C₃₋₇-Cycloalkyl-C₁₋₄-alkyl, C₁₋₆-Alkylcarbonyl-C₁₋₃-alkyl, C₆₋₁₀-Aryl-C₁₋₄-alkyl, C₅₋₁₀-Heteroaryl-C₁₋₄-alkyl, C₃₋₇-Cycloalkyl, C₁₋₆-Alkylcarbonyl, C₃₋₇-cycloalkyl-carbonyl, Cyan, Aminocarbonyl, Carboxy, Amino, C₁₋₄-Alkylcarbonylamino, C₁₋₄-Alkylsulfonylamino, C₁₋₄-Alkyloxy, C₆₋₁₀-Aryl, C₅₋₁₀-Heteroaryl, Hydroxy.
wobei alle genannten Alkyl-, Alkenyl-, Alkinyl- und Cycloalkyl-Reste teilweise oder vollständig fluoriert und/oder ein-, zwei- oder dreifach mit gleichen oder verschiedenen Substituenten ausgewählt aus C₁₋₃-Alkyl, Cyan, Hydroxy, C₁₋₃-Alkyloxy, Amino, C₁₋₃-Alkylamino, Di-(C₁₋₃-Alkyl)amino, Acetylamino, Methylsulfonylamino, C₆₋₁₀-Aryl und C₅₋₁₀-Heteroaryl substituiert sein können,
wobei in den genannten Alkyl-Resten optional eine oder zwei CH₂-Gruppe gegen O und/oder NR^{N} ausgetauscht sind,
wobei in den genannten Cycloalkyl-Resten eine oder zwei CH₂-Gruppen unabhängig voneinander durch NR^{N}, O, S, CO, SO oder SO₂ und eine oder zwei CH-Gruppen durch N ersetzt sein können, und
R³ ausgewählt ist aus Wasserstoff, Halogen, C₁₋₄-Alkyl, C₂₋₆-Alkinyl, C₂₋₆-Alkenyl, C₃₋₇-Cycloalkyl-C₁₋₃-alkyl, C₃₋₆-Cycloalkyl, Trifluormethyl, Difluormethyl, Hydroxy, C₁₋₄-Alkyloxy, Tirfluormethoxy, Difluormethoxy, Cyan, Aminocarbonyl, C₁₋₄-Alkyl-aminocarbonyl, Di-(C₁₋₃-Alkyl)-aminocarbonyl, Carboxy, C₁₋₄-Alkyloxycarbonyl, Amino, C₁₋₄-Alkylamino, Di-(C₁₋₃-alkyl)-amino, C₁₋₄-Alkylcarbonylamino, C₁₋₄-Alkylsulfonylamino, wobei R³ nicht Wasserstoff ist, wenn R¹ die Bedeutung Wasserstoff hat,
R⁴ ausgewählt ist aus Wasserstoff, C₁₋₄-Alkyl, C₃₋₆-Cycloalkyl, Trifluoromethyl, Difluormethyl, Cyan, Carboxy, Aminocarbonyl, C₁₋₃Alkylaminocarbonyl, Di-(C₁₋₃-alkyl)aminocarbonyl, C₁₋₄-Alkyloxycarbonyl, Amino, C₁₋₃-Alkylamino, Di-(C₁₋₃-alkyl)amino, Hydroxy, C₁₋₄-Alkyloxy, C₁₋₄-Alkylsulfanyl,
R⁵ C₁₋₄-Alkyl ist,
R⁶ ausgewählt ist aus Dimethylamino-C₁₋₃-alkyl, Cyclopropylamino-C₁₋₃-alkyl, Pyrrolidinyl-C₁₋₃-alkyl, Hydroxy-C₁₋₃-alkyl, Methylsulfanyl-C₁₋₃-alkyl, Methylsulfinyl-C₁₋₃-alkyl, Methylsulfonyl-C₁₋₃-alkyl, C₁₋₄-Alkylcarbonylamino, C₃₋₅-Cycloalkyl-carbonylamino, C₁₋₄-Alkylsulfonylamino, C₃₋₅-Cycloalkylsulfonylamino, C₁₋₃-Alkylsulfinyl und C₁₋₃-Alkylsulfonyl,
R⁷ ausgewählt ist aus C₄-Alkyl, Trifluormethyl, Pentafluorethyl,
X,Y unabhängig voneinander N oder CR₁₃, wobei X und Y in der selben Bedeutung N ausgeschlossen ist und R₁₃ ausgewählt ist aus Wasserstoff, Fluor, Chlor, C₁₋₄-Alkyl, Trifluormethyl, Hydroxy, C₁₋₄-Alkyloxy, Trifluormethoxy, Difluormethoxy, Cyano,
R^{N} ausgewählt ist aus H, C₁₋₄-Alkyl, 1,1,1-Trifluorethyl, Cyanmethyl, Acetyl, Methylsulfonyl, C₆₋₁₀-Arylmethyl,
L ausgewählt ist aus der Gruppe bestehend aus Fluor, Chlor, Brom, C₁₋₃-Alkyl, Difluormethyl, Trifluormethyl, Hydroxy, C₁₋₃-Alkoxy, Difluormethoxy, Trifluormethoxy, Acetylamino, Methylsulfonylamino und Cyan,
wobei unter den bei der Definition der vorstehend genannten Reste erwähnten C₅₋₁₀-Arylgruppen Phenyl- oder Naphthylgruppen zu verstehen sind, welche unabhängig von einander ein- oder zweifach mit gleichen oder verschiedenen Resten L substituiert sein können; und
unter den bei der Definition der vorstehend erwähnten Reste erwähnten Heteroarylgruppen eine Pyrrolyl-, Furanyl-, Thienyl-, Pyridyl-, Indolyl-, Benzofuranyl-, Benzothiophenyl-, Chinolinyl- oder Isochinolinylgruppe zu verstehen ist,
oder eine Pyrrolyl-, Furanyl-, Thienyl- oder Pyridylgruppe zu verstehen ist, in der eine oder zwei Methingruppen durch Stickstoffatome ersetzt sind,
oder eine Indolyl-, Benzofuranyl-, Benzothiophenyl-, Chinolinyl- oder Isochinolinylgruppe zu verstehen ist, in der eine bis drei Methingruppen durch Stickstoffatome ersetzt sind,
wobei die vorstehend erwähnten Heteroarylgruppen unabhängig voneinander ein- oder zweifach mit gleichen oder verschiedenen Resten L substituiert sein können;
wobei, soweit nichts anderes erwähnt wurde, die vorstehend erwähnten Alkylgruppen geradkettig oder verzweigt sein können,
deren Tautomere, deren Stereoisomere, deren Gemische und deren Salze, insbesondere deren physiologisch verträglichen Salze.

3. Heteroaryloxy-substituierte Benzoesäureamide gemäß einem der vorstehenden Ansprüche, **dadurch gekennzeichnet, dass**
R¹ ausgewählt ist aus Wasserstoff, Fluor, Chlor, Brom, C₁₋₄-Alkyl, Trifluormethyl, C₃₋₆-Cycloalkyl, Cyan, Aminocarbonyl, C₁₋₄-Alkyl-aminocarbonyl, Di-(C₁₋₃-Alkyl)-aminocarbonyl, Carboxy, C₁₋₄Alkyloxycarbonyl, Amino, C₁₋₄-Alkylamino, Di-(C₁₋₃-alkyl)-amino, Pyrrolidin-1-yl, Piperidin-1-yl, Morpholin-4-yl, Acetylamino, Methylsulfonylamino, Hydroxy,C₁₋₄-Alkyloxy, Trifluormethoxy, Difluormethoxy und C₁₋₄-Alkylsulfonyl,
wobei die genannten Alkyl-, Cycloalkyl- und N-Heterocycloalkyl-Reste ein- oder zweifach mit gleichen oder verschiedenen Substituenten ausgewählt aus Cyan, Hydroxy, C₁₋₃- Alkyloxy, Acetylamino, Methylsulfonylamino und C₁₋₃-Alkyl substituiert sein können, und
wobei in den genannten Cycloalkyl-Resten ein oder zwei Methylengruppen unabhängig voneinander durch O, CO oder SO₂ und in den genannten N-Heterocycloalkyl-Resten eine Methylengruppe durch CO oder SO₂ substituiert sein können, und
wobei R¹ nicht Wasserstoff ist, wenn R³ die Bedeutung Wasserstoff hat.

4. Heteroaryloxy-substituierte Benzoesäureamide gemäß einem oder mehreren der vorhergehenden Ansprüche, **dadurch gekennzeichnet, dass**
R² ausgewählt ist aus Wasserstoff, C₁₋₄-Alkyl, C₃₋₆-Cycloalkyl-C₁₋₃-alkyl, C₁₋₃-Alkylamino-C₁₋₃-alkyl, C₃₋₈-Cycloalkylamino-C₁₋₃-alkyl, Pyrrolidinyl-C₁₋₃-alkyl, Piperidin-C₁₋₃-alkyl, Azepanyl-C₁₋₃-alkyl, Piperazinyl-C₁₋₃-alkyl, Morpholinyl-C₁₋₃-alkyl, Homopiperazinyl-C₁₋₃-alkyl, Pyrrolidin-3-ylamino-C₁₋₃-alkyl, Piperidin-3-ylamino-C₁₋₃-alkyl, Piperidin-4-ylamino-C₁₋₃-alkyl, C₃₋₆-Cycloalkyl, Trifluormethyl, Cyan, Aminocarbonyl, C₁₋₄-Alkyl-aminocarbonyl, Di-(C₁₋₃-Alkyl)-aminocarbonyl, Piperazin-1-ylcarbonyl, Morpholin-4-ylcarbonyl, Carboxy, C₁₋₄-Alkyloxycarbonyl, Amino, C₁₋₄-Alkylamino, C₃₋₆-Cycloalkylamino, C₃₋₆-Cycloalkyl-C₁₋₃-alkylamino, Pyrrolidin-1-yl, Piperidin-1-yl, Piperazin-1-yl, Morpholin-4-yl, C₁₋₃-Alkyl-amino-C₂₋₃-alkylamino, Pyrrolidin-3-ylamino, Piperidin-3-ylamino, Piperidin-4-ylamino, Pyrrolidinyl-C₁₋₃-alkylamino, Piperidin-C₁₋₃alkyl-amino, Piperazinyl-C₁₋₃-alkyl-amino, Morpholinyl-C₁₋₃-alkylamino, Homopiperazinyl-C₁₋₃-alkyl-amino, Acetylamino, Methylsulfonylamino, Hydroxy, C₁₋₄-Alkyloxy, Trifluormethoxy, Difluormethoxy, C₃₋₆-Cycloalkyloxy, C₃₋₆-Cycloalkyl-C₁₋₃-alkyloxy, C₁₋₃-Alkylamino-C₂₋₃-alkyloxy, Di-(C₁₋₃-Alkyl)-amino-C₂₋₃-alkyloxy, Pyrrolidinyl-C₂₋₃-alkyloxy, Piperidin-C₂₋₃-alkyloxy, Piperazinyl-C₂₋₃-alkyloxy, Morpholinyl-C₂₋₃-alkyloxy, Homopiperazinyl-C₂₋₃-alkyloxy,
wobei die oben genannten Alkyl-, Cycloalkyl- und N-Heterocycloalkyl-Reste ein- oder zweifach mit gleichen oder verschiedenen Substituenten ausgewählt aus C₁₋₃-Alkyl, Cyan, Amino, C₁₋₃-Alkylamino, Di-(C₁₋₃-alkyl)amino. Acetylamino, Methylsulfonylamino, Hydroxy und C₁₋₃-Alkyloxy substituiert sein können, und
wobei in den oben genannten Cycloalkyl-Resten ein oder zwei Methylen-gruppen unabhängig voneinander durch O und/oder CO und in den genannten N-Heterocycloalkyl-Resten eine Methylengruppe durch CO oder SO₂ substituiert sein können, und
wobei optional alle in den oben genannten Resten beinhalteten NH-Gruppen durch N-Me, N-Et, N-*i*Pr, N-Acetyl und N-SO₂Me ersetzt sind.

5. Heteroaryloxy-substituierte Benzoesäureamide gemäß einem oder mehreren der vorhergehenden Ansprüche, **dadurch gekennzeichnet, dass** R³ ausgewählt ist aus Wasserstoff, C₁₋₃-Alkyl, C₃₋₆-Cycloalkyl, Fluor, Chlor, Brom, Trifluormethyl, Difluormethyl, Hydroxy, C₁₋₃-Alkyloxy, Trifluormethoxy, Difluormethoxy und Cyan , wobei R³ nicht Wasserstoff ist, wenn R¹ die Bedeutung Wasserstoff hat.

6. Heteroaryloxy-substituierte Benzoesäureamide gemäß einem oder mehreren der vorhergehenden Ansprüche, **dadurch gekennzeichnet, dass** R⁴ ausgewählt ist aus Wasserstoff, C₁₋₃-Alkyl, Trifluoromethyl, Carboxy, Aminocarbonyl, Methylaminocarbonyl, Dimethylamino-carbonyl, Amino, C₁₋₃-Alkylamino, Di-(C₁₋₃-alkyl)-amino, Hydroxy und
C₁₋₃-Alkyloxy.

7. Heteroaryloxy-substituierte Benzoesäureamide gemäß einem oder mehreren der vorhergehenden Ansprüche, **dadurch gekennzeichnet, dass** R⁵ ausgewählt ist aus Methyl, Ethyl, n-Propyl und Isopropyl.

8. Heteroaryloxy-substituierte Benzoesäureamide gemäß einem oder mehreren der vorhergehenden Ansprüche, **dadurch gekennzeichnet, dass** R⁶ ausgewählt ist aus Dimethylaminomethyl, Cyclopropylaminomethyl, Pyrrolidin-1-ylamino, Hydroxymethyl, Methylsulfanylmethyl, Methylsulfinylmethyl, Methylsulfonylmethyl, n-Butylcarbonyl-amino, isopropylcarbonyl-amino, Isobutylcarbonylamino, Cyclopropylcarbonylamino, Methylsulfonylamino, Isopropylsulfonylamino, n-Butylsulfonylamino, Isobutylsulfonyl-amino, Cyclopropyl-sulfonylamino, Methylsulfinyl und Methylsulfonyl.

9. Heteroaryloxy-substituierte Benzoesäureamide gemäß einem oder mehreren der vorhergehenden Ansprüche, **dadurch gekennzeichnet, dass** R⁷ ausgewählt ist aus Isobutyl, tert-Butyl, Trifluormethyl und Pentafluorethyl.

10. Heteroaryloxy-substituierte Benzoesäureamide gemäß einem oder mehreren der vorhergehenden Ansprüche, **dadurch gekennzeichnet, dass** X und Y unabhängig voneinander ausgewählt aus N, C-H, C-F, C-Cl, C-C₁₋₃-alkyl und C₁₋₃-alkyloxy sind, mit der Einschränkung, dass X und Y nicht in der selben Bedeutung N vorkommen,

11. Verbindungen nach Anspruch 1 ausgewählt unter
N-(5-tert-Butyl-3-methansulfonylamino-2-methoxy-phenyl)4-methyl-3-(pyridin-4-yloxy)-benzamid;
N-(5-tert-Butyl-3-methansulfonylamino-2-methoxy-phenyl)-4-methyl-3-(2-methylamino-pyrimidin-4-yloxy)-benzamid;
N-(5-tert-Butyl-3-methansulfonylamino-2-methoxy-phenyl)-4-methyl-3-(6-methylamino-pyrimidin-4-yloxy)-benzamid;
N-(5-tert-Butyl-3-methansulfonylamino-2-methoxy-phenyl)-4-methyl-3-[2-(2-pyrrolidin-1-yl-ethylamino)-pyrimidin-4-yloxy]-benzamid;
N-(5-tert-Butyl-3-methansulfonylamino-2-methoxy-phenyl)-3-[6-(2-hydroxy-ethylamino)-pyrimidin-4-yloxy]-4-methyl-benzamid;
N-(5-tert-Butyl-3-methansulfonylamino-2-methoxy-phenyl)-3-[6-(2-dimethylamino-ethylamino)-pyrimidin-4-yloxy]-4-methyl-benzamid;
N-(5-tert-Butyl-3-methansulfonylamino-2-methoxy-phenyl)-4-methyl-3-[6-(3-oxo-piperazin-1-yl)-pyrimidin-4-yloxy]-benzamid;
N-(5-tert-Butyl-3-methansulfonylamino-2-methoxy-phenyl)-4-methyl-3-(6-pyrrolidin-1-yl-pyrimidin-4-yloxy)-benzamid;
N-(5-tert-Butyl-3-methansultonylamino-2-methoxy-phenyl)-4-methyl-3-(6-morpholin-4-yl-pyrimidin-4-yloxy)-benzamid;
(*R*)-N-(5-tert-Butyl-3-methansulfonylamino-2-methoxy-phenyl)-3-[2-(3-dimethylamino-pyrrolidin-1-yl)-pyrimidin-4-yloxy]-4-methyl-benzamid;
(*S*)-N-(5-tert-Butyl-3-methansulfonylamino-2-methoxy-phenyl)-3-[2-(3-dimethylamino-pyrrolidin-1-yl)-pyrimidin-4-yloxy]-4-methyl-benzamid;
N-(5-tert-Butyl-3-methansulfonylamino-2-methoxy-phenyl)-3-[2-(4-dimethylamino-piperidin-1-yl)-pyrimidin-4-yloxy]-4-methyl-benzamid;
N-(5-tert-Butyl-3-methansulfonylamino-2-methoxy-phenyl)-4-methyl-3-[2-(1-methylpiperidin-4-ylamino)-pyrimidin-4-yloxy]-benzamid;
N-(5-tert-Butyl-3-methansulfonylamino-2-methoxy-phenyl)-4-methyl-3-(2-piperazin-1-yl-pyrimidin-4-yloxy)-benzamid;
N-(5-tert-Butyl-3-methansulfonylamino-2-methoxy-phenyl)-4-methyl-3-(6-piperazin-1-yl-pyrimidin-4-yloxy)-benzamid;
N-(5-tert-Butyl-3-methansulfonylamino-2-methoxy-phenyl)-4-methyl-3-[6-(piperazin-l-carbonyl)-pyrimidin-4-yloxy]-benzamid;
N-(5-tert-Butyl-3-methansulfonylamino-2-methoxy-phenyl)-4-chlor-3-[6-(piperazin-1-carbonyl)-pyrimidin-4-yloxy]-benzamid;
N-(5-tert-Butyl-3-methansulfonylamino-2-methoxy-phenyl)-3-(2-piperazin-1-yl-pyrimidin-4-yloxy)-4-trifluormethyl-benzamid;
N-(5-tert-Butyl-3-methansulfonylamino-2-methoxy-phenyl)-4-chlor-3-(2-piperazin-1-yl-pyrimidin-4-yloxy)-benzamid;
N-(5-tert-Butyl-3-methansulfonylamino-2-methoxy-phenyl)-4-methyl-3-(2-piperazin-1-ylmethyl-pyrimidin-4-yloxy)-benzamid;
N-(5-tert-Butyl-3-methansulfonylamino-2-methoxy-phenyl)-4-methyl-3-(6-piperazin-1-ylmethyl-pyrimidin-4-yloxy)-benzamid;
N-(5-tert-Butyl-3-methansulfonylamino-2-methoxy-phenyl)-4-methoxy-3-(2-piperazin-1-yl-pyrimidin-4-yloxy)-benzamid;
N-(5-tert-Butyl-3-methansulfonylamino-2-methoxy-phenyl)-4-methyl-3-(2-piperazin-1-ylmethyl-pyridin-4-yloxy)-benzamid;
N-(5-tert-Butyl-3-methansulfonylamino-2-methoxy-phenyl)-4-fluor-3-(2-piperazin-1-yl-pyrimidin-4-yloxy)-benzamid;
N-(5-tert-Butyl-3-methansulfonylamino-2-methoxy-phenyl)-4-brom-3-(2-piperazin-1-yl-pyrimidin-4-yloxy)-benzamid;
N-(5-tert-Butyl-3-methansulfonylamino-2-methoxy-phenyl)-3-chlor-4-methyl-5-(2-piperazin-1-yl-pyrimidin-4-yloxy)-benzamid;
N-(5-tert-Butyl-3-methansulfonylamino-2-methoxy-phenyl)-3-fluor-4-methyl-5-(2-piperezin-1-yl-pyrimidin-4-yloxy)-benzamid;
N-(5-tert-Butyl-3-methansulfonylamino-2-methoxy-phenyl)-4-methyl-3-[2-(N-methyl-N-piperidin-4-yl-amino)-pyrimidin-4-yloxy]-benzamid;
N-(5-tert-Butyl-3-methansulfonylamino-2-methoxy-phenyl)-4-ethyl-5-(2-piperazin-1-yl-pyrimidin-4-yloxy)-benzamid;
N-(5-tert-Butyl-3-methansulfonylamino-2-methoxy-phenyl)-4-methyl-3-[2-(piperidin-4-ylamino)-pyrimidin-4-yloxy]-benzamid;
N-(5-tert-Butyl-3-methansulfonylamino-2-methoxy-phenyl)-4-methyl-3-[6-(piperidin-4-ylamino)-pyrimidin-4-yloxy]-benzamid;
N-(5-tert-Butyl-3-methansulfonylamino-2-methoxy-phenyl)-4-methyl-3-(6-piperidin-4-ylmethyl-pyrimidin-4-yloxy)-benzamid;
N-(5-tert-Butyl-3-methansulfonylamino-2-methoxy-phenyl)-4-methyl-3-(2-piperidin-4-ylmethyl-pyrimidin-4-yloxy)-benzamid;
N-(5-tert-Butyl-3-methansulfonylamino-2-methoxy-phenyl)-4-methyl-3-(2-piperidin-4-ylmethyl-pyridin-4-yloxy)-benzamid;
N-(5-tert-Butyl-3-methansulfonylamino-2-methoxy-phenyl)-3-methyl-5-[2-(piperazin-1-carbonyl)-pyrimidin-4-yloxy]-benzamid;
N-(5-tert-Butyl-3-methansulfonylamino-2-methoxy-phenyl)-3-methyl-5-(2-piperazin-1-ylmethyl-pyrimidin-4-yloxy)-benzamid;
N-(5-tert-Butyl-3-methansulfonylamino-2-methoxy-phenyl)-3-methyl-5-(pyridin-4-yloxy)-benzamid;
6-[5-(5-tert-Butyl-3-methansulfonylamino-2-methoxy-phenylaminocarbonyl)-2-methyl-phenoxy]-pyrimidin-4-carbonsäure-methylamid;
N-(5-tert-Butyl-3-methansulfonylamino-2-methoxy-phenyl)-4-methyl-3-[6-(morpholin-4-carbonyl)-pyrimidin-4-yloxy]-benzamid;
N-(5-tert-Butyl-3-methansulfonylamino-2-methoxy-phenyl)-4-methyl-3-[6-(4-methylpiperazin-1-carbonyl)-pyrimidin-4-yloxy]-benzamid;
N-(5-tert-Butyl-3-methansulfonylamino-2-methoxy-phenyl)-4-methyl-3-(6-morpholin-4-ylmethyl-pyrimidin-4-yloxy)-benzamid;
N-(5-tert-Butyl-3-methansulfonylamino-2-methoxy-phenyl)-4-methyl-3-(6-methylaminomethyl-pyrimidin-4-yloxy)-benzamid;
N-(5-tert-Butyl-3-methansulfonylamino-2-methoxy-phenyl)-4-methyl-3-[6-(4-methylpiperazin-1-ylmethyl)-pyrimidin-4-yloxy]-benzamid;
N-(5-tert-Butyl-3-methansulfonylamino-2-methoxy-phenyl)-4-methyl-3-(2-morpholin-4-ylmethyl-pyrimidin-4-yloxy)-benzamid;
N-(5-tert-Butyl-3-methansulfonylamino-2-methoxy-phenyl)-4-methyl-3-(2-methylaminomethyl-pyrimidin-4-yloxy)-benzamid;
N-(5-tert-Butyl-3-methansulfonylamino-2-methoxy-phenyl)-4-methyl-3-[2-(3-oxo-piperazin-1-ylmethyl)-pyrimidin-4-yloxy]-benzamid;
N-(5-tert-Butyl-3-methansulfonylamino-2-methoxy-phenyl)-4-methyl-3-[2-(4-methylpiperazin-1-ylmethyl)-pyrimidin-4-yloxy]-benzamid;
N-(5-tert-Butyl-3-methansulfonylamino-2-methoxy-phenyl)-4-methyl-3-[6-(3-oxo-piperazin-1-ylmethyl)-pyrimidin-4-yloxy]-benzamid;
N-(5-tert-Butyl-3-methansulfonylamino-2-methoxy-phenyl)-4-methyl-3-(6-piperazin-1-ylmethyl-pyrimidin-4-yloxy)-benzamid;
N-(5-tert-Butyl-3-methansulfonylamino-2-methoxy-phenyl)-4-methyl-3-(2-morpholin-4-ylmethyl-pyridin-4-yloxy)-benzamid;
N-(5-tert-Butyl-3-methansulfonylamino-2-methoxy-phenyl)-4-methyl-3-[2-(4-methylpiperazin-1-ylmethyl)-pyridin-4-yloxy]-benzamid;
(*S*)-N-(5-tert-Butyl-3-methansulfonylamino-2-methoxy-phenyl)-4-methyl-3-{2-[(tetrahydro-furan-3-ylamino)-methyl]-pyridin-4-yloxy}-benzamid;
(*R*)-N-(5-tert-Butyl-3-methansulfonylamino-2-methoxy-phenyl)-3-[2-(3-hydroxy-pyrrolidin-1-ylmethyl)-pyridin-4-yloxy]-4-methyl-benzamid;
(*S*)-N-(5-tert-Butyl-3-methansulfonylamino-2-methoxy-phenyl)-3-[2-(3-hydroxy-pyrrolidin-1-ylmethyl)-pyridin-4-yloxy]-4-methyl-benzamid;
N-(5-tert-Butyl-3-methansulfonylamino-2-methoxy-phenyl)-4-methyl-3-(2-methylaminomethyl-pyridin-4-yloxy)-benzamid;
(*S*)-N-(5-tert-Butyl-3-methansulfonylamino-2-methoxy-phenyl)-4-methyl-3-{2-[(tetrahydro-furan-3-ylamino)-methyl]-pyridin-4-yloxy}-benzamid;
N-(5-tert-Butyl-3-methansulfonylamino-2-methoxy-phenyl)-4-methyl-3-[2-(4-methyl-homopiperazin-1-ylmethyl)-pyrimidin-4-yloxy]-benzamid;
N-(5-tert-Butyl-3-methansulfonylamino-2-methoxy-phenyl)-4-methyl-3-{2-[(1-methylpiperidin-4-ylamino)-methyl]-pyrimidin-4-yloxy}-benzamid;
N-(5-tert-Butyl-3-methansulfonylamino-2-methoxy-phenyl)-4-methyl-3-{2-[(1-methylpiperidin-3-ylamino)-methyl]-pyrimidin-4-yloxy}-benzamid;
N-(5-tert-Butyl-3-methansulfonylamino-2-methoxy-phenyl)-4-methyl-3-(2-{[methyl-(1-methyl-pyrrolidin-3-yl)-amino]-methyl}-pyrimidin-4-yloxy)-benzamid;
N-(5-tert-Butyl-3-methansulfonylamino-2-methoxy-phenyl)-4-methyl-3-[2-(4-methyl-homopiperazin-1-ylmethyl)-pyridin-4-yloxy]-benzamid;
(*S*)-N-(5-tert-Butyl-3-methansulfonylamino-2-methoxy-phenyl-3-[2-(3-dimethylamino-pyrrolidin-1-ylmethyl)-pyrimidin-4-yloxy]-4-methyl-benzamid;
(*R*)-N-(5-tert-Butyl-3-methansulfonylamino-2-methoxy-phenyl)-3-[2-(3-dimethylamino-pyrrolidin-1-ylmethyl)-pyrimidin-4-yloxy]-4-methyl-benzamid;
N-(5-tert-Butyl-3-methansulfonylamino-2-methoxy-phenyl)-4-methyl-3-{2-[(1-methylpyrrolidin-3-ylamino)-methyl]-pyrimidin-4-yloxy}-benzamid;
N-(5-tert-Butyl-3-methansulfonylamino-2-methoxy-phenyl)-3-(2-{[N-(2-dimethylaminoethyl)-N-methyl-amino]-methyl}-pyrimidin-4-yloxy)-4-methyl-benzamid;
N-(5-tert-Butyl-3-methansulfonylamino-2-methoxy-phenyl)-3-{2-[(2-dimethylamino-ethylamino)-methyl]-pyrimidin-4-yloxy}-4-methyl-benzamid;
N-(5-tert-Butyl-3-methansulfonylamino-2-methoxy-phenyl)-3-(2-{[(2-dimethylaminoethyl)-methyl-amino]-methyl}-pyridin-4-yloxy)-4-methyl-benzamid;
N-(5-tert-Butyl-3-methansulfonylamino-2-methoxy-phenyl)-3-{2-[(2-dimethylamino-ethylamino)-methyl]-pyridin-4-yloxy}-4-methyl-benzamid;
N-(5-tert-Butyl-3-methansulfonylamino-2-methoxy-phenyl)-3-methyl-5-[2-(4-methylpiperazin-1-ylmethyl)-pyrimidin-4-yloxy]-benzamid;
N-(5-tert-Butyl-3-methansulfonylamino-2-methoxy-phenyl)-3-{6-[(2-dimethylamino-ethylamino)-methyl]-pyrimidin-4-yloxy}-4-methyl-benzamid;
N-(5-tert-Butyl-3-methansulfonylamino-2-methoxy-phenyl)-3-(6-{[(2-dimethylaminoethyl)-methyl-amino]-methyl}-pyrimidin-4-yloxy)-4-methyl-benzamid;
N-(5-tert-Butyl-3-methansulfonylamino-2-methoxy-phenyl)-4-methyl-3-[6-(1-methylpiperidin-4-ylmethyl)-pyrimidin-4-yloxy]-benzamid,
N-(5-tert-Butyl-3-methansulfonylamino-2-methoxy-phenyl)-4-methyl-3-[2-(1-methylpiperidin-4-ylmethyl)-pyrimidin-4-yloxy]-benzamid;
N-(5-tert-Butyl-3-methansulfonylamino-2-methoxy-phenyl)-4-methyl-3-[2-(1-methylpiperidin-4-ylmethyl)-pyridin-4-yloxy]-benzamid;
N-(5-tert-Butyl-3-methansulfonylamino-2-methoxy-phenyl)-4-methyl-3-[2-(pyrrolidin-3-ylamino)-pyrimidin-4-yloxy]-benzamid;
N-(5-tert-Butyl-3-methansulfonylamino-2-methoxy-phenyl)-4-chlor-3-[2-(4-methylpiperazin-1-ylmethyl)-pyrimidin-4-yloxy]-benzamid;
N-(5-tert-Butyl-3-methansulfonylamino-2-methoxy-phenyl)-3-chlor-4-methyl-5-[2-(4-methyl-piperazin-1-ylmethyl)-pyrimidin-4-yloxy]-benzamid;
N-(5-tert-Butyl-3-methansulfonylamino-2-methoxy-phenyl)-4-chlor-3-[6-(1-methylpiperidin-4-ylamino)-pyrimidin-4-yloxy]-benzamid;
N-(5-tert-Butyl-3-methansulfonylamino-2-methoxy-phenyl)-4-chlor-3-[2-(1-methylpiperidin-4-ylamino)-pyridin-4-yloxy]-benzamid;
N-(5-tert-Butyl-3-methansulfonylamino-2-methoxy-phenyl)-4-chlor-3-[6-(piperidin-4-ylamino)-pyrimidin-4-yloxy]-benzamid;
N-(5-tert-Butyl-3-methansulfonylamino-2-methoxy-phenyl)-4-chlor-3-[2-(1-methylpiperidin-4-ylamino)-pyrimidin-4-yloxy]-benzamid;
N-(5-tert-Butyl-3-methansulfonylamino-2-methoxy-phenyl)-4-methyl-3-{2-[methyl-(1-methyl-piperidin-4-yl)-amino]-pyrimidin-4-yloxy}-benzamid;
N-(5-tert-Butyl-3-methanesulfonylamino-2-methoxy-phenyl)-4-methyl-3-{6-[methyl-(1-methyl-piperidin-4-yl)-amino]-pyrimidin-4-yloxy}-benzamid;
N-(5-tert-Butyl-3-methansulfonylamino-2-methoxy-phenyl)-4-chlor-3-[2-(piperidin-4-ylamino)-pyrimidin-4-yloxy]-benzamid;
N-(5-tert-Butyl-3-methansulfonylamino-2-methoxy-phenyl)-4-chlor-3-(2-piperidin-4-ylmethyl-pyridin-4-yloxy)-benzamid;
N-(5-tert-Butyl-3-methansulfonylamino-2-methoxy-phenyl)-4-chlor-3-(2-piperidin-4-ylmethyl-pyrimidin-4-yloxy)-benzamid;
N-(5-tert-Butyl-3-methansulfonylamino-2-methoxy-phenyl)-4-chlor-3-{6-[(2-dimethylamino-ethylamino)-methyl]-pyrimidin-4-yloxy}-benzamid;
N-(5-tert-Butyl-3-methansulfonylamino-2-methoxy-phenyl)-4-chlor-3-(6-{[(2-dimethylamino-ethyl)-methyl-amino]-methyl}-pyrimidin-4-yloxy)-benzamid;
N-(5-tert-Butyl-3-methansulfonylamino-2-methoxy-phenyl)-4-chlor-3-[2-(piperidin-4-ylamino)-pyridin-4-yloxy]-benzamid;
N-(5-tert-Butyl-3-methansulfonylamino-2-methoxy-phenyl)-4-chlor-3-(6-piperazin-1-ylmethyl-pyrimidin-4-yloxy)-benzamid:
N-(5-tert-Butyl-3-methansulfonylamino-2-methoxy-phenyl)-4-chlor-3-(6-piperidin-4-ylmethyl-pyrimidin-4-yloxy)-benzamid;
N-(5-tert-Butyl-3-methansulfonylamino-2-methoxy-phenyl)-4-chlor-3-[6-(4-methylpiperazin-1-ylmethyl)-pyrimidin-4-yloxy]-benzamid;
N-(5-tert-Butyl-3-methansulfonylamino-2-methoxy-phenyl)-4-chlor-3-[6-(1-methylpiperidin-4-ylmethyl)-pyrimidin-4-yloxy]-benzamid;
N-(5-tert-Butyl-3-methansulfonylamino-2-methoxy-phenyl)-4-chlor-3-[2-(1-methylpiperidin-4-ylmethyl)-pyrimidin-4-yloxy]-benzamid;
N-(5-tert-Butyl-3-methansulfonylamino-2-methoxy-phenyl)-4-chlor-3-(2-{[(2-dimethylamino-ethyl)-methyl-amino]-methyl}-pyridin-4-yloxy)-benzamid;
N-(5-tert-Butyl-3-methansulfonylamino-2-methoxy-phenyl)-3-[2-(*trans*-4-dimethylamino-cyclohexylamino)-pyrimidin-4-yloxy]-4-methyl-benzamid;
N-(5-tert-Butyl-3-methansulfonylamino-2-methoxy-phenyl)-4-chlor-3-{2-[(2-dimethylamino-ethylamino)-methyl]-pyridin-4-yloxy}-benzamid;
N-(5-tert-Butyl-3-methansulfonylamino-2-methoxy-phenyl)-4-chlor-3-(2-piperazin-1-ylmethyl-pyridin-4-yloxy)-benzamid;
N-(5-tert-Butyl-3-methansulfonylamino-2-methoxy-phenyl)-4-methyl-3-{2-[methyl-(1-methyl-pyrrolidin-3-yl)-amino]-pyrimidin-4-yloxy}-benzamid;
N-(5-tert-Butyl-3-methansulfonylamino-2-methoxy-phenyl)-4-chlor-3-[2-(4-methylpiperazin-1-ylmethyl)-pyridin-4-yloxy]-benzamid;
N-(5-tert-Butyl-3-methansulfonylamino-2-methoxy-phenyl)-4-methyl-3-{6-[methyl-(1-methyl-pyrrolidin-3-yl)-amino]-pyrimidin-4-yloxy}-benzamid;
N-(5-tert-Butyl-3-methansulfonylamino-2-methoxy-phenyl)-4-chlor-3-[2-(4-methyl-[1,4]diazepan-1-ylmethyl)-pyrimidin-4-yloxy]-benzamid;
N-(5-tert-Butyl-3-methansulfonylamino-2-methoxy-phenyl)-4-chlor-3-(2-{[(2-dimethylamino-ethyl)-methyl-amino]-methyl}-pyrimidin-4-yloxy)-benzamid;
N-(5-tert-Butyl-3-methansulfonylamino-2-methoxy-phenyl)-4-chlor-3-{2-[(2-dimethylamino-ethylamino)-methyl]-pyrimidin-4-yloxy}-benzamid;
N-(5-tert-Butyl-3-methansulfonylamino-2-methoxy-phenyl)-4-chlor-3-[6-(4-methyl-[1,4]diazepan-1-ylmethyl)-pyrimidin-4-yloxy]-benzamid;
N-(5-tert-Butyl-3-methansulfonylamino-2-methoxy-phenyl)-4-chlor-3-(2-piperazin-1-ylmethyl-pyrimidin-4-yloxy)-benzamid;
N-(5-tert-Butyl-3-methansulfonylamino-2-methoxy-phenyl)-4-chlor-3-(6-[1,4]diazepan-1-ylmethyl-pyrimidin-4-yloxybenzamid;
N-(5-tert-Butyl-3-methansulfonylamino-2-methoxy-phenyl)-4-chlor-3-[2-(4-methyl-[1,4]diazepan-1-ylmethyl)-pyridin-4-yloxy]-benzamid;
N-(5-tert-Butyl-3-methansulfonylamino-2-methoxy-phenyl)-4-chlor-3-(2-[1,4]diazepan-1-ylmethyl-pyridin-4-yloxy)-benzamid;
N-(5-tert-Butyl-3-methansulfonylamino-2-methoxy-phenyl)-4-chlor-3-[2-(1-methylpiperidin-4-ylmethyl)-pyridin-4-yloxy]-benzamid;
N-(5-tert-Butyl-3-methansulfonyl-2-methoxy-phenyl)-4-methyl-3-(2-piperidin-4-ylmethyl-pyrimidin-4-yloxy)-benzamid;
N-(5-tert-Butyl-3-methansulfonylamino-2-methoxy-phenyl)-3-(2-[1,4]diazepan-1-ylmethyl-pyridin-4-yloxy)-4-methyl-benzamid;
N-(5-tert-Butyl-2-methoxy-3-methylsulfanylmethyl-phenyl)-4-methyl-3-(2-piperidin-4-ylmethyl-pyridin-4-yloxy)-benzamid;
N-(5-tert-Butyl-3-methansulfonylmethyl-2-methoxy-phenyl)-4-methyl-3-(2-piperidin-4-ylmethyl-pyridin-4-yloxy)-benzamid;
N-(5-tert-Butyl-3-methansulfinylmethyl-2-methoxy-phenyl)-4-methyl-3-(2-piperidin-4-ylmethyl-pyridin-4-yloxy)-benzamid;
N-[5-tert-Butyl-3-(cyclopropancarbonyl-amino)-2-methoxy-phenyl]-4-methyl-3-(2-piperidin-4-ylmethyt-pyndin-4-yloxy)-benzamid;
N-(5-tert-Butyl-2-isopropoxy-3-methansulfonylamino-phenyl)-4-methyl-3-(2-piperidin-4-ylmethyl-pyridin-4-yloxy)-benzamid;
N-(5-tert-Butyl-2-ethoxy-3-methansulfonylamino-phenyl)-4-methyl-3-(2-piperidin-4-ylmethyl-pyridin-4-yloxy)-benzamid;
N-[5-tert-Butyl-2-methoxy-3-(2-methyl-propan-1-sulfinylamino)-phenyl]-4-methyl-3-(2-piperidin-4-ylmethyl-pyridin-4-yloxy)-benzamid;
N-[3-(Butan-1-sulfonylamino)-5-tert-butyl-2-methoxy-phenyl]-4-methyl-3-(2-piperidin-4-ylmethyl-pyridin-4-yloxy)-benzamid;
N-(5-tert-Butyl-2-methoxy-3-pentanoylamino-phenyl)-4-methyl-3-(2-piperidin-4-ylmethyl-pyridin-4-yloxy)-benzamid;
N-(5-tert-Butyl-3-methansulfonyl-2-methoxy-phenyl)-4-methyl-3-(2-piperidin-4-ylmethyl-pyridin-4-yloxy)-benzamid;
N-[5-tert-Butyl-2-methoxy-3-(3-methyl-butyrylamino)-phenyl]-4-methyl-3-(2-piperidin-4-ylmethyl-pyridin-4-yloxy)-benzamid;
N-(5-tert-Butyl-3-isobutyrylamino-2-methoxy-phenyl)-4-methyl-3-(2-piperidin-4-ylmethyl-pyridin-4-yloxy)-benzamid;
N-(5-tert-Butyl-3-methansulfinyl-2-methoxy-phenyl)-4-methyl-3-(2-piperidin-4-ylmethyl-pyridin-4-yloxy)-benzamid;
N-(5-tert-Butyl-3-methansulfinylmethyl-2-methoxy-phenyl)-4-methyl-3-(2-piperidin-4-ylmethyl-pyridin-4-yloxy)-benzamid;
N-(5-tert-Butyl-3-methansulfinylmethyl-2-methoxy-phenyl)-4-methyl-3-(2-piperidin-4-ylmethyl-pyridin-4-yloxy)-benzamid;
N-(5-tert-Butyl-3-cyclopropansulfonylamino-2-methoxy-phenyl)-4-methyl-3-(2-piperidin-4-ylmethyl-pyridin-4-yloxy)-benzamid;
3-(2-Azepan-4-ylmethyl-pyridin-4-yloxy)-N-(5-tert-butyl-3-methansulfonylamino-2-methoxy-phenyl)-4-methyl-benzamid;
3-(2-Azepan-4-ylmethyl-pyrimidin-4-yloxy)-N-(5-tert-butyl-3-methansulfonylamino-2-methoxy-phenyl)-4-methyl-benzamid;
N-(5-tert-Butyl-3-methansulfonylamino-2-methoxy-phenyl)-4-methyl-3-[2-(1-methyl-azepan-4-ylmethyl)-pyridin-4-yloxy]-benzamid;
N-(5-tert-Butyl-3-methansulfonylamino-2-methoxy-phenyl)-4-methyl-3-[2-(1-methyl-azepan-4-ylmethyl)-pyrimidin-4-yloxy]-benzamid;
3-(6-Azepan-4-ylmethyl-pyrimidin-4-yloxy)-N-(5-tert-butyl-3-methanesulfonylamino-2-methoxy-phenyl)-4-methyl-benzamid;
N-(5-tert-Butyl-3-methansulfonylamino-2-methoxy-phenyl)-4-methyl-3-[6-(1-methyl-azepan-4-ylmethyl)-pyrimidin-4-yloxy]-benzamid;
N-(3-Methanesulfonylamino-2-methoxy-5-pentafluoroethyl-phenyl)-4-methyl-3-(2-piperidin-4-ylmethyl-pyridin-4-yloxy)-benzamide;
N-(5-tert-Butyl-3-hydroxymethyl-2-methoxy-phenyl)-4-methyl-3-[2-(4-methyl-piperazin-1-ylmethyl)-pyrimidin-4-yloxy]-benzamid;
N-(5-tert-Butyl-3-methansulfinyl-2-methoxy-phenyl)-4-methyl-3-[2-(4-methyl-piperazin-1-ylmethyl)-pyrimidin-4-yloxy]-benzamid;
N-(5-tert-Butyl-3-dimethylaminomethyl-2-methoxy-phenyl)-4-methyl-3-(2-piperidin-4-ylmethyl-pyridin-4-yloxy)-benzamid;
N-(5-tert-Butyl-3-methansulfonylamino-2-methoxy-phenyl)-4-methyl-3-{2-[4-(2,2,2-trifluor-ethyl)-piperazin-1-ylmethyl]-pyridin-4-yloxy}-benzamid;
N-(5-tert-Butyl-3-methansulfonylamino-2-methoxy-phenyl)-3-[2-(4-cyanmethyl-piperazin-1-ylmethyl)-pyridin-4-yloxy]-4-methyl-benzamid;
N-(5-tert-Butyl-2-methoxy-3-pyrrolidin-1-ylmethyl-phenyl)-4-methyl-3-(2-piperidin-4-ylmethyl-pyridin-4-yloxy)-benzamid;
N-(5-tert-Butyl-3-hydroxymethyl-2-methoxy-phenyl)-4-methyl-3-(2-piperazin-1-ylmethyl-pyridin-4-yloxy)-benzamid;
N-(5-tert-Butyl-3-hydroxymethyl-2-methoxy-phenyl)-4-methyl-3-[2-(4-methyl-piperazin-1-ylmethyl)-pyridin-4-yloxy]-benzamid;
N-(5-tert-Butyl-3-hydroxymethyl-2-methoxy-phenyl)-3-(2-[1,4]diazepan-1-ylmethyl-pyridin-4-yloxy)-4-methyl-benzamid;
N-(5-tert-Butyl-3-hydroxymethyl-2-methoxy-phenyl)-4-methyl-3-[2-(4-methyl-[1,4]diazepan-1-ylmethyl)-pyridin-4-yloxyl-benzamid;
N-(5-tert-Butyl-3-cyclopropylaminomethyl-2-methoxy-phenyl)-4-methyl-3-(2-piperidin-4-ylmethyl-pyridin-4-yloxy)-benzamid
deren Tautomere, deren Stereoisomere und deren Gemische.

12. Physiologisch verträgliche Salze der Verbindungen nach einem oder mehreren der Ansprüche 1 bis 11 mit anorganischen oder organischen Säuren.

13. Verbindung nach einem oder mehreren der Ansprüche 1 bis 11 oder physiologisch verträgliche Salze gemäß Anspruch 12 zur Verwendung als Arzneimittel.

14. Arzneimittel, enthaltend eine Verbindung nach einem oder mehreren der Ansprüche 1 bis 11 oder ein physiologisch verträgliches Salz gemäß Anspruch 12 neben gegebenenfalls einem oder mehreren inerten Trägerstoffen, Konservierungs- und/oder Verdünnungsmitteln.

15. Verwendung mindestens einer Verbindung nach einem oder mehreren der Ansprüche 1 bis 11 oder eines physiologisch verträglichen Salzes gemäß Anspruch 12, zur Herstellung eines Arzneimittels, das zur Behandlung oder Prophylaxe von Atemwegserkrankungen geeignet ist.

16. Verwendung nach Anspruch 15, **dadurch gekennzeichnet, dass** es sich bei der Atemwegserkrankung um COPD oder Asthma handelt.

17. Verfahren zur Herstellung eines Arzneimittels gemäß Anspruch 14, **dadurch gekennzeichnet, dass** auf nicht-chemischem Wege eine Verbindung nach mindestens einem der Ansprüche 1 bis 11 oder ein physiologisch verträgliches Salz gemäß Anspruch 12 in einen oder mehrere inerte Trägerstoffe, Konservierungs- und/oder Verdünnungsmittel eingearbeitet wird.

18. Verfahren zur Herstellung der Verbindungen der allgemeinen Formel I gemäß den Ansprüchen 1 bis 11, **dadurch gekennzeichnet, dass** eine Verbindung der allgemeinen Formel II, 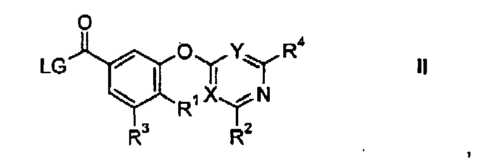 in der R¹ bis R⁴, X und Y die in den Ansprüchen 1 bis 6 und 10 angegebenen Bedeutungen besitzen und LG
Fluor, Chlor, Brom, Cyan, C₁₋₁₀-Alkoxy, C₁₋₆-Alkylsulfanyl, C₂₋₄-Alkenyl-oxy, C₂₋₄-Alkinyloxy, Benzotriazol-1-yloxy, [1,2,3]Triazolo[4,5-b]pyridin-3-yloxy, C₅₋₁₀-Heteroaryl (über N an II gebunden), Succinyl-N-oxy. C₁₋₄-Alkylcarbonyloxy, Di-(C₁₋₄-alkyl)aminocarbonyloxy, Pyrrol-1-ylcarbonyloxy, Piperidin-1-yl-carbonyloxy, Morpholin-4-ylcarbonyloxy, Tri-(C₁₋₄-alkyl)carbamimidoyloxy, N,N,N',N'-Tetra-(C₁₋₄-alkyl)-uronium-O-yl, N,N'-Dicyclohexyluron-O-yl, N-(3-Dimethylaminopropyl)-N'-ethyl-uronyl, Di-(C₁₋₄-alkyloxy)-phosphoryloxy, Bis(di-C₁₋₄-alkylamino)-phosphoryloxy, Dipyrrolidinyl-phosphoryloxy, C₆₋₁₀-Arylsulfanyl, C₅₋₁₀-Heteroarylsulfanyl, C₆₋₁₀-Aryloxy oder C₅₋₁₀-Heteroaryloxy ist,
wobei alle in der Definition erwähnten Alkyl-, Alkenyl- und Alkinyl-Gruppen mit Fluor, Chlor, C₁₋₃-Alkyl und/oder C₁₋₃-Alkoxy einfach oder mehrfach substituiert sein können,
wobei alle in der Definition erwähnten Aryl-Gruppen für Phenyl oder Naphthyl und alle Heteroaryl-Gruppen für Pyridinyl, Pyrimidinyl, Triazinyl, Imidazolyl, Pyrazolyl, Triazolyl, Tetrazolyl stehen, die, sowohl Aryl- als auch Heteroaryl-Gruppen, optional einfach oder mehrfach mit identischen oder unterschiedlichen Resten ausgewählt von Fluor, Chlor, Brom, C₁₋₃-Alkyl, C₁₋₃Alkyloxy, Nitro, Cyan und Di-(C₁₋₃-alkyl)amino substituiert sind,
mit einem Anilin der allgemeinen Formel 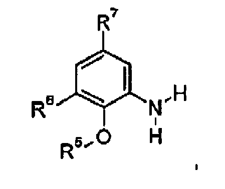 in der R⁵, R⁶ und R⁷ die in den Ansprüchen 1, 2, 7, 8 und 9 angegebenen Bedeutungen besitzen
optional in Gegenwart einer Base und/oder eines Additivs wie z.B. Triethylamin, Pyridin, Ethyldiisopropylamin, 4-Dimethylaminopyridin, Kaliumcarbonat oder 1-Hydroxybenzotriazol,
zur Reaktion gebracht wird; und
erforderlichenfalls ein bei den vorstehend unter a) und b) beschriebenen Umsetzungen verwendeter Schutzrest wieder abgespalten wird und/oder
falls gewünscht eine so erhaltene Verbindung der allgemeinen Formel I in ihre Stereoisomere aufgetrennt wird und/oder
falls gewünscht eine so erhaltene Verbindung der allgemeinen Formel I in ihre Salze, insbesondere für die pharmazeutische Anwendung in ihre physiologisch verträglichen Salze, überführt wird.

19. Verfahren zur Herstellung der Verbindungen der allgemeinen Formel I gemäß den Ansprüchen 1 bis 11, **dadurch gekennzeichnet, dass** eine Verbindung der allgemeinen
Formel III 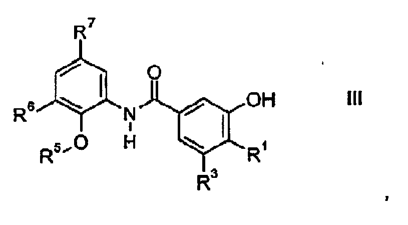 in der R¹, R³, R⁵, R⁶ und R⁷ die in den Ansprüchen 1 bis 3, 5 und 7 bis 9, angegebenen Bedeutungen besitzen,
mit einer Verbindung der allgemeinen Formel IV 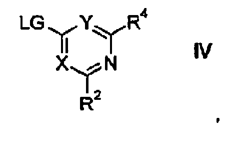 in der R² und R⁴ die in den Ansprüchen 1, 2, 4, 6 und 10 angegebenen Bedeutungen besitzen und LG eine Austrittsgruppe darstellt, insbesondere bedeutet LG F, Cl, Br, I, O-C₁₋₆-Alkyl, O-C₆₋₁₀-Aryl, Si(O)ₙ-C₁₋₄-Alkyl, S(O)ₘ-C₅₋₁₀-Aryl, OSO₂-C₁₋₄-Alkyl, OSO₂-C₆₋₁₀-Aryl, NO₂,
wobei alle erwähnten Alkyl-Gruppen optional mit Fluor, C₁₋₃-Alkyl und/oder C₁₋₃-Alkoxy einfach oder mehrfach substituiert sind, und wobei alle erwähnten Aryl-Gruppen für Phenyl oder Naphthyl stehen, die optional einfach oder mehrfach mit identischen oder unterschiedlichen Resten ausgewählt aus Fluor, Chlor, C₁₋₃-Alkyl, C₁₋₃-Alkyloxy, Nitro und Cyan substituiert sind,
wobei n und m unabhängig voneinander 0, 1 oder 2 sein können,
in Gegenwart einer Base, z.B. NaH, KH, KO*t*Bu, NaO*t*Bu, NaOMe, NaOEt, NaO*i*Pr, KF, K₂CO₃, Cs₂CO₃, Pyridin, 4-Dimethylaminopyridin, NEt₃ oder EtN*i*Pr₂,
optional in Gegenwart eines Katalysators, z.B. eines Cu- oder Pd-Komplexes, umgesetzt wird: und
erforderlichenfalls ein bei den vorstehend unter a) und b) beschriebenen Umsetzungen verwendeter Schutzrest wieder abgespalten wird und/oder
falls gewünscht eine so erhaltene Verbindung der allgemeinen Formel I in ihre Stereoisomere aufgetrennt wird und/oder
falls gewünscht eine so erhaltene Verbindung der allgemeinen Formel I in ihre Salze, insbesondere für die pharmazeutische Anwendung in ihre physiologisch verträglichen Salze, überführt wird.

## Claims

1. Heteroaryloxy-substituted benzoic acid amides of general formula I 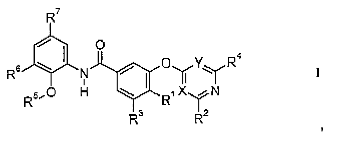 wherein
R¹ is L₁-R₈,
where L₁ is selected from a bond, O, CO, NH, CONH, NHCO, SO, SO₂, SO₂NH, NHSO₂ and C₁-C₄-akylene,
where R₈ is selected from hydrogen, halogen, C₁₋₆-alkyl, C₂₋₆-alkynyl, C₂₋₆-alkenyl, C₃₋₆-cycloalkyl, C₃₋₈-heterocycloalkyl, C₆₋₁₀-aryl, C₅₋₁₀-heteroaryl, cyano, carboxy, C₁₋₄-alkyloxycarbonyl, nitro, amino, di-(C₁₋₄-alkyl)-amino, hydroxy, C₁₋₆-alkylsulphanyl,
wherein R₆ may be partly or completely fluorinated and/or may be substituted by one or more substituents L, and wherein in heterocycloalkyl groups a methylene group may be substituted by CO, SO or SO₂ and
wherein R¹ is not hydrogen if R³ denotes hydrogen, and
R² is L₂-R₉,
wherein L₂ is selected from a bond, O, CO, NH, CONH, NHCO, SO, SO₂, SO₂NH, NHSO₂ and C₁-C₄-alkylene, wherein R₉ is selected from hydrogen, halogen, C₁₋₆-alkyl, C₂₋₆-alkynyl, C₂₋₆-alkenyl, C₃₋₈-cycloalkyl, C₄₋₈-cycloalkenyl, C₃₋₈-heterocycloalkyl, C₆₋₁₀-aryl, C₅₋₁₀-heteroaryl, cyano, carboxy, C₁₋₄-alkyloxycarbonyl, nitro, amino, di-(C₁₋₄-alkyl)-amino, hydroxy, C₁₋₆-alkylsulphanyl,
wherein R₉ may be partly or completely fluorinated and/or may be substituted by one or more substituents L, and
wherein in the alkyl groups mentioned optionally one or two CH₂ groups may be exchanged for O and/or NR^{N},
wherein in the cycloalkyl groups mentioned one or two CH₂ groups may be replaced independently of one another by NR^{N}, O, S, CO, SO or SO₂ and one or two CH groups may be replaced by N, and
R³ is L₃-R₁₀
wherein L₃ is selected from a bond, O, CO, NH, CONH, NHCO, SO, SO₂, SO₂NH, NHSO₂ and C₁-C₄-alkylene
wherein R₁₀ is selected from hydrogen, halogen, C₁₋₆-alkyl, C₂₋₆-alkynyl, C₂₋₆-alkenyl, C₃₋₈-cycloalkyl, C₄₋₈-cycloalkenyl, C₃₋₈-heterocycloalkyl, C₅₋₁₀-aryl, C₅₋₁₀-heteroaryl, cyano, carboxy, C₁₋₄-alkyloxycarbonyl, nitro, amino, di-(C₁₋₄-alkyl)-amino, hydroxy, C₁₋₆-alkylsulphanyl
wherein R₁₀ may be partly or completely fluorinated and/or may be substituted by one or more substituents L, and
wherein R³ is not hydrogen if R¹ denotes hydrogen,
R⁴ is L₄-R₁₁
wherein L₄ is selected from a bond, O, CO, NH, CONH, NHCO,
wherein R₁₁ is selected from hydrogen, halogen, amino, C₁₋₆-alkyl, C₃₋₈cycloalkyl, C₃₋₈-heterocycloalkyl, trifluoromethyl, difluoromethyl, cyano, carboxy, C₁₋₄-alkyloxycarbonyl, di-(C₁₋₄-alkyl)-amino, C₁₋₄-alkylsulphanyl,
R⁵ is C₁₋₆-alkyl,
R⁶ is selected from dimethylamino-C₁₋₃-alkyl, cyclopropylamino-C₁₋₃-alkyl, pyrrolidinyl-C₁₋₃-alkyl, hydroxy-C₁₋₃-alkyl, methylsulphanyl-C₁₋₃-alkyl, methylsulphinyl-C₁₋₃-alkyl, methylsulphonyl-C₁₋₃-alkyl, C₁₋₄-alkylcarbonylamino, C₃₋₅-cycloalkyl-carbonylamino, C₁₋₄-alkylsulphonylamino, C₃₋₅-cycloalkylsulphonylamino, C₁₋₃-alkylsulphinyl and C₁₋₃-alkylsulphonyl,
R⁷ is C₁₋₆-alkyl, trifluoromethyl, pentafluoroethyl,
X,Y independently of one another denote N or C-R₁₃, wherein X and Y cannot both be N and R₁₃ is selected from hydrogen, fluorine, chlorine, C₁₋₄-alkyl, trifluoromethyl, OH, C₁₋₄-alkyloxy, trifluoromethoxy, difluoromethoxy and cyano,
R^{N} is selected from H, C₁₋₄-alkyl, 1,1,1-trifluoroethyl, cyanomethyl, acetyl, methylsulphonyl, C₆₋₁₀-arylmethyl,
L is selected from among halogen, cyano, C₁₋₆-alkyl, C₁₋₆-alkyloxy, amino, C₁₋₃-alkylamino, di-(C₁₋₉-alkyl)-amino, C₁₋₄-alkylcarbonylamino, difluoromethyl, C₁₋₄-alkylsulphonylamino trifluoromethyl, hydroxy, C₁₋₃-alkoxy, difluoromethoxy, trifluoromethoxy, acetylamino, C₆₋₁₀-aryl and C₅₋₁₀-heteroaryl.

2. Heteroaryloxy-substituted benzoic acid amides according to claim 1 wherein
R¹ is selected from hydrogen, halogen, C₁₋₆-alkyl, C₂₋₆-alkynyl, C₂₋₆-alkenyl, C₃₋₇-cycloalkyl, C₃₋₇-cycloalkyl-C₁₋₃-alkyl, C₆₋₁₀-aryl, C₅₋₁₀-heteroaryl, C₆₋₁-aryl-C₁₋₃-alkyl, C₅₋₁₀-heteroaryl-C₁₋₃-alkyl, C₁₋₄-alkylcarbonyl, cyano, aminocarbonyl, C₁₋₄-alkyl-aminocarbonyl, di-(C₁₋₃-alkyl)-aminocarbonyl, pyrrolidin-1-ylcarbonyl, piperidin-1-ylcarbonyl, morpholin-4-ylcarbonyl, piperazin-1-ylcarbonyl, 4-(C₁₋₄-alkyl)-piperazin-1-ylcarbonyl, carboxy, C₁₋₄-alkyloxycarbonyl, nitro, amino, C₁₋₄-alkylamino, di-(C₁₋₃-alkyl)-amino, pyrrolidin-1-yl, piperidin-1-yl, morpholin-4-yl, piperazin-1-yl, 4-(C₁₋₄-alkyl)piperazin-1-yl, C₁₋₄-alkylcarbonylamino, C₁₋₄-alkylsulphonylamino, hydroxy, C₁₋₆-alkyloxy, C₃₋₇-cycloalkyloxy, C₆₋₁₀-aryloxy, C₁₋₄-alkylsulphanyl, C₁₋₄-alkylsulphinyl and C₁₋₄-alkylsulphonyl,
wherein alkyl, alkenyl, alkynyl, cycloalkyl and heterocycloalkyl groups may be partly or completely fluorinated and/or mono- or disubstituted by identical or different substituents selected from cyano, hydroxy, C₁₋₃-alkyloxy, acetylamino, methylsulphonylamino and C₁₋₃-alkyl, and
wherein in cycloalkyl groups one or two methylene groups may be substituted independently of one another by O, S, CO, SO or SO₂, and
wherein in N-heterocycloalkyl groups a methylene group may be substituted by CO or SO₂,
wherein R¹ is not hydrogen if R³ denotes hydrogen, and
R² is selected from hydrogen, C₁₋₈-alkyl, C₂₋₆-alkenyl, C₂₋₆-alkynyl, C₃₋₇-cycloalkyl-C₁₋₄-alkyl, C₁₋₆-alkyloarbonyl-C₁₋₃-alkyl, C₆₋₁₀-aryl-C₁₋₄-alkyl, C₅₋₁₀-heteroaryl-C₁₋₄-alkyl, C₃₋₇-cycloalkyl, C₁₋₆-alkylcarbonyl, C₃₋₇-cyoloalkyl-carbonyl, cyano, aminocarbonyl, carboxy, amino, C₁₋₄-alkylcarbonylamino, C₁₋₄-alkylsulphonylamino, C₁₋₄-alkyloxy, C₆₋₁₀-aryl, C₅₋₁₀-heteroaryl, hydroxy,
wherein all the above-mentioned alkyl, alkenyl, alkynyl and cycloalkyl groups may be partly or completely fluorinated and/or mono-, di- or trisubstituted by identical or different substituents selected from C₁₋₃-alkyl, cyano, hydroxy, C₁₋₃-alkyloxy, amino, C₁₋₃-alkylamino, di-(C₁₋₃-alkyl)amino, acetylamino, methylsulphonylamino, C₆₋₁₀-aryl and C₅₋₁₀-heteroaryl,
wherein in the alkyl groups mentioned one or two CH₂ groups may optionally be exchanged for O and/or NR^{N},
wherein in the cycloalkyl groups mentioned one or two CH₂ groups may be replaced independently of one another by NR^{N}, O, S, CO, SO or SO₂ and one or two CH groups may be replaced by N, and
R³ is selected from hydrogen, halogen, C₁₋₄-alkyl, C₂₋₆-alkynyl, C₂₋₆-alkenyl, C₃₋₇-cycloalkyl-C₁₋₃-alkyl, C₃₋₆-cycloalkyl, trifluoromethyl, difluoromethyl, hydroxy, C₁₋₄-alkyloxy, trifluoromethoxy, diffluoromethoxy, cyano, aminocarbonyl, C₁₋₄-alkylaminocarbonyl, di-(C₁₋₃-alkyl)-aminocarbonyl, carboxy, C₁₋₄-alkyloxycarbonyl, amino, C₁₋₄-alkylamino, di-(C₁₋₃-alkyl)-amino, C₁₋₄-alkylcarbonylamino, C₁₋₄-alkylsulphonylamino, wherein R³ is not hydrogen if R¹ denotes hydrogen,
R⁴ is selected from hydrogen, C₁₋₄-alkyl, C₃₋₆-cycloalkyl, trifluoromethyl, difluoromethyl, cyano, carboxy, aminocarbonyl, C₁₋₃-alkylaminocarbonyl, di-(C₁₋₃-alkyl)aminocarbonyl, C₁₋₄-alkyloxycarbonyl, amino, C₁₋₃-alkylamino, di-(C₁₋₃-alkyl)amino, hydroxy, C₁₋₄-alkyloxy, C₁₋₄-alkylsulphanyl,
R⁵ is C₁₋₄-alkyl,
R⁶ is selected from dimethylamino-C₁₋₃-alkyl, cyclopropylamino-C₁₋₃-alkyl, pyrrolidinyl-C₁₋₃-alkyl, hydroxy-C₁₋₃-alkyl, methylsulphanyl-C₁₋₃-alkyl, methylsulphinyl-C₁₋₃-alkyl, methylsulphonyl-C₁₋₃-alkyl, C₁₋₄-alkylcarbonylamino, C₃₋₅-cycloalkyl-carbonylamino, C₁₋₄-alkylsulphonylamino, C₃₋₅-cycloalkylsulphonylamino, C₁₋₃-alkylsulphinyl and C₁₋₃-alkylsulphonyl,
R⁷ is selected from C₄-alkyl, trifluoromethyl, pentafluoroethyl,
X,Y independently of one another denote N or CR₁₃, while X and Y cannot both represent N and R₁₃ is selected from hydrogen, fluorine, chlorine, C₁₋₄-alkyl, trifluoromethyl, hydroxy, C₁₋₄-alkyloxy, trifluoromethoxy, difluoromethoxy, cyano,
R^{N} is selected from H, C₁₋₄-alkyl, 1,1,1-trifluoroethyl, cyanomethyl, acetyl, methylsulphonyl, C₆₋₁₀-arylmethyl,
L is selected from among fluorine, chlorine, bromine, C₁₋₃-alkyl, difluoromethyl, trifluoromethyl, hydroxy, C₁₋₃-alkoxy, diffluoromethoxy, trifluoromethoxy, acetylamino, methylsulphonylamino and cyano,
wherein by the C₅₋₁₀-aryl groups mentioned in the definition of the above groups are meant phenyl or naphthyl groups, which may be mono- or disubstituted independently of one another by identical or different groups L; and
by the heteroaryl groups mentioned in the definition of the above-mentioned groups are meant a pyrrolyl, furanyl, thienyl, pyridyl, indolyl, benzofuranyl, benzothiophenyl, quinolinyl or isoquinolinyl group,
or a pyrrolyl, furanyl, thienyl or pyridyl group is meant, wherein one or two methyne groups are replaced by nitrogen atoms,
or an indolyl, benzofuranyl, benzothiophenyl, quinolinyl or isoquinolinyl group is meant, wherein one to three methyne groups are replaced by nitrogen atoms,
wherein the above-mentioned heteroaryl groups may be mono- or disubstituted independently of one another by identical or different groups L;
wherein, unless stated otherwise, the above-mentioned alkyl groups may be straight-chain or branched,
the tautomers, the stereoisomers, the mixtures and the salts thereof, particularly the physiologically acceptable salts thereof.

3. Heteroaryloxy-substituted benzoic acid amides according to one of the preceding claims, **characterised in that**
R¹ is selected from hydrogen, fluorine, chlorine, bromine, C₁₋₄-alkyl, trifluoromethyl, C₃₋₆-cycloalkyl, cyano, aminocarbonyl, C₁₋₄-alkyl-aminocarbonyl, di-(C₁₋₃-alkyl)-aminocarbonyl, carboxy, C₁₋₄-alkyloxycarbonyl, amino, C₁₋₄-alkylamino, di-(C₁₋₃-alkyl)-amino, pyrrolidin-1-yl, piperidin-1-yl, morpholin-4-yl, acetylamino, methylsulphonylamino, hydroxy, C₁₋₄-alkyloxy, trifluoromethoxy, difluoromethoxy and C₁₋₄-alkylsulphonyl,
wherein the above-mentioned alkyl, cycloalkyl and N-heterocycloalkyl groups may be mono- or disubstituted by identical or different substituents selected from cyano, hydroxy, C₁₋₃-alkyloxy, acetylamino, methylsulphonylamino and C₁₋₃-alkyl, and
wherein in the cycloalkyl groups mentioned one or two methylene groups may be substituted independently of one another by O, CO or SO₂ and in the above-mentioned N-heterocycloalkyl groups a methylene group may be substituted by CO or SO₂, and
wherein R¹ is not hydrogen if R³ denotes hydrogen.

4. Heteroaryloxy-substituted benzoic acid amides according to one of the preceding claims, **characterised in that**
R² is selected from hydrogen, C₁₋₄-alkyl, C₃₋₆-cycloalkyl-C₁₋₃-alkyl, C₁₋₃-alkylamino-C₁₋₃-alkyl, C₃₋₆-cycloalkylamino-C₁₋₃-alkyl, pyrrolidinyl-C₁₋₃-alkyl, piperidin-C₁₋₃-alkyl, azepanyl-C₁₋₃-alkyl, piperazinyl-C₁₋₃-alkyl, morpholinyl-C₁₋₃-alkyl, homopiperazinyl-C₁₋₃-alkyl, pyrrolidin-3-ylamino-C₁₋₃-alkyl, piperidin-3-ylamino-C₁₋₃-alkyl, piperidin-4-ylamino-C₁₋₃-alkyl, C₃₋₆-cycloalkyl, trifluoromethyl, cyano, aminocarbonyl, C₁₋₄-alkyl-aminocarbonyl, di-(C₁₋₃-alkyl)-aminocarbonyl, piperazin-1-ylcarbonyl, morpholin-4-ylcarbonyl, carboxy, C₁₋₄-alkyloxycarbonyl, amino, C₁₋₄-alkylamino, C₃₋₆-cycloalkylamino, C₃₋₆-cycloalkyl-C₁₋₃-alkylamino, pyrrolidin-1-yl, piperidin-1-yl, piperazin-1-yl, morpholin-4-yl, C₁₋₃-alkyl-amino-C₂₋₃-alkylamino, pyrrolidin-3-ylamino, piperidin-3-ylamino, piperidin-4-ylamino, pyrrolidinyl-C₁₋₃-alkyl-amino, piperidln-C₁₋₃-alkyl-amino, piperazinyl-C₁₋₃-alkyl-amino, morpholinyl-C₁₋₃-alkyl-amino, homopiperazinyl-C₁₋₃-alkyl-amino, acetylamino, methylsulphonylamino, hydroxy, C₁₋₄-alkyloxy, trifluoromethoxy, difluoromethoxy, C₃₋₆-cycloalkyloxy, C₃₋₆-cycloalkyl-C₁₋₃-alkyloxy, C₁₋₃-alkylamino-C₂₋₃-alkyloxy, di-(C₁₋₃-alky)-amino-C₂₋₃-alkyloxy, pyrrolidinyl-C₂₋₃-alkyloxy, piperidin-C₂₋₃-alkyloxy, piperazinyl-C₂₋₃-alkyloxy, morpholinyl-C₂₋₃-alkyloxy, homopiperazinyl-C₂₋₃-alkyloxy,
wherein the above mentioned alkyl, cycloalkyl and N-haterocycloalkyl groups may be mono- or disubstituted by identical or different substituents selected from C₁₋₃-alkyl, cyano, amino, C₁₋₃-alkylamino, di-(C₁₋₃-alkyl)amino, acetylamino, methylsulphonylamino, hydroxy and C₁₋₃-alkyloxy, and
wherein in the above mentioned cycloalkyl groups one or two methylene groups may be substituted independently of one another by O and/or CO and in the above-mentioned N-heterocycloalkyl groups a methylene group may be substituted by CO or SO₂, and
wherein optionally all the NH groups contained in the above mentioned groups are replaced by N-Me, N-Et, N-*i*Pr, N-acetyl and N-SO₂Me.

5. Heteroaryloxy-substituted benzoic acid amides according to one or more of the preceding claims, **characterised in that** R³ is selected from hydrogen, C₁₋₃-alkyl, C₃₋₆-cycloalkyl, fluorine, chlorine, bromine, trifluoromethyl, difluoromethyl, hydroxy, C₁₋₃-alkyloxy, trifluoromethoxy, difluoromethoxy and cyano, wherein R³ is not hydrogen if R' denotes hydrogen.

6. Heteroaryloxy-substituted benzoic acid amides according to one or more of the preceding claims, **characterised in that** R⁴ is selected from hydrogen, C₁₋₃-alkyl, trifluoromethyl, carboxy, aminocarbonyl, methylaminocarbonyl, dimethylamino-carbonyl, amino, C₁₋₃-alkylamino, di-(C₁₋₃-alkyl)-amino, hydroxy and C₁₋₃-alkyloxy.

7. Heteroaryloxy-substituted benzoic acid amides according to one or more of the preceding claims, **characterised in that** R⁵ is selected from methyl, ethyl, n-propyl and isopropyl.

8. Heteroaryloxy-substituted benzoic acid amides according to one or more of the preceding claims, **characterised in that** R⁶ is selected from dimethylaminomethyl, cyclopropylaminomethyl, pyrrolidin-1-ylamino, hydroxymethyl, methylsulphanylmethyl, methylsulphinylmethyl, methylsulphonylmethyl, n-butylcarbonyl-amino, isopropylcarbonyl-amino, isobutylcarbonylamino, cyclopropylcarbonylamino, methylsulphonylamino, isopropylsulphonylamino, n-butylsulphonylamino, isobutylsulphonyl-amino, cyclopropyl-sulphonylamino, methylsulphinyl and methylsulphonyl.

9. Heteroaryloxy-substituted benzoic acid amides according to one or more of the preceding claims, **characterised in that** R⁷ is selected from isobutyl, tert-butyl, trifluoromethyl and pentafluoroethyl.

10. Heteroaryloxy-substituted benzoic acid amides according to one or more of the preceding claims, **characterised in that** X and Y are selected independently of one another from N, C-H, C-F, C-Cl, C-C₁₋₃-alkyl and C₁₋₃-alkyloxy, with the restriction that X and Y do not both represent N.

11. Compounds according to claim 1, selected from among
N-(5-tert-butyl-3-methanesulphonylamino-2-methoxy-phenyl)-4-methyl-3-(pyridin-4-yloxy)-benzamide;
N-(5-tert-butyl-3-methanesulphonylamino-2-methoxy-phenyl)-4-methyl-3-(2-methylamino-pyrimidin-4-yloxy)-benzamide;
N-(5-tert-butyl-3-methanesulphonylamino-2-methoxy-phenyl)-4-methyl-3-(6-methylamino-pyrimidin-4-yloxy)-benzamide;
N-(5-tert-butyl-3-methanesulphonylamino-2-methoxy-phenyl)-4-methyl-3-[2-(2-pyrrolidin-1-yl-ethylamino)-pyrimidin-4-yloxy]-benzamide;
N-(5-tert-butyl-3-methanasulphonylamino-2-methoxy-phenyl)-3-[6-(2-hydroxy-ethylamino)-pyrimidin-4-yloxy]-4-methyl-benzamide;
N-(5-tert-butyl-3-methanesulphonylamino-2-methoxy-phenyl)-3-[6-(2-dimethylamino-ethylamino)-pyrimidin-4-yloxy]-4-methyl-benzamide;
N-(5-tert-butyl-3-methanesulphonylamino-2-methoxy-phenyl)-4-methyl-3-[6-(3-oxo-piperazin-1-yl)-pyrimidin-4-yloxy]-benzamide;
N-(5-tert-butyl-3-methanesulphonylamino-2-methoxy-phenyl)-4-methyl-3-(6-pyrrolidin-1-yl-pyrimidin-4-yloxy)-benzamide;
N-(5-tert-butyl-3-methanesulphonylamino-2-methoxy-phenyl)-4-methyl-3-(6-morpholin-4-yl-pyrimidin-4-yloxy)-benzamide;
(*R*)-N-(5-tert-butyl-3-methanesulphonylamino-2-methoxy-phenyl)-3-[2-(3-dimethylamino-pyrrolidin-1-yl)-pyrimidin-4-yloxy]-4-methyl-benzamide;
(*S*)-N-(5-tert-butyl-3-methanesulphonylamino-2-methoxy-phenyl)-3-[2-(3-dimethylamino-pyrrolidin-1-yl)-pyrimidin-4-yloxy]-4-methyl-benzamide;
N-(5-tert-butyl-3-methanesulphonylamino-2-methoxy-phenyl)-3-[2-(4-dimethylamino-piperidin-1-yl)-pyrimidin-4-yloxy]-4-methyl-benzamide;
N-(5-tert-butyl-3-methanesulphonylamino-2-methoxy-phenyl)-4-methyl-3-[2-(1-methyl-piperidin-4-ylamino)-pyrimidin-4-yloxy]-benzamide;
N-(6-tert-butyl-3-methanesulphonylamino-2-methoxy-phenyl)-4-methyl-3-(2-piperazin-1-yl-pyrimidin-4-yloxy)-benzamide;
N-(5-tert-butyl-3-methanesulphonylamino-2-methoxy-phenyl)-4-methyl-3-(6-piperazin-1-yl-pyrimidin-4-yloxy)-benzamide;
N-(5-tert-butyl-3-methanesulphonylamino-2-methoxy-phenyl)-4-methyl-3-[6-(piperazine-1-carbonyl)-pyrimidin-4-yloxy]-benzamide;
N-(5-tert-butyl-3-methanesulphonylamino-2-methoxy-phenyl)-4-chloro-3-[6-(piperazine-1-carbonyl)-pyrimidin-4-yloxy]-benzamide;
N-(5-tert-butyl-3-methanesulphonylamino-2-methoxy-phenyl)-3-(2-piperazin-1-yl-pyrimidin-4-yloxy)-4-trifluoromethyl-benzamide;
N-(5-tert-butyl-3-methanesulphonylamino-2-methoxy-phenyl)-4-chloro-3-(2-piperazin-1-yl-pyrimidin-4-yloxy)-benzamide;
N-(5-tert-butyl-3-methanesulphonylamino-2-methoxy-phenyl)-4-methyl-3-(2-piperazin-1-ylmethyl-pyrimidin-4-yloxy)-benzamide;
N-(5-tert-butyl-3-methanesulphonylamino-2-methoxy-phenyl)-4-methyl-3-(6-piperazin-1-ylmethyl-pyrimidin-4-yloxy)-benzamide;
N-(5-tert-butyl-3-methanesulphonylamino-2-methoxy-phenyl)-4-methoxy-3-(2-piperazin-1-yl-pyrimidin-4-yloxy)-benzamide;
N-(5-tert-butyl-3-methanesulphonylamino-2-methoxy-phenyl)-4-methyl-3-(2-piperazin-1-ylmethyl-pyridin-4-yloxy)-benzamide;
N-(5-tert-butyl-3-methanesulphonylamino-2-methoxy-phenyl)-4-fluoro-3-(2-piperazin-1-yl-pyrimidin-4-yloxy)-benzamide;
N-(5-tert-butyl-3-methanesulphonylamino-2-methoxy-phenyl)-4-bromo-3-(2-piperazin-1-yl-pyrimidin-4-yloxy)-benzamide;
N-(5-tert-butyl-3-methanesulphonylamino-2-methoxy-phenyl)-3-chloro-4-methyl-5-(2-piperazin-1-yl-pyrimidin-4-yloxy)-benzamide;
N-(5-tert-butyl-3-methanesulphonylamino-2-methoxy-phenyl)-3-fluoro-4-methyl-5-(2-piperazin-1-yl-pyrimidin-4-yloxy)-benzamide;
N-(5-tert-butyl-3-methanesulphonylamino-2-methoxy-phenyl)-4-methyl-3-[2-(N-methyl-N-piperidin-4-yl-amino)-pyrimidin-4-yloxy]-benzamide;
N-(5-tert-butyl-3-methanesulphonylamino-2-methoxy-phenyl)-4-ethyl-5-(2-piperazin-1-yl-pyrimidin-4-yloxy)-benzamide;
N-(5-tert-butyl-3-methanesulphonylamino-2-methoxy-phenyl)-4-methyl-3-[2-(piperidin-4-ylamino)-pyrimidin-4-yloxy]-benzamide;
N-(5-tert-butyl-3-methanesulphonylamino-2-methoxy-phenyl)-4-methyl-3-[6-(piperidin-4-ylamino)-pyrimidin-4-yloxy]-benzamide;
N-(5-tert-butyl-3-methanesulphonylamino-2-methoxy-phenyl)-4-methyl-3-(6-piperidin-4-ylmethyl-pyrimidin-4-yloxy)-benzamide;
N-(5-tert-butyl-3-methanesulphonylamino-2-methoxy-phenyl)-4-methyl-3-(2-piperidin-4-ylmethyl-pyrimidin-4-yloxy)-benzamide;
N-(5-tert-butyl-3-methanesulphonylamino-2-methoxy-phenyl)-4-methyl-3-(2-piperidin-4-ylmethyl-pyridin-4-yloxy)-benzamide;
N-(5-tert-butyl-3-methanesulphonylamino-2-methoxy-phenyl)-3-methyl-5-[2-(piperazine-1-carbonyl)-pyrimidin-4-yloxy]-benzamide;
N-(5-tert-butyl-3-methanesulphonylamino-2-methoxy-phenyl)-3-methyl-5-(2-piperazin-1-ylmethyl-pyrimidin-4-yloxy)-benzamide;
N-(5-tert-butyl-3-methanesulphonylamino-2-methoxy-phenyl)-3-methyl-5-(pyridin-4-yloxy)-benzamide;
6-[5-(5-tert-butyl-3-methanesulphonylamino-2-methoxyphenylaminocarbonyl)-2-methyl-phenoxy]-pyrimidine-4-carboxylic acid-methylamide;
N-(5-tert-butyl-3-methanesulphonylamino-2-methoxy-phenyl)-4-methyl-3-[6-(morpholine-4-carbonyl)-pyrimidin-4-yloxy]-benzamide;
N-(5-tert-butyl-3-methanesulphonylamino-2-methoxy-phenyl)-4-methyl-3-[6-(4-methyl-piperazine-1-carbonyl)-pyrimidin-4-yloxy]-benzamide;
N-(5-tert-butyl-3-methanesulphonylamino-2-methoxy-phenyl)-4-methyl-3-(6-morpholin-4-ylmethyl-pyrimidin-4-yloxy)-benzamide;
N-(5-tert-butyl-3-methanesulphonylamino-2-methoxy-phenyl)-4-methyl-3-(6-methylaminomethyl-pyrimidin-4-yloxy)-benzamide;
N-(5-tert-butyl-3-methanesulphonylamino-2-methoxy-phenyl)-4-methyl-3-[6-(4-methyl-piperazin-1-ylmethyl)-pyrimidin-4-yloxy]-benzamide;
N-(5-tert-butyl-3-methanesulphonylamino-2-methoxy-phenyl)-4-methyl-3-(2-morpholin-4-ylmethyl-pyrimidin-4-yloxy)-benzamide;
N-(5-tert-butyl-3-methanesulphonylamino-2-methoxy-phenyl)-4-methyl-3-(2-methylaminomethyl-pyrimidin-4-yloxy)-benzamide;
N-(5-tert-butyl-3-methanesulphonylamino-2-methoxy-phenyl)-4-methyl-3-[2-(3-oxo-piperazin-1-ylmethyl)-pyrimidin-4-yloxy]-benzamide;
N-(5-tert-butyl-3-methanesulphonylamino-2-methoxy-phenyl)-4-methyl-3-[2-(4-methyl-piperazin-1-ylmethyl)-pyrimidin-4-yloxy]-benzamide;
N-(5-tert-butyl-3-methanesulphonylamino-2-methoxy-phenyl)-4-methyl-3-[6-(3-oxo-piperazin-1-ylmethyl)-pyrimidin-4-yloxy]-benzamide;
N-(5-tert-butyl-3-methanesulphonylamino-2-methoxy-phenyl)-4-methyl-3-(6-piperazin-1-ylmethyl-pyrimidin-4-yloxy)-benzamide;
N-(5-tert-butyl-3-methanesulphonylamino-2-methoxy-phenyl)-4-methyl-3-(2-morpholin-4-ylmethyl-pyridin-4-yloxy)-benzamide;
N-(5-tert-butyl-3-methanesulphonylamino-2-methoxy-phenyl)-4-methyl-3-[2-(4-methyl-piperazin-1-ylmethyl)-pyridin-4-yloxy]-benzamide;
(*S*)-N-(5-tert-butyl-3-methanesulphonylamino-2-methoxy-phenyl)-4-methyl-3-{2-[(tetrahydro-furan-3-ylamino)-methyl]-pyridin-4-yloxy}-benzamide;
(*R*)-N-(5-tert-butyl-3-methanesulphonylamino-2-methoxy-phenyl)-3-[2-(3-hydroxy-pyrrolidin-1-ylmethyl)-pyridin-4-yloxy]-4-methyl-benzamide;
(*S*)-N-(5-tert-butyl-3-methanesulphonylamino-2-methoxy-phenyl)-3-[2-(3-hydroxy-pyrrolidin-1-ylmethyl)-pyridin-4-yloxy]-4-methyl-benzamide;
N-(5-tert-butyl-3-methanesulphonylamino-2-methoxy-phenyl)-4-methyl-3-(2-methylaminomethyl-pyridin-4-yloxy)-benzamide;
(*S*)-N-(5-tert-butyl-3-methanesulphonylamino-2-methoxy-phenyl)-4-methyl-3-{2-[(tetrahydro-furan-3-ylamino)-methyl]-pyridin-4-yloxy}-benzamide;
N-(5-tert-butyl-3-methanesulphponylamino-2-methoxy-phenyl)-4-methyl-3-[2-(4-methyl-homopiperazin-1-ylmethyl)-pyrimidin-4-yloxy]-benzamide;
N-(5-tert-butyl-3-methanesulphonylamino-2-methoxy-phenyl)-4-methyl-3-{2-[(1-methyl-piperidin-4-ylamino)-methyl]-pyrimidin-4-yloxy}-benzamide;
N-(5-tert-butyl-3-methanesulphonylamino-2-methoxy-phenyl)-4-methyl-3-{2-[(1-methyl-piperidin-3-ylamino)-methyl]-pyrimidin-4-yloxy}-benzamide;
N-(5-tert-butyl-3-methanesulphonylamino-2-methoxy-phenyl)-4-methyl-3-(2-{[methyl-(1-methyl-pyrrolidin-3-yl)-amino]-methyl}-pyrimidin-4-yloxy)-benzamide;
N-(5-tert-butyl-3-methanesulphonylamino-2-methoxy-phenyl)-4-methyl-3-[2-(4-methyl-homopiperazin-1-ylmethyl)-pyridin-4-yloxy]-benzamide;
(*S*)-N-(5-tert-butyl-3-methanesulphonylamino-2-methoxy-phenyl)-3-[2-(3-dimethylamino-pyrrolidin-1-ylmethyl)-pyrimidin-4-yloxy]-4-methyl-benzamide;
(*R*)-N-(5-tert-butyl-3-methanesulphonylamino-2-methoxy-phenyl)-3-[2-(3-dimethylamino-pyrrolidin-1-ylmethyl)-pyrimidin-4-yloxy]-4-methyl-benzamide;
N-(5-tert-butyl-3-methanesulphonylamino-2-methoxy-phenyl)-4-methyl-3-{2-[(1-methyl-pyrrolidin-3-ylamino)-methyl]-pyrimidin-4-yloxy}-benzamide;
N-(5-tert-butyl-3-methanesulphonylamino-2-methoxy-phenyl)-3-(2-{[N-(2-dimethylamino-ethyl)-N-methyl-amino]-methyl}-pyrimidin-4-yloxy)-4-methyl-benzamide;
N-(5-tert-butyl-3-methanesulphonylamino-2-methoxy-phenyl)-3-{2-[(2-dimethylamino-ethylamino)-methyl]-pyrimidin-4-yloxy}-4-methyl-benzamide;
N-(5-tert-butyl-3-methanesulphonylamino-2-methoxy-phenyl)-3-(2-{[(2-dimethylamino-ethyl)-methyl-amino]-methyl}-pyridin-4-yloxy)-4-methyl-benzamide;
N-(5-tert-butyl-3-methanesulphonylamino-2-methoxy-phenyl)-3-{2-[(2-dimethylamino-ethylamino)-methyl]-pyridin-4-yloxy}-4-methyl-benzamide;
N-(5-tert-butyl-3-methanesulphonylamino-2-methoxy-phenyl)-3-methyl-5-[2-(4-methyl-piperazin-1-ylmethyl)-pyrimidin-4-yloxy]-benzamide;
N-(5-tert-butyl-3-methanesulphonylamino-2-methoxy-phenyl)-3-{6-[(2-dimethylamino-ethylamino)-methyl]-pyrimidin-4-yloxy}-4-methyl-benzamide;
N-(5-tert-butyl-3-methanesulphonylamino-2-methoxy-phenyl)-3-(6-{[(2-dimethylamino-ethyl)-methyl-amino]-methyl}-pyrimidin-4-yloxy)-4-methyl-benzamide;
N-(5-tert-butyl-3-methanesulphonylamino-2-methoxy-phenyl)-4-methyl-3-[6-(1-methyl-piperidin-4-ylmethyl)-pyrimidin-4-yloxy]-benzamide;
N-(5-tert-butyl-3-methanesulphonylamino-2-methoxy-phenyl)-4-methyl-3-[2-(1-methyl-piperidin-4-ylmethyl)-pyrimidin-4-yloxy]-benzamide;
N-(5-tert-butyl-3-methanesulphonylamino-2-methoxy-phenyl)-4-methyl-3-[2-(1-methyl-piperidln-4-ylmethyl)-pyridin-4-yloxy]-benzamide;
N-(5-tert-butyl-3-methanesulphonylamino-2-methoxy-phenyl)-4-methyl-3-[2-(pyrrolidin-3-ylamino)-pyrimidin-4-yloxy]-benzamide;
N-(5-tert-butyl-3-methanesulphonylamino-2-methoxy-phenyl)-4-chloro-3-[2-(4-methyl-piperazin-1-ylmethyl)-pyrimidin-4-yloxy]-benzamide;
N-(5-tert-butyl-3-methanesulphonylamino-2-methoxy-phenyl)-3-chloro-4-methyl-5-[2-(4-methyl-piperazin-1-ylmethyl)-pyrimidin-4-yloxy]-benzamide;
N-(5-tert-butyl-3-methanesulphonylamino-2-methoxy-phenyl)-4-chloro-3-[6-(1-methyl-piperidin-4-ylamino)-pyrimidin-4-yloxy]-benzamide;
N-(5-tert-butyl-3-methanesulphonylamino-2-methoxy-phenyl)-4-chloro-3-[2-(1-methyl-piperidin-4-ylamino)-pyridin-4-yloxy]-benzamide;
N-(5-tert-butyl-3-methanesulphonylamino-2-methoxy-phenyl)-4-chloro-3-[6-(piperidin-4-ylamino)-pyrimidin-4-yloxy]-benzamide;
N-(5-tert-butyl-3-methanesulphonylamino-2-methoxy-phenyl)-4-chloro-3-[2-(1-methyl-piperidin-4-ylamino)-pyrimidin-4-yloxy]-benzamide;
N-(5-tert-butyl-3-methanesulphonylamino-2-methoxy-phenyl)-4-methyl-3-{2-[methyl-(1-methyl-piperidin-4-yl)-amino]-pyrimidin-4-yloxy}-benzamide;
N-(5-tert-butyl-3-methanesulphonylamino-2-methoxy-phenyl)-4-methyl-3-{6-[methyl-(1-methyl-piperidin-4-yl)-amino]-pyrimidin-4-yloxy}-benzamide;
N-(5-tert-butyl-3-methanesulphonylamino-2-methoxy-phenyl)-4-chloro-3-[2-(piperidin-4-ylamino)-pyrimidin-4-yloxy]-benzamide;
N-(5-tert-butyl-3-methanesulphonylamino-2-methoxy-phenyl)-4-chloro-3-(2-piperidin-4-ylmethyl-pyridin-4-yloxy)-benzamide;
N-(5-tert-butyl-3-methanesulphonylamino-2-methoxy-phenyl)-4-chloro-3-(2-piperidin-4-ylmethyl-pyrimidin-4-yloxy)-benzamide;
N-(5-tert-butyl-3-methanesulphonylamino-2-methoxy-phenyl)-4-chloro-3-{6-[(2-dimethylamino-ethylamino)-methyl]-pyrimidin-4-yloxy}-benzamide;
N-(5-tert-butyl-3-methanesulphonylamino-2-methoxy-phenyl)-4-chloro-3-(6-{[(2-dimethylamino-ethyl)-methyl-amino]-methyl}-pyrimidin-4-yloxy)-benzamide;
N-(5-tert-butyl-3-methanesulphonylamino-2-methoxy-phenyl)-4-chloro-3-[2-(piperidin-4-ylamino)-pyridin-4-yloxy]-benzamide;
N-(5-tert-butyl-3-methanesulphonylamino-2-methoxy-phenyl)-4-chloro-3-(6-piperazin-1-ylmethyl-pyrimidin-4-yloxy)-benzamide;
N-(5-tert-butyl-3-methanesulphonylamino-2-methoxy-phenyl)-4-chloro-3-(6-piperidin-4-ylmethyl-pyrimidin-4-yloxy)-benzamide;
N-(5-tert-butyl-3-methanesulphonylamino-2-methoxy-phenyl)-4-chloro-3-[6-(4-methyl-piperazin-1-ylmethyl)-pyrimidin-4-yloxy]-benzamide;
N-(5-tert-butyl-3-methanesulphonylamino-2-methoxy-phenyl)-4-chloro-3-[6-(1-methyl-piperidin-4-ylmethyl)-pyrimidin-4-yloxy]-benzamide;
N-(5-tert-butyl-3-methanesulphonylamino-2-methoxy-phenyl)-4-chloro-3-[2-(1-methyl-piperidin-4-ylmethyl)-pyrimidin-4-yloxy]-benzamide;
N-(5-tert-butyl-3-methanesulphonylamino-2-methoxy-phenyl)-4-chloro-3-(2-{[(2-dimethylamino-ethyl)-methyl-amino]-methyl}-pyridin-4-yloxy)-benzamide;
N-(5-tert-butyl-3-methanesulphonylamino-2-methoxy-phenyl)-3-[2-(*trans-*4-dimethylamino-cyclohexylamino)-pyrimidin-4-yloxy]-4-methyl-benzamide;
N-(5-tert-butyl-3-methanesulphonylamino-2-methoxy-phenyl)-4-chloro-3-{2-[(2-dimethylamino-ethylamino)-methyl]-pyridin-4-yloxy}-benzamide;
N-(5-tert-butyl-3-methanesulphonylamino-2-methoxy-phenyl)-4-chloro-3-(2-piperazin-1-ylmethyl-pyridin-4-yloxy)-benzamide;
N-(5-tert-butyl-3-methanesulphonylamino-2-methoxy-phenyl)-4-methyl-3-{2-[methyl-(1-methyl-pyrrolidin-3-yl)-amino]-pyrimidin-4-yloxy}-benzamide;
N-(5-tert-butyl-3-methanesulphonylamino-2-methoxy-phenyl)-4-chloro-3-[2-(4-methyl-piperazin-1-ylmethyl)-pyridin-4-yloxy]-benzamide;
N-(5-tert-butyl-3-methanesulphonylamino-2-methoxy-phenyl)-4-methyl-3-{6-[methyl-(1-methyl-pyrrolidin-3-yl)-amino]-pyrimidin-4-yloxy}-benzamide;
N-(5-tert-butyl-3-methanesulphonylamino-2-methoxy-phenyl)-4-chloro-3-[2-(4-methyl-[1,4]diazepan-1-ylmethyl)-pyrimidin-4-yloxy]-benzamide;
N-(5-tert-butyl-3-methanesulphonylamino-2-methoxy-phenyl)-4-chloro-3-(2-{[(2-dimethylamino-ethyl)-methyl-amino]-methyl}-pyrimidin-4-yloxy)-benzamide;
N-(5-tert-butyl-3-methanesulphonylamino-2-methoxy-phenyl)-4-chloro-3-{2-[(2-dimethylamino-ethylamino)-methyl]-pyrimidin-4-yloxy}-benzamide;
N-(5-tert-butyl-3-methanesulphonylamino-2-methoxy-phenyl)-4-chloro-3-[6-(4-methyl-[1,4]diazepan-1-ylmethyl)-pyrimidin-4-yloxy]-benzamide;
N-(5-tert-butyl-3-methanesulphonylamino-2-methoxy-phenyl)-4-chloro-3-(2-piperazin-1-ylmethyl-pyrimidin-4-yloxy)-benzamide;
N-(5-tert-butyl-3-methanesulphonylamino-2-methoxy-phenyl)-4-chloro-3-(6-[1,4]diazepan-1-ylmethyl-pyrimidin-4-yloxy)-benzamide;
N-(5-tert-butyl-3-methanesulphonylamino-2-methoxy-phenyl)-4-chloro-3-[2-(4-methyl-[1,4]diazepan-1-ylmethyl)-pyridin-4-yloxy]-benzamide;
N-(5-tert-butyl-3-methanesulphonylamino-2-methoxy-phenyl)-4-chloro-3-(2-[1,4]diazepan-1-ylmethyl-pyridin-4-yloxy)-benzamide;
N-(5-tert-butyl-3-methanesulphonylamino-2-methoxy-phenyl)-4-chloro-3-[2-(1-methyl-piperidin-4-ylmethyl)-pyridin-4-yloxy]-benzamide;
N-(5-tert-butyl-3-mathanesulphonyl-2-methoxy-phenyl)-4-methyl-3-(2-piperidin-4-ylmethyl-pyrimidin-4-yloxy)-benzamide;
N-(5-tert-butyl-3-methanesulphonylamino-2-methoxy-phenyl)-3-(2-[1,4]diazepan-1-ylmethyl-pyridin-4-yloxy)-4-methyl-benzamide;
N-(5-tert-butyl-2-methoxy-3-methylsulphanylmethyl-phenyl)-4-methyl-3-(2-piperidin-4-ylmethyl-pyridin-4-yloxy)-benzamide;
N-(5-tert-butyl-3-methanesulphonylmethyl-2-methoxy-phenyl)-4-methyl-3-(2-piperidin-4-ylmethyl-pyridin-4-yloxy)-benzamide;
N-(5-tert-butyl-3-methanesulphinylmethyl-2-methoxy-phenyl)-4-methyl-3-(2-piperidin-4-ylmethyl-pyridin-4-yloxy)-benzamide;
N-[5-tert-butyl-3-(cyclopropanecarbonyl-amino)-2-methoxy-phenyl]-4-methyl-3-(2-piperidin-4-ylmethyl-pyridin-4-yloxy)-benzamide;
N-(5-tert-butyl-2-isopropoxy-3-methanesulphonylamino-phenyl)-4-methyl-3-(2-piperidin-4-ylmethyl-pyridin-4-yloxy)-benzamide;
N-(5-tert-butyl-2-ethoxy-3-methanesulphonylamino-phenyl)-4-methyl-3-(2-piperidin-4-ylmethyl-pyridin-4-yloxy)-benzamide;
N-[5-tert-butyl-2-methoxy-3-(2-methyl-propane-1-sulphinylamino)-phenyl]-4-methyl-3-(2-piperidin-4-ylmethyl-pyridin-4-yloxy)-benzamide;
N-[3-(butan-1-sulphonylamino)-5-tert-butyl-2-methoxy-phenyl]-4-methyl-3-(2-piperidin-4-ylmethyl-pyridin-4-yloxy)-benzamide;
N-(5-tert-butyl-2-methoxy-3-pentanoylamino-phenyl)-4-methyl-3-(2-piperidin-4-ylmethyl-pyridin-4-yloxy)-benzamide;
N-(5-tert-butyl-3-methanesulphonyl-2-methoxy-phenyl)-4-methyl-3-(2-piperidin-4-ylmethyl-pyridin-4-yloxy)-benzamide;
N-[5-tert-butyl-2-methoxy-3-(3-methyl-butyrylamino)-phenyl]-4-methyl-3-(2-piperidin-4-ylmethyl-pyridin-4-yloxy)-benzamide;
N-(5-tert-butyl-3-isobutyrylamino-2-methoxy-phenyl)-4-methyl-3-(2-piperidin-4-ylmethyl-pyridin-4-yloxy)-benzamide;
N-(5-tert-butyl-3-methanesulphinyl-2-methoxy-phenyl)-4-methyl-3-(2-piperidin-4-ylmethyl-pyridin-4-yloxy)-benzamide;
N-(5-tert-butyl-3-methanesulphinylmethyl-2-methoxy-phenyl)-4-methyl-3-(2-piperidin-4-ylmethyl-pyridin-4-yloxy)-benzamide;
N-(5-tert-butyl-3-methanesulphinylmethyl-2-methoxy-phenyl)-4-methyl-3-(2-piperidin-4-ylmethyl-pyridin-4-yloxy)-benzamide;
N-(5-tert-butyl-3-cyclopropanesulphonylamino-2-methoxy-phenyl)-4-methyl-3-(2-piperidin-4-ylmethyl-pyridin-4-yloxy)-benzamide;
3-(2-azepan-4-ylmethyl-pyridin-4-yloxy)-N-(5-tert-butyl-3-methanesulphonylamino-2-methoxy-phenyl)-4-methyl-benzamide;
3-(2-azepan-4-ylmethyl-pyrimidin-4-yloxy)-N-(5-tert-butyl-3-methanesulphonylamino-2-methoxy-phenyl)-4-methyl-benzamide;
N-(5-tert-butyl-3-methanesulphonylamino-2-methoxy-phenyl)-4-methyl-3-[2-(1-methyl-azepan-4-ylmethyl)-pyridin-4-yloxy]-benzamide;
N-(5-tert-butyl-3-methanesulphonylamino-2-methoxy-phenyl)-4-methyl-3-[2-(1-methyl-azepan-4-ylmethyl)-pyrimidin-4-yloxy]-benzamide;
3-(6-azepan-4-ylmethyl-pyrimidin-4-yloxy)-N-(5-tert-butyl-3-methanesulphonylamino-2-methoxy-phenyl)-4-methyl-benzamide;
N-(5-tert-butyl-3-methanesulphonylamino-2-methoxy-phenyl)-4-methyl-3-[6-(1-methyl-azepan-4-ylmethyl)-pyrimidin-4-yloxy]-benzamide;
N-(3-methanesulphonylamino-2-methoxy-5-pentafluoroethyl-phenyl)-4-methyl-3-(2-piperidin-4-ylmethyl-pyridin-4-yloxy)-benzamide;
N-(5-tert-butyl-3-hydroxymethyl-2-methoxy-phenyl)-4-methyl-3-[2-(4-methyl-piperazin-1-ylmethyl)-pyrimidin-4-yloxy]-benzamide;
N-(5-tert-butyl-3-methanesulphinyl-2-methoxy-phenyl)-4-methyl-3-[2-(4-methyl-piperazin-1-ylmethyl)-pyrimidin-4-yloxy]-benzamide;
N-(5-tert-butyl-3-dimethylaminomethyl-2-methoxy-phenyl)-4-methyl-3-(2-piperidin-4-ylmethyl-pyridin-4-yloxy)-benzamide;
N-(5-tert-butyl-3-methanesulphonylamino-2-methoxy-phenyl)-4-methyl-3-{2-[4-(2,2,2-trifluoro-ethyl)-piperazin-1-ylmethyl]-pyridin-4-yloxy}-benzamide;
N-(5-tert-butyl-3-methanesulphonylamino-2-methoxy-phenyl)-3-[2-(4-cyanomethyl-piperazin-1-ylmethyl)-pyridin-4-yloxy]-4-methyl-benzamide;
N-(5-tert-butyl-2-methoxy-3-pyrrolidin-1-ylmethyl-phenyl)-4-methyl-3-(2-piperidin-4-ylmethyl-pyridin-4-yloxy)-benzamide;
N-(5-tert-butyl-3-hydroxymethyl-2-methoxy-phenyl)-4-methyl-3-(2-piperazin-1-ylmethyl-pyridin-4-yloxy)-benzamide;
N-(5-tert-butyl-3-hydroxymethyl-2-methoxy-phenyl)-4-methyl-3-[2-(4-methyl-piperazin-1-ylmethyl)-pyridin-4-yloxy]-benzamide;
N-(5-tert-butyl-3-hydroxymethyl-2-methoxy-phenyl)-3-(2-[1,4]diazepan-1-ylmethyl-pyridin-4-yloxy)-4-methyl-benzamide;
N-(5-tert-butyl-3-hydroxymethyl-2-methoxy-phenyl)-4-methyl-3-[2-(4-methyl-[1,4]diazepan-1-ylmethyl)-pyridin-4-yloxy]-benzamide;
N-(5-tert-butyl-3-cyclopropylaminomethyl-2-methoxy-phenyl)-4-methyl-3-(2-piperidin-4-ylmethyl-pyridin-4-yloxy)-benzamide
the tautomers, the stereoisomers and the mixtures thereof.

12. Physiologically acceptable salts of the compounds according to one or more of claims 1 to 11 with inorganic or organic acids.

13. Compound according to one or more of claims 1 to 11 or a physiologically acceptable salt according to claim 12 for use as a medicament.

14. Medicaments, containing a compound according to one or more of claims 1 to 11 or a physiologically acceptable salt according to claim 12 optionally together with one or more inert carriers, preservatives and/or diluents.

15. Use of at least one compound according to one or more of claims 1 to 11 or of a physiologically acceptable salt according to claim 12, for preparing a medicament that is suitable for the treatment or prevention of respiratory complaints.

16. Use according to claim 15, **characterised in that** the respiratory complaint is COPD or asthma.

17. Method for preparing a medicament according to claim 14, **characterised in that** a compound according to at least one of claims 1 to 11 or a physiologically acceptable salt according to claim 12 is incorporated by a non-chemical method in one or more inert carriers, preservatives and/or diluents.

18. Process for preparing the compounds of general formula I according to claims 1 to 11, **characterised in that** a compound of general formula II, 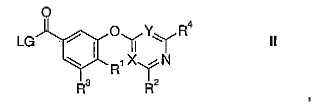 wherein R' to R⁴, X and Y have the meanings given in claims 1 to 6 and 10, and LG denotes fluorine, chlorine, bromine, cyano, C₁₋₁₀-alkoxy, C₁₋₆-alkylsulphanyl, C₂₋₄-alkenyl-oxy, C₂₋₄-alkynyloxy, benzotriazol-1-yloxy, [1,2,3]triazolo[4,5-b]pyridin-3-yloxy, C₅₋₁₀-hoteroaryl (linked to II via N), succinyl-N-oxy, C₁₋₄-alkylcarbonyloxy, di-(C₁₋₄-alkyl)aminocarbonyloxy, pyrrol-1-ylcarbonyloxy, piperidin-1-yl-carbonyloxy, morpholin-4-ylcarbonyloxy, tri-(C₁₋₄-alkyl)carbamimidoyloxy, N,N,N',N'-tetra-(C₁₋₄-alkyl)-uronium-O-yl, N,N'-dicyclohexyluron-O-yl, N-(3-dimethylaminopropyl)-N'-ethyl-uronyl, di-(C₁₋₄-alkyloxy)-phosphoryloxy, bis(di-C₁₋₄-alkylamino)-phosphoryloxy, dipyrrolidinyl-phosphoryloxy, C₆₋₁₀-arylsulphanyl, C₅₋₁₀-heteroarylsulphanyl, C₆₋₁₀-aryloxy or C₅₋₁₀-heteroaryloxy,
wherein all the alkyl, alkenyl and alkynyl groups mentioned in the definition may be mono- or polysubstituted by fluorine, chlorine, C₁₋₃-alkyl and/or C₁₋₃-alkoxy,
wherein all the aryl groups mentioned in the definition represent phenyl or naphthyl and all the heteroaryl groups represent pyridinyl, pyrimidinyl, triazinyl, imidazolyl, pyrazolyl, triazolyl or tetrazolyl, which, both aryl and heteroaryl groups, are optionally mono- or polysubstituted by identical or different groups selected from among fluorine, chlorine, bromine, C₁₋₃-alkyl, C₁₋₃-alkyloxy, nitro, cyano and di-(C₁₋₃-alkyl)amino,
is reacted with an aniline of general formula 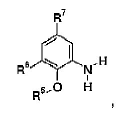 wherein R⁵, R⁶ and R⁷ have the meanings given in claims 1, 2, 7, 8 and 9,
optionally in the presence of a base and/or an additive such as e.g. triethylamine, pyridine, ethyldiisopropylamine, 4-dimethylaminopyridine, potassium carbonate or 1-hydroxybenzotriazole; and
if necessary any protecting group used In the reactions described hereinbefore under a) and b) is cleaved again, and/or
if desired a compound of general formula I thus obtained is resolved into its stereoisomers and/or
if desired a compound of general formula I thus obtained is converted into the salts thereof, particularly for pharmaceutical use into the physiologically acceptable salts thereof.

19. Process for preparing the compounds of general formula I according to claims 1 to 11, **characterised in that** a compound of general formula III 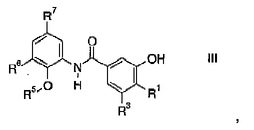 wherein R¹, R³, R⁵, R⁶ and R⁷ have the meanings given in claims 1 to 3, 5 and 7 to 9,
is reacted with a compound of general formula IV 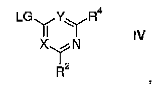 wherein R² and R⁴ have the meanings given in claims 1, 2, 4, 6 and 10 and LG denotes a leaving group, in particular LG denotes
F, Cl, Br, I, O-C₁₋₆-alkyl, O-C₆₋₁₀-aryl, S(O)ₙ-C₁₋₄-alkyl, S(O)ₘ-C₅₋₁₀-aryl, OSO₂-C₁₋₄-alkyl, OSO₂-C₆₋₁₀-aryl, NO₂,
while all the above-mentioned alkyl groups are optionally mono- or polysubstituted by fluorine, C₁₋₃-alkyl and/or C₁₋₃-alkoxy, and wherein all the above-mentioned aryl groups represent phenyl or naphthyl, which may optionally be mono- or polysubstituted by identical or different groups selected from fluorine, chlorine, C₁₋₃-alkyl, C₁₋₃-alkyloxy, nitro and cyano,
wherein n and m independently of one another may be 0, 1 or 2,
in the presence of a base, e.g. NaH, KH, KO*t*Bu, NaO*t*Bu, NaOMe, NaOEt, NaO*i*Pr, KF, K₂CO₃, Cs₂CO₃, pyridine, 4-dimethylaminopyridine, NEt₃ or EtN*i*Pr₂,
optionally in the presence of a catalyst, e.g. a Cu or Pd complex; and
if necessary any protecting group used in the reactions described hereinbefore under a) and b) is cleaved again, and/or
if desired a compound of general formula I thus obtained is resolved into its stereoisomers, and/or
if desired a compound of general formula I thus obtained is converted into the salts thereof, particularly for pharmaceutical use into the physiologically acceptable salts thereof.

## Revendications

1. Amide d'acide benzoïque à substitution hétéroaryloxy, de formule générale I 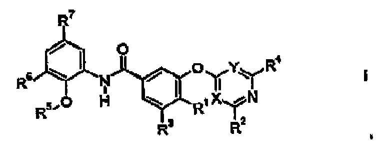 dans laquelle
R¹ est L₁-R₈,
où L₁ est choisi parmi une liaison, O, CO, NH, CONH, NHCO, SO, SO₂, SO₂NH, NHSO₂ et alkylène en C₁ à C₄,
où Rₐ est choisi parmi un atome d'hydrogène, d'halogène, un groupe alkyle en C₁ à C₆, alcinyle en C₂ à C₆, alcényle en C₂ à C₆, cycloalkyle en C₃ à C₆, hétérocycloalkyle en C₃ à C₈, aryle en C₅ à C₁₀, hétéroaryle en C₅ à C₁₀, cyano, carboxy, alkyloxycarbonyle en C₁ à C₄, nitro, amino, di-(alkyle en C₁ à C₄)-amino, hydroxy, alkylsulfanyle en C₁ à C₆, où R₈ peut être partiellement ou complètement fluoré et/ou peut être substitué par un ou plusieurs substituants L, et où dans des radicaux hétérocycloalkyle, un groupe méthylène peut être substitué par CO, SO ou SO₂, et
R¹ n'étant pas un atome d'hydrogène lorsque R³ a la signification hydrogène, et
R² est L₂-R₉,
où L₂ est choisi parmi une liaison, O, CO, NH, CONH, NHCO, SO, SO₂, SO₂NH, NHSO₂ et un groupe alkylène en C₁ à C₄, où R₉ est choisi parmi un atome d'hydrogène, d'halogène, un groupe alkyle en C₁ à C₆, alcinyle en C₂ à C₆, alcényle en C₂ à C₆, cycloalkyle en C₃ à C₈, cycloalcényle en C₄ à C₈, hétérocycloalkyle en C₃ à C₈, aryle en C₅ à C₁₀, hétéroaryle en C₅ à C₁₀, cyano, carboxy, alkyloxycarbonyle en C₁ à C₄, nitro, amino, di-(alkyle en C₁ à C₄)-amino, hydroxy, alkylsulfanyle en C₁ à C₆,
où R₉ peut être partiellement ou complètement fluoré et/ou peut être substitué par un ou plusieurs substituants L, et
où dans les radicaux alkyle mentionnés, éventuellement un ou deux groupes CH₂ sont échangés contre O et/ou NR^{N},
où dans les radicaux cycloalkyle mentionnés, un ou deux groupes CH₂ peuvent être remplacés indépendamment l'un de l'autre par NR^{N}, O, S, CO, SO ou SO₂ et un ou deux groupes CH peuvent être remplacés par N, et
R³ est L₃-R₁₀,
où L₃ est choisi parmi une liaison, O, CO, NH, CONH, NHCO, SO, SO₂, SO₂NH, NHSO₂ et un groupe alkylène en C₁ à C₄,
où R₁₀ est choisi parmi un atome d'hydrogène, d'halogène, un groupe alkyle en C₁ à C₆, alcinyle en C₂ à C_{6,} alcényle en C₂ à C₆, cycloalkyle en C₃ à C₈, cycoalcényle en C₄ à C₈, hétérocycloalkyle en C₃ à C₈, aryle en C₅ à C₁₀, hétéroaryle en C₅ à C₁₀, cyano, carboxy, alkyloxycarbonyle en C₁ à C₄, nitro, amino, di-(alkyle en C₁ à C₄)-amino, hydroxy, alkylsulfanyle en C₁ à C₆,
où R₁₀ peut être partiellement ou complètement fluoré et/ou peut être substitué par un ou plusieurs substituants L, et
où R³ n'est pas un atome d'hydrogène lorsque R¹ a la signification hydrogène, et
R₄ est L₄-R₁₁,
où L₄ est choisi parmi une liaison, O, CO, NH, CONH, NHCO,
où R₁₁ est choisi parmi un atome d'hydrogène, d'halogëne, un groupe amino, alkyle en C₁ à C₆, cycloalkyle en C₃ à C₈, hétérocycloalkyle en C₃ à C₈, trifluorométhyle, difluorométhyle, cyano, carboxy, alkyloxycarbonyle en C₁ à C₄, di-(alkyle en C₁ à C₄)-amino, alkylsulfanyle en C₁ à C₄,
R⁵ est un groupe alkyle en C₁ à C₆,
R⁶ est choisi parmi les groupes diméthylamino-alkyle en C₁ à C₃, cyclopropylamino-alkyle en C₁ à C₃,
pyrrolidinyl-alkyle en C₁ à C₃, hydroxy-alkyle en C₁ à C₃, méthylsulfanyl-alkyle en C₁ à C₃, méthylsulfinyl-alkyle en C₁ à C₃, méthylsulfonyl-alkyle en C₁ à C₃, alkylcarbonylamino en C₁ à C₄, cycloalkyle en C₃ à C₅-carbonylamino, alkylsulfonylamino en C₁ à C₄, cycloalkylsulfonylamino en C₃ à C₅, alkylsulfinyle en C₁ à C₃ et alkylsulfonyle en C₁ à C₃,
R⁷ est un groupe alkyle en C₁ à C₆, trifluorométhyle, pentafluoroéthyle,
X, Y sont indépendamment l'un de l'autre, N ou C-R₁₃, où X et Y ne peuvent pas être tous les deux N et R₁₃ est choisi parmi un atome d'hydrogène, de fluor, de chlore, un groupe alkyle en C₁ à C₄, trifluorométhyle, OH, alkyloxy en C₁ à C₄, trifluorométhoxy, difluorométhoxy et cyano,
R^{N} est choisi parmi H, un groupe alkyle en C₁ à C₄, 1,1,1-trifluoroéthyle, cyanométhyle, acétyle, méthylsulfonyle, arylméthyle en C₆ à C₁₀,
L est choisi dans le groupe consistant en atome d'halogène, en groupes cyano, alkyle en C₁ à C₆, alkyloxy en C₁ à C₆, amino, alkylamino en C₁ à C₃, di-(alkyle en C₁ à C₃)-amino, alkylcarbonylamino en C₁ à C₄, difluorométhyle, alkylsulfonylamino en C₁ à C₄, trifluorométhyle, hydroxy, alcoxy en C₁ à C₃, difluorométhoxy, trifluorométhoxy, acétylamino, aryle en C₅ à C₁₀ et hétéroaryle en C₅ à C₁₀.

2. Amide d'acide benzoïque à substitution hétéroaryloxy selon la revendication 1, dans lequel
R¹ est choisi parmi un atome d'hydrogène, d'halogène, un groupe alkyle en C₁ à C₆, alcinyle en C₂ à C₆, alcényle en C₂ à C₆, cycloalkyle en C₃ à C₇, cycloalkyle en C₃ à C₇-alkyle en C₁ à C₃, aryle en C₅ à C₁₀, hétéroaryle en C₅ à C₁₀, aryle en C₈ à C₁₀-alkyle en C₁ à C₃, hétéroaryle en C₅ à C₁₀-alkyle en C₁ à C₃, alkylcarbonyle en C₁ à C₄, cyano, aminocarbonyle, alkyle en C₁ à C₄-aminocarbonyle, di-(alkyle en C₁ à C₃)-aminocarbonyle, pyrrolidin-1-ylcarbonyle, pipéridin-1-ylcarbonyle, morpholin-4-ylcarbonyle, pipérazin-1-ylcarbonyle, 4-(alkyle en C₁ à C₄)-pipérazin-1-ylcarbonyle, carboxy, alkyloxycarbonyle en C₁ à C₄, nitro, amino, alkylamino en C₁ à C₄, di-(alkyle en C₁ à C₃)-amino, pyrrolidin-1-yle, pipéridin-1-yle, morpholin-4-yle, pipérazin-1-yle, 4-(alkyle en C₁ à C₄)pipérazin-1-yle, alkylcarbonylamino en C₁ à C₄, alkyloulfonylamino en C₁ à C₄, hydroxy, alkyloxy en C₁ à C₆, cycloalkyloxy en C₃ à C₇, aryloxy en C₆ à C₁₀, alkylsulfanyle en C₁ à C₄, alkylsulfinyle en C₁ à C₄ et alkylsulfonyle en C₁ à C₄,
où les radicaux alkyle, alcényle, alcinyle, cycloalkyle et hétérocycloalkyle peuvent être partiellement ou complètement fluorés et/ou peuvent être mono- ou disubstitués par des substituants identiques ou différents choisis parmi les groupes cyano, hydroxy, alkyloxy en C₁ à C₃, acétylamino, méthylsulfonylamino et alkyle en C₁ à C₃, et
où dans les radicaux cycloalkyle, un ou deux groupes méthylène peuvent être substitués indépendamment l'un de l'autre par O, S, CO, SO ou SO₂, et
ou dans les radicaux N-hétérocycloalkyle, un groupe méthylène peut être substitué par CO ou SO₂, où R¹ n'est pas un atome d'hydrogène lorsque R³ a la signification hydrogène, et
R² est choisi parmi un atome d'hydrogène, un groupe alkyle en C₁ à C₆, alcényle en C₂ à C₆, alcinyle en C₂ à C₆, cycloalkyle en C₃ à C₇-alkyle en C₁ à C₄, alkylcarbonyle en C₁ à C₆-alkyle en C₁ à C₃, aryle en C₅ à C₁₀-alkyle en C₁ à C₄, hétéroaryle en C₅ à C₁₀-alkyle en C₁ à C₄, cycloalkyle en C₃ à C₇, alkylcarbonyle en C₁ à C₆, cycloalkyle en C₃ à C₇-carbonyle, cyano, aminocarbonyle, carboxy, amino, alkylcarbonylamino en C₁ à C₄, alkylsulfonylamino en C₁ à C₄, alkyloxy en C₁ à C₄, aryle en C₆ à C₁₀, hétéroaryle en C₅ à C₁₀, hydroxy,
où tous les radicaux alkyle, alcényle, alcinyle et cycloalkyle mentionnés peuvent être partiellement ou complètement fluorés et/ou peuvent être mono, di- ou tri-substitué par des substituants identiques ou différents choisis parmi les groupes alkyle en C₁ à C₃, cyano, hydroxy, alkyloxy en C₁ à C₃, amino, alkylamino en C₁ à C₃, di-(alkyle en C₁ à C₃)amino, acétylamino, méthylsulfonylamino, aryle en C₆ à C₁₀ et hétéroaryle en C₅ à C₁₀,
où dans les radicaux alkyle mentionnés, éventuellement un ou deux groupes CH₂ sont échangés contre O et/ou NR^{N},
où dans les radicaux cycloalkyle mentionnés, un ou deux groupes CH₂ peuvent être remplacés indépendamment l'un de l'autre par NR^{N}, O, S, CO, SO ou SO₂ et un ou deux groupes CH peuvent être remplacés par N, et
R³ est choisi parmi un atome d'hydrogène, d'halogène, un groupe alkyle en C₁ à C₄, alcinyle en C₂ à C₆, alcényle en C₂ à C₆, cycloalkyle en C₃ à C₇-alkyle en C₁ à C₃, cycloalkyle en C₃ à C₅, trifluorométhyle, difluorométhyle, hydroxy, alkyloxy en C₁ à C₄, trifluorométhoxy, difluorométhoxy, cyano, aminocarbonyle, alkyle en C₁ à C₄-aminocarbonyle, di-(alkyle en C₁ à C₃)-aminocarbonyle, carboxy, alkyloxycarbonyle en C₁ à C₄, amino, alkylamino en C₁ à C₄, di-(alkyle en C₁ à C₃)-amino, alkylcarbonylamino en C₁ à C₄, alkylsulfonylamino en C₁ à C₄, R³ n'étant pas un atome d'hydrogène lorsque R¹ a la signification hydrogène,
R⁴ est choisi parmi un atome d'hydrogène, un groupe alkyle en C₁ à C₄, cycloalkyle en C₃ à C₆, trifluorométhyle, difluorométhyle, cyano, carboxy, aminocarbonyle, alkylaminocarbonyle en C₁ à C₃, di-(alkyle en C₁ à C₃)aminocarbonyle, alkyloxycarbonyle en C₁ à C₄, amino, alkylamino en C₁ à C₃, di- (alkyle en C₁ à C₃)amino, hydroxy, alkyloxy en C₁ à C₄, alkylsulfanyle en C₁ à C₄,
R⁵ est un groupe alkyle en C₁ à C₄,
R⁶ est choisi parmi les groupes diméthylamino-alkyle en C₁ à C₃, cyclopropylamino-alkyle en C₁ à C₃, pyrrolidinyl-alkyle en C₁ à C₃, hydroxy-alkyle en C₁ à C₃, méthylsulfanyl-alkyle en C₁ à C₃, méthylsulfinyl-alkyle en C₁ à C₃, méthylsulfonyl-alkyle en C₁ à C₃, alkylcarbonylamino en C₁ à C₆, cycloalkyle en C₃ à C₅-carbonylamino, alkylsulfonylamino en C₁ à C₄, cycloalkylsulfonylamino en C₃ à C₅, alkylsulfinyle en C₁ à C₃ et alkylsulfonyle en C₁ à C₃,
R₇ est choisi parmi les groupes alkyle en C₄, trifluorométhyle, pentafluoroéthyle,
X, Y sont indépendamment l'un de l'autre, N ou CR₁₃, où X et Y ayant la même signification, N est exclu et R₁₃ est choisi parmi un atome d'hydrogène, de fluor, de chlore, un groupe alkyle en C₁ à C₄, trifluorométhyle, hydroxy, alkyloxy en C₁ à C₄, trifluorométhoxy, difluorométhoxy, cyano,
R^{N} est choisi parmi H, les groupes alkyle en C₁ à C₄, 1,1,1-trifluoroéthyle, cyanométhyle, acétyle, méthylsulfonyle, arylméthyle en C₆ à C₁₀,
L est choisi dans le groupe consistant en atome de fluor, de chlore, de brome, un groupe alkyle en C₁ à C₃, difluorométhyle, trifluorométhyle, hydroxy, alcoxy en C₁ à C₃, difluorométhoxy, trifluorométhoxy, acétylamino, méthylsulfonylamino et cyano,
où les groupes aryle en C₅ à C₁₀ mentionnés dans la définition des radicaux précédemment cités désignent des groupes phényle ou naphtyle, qui peuvent être indépendamment les uns des autres, mono- ou disubstitués par des radicaux L identiques ou différents, et
les groupes hétéroaryle mentionnés dans la définition des radicaux cités précédemment désignent un groupe pyrrolyle, furanyle, thiényle, pyridyle, indolyle, benzofuranyle, benzothiophényle, quinolinyle ou isoquinolinyle,
ou désignent un groupe pyrrolyle, furanyle, thiényle ou pyridyle, dans lequel un ou deux groupes méthine sont remplacés par des atomes d'azote,
ou désignent un groupe indolyle, benzofuranyle, benzothiophényle, quinolinyle ou isoquinolinyle, dans lequel un à trois groupes méthine sont remplacés par des atomes d'azote,
où les groupes hétéroaryle mentionnés précédemment peuvent être mono- ou disubstitués indépendamment les uns des autres par des radicaux L identiques ou différents ;
où, sauf indication contraire, les groupes alkyle mentionnés précédemment peuvent être à chaîne linéaire ou ramifiée,
leurs tautomères, leurs stéréo-isomères, leurs mélanges et leurs sels, en particulier leurs sels physiologiquement acceptables.

3. Amide d'acide benzoïque à substitution hétéroaryloxy selon l'une des revendications précédentes, **caractérisé en ce que**
R¹ est choisi parmi un atome d'hydrogène, de fluor, de chlore, de brome, un groupe alkyle en C₁ à C₄, trifluorométhyle, cycloalkyle en C₃ à C₆, cyano, aminocarbonyle, alkyle en C₁ à C₄-aminocarbonyle, di-(alkyle en C₁ à C₃)-aminocarbonyle, carboxy, alkyloxycarbonyle en C₁ à C₄, amino, alkylamino en C₁ à C₄, di-(alkyle en C₁ à C₃)-amino, pyrrolidin-1-yle, pipéridin-1-yle, morpholin-4-yle, acétylamino, méthylsulfonylamino, hydroxy, alkyloxy en C₁ à C₄, trifluorométhoxy, difluorométhoxy et alkylsulfonyle en C₁ à C₄,
où les radicaux alkyle, cycloalkyle et N-hétérocycloalkyle cités peuvent être mono- ou disubstitués par des substituants identiques ou différents choisis parmi les groupes cyano, hydroxy, alkyloxy en C₁ à C₃, acétylamino, méthylsulfonylamino et alkyle en C₁ à C₃, et
où dans les radicaux cycloalkyle cités, un ou deux groupes méthylène peuvent être substitués indépendamment l'un de l'autre par O, CO ou SO₂ et dans les radicaux N-hétérocycloalkyle cités, un groupe méthylène peut être substitué par CO ou SO₂, et
où R¹ n'est pas un atome d'hydrogène lorsque R³ a la signification hydrogène.

4. Amide d'acide benzoïque à substitution hétéroaryloxy selon l'une ou plusieurs des revendications précédentes, **caractérisé en ce que**
R² est choisi parmi un atome d'hydrogéne, les groupes alkyle en C₁ à C₄, cycloalkyle en C₃ à C₆-alkyle en C₁ à C₃, alkyle en C₁ à C₃-amino-alkyle en C₁ à C₃, cycloalkylamino en C₃ à C₈-alkyle en C₁ à C₃, pyrrolidinyl-alkyle en C₁ à C₃, pipéridin-alkyle en C₁ à C₃, azépanyl-alkyle en C₁ à C₃, pipérazinyl-alkyle en C₁ à C₃, morpholinyl-alkyle en C₁ à C₃, homopipérazinyl-alkyle en C₁ à C₃, pyrrolidin-3-ylamino-alkyle en C₁ à C₃, pipéridin-3-ylamino-alkyle en C₁ à C₃, pipéridin-4-ylamino-alkyle en C₁ à C₃, cycloalkyle en C₃ à C₆, trifluorométhyle, cyano, aminocarbonyle, alkyle en C₁ à C₄-aminocarbonyle, di-(alkyle en C₁ à C₃)-aminocarbonyle, pipérazin-1-ylcarbonyle, morpholin-4-ylcarbonyle, carboxy, alkyloxycarbonyle en C₁ à C₄, amino, alkylamino en C₁ à C₄, cycloalkylamino en C₃ à C₆, cycloalkyle en C₃ à C₆-alkylamino en C₁ à C₃, pyrrolidin-1-yle, pipéridin-1-yle, pipérazin-1-yle, morpholin-4-yle, alkyle en C₁ à C₃-amino-alkylamino en C₂ à C₃, pyrrolidin-3-ylamino, pipéridin-3-ylamino, pipéridin-4-ylamino, pyrrolidinyl-alkyle en C₁ à C₃-amino, pipéridin-alkyle en C₁ à C₃-amino, pipérazinyl-alkyle en C₁ à C₃-amino, morpholinyl-alkyle en C₁ à C₃-amino, homopipérazinyl-alkyle en C₁ à C₃-amino, acétylamino, méthylsulfonylamino, hydroxy, alkyloxy en C₁ à C₄, trifluorométhoxy, difluorométhoxy, cycloalkyloxy en C₃ à C₆, cycloalkyle en C₃ à C₆-alkyloxy en C₁ à C₃, alkylamino en C₁ à C₃-alkyloxy en C₂ à C₃, di-(alkyle en C₁ à C₃)-amino-alkyloxy en C₂ à C₃, pyrrolidinyl-alkyloxy en C₂ à C₃, pipéridin-alkyloxy en C₂ à C₃, pipérazinyl-alkyloxy en C₂ à C₃, morpholinyl-alkyloxy en C₂ à C₃, homopipérazinyl-alkyloxy en C₂ à C₃,
où les radicaux alkyle, cycloalkyle et N-hétérocycloalkyle mentionnés ci-dessus peuvent être mono ou di-substitués par des substituants identiques ou différents choisis parmi les groupes alkyle en C₁ à C₃, cyano, amino, alkylamino en C₁ à C₃, di-(alkyle en C₁ à C₃)amino, acétylamino, méthylsulfonylamino, hydroxy et alkyloxy en C₁ à C₃, et
où dans les radicaux cycloalkyle mentionnés ci-dessus, un ou deux groupes méthylène peuvent être substitués indépendamment l'un de l'autre par O et/ou CO et dans les radicaux N-hétérocycloalkyle mentionnés, un groupe méthylène peut être substitué par CO ou SO₂, et
où éventuellement, tous les groupes NH contenus dans les radicaux mentionnés ci-dessus sont remplacés par N-Me, N-Et, N-/*i*Pr, N-acétyle et N-SO₂Me.

5. Amide d'acide benzoïque à substitution hétéroaryloxy selon une ou plusieurs des revendications précédentes, **caractérisé en ce que** R³ est choisi parmi un atome d'hydrogène, un groupe alkyle en C₁ à C₃, cycloalkyle en C₃ à C₆, un atome de fluor, de chlore, de brome, un groupe trifluorométhyle, difluorométhyle, hydroxy, alkyloxy en C₁ à C₃, trifluorométhoxy, difluorométhoxy et cyano, R³ n'étant pas un atome d'hydrogène lorsque R¹ a la signification hydrogène.

6. Amide d'acide benzoïque à substitution hétéroaryloxy selon une ou plusieurs des revendications précédentes, **caractérisé en ce que** R⁴ est choisi parmi un atome d'hydrogène, un groupe alkyle en C₁ à C₃, trifluorométhyle, carboxy, aminocarbonyle, méthylaminocarbonyle, diméthylamino-carboinyle, amino, alkylamino en C₁ à C₃, di-(alkyle en C₁ à C₃)-amino, hydroxy et alkyloxy en C₁ à C₃.

7. Amide d'acide benzoïque à substitution hétéroaryloxy selon une ou plusieurs des revendications précédentes, **caractérisé en ce que** R⁵ est choisi parmi les groupes méthyle, éthyle, n-propyle et isopropyle.

8. Amide d'acide benzoïque substitution hétéroaryloxy selon une ou plusieurs des revendications précédentes, **caractérisé en ce que** R⁶ est choisi parmi les groupes diméthylaminométhyle, cyclopropylaminométhyle, pyrrolidin-1-ylamino, hydroxyméthyle, méthylsulfanylméthyle, méthylsulfinylméthyle, méthylsulfonylméthyle, n-butylcarbonyl-amino, isopropylcarbonyl-amino, isobutylcarbonylamino, cyclopropylcarbonylamino, méthylsulfonylamino, isopropylsulfonylamino, n-butylsulfonylamino, isobutylsulfonyl-amino, cyclopropyl-sulfonylamino, méthylsulfinyle et méthylsulfonyle.

9. Amide d'acide benzoïque à substitution hétéroaryloxy selon une ou plusieurs des revendications précédentes, **caractérisé en ce que** R⁷ est choisi parmi les groupes isobutyle, tert-butyle, trifluorométhyle et pentafluoroéthyle.

10. Amide d'acide benzoïque à substitution hétéroaryloxy selon une ou plusieurs des revendications précédentes, **caractérisé en ce que** X et Y sont choisis indépendamment l'un de l'autre parmi N, C-H, C-F, C-Cl, C-alkyle en C₁ à C₃ et alkyloxy en C₁ à C₃, à condition que X et Y ne soient pas présents dans la même signification N.

11. Composés selon la revendication 1, choisis parmi :
N-(5-tert-butyl-3-méthanesulfonylamino-2-méthoxy-phényl)-4-méthyl-3-(pyridin-4-yloxy)-benzamide ;
N-(5-tert-butyl-3-méthanesulfonylamino-2-méthoxy-phényl)-4-méthyl-3-(2-méthylamino-pyridin-4-yloxy)-benzamide ;
N-(5-tert-butyl-3-méthanesulfonylamino-2-méthoxy-phényl)-4-méthyl-3-(6-méthylamino-pyridin-4-yloxy)-benzamide ;
N-(5-tert-butyl-3-méthanesulfonylamino-2-méthoxy-phényl)-4-méthyl-3-[2-(2-pyrrolidin-1-yl-éthylamino)-pyrimidin-4-yloxy)-benzamide ;
N-(5-tert-butyl-3-méthanesulfonylammo-2-méthoxy-phényl)-3-[6-(2-hydroxy-éthylamino)-pyrimidin-4-yloxy)-4-méthyl-benzamide ;
N-(5-tert-butyl-3-méthanosulfonylamino-2-méthoxy-phényl)-3-[6-(2-diméthylamino-éthylamino)-pyrimidin-4-yloxy)-4-méthyl-benzamide ;
N-(5-tert-butyl-3-méthanesulfonylamino-2-méthoxy-phényl)-4-méthyl-3-[6-(3-oxo-pipérazin-1-yl)-pyrimidin-4-yloxy)-benzamide ;
N-(5-tert-butyl-3-méthanesulfonylamino-2-méthoxy-phényl)-4-méthyl-3-(6-pyrrolidin-1-yl-pyrimidin-4-yloxy)-benzamide ;
N-5-tert-butyl-3-méthanesulfonylamino-2-méthoxy-phényl)-4-méthyl-3-(6-morpholin-4-yl-pyrimidin-4-yloxy)-benzamide ;
*(R)*-N-(5-tert-butyl-3-méthanesulfonylamino-2-méthoxy-phényl)-3-[2-(3-diméthylamino-pyrrolidin-1-yl)-pyrimidin-4-yloxy]-4-méthyl-benzamide ;
*(S)*-N-(5-tert-butyl-3-méthanesulfonylamino-2-méthoxy-phényl)-3-[2-(3-diméthylamino-pyrrolidin-1-yl)-pyrimidin-4-yloxy]-4-méthyl-benzamide ;
N-(5-tert-butyl-3-méthanesulfonylamino-2-méthoxy-phényl)-3-[2-(4-diméthylamino-pipéridin-1-yl)-pyrimidin-4-yloxy]-4-méthyl-benzamide ;
N-(5-tert-butyl-3-méthanesulfonylamino-2-méthoxy-phényl)-4-méthyl-3-[2-(1-méthyl-pipéridin-4-ylamino)-pyrimidin-4-yloxy]-benzamide ;
N-(5-tert-butyl-3-méthanesulfonylamino-2-méthoxy-phényl)-4-méthyl-3-(2-pipéridin-1-yl-pyrimidin-4-yloxy]-benzamide ;
N-(5-tert-butyl-3-méthanesulfonylamino-2-méthoxy-phényl)-4-méthyl-3-(6-pipérazin-1-yl-pyrimidin-4-yloxy]-benzamide ;
N-(5-tert-butyl-3-méthanesulfonylamino-2-méthoxy-phényl)-4-méthyl-3-[6-(pipérazin-1-carbonyl)-pyrimidin-4-yloxy]-benzamide ;
N-(5-tert-butyl-3-méthanesulfonylamino-2-méthoxy-phényl)-4-chloro-3-[6-(pipérazin-1-carbonyl)-pyrimidin-4-yloxy]-benzamide ;
N-(5-tert-butyl-3-méthanesulfonylamino-2-méthoxy-phényl)-3-(2-pipérazin-1-yl-pyrimidin-4-yloxy)-4-trifluorométhyl-benzamide ;
N-(5-tert-butyl-3-méthanesulfonylamino-2-méthoxy-phényl)-4-chloro-3-(2-pipérazin-1-yl-pyrimidin-4-yloxy)-benzamide ;
N-(5-tert-butyl-3-méthanesulfonylamino-2-méthoxy-phényl)-4-méthyl-3-(2-pipérazin-1-ylméthyl-pyrimidin-4-yloxy)-benzamide ;
N-(5-tert-butyl-3-méthanesulfonylamino-2-méthoxy-phényl)-4-méthyl-3-(6-pipérazin-1-ylméthyl-pyrimidin-4-yloxy)-benzamide ;
N-(5-tert-butyl-3-méthanesulfonylamino-2-méthoxy-phényl)-4-méthoxy-3-(2-pipérazin-1-ylméthyl-pyrimidin-4-yloxy)-benzamide ;
N-(5-tert-butyl-3-méthanesulfonylamino-2-méthoxy-phényl)-4-méthyl-3-(2-pipérazin-1-ylméthyl-pyridin-4-yloxy)-benzamide ;
N-(5-tert-butyl-3-méthanesulfonylamino-2-méthoxy-phényl)-4-fluoro-3-(2-pipérazin-1-yl-pyrimidin-4-yloxy)-benzamide ;
N-(5-tert-butyl-3-méthanesulfonylamino-2-méthoxy-phényl)-4-bromo-3-(2-pipérazin-1-yl-pyrimidin-4-yloxy)-benzamide ;
N-(5-tert-butyl-3-méthanesulfonylamino-2-méthoxy-phényl)-3-chloro-4-méthyl-5-(2-pipérazin-1-yl-pyrimidin-4-yloxy)-benzamide ;
N-(5-tert-butyl-3-méthanesulfonylamino-2-méthoxy-phényl-)-3-fluoro-4-méthyl-5-(2-pipérazin-1-yl-pyrimidin-4-yloxy)-benzamide ;
N-(5-tert-butyl-3-méthanesulfonylamino-2-méthoxy-phényl)-4-méthyl-3-[2-(N-méthyl-N-pipéridin-4-yl-amino)-pyrimidin-4-yloxy]-benzamide ;
N-(5-tert-butyl-3-méthanesulfonylamino-2-méthoxy-phényl)-4-éthyl-5-(2-pipérazin-1-yl-pyrimidin-4-yloxy)-benzamide ;
N-(5-tert-butyl-3-méthanesulfonylamino-2-méthoxy-phényl)-4-méthyl-3-[2-(pipéridin-4-yl-amino)-pyrimidin-4-yloxy]-benzamide ;
N-(5-tert-butyl-3-méthanesulfonylamino-2-méthoxy-phényl)-4-méthyl-3-[6-(pipéridin-4-yl-amino)-pyrimidin-4-yloxy]-benzamide ;
N-(5-tert-butyl-3-méthanesulfonylamino-2-méthoxy-phényl)-4-méthyl-3-(6-pipéridin-4-ylméthyl-pyrimidin-4-yloxy)-benzamide ;
N-(5-tert-butyl-3-méthanesulfonylamino-2-méthoxy-phényl)-4-méthyl-3-(2-pipéridin-4-ylméthyl-pyrimidin-4-yloxy)-benzamide ;
N-(5-tert-butyl-3-méthanesulfonylamino-2-méthoxy-phényl)-4-méthyl-3-(2-pipéridin-4-ylméthyl-pyridin-4-yloxy)-benzamide ;
N-(5-tert-butyl-3-méthanesulfonylamino-2-méthoxy-phényl)-3-méthyl-5-[2-(pipérazin-1-carbonyl)-pyrimidin-4-yloxy]-benzamide ;
N-(5-tert-butyl-3-méthanesulfonylamino-2-méthoxy-phényl)-3-méthyl-5-(2-pipérazin-1-ylméthyl-pyrimidin-4-yloxy)-benzamide ;
N-(5-tert-butyl-3-méthanesulfonylamino-2-méthoxy-phényl)-3-méthyl-5-(pyridin-4-yloxy)-benzamide ;
méthylamide d'acide 6-[5-(5-tert-butyl-3-méthanesulfonylamino-2-méthoxy-phénylaminocarbonyl)-2-méthyl-phénoxy]-pyrimidin-4-carboxylique ;
N-(5-tert-butyl-3-méthanesulfonylamino-2-méthoxy-phényl)-4-méthyl-3-[6-(morpholin-4-carbonyl)-pyrimidin-4-yloxy]-benzamide ;
N-(5-tert-butyl-3-méthanesulfonylamino-2-méthoxy-phényl)-4-méthyl-3-[6-(4-méthyl-pipérazin-1-carbonyl)-pyrimidin-4-yloxy]-benzamide ;
N-(5-tert-butyl-3-méthanesulfonylamino-2-méthoxy-phényl)-4-méthyl-3-(6-morpholin-4-ylméthyl-pyrimidin-4-yloxy)-benzamide ;
N-(5-tert-butyl-3-méthanesulfonylamino-2-méthoxy-phényl)-4-méthyl-3-(6-méthylaminométhyl-pyrimidin-4-yloxy)-benzamide ;
N-(5-tert-butyl-3-méthanesulfonylamino-2-méthoxy-phényl)-4-méthyl-3-[6-(4-méthyl-pipérazin-1-ylméthyl)-pyrimidin-4-yloxy]-benzamide ;
N-(5-tert-butyl-3-méthanesulfonylamino-2-méthoxy-phényl)-4-méthyl-3-(2-morpholin-4-ylméthyl-pyrimidin-4-yloxy)-benzamide ;
N-(5-tert-butyl-3-méthanesulfonylamino-2-méthoxy-phényl)-4-méthyl-3-(2-méthylaminométhyl-pyrimidin-4-yloxy)-benzamide ;
N-(5-tert-butyl-3-méthanesulfonylamino-2-méthoxy-phényl)-4-méthyl-3-[2-(3-oxo-pipérazin-1-ylméthyl)-pyrimidin-4-yloxy]-benzamide ;
N-(5-tert-butyl-3-méthanesulfonylamino-2-méthoxy-phényl)-4-méthyl-3-[2-(4-méthyl-pipérazin-1-ylméthyl)-pyrimidin-4-yloxy]-benzamide ;
N-(5-tert-butyl-3-méthanesulfonylamino-2-méthoxy-phényl)-4-méthyl-3-[6-(3-oxo-pipérazin-1-ylméthyl)-pyrimidin-4-yloxy]-benzamide ;
N-(5-tert-butyl-3-méthanesulfonylamino-2-méthoxy-phényl)-4-méthyl-3-(6-pipérazin-1-ylméthyl-pyrimidin-4-yloxy)-benzamide ;
N-(5-tert-butyl-3-méthanesulfonylamino-2-méthoxy-phényl)-4-méthyl-3-(2-morpholin-4-ylméthyl-pyridin-4-yloxy)-benzamide ;
N-(5-tert-butyl-3-méthanesulfonylamino-2-méthoxy-phényl)-4-méthyl-3-[2-(4-méthyl-pipérazin-1-ylméthyl)pyridin-4-yloxy]-benzamide ;
*(S)*-N-(5-tert-butyl-3-méthanesulfonylamino-2-méthoxy-phényl)-4-méthyl-3-{2-[(tétrahydro-furan-3-ylamino)-méthyl]-pyridin-4-yloxy}-benzamide ;
*(R)*-N-(5-tert-butyl-3-méthanesulfonylamino-2-méthoxy-phényl)-3-[2-(3-hydroxy-pyrrolidin-1-ylméthyl)pyridin-4-yloxy]-4-méthyl-benzamide ;
*(S)*-N-(5-tert-butyl-3-méthanesulfonylamino-2-méthoxy-phényl)-3-[2-(3-hydroxy-pyrrolidin-1-ylméthyl)pyridin-4-yloxy]-4-méthyl-benzamide ;
N-(5-tert-butyl-3-méthanesulfonylamino-2-méthoxy-phényl)-4-méthyl-3-(2-méthylaminométhyl-pyridin-4-yloxy)-benzamide ;
*(S)*-N-(5-tert-butyl-3-méthanesulfonylamino-2-méthoxy-phényl)-4-méthyl-3-{2-[(tétrahydro-furan-3-ylamino)-méthyl]-pyridin-4-yloxy}-benzamide ;
N-(5-tert-butyl-3-méthanesulfonylamino-2-méthoxy-phényl)-4-méthyl-3-[2-(4-méthyl-homopipérazin-1-ylméthyl)pyrimidin-4-yloxy]-benzamide ;
N-(5-tert-butyl-3-méthanesulfonylamino-2-méthoxy-phényl)-4-méthyl-3-{2-[(1-méthyl-pipéridin-4-ylamino)-méthyl]-pyrimidin-4-yloxy}-benzamide ;
N-(5-tert-butyl-3-méthanesulfonylamino-2-méthoxy-phényl)-4-méthyl-3-{2-[(1-méthyl-pipéridin-3-ylamino)-méthyl]-pyrimidin-4-yloxy}-benzamide ;
N-(5-tert-butyl-3-méthanesulfonylamino-2-méthoxy-phényl)-4-méthyl-3-(2-{(méthyl-(1-méthyl-pyrrolidin-3-yl)amino]-méthyl}-pyrimidin-4-yloxy)-benzamide ;
N-(5-tert-butyl-3-méthanesulfonylamino-2-méthoxy-phényl)-4-méthyl-3-[2-(4-méthyl-homopipipérazin-1-ylméthyl)-pyridin-4-yloxy]-benzamide ;
*(S)*-N-(5-tert-butyl-3-méthanesulfonylamino-2-méthoxy-phényl)-3-[2-(3-diméthylamino-pyrrolidin-1-ylméthyl)pyrimidin-4-yloxy] -4-méthyl-benzamide ;
*(R)*-N-(5-tert-butyl-3-méthanesulfonylamino-2-méthoxy-phényl)-3-[2-(3-diméthylamino-pyrrolidin-1-ylméthyl)pyrimidin-4-yloxy]-4-méthyl-benzamide ;
N-(5-tert-butyl-3-méthanesulfonylamino-2-méthoxy-phényl)-4-méthyl-3-{2-[(1-méthyl-pyrrolidin-3-ylamino)-méthyl]-pyrimidin-4-yloxy}-benzamide ;
N-(5-tert-butyl-3-méthanesulfonylamino-2-méthoxy-phényl)-3-(2-{(N-(2-diméthylamino-éthyl)-N-méthyl-amino]-méthyl}-pyrimidin-4-yloxy)}-4-méthyl-benzamide ;
N-(5-tert-butyl-3-méthanesulfonylamino-2-méthoxy-phényl)-3-{2-[(2-diméthylamino-éthylamino)-méthyl]-pyrimidin-4-yloxy}-4-méthyl-benzamide ;
N-(5-tert-butyl-3-méthanesulfonylamino-2-méthoxy-phényl)-3-(2-{[(2-diméthylamino-éthyl)-méthyl-aminol-méthyl}-pyridin-4-yloxy)-4-méthyl-benzamide ;
N-(5-tert-butyl-3-méthanesulfonylamino-2-méthoxy-phényl)-3-{2-[(2-diméthylamino-éthylamino)-méthyl]-pyridin-4-yloxy}-4-méthyl-benzamide ;
N-(5-tert-butyl-3-méthanesulfonylamino-2-méthoxy-phényl)-3-méthyl-5-[2-(4-méthyl-pipérazin-1-ylméthyl)-pyrimidin-4-yloxy}-benzamide ;
N-(5-tert-butyl-3-méthanesulfonylamino-2-méthoxy-phényl)-3-{6-[(2-diméthylamino-éthylamino)-méthyl]-pyrimidin-4-yloxy}-4-méthyl-benzamide ;
N-(5-tert-butyl-3-méthanesulfonylamino-2-méthoxy-phényl)-3-(6-{[(2-diméthylamino-éthyl)-méthyl-amino]-méthyl}-pyrimidin-4-yloxy)-4-méthyl-benzamide ;
N-(5-tert-butyl-3-méthanesulfonylamino-2-méthoxy-phényl)-4-méthyl-3-[6-(1-méthyl-pipéridin-4-ylméthyl)-pyrimidin-4-yloxy]-benzamide ;
N-(5-tert-butyl-3-méthanesulfonylamino-2-méthoxy-phényl)-4-méthyl-3-[2-(1-méthyl-pipéridin-4-ylméthyl)-pyrimidin-4-yloxy]-benzamide ;
N-(5-tert-butyl-3-méthanesulfonylamino-2-méthoxy-phényl)-4-méthyl-3-[2-(1-méthyl-pipéridin-4-ylméthyl)-pyridin-4-yloxy]-benzamide ;
N-(5-tert-butyl-3-méthanesulfonylamino-2-méthoxy-phényl)-4-méthyl-3-[2-(pyrrolidin-3-ylamino)-pyrimidin-4-yloxy]-benzamide ;
N-(5-tert-butyl-3-méthanesulfonylamino-2-méthoxy-phényl)-4-chloro-3-[2-(4-méthyl-pipérazin-1-ylméthyl)-pyrimidin-4-yloxy]-benzamide ;
N-(5-tert-butyl-3-méthanesulfonylamino-2-méthoxy-phényl)-3-chloro-4-méthyl-5-[2-(4-méthyl-pipérazin-1-ylméthyl)-pyrimidin-4-yloxy]-benzamide ;
N-(5-tert-butyl-3-méthanesulfonylamino-2-méthoxy-phényl)-4-chloro-3-[6-(1-méthyl-pipéridin-4-ylamino)-pyrimidin-4-yloxy]-benzamide ;
N-(5-tert-butyl-3-méthanesulfonylamino-2-méthoxy-phényl)-4-chloro-3-[2-(1-méthyl-pipéridin-4-ylamino)-pyridin-4-yloxy]-benzamide ;
N-(5-tert-butyl-3-méthanesulfonylamino-2-méthoxy-phényl)-4-chloro-3-[6-(pipéridin-4-ylamino)-pyrimidin-4-yloxy]-benzamide ;
N-(5-tert-butyl-3-méthanesulfonylamino-2-méthoxy-phényl)-4-chloro-3-[2-(1-méthyl-pipéridin-4-ylamino)-pyrimidin-4-yloxy]-benzamide ;
N-(5-tert-butyl-3-méthanesulfonylamino-2-méthoxy-phényl)-4-méthyl-3-{2-[méthyl-(1-méthyl-pipéridin-4-yl)amino]-pyrimidin-4-yloxy}-benzamide ;
N-(5-tert-butyl-3-méthanesulfonylamino-2-méthoxy-phényl)-4-méthyl-3-{6-[méthyl-(1-méthyl-pipéridin-4-yl)amino]-pyrimidiri-4-yloxy}-benzamide ;
N-(5-tert-butyl-3-méthanesulfonylamino-2-méthoxy-phényl)-4-chloro-3-[2-(pipéridin-4-ylamino)-pyrimidin-4-yloxy]-benzamide ;
N-(5-tert-butyl-3-méthanesulfonylamino-2-méthoxy-phényl)-4-chloro-3-[2-(pipéridin-4-ylméthyl-pyridin-4-yloxy]-benzamide ;
N-(5-tert-butyl-3-méthanesulfonylamino-2-méthoxy-phényl)-4-chloro-3-(2-pipéridin-4-ylméthyl-pyrimidin-4-yloxy)-benzamide ;
N-(5-tert-butyl-3-méthanesulfonylamino-2-méthoxy-phényl)-4-chloro-3-{6-[(2-diméthylamino-éthylamino)-mëthyl]-pyrimidin-4-yloxy}-benzamide ;
N-(5-tert-butyl-3-méthanesulfonylamino-2-méthoxy-phényl)-4-chloro-3-(6-{[(2-diméthylamino-éthyl)-méthyl-amino]-méthyl}-pyrimidin-4-yloxy)-benzamide ;
N-(5-tert-butyl-3-méthanesulfonylamino-2-méthoxy-phényl)-4-chloro-3-[2-(pipéridin-4-ylamino)pyridin-4-yloxy]-benzamide ;
N-(5-tert-butyl-3-méthanesulfonylamino-2-méthoxy-phényl)-4-chloro-3-(6-pipérazin-1-ylméthyl-pyrimidin-4-yloxy)-benzamide ;
N-(5-tert-butyl-3-méthanesulfonylamino-2-méthoxy-phényl)-4-chloro-3-(6-pipéridin-4-ylméthyl-pyrimidin-4-yloxy)-benzamide ;
N- (5-tert-butyl-3-méthanesulfonylamino-2-méthoxy-phényl)-4-chloro-3-[6-(4-méthyl-pipérazin-1-ylméthyl)-pyrimidin-4-yloxy]-benzamide ;
N-(5-tert-butyl-3-méthanesulfonylamino-2-méthoxy-phényl)-4-chloro-3-[6-(1-méthyl-pipéridin-4-ylméthyl)-pyrimidin-4-yloxy]-benzamide ;
N-(5-tert-butyl-3-méthanesulfonylamino-2-méthoxy-phényl)-4-chloro-3-[2-(1-méthyl-pipéridin-4-ylméthyl)-pyrimidin-4-yloxy]-benzamide ;
N-(5-tert-butyl-3-méthanesulfonylamino-2-méthoxy-phényl)-4-chloro-3-(2-{[(2-diméthylamino-éthyl)-méthyl-amino]-méthyl}-pyridin-4-yloxy)-benzamide ;
N-(5-tert-butyl-3-méthanesulfonylamino-2-méthoxy-phényl)-3-[2-(*trans*-4-diméthylamino-cyclohexylamino)-pyrimidin-4-yloxy]-4-méthyl-benzamide ;
N-(5-tert-butyl-3-méthanesulfonylamino-2-méthoxy-phényl)-4-chloro-3-[2-{(2-diméthylamino-éthylamino)-méthyl]-pyridin-4-yloxy}-benzamide ;
N-(5-tert-butyl-3-méthanesulfonylamino-2-méthoxy-phényl)-4-chloro-3-(2-pipérazin-1-ylméthyl-pyridin-4-yloxy)-benzamide ;
N-(5-tert-butyl-3-méthanesulfonylamino-2-méthoxy-phényl)-4-méthyl-3-{2-[méthyl-(1-méthyl-pyrrolidin-3-yl)amino]-pyrimidin-4-yloxy}-benzamide ;
N-(5-tert-butyl-3-méthanesulfonylamino-2-méthoxy-phényl)-4-chloro-3-[2-(4-méthyl-pipérazin-1-ylméthyl)-pyridin-4-yloxy]-benzamide ;
N-(5-tert-butyl-3-méthanesulfonylamino-2-méthoxy-phényl)-4-méthyl-3-{6-[méthyl-(1-méthyl-pyrrolidin-3-yl)-amino]-pyrimidin-4-yloxy}-benzamide ;
N-(5-tert-butyl-3-méthanesulfonylamino-2-méthoxy-phényl)-4-chloro-3-[2-(4-méthyl-[1,4]diazépan-1-ylméthyl)-pyrimidin-4-yloxy]-benzamide ;
N-(5-tert-butyl-3-méthanesulfonylamino-2-méthoxy-phényl)-4-chloro-3-(2-{[(2-diméthylamino-éthyl)-méthyl-amino]-méthyl}-pyrimidin-4-yloxy)-benzamide ;
N-(5-tert-butyl-3-méthanesulfonylamino-2-méthoxy-phényl)-4-chloro-3-{2-[(2-diméthylamino-éthylamino)-méthyl]-pyrimidin-4-yloxy}-benzamide ;
N-(5-tert-butyl-3-méthanesulfonylamino-2-méthoxy-phényl)-4-chloro-3-[6-(4-méthyl-[1,4]diazépan-1-ylméthyl)-pyrimidin-4-yloxy]-benzamide ;
N-(5-tert-butyl-3-méthanesulfonylamino-2-méthoxy-phényl)-4-chloro-3-(2-pipérazin-1-ylméthyl-pyrimidin-4-yloxy)-benzamide ;
N-(5-tert-butyl-3-méthanesulfonylamino-2-méthoxy-phényl)-4-chloro-3-(6-[1,4]diazépan-1-ylméthyl-)-pyrimidin-4-yloxy]-benzamide ;
N-(5-tert-butyl-3-méthanesulfonylamino-2-méthoxy-phényl)-4-chloro-3-[2-(4-méthyl-[1,4]diazépan-1-ylméthyl)-pyridin-4-yloxy]-benzamide ;
N-(5-tert-butyl-3-méthanesulfonylamino-2-méthoxy-phényl)-4-chloro-3-(2-[1,4]diazépan-1-ylméthyl)-pyridin-4-yloxy)-benzamide ;
N-(5-tert-butyl-3-méthanesulfonylamino-2-méthoxy-phényl)-4-chloro-3-[2-(1-méthylpipéridin-4-ylméthyl)-pyridin-4-yloxy]-benzamide ;
N-(5-tert-butyl-3-méthanesulfonylamino-2-méthoxy-phényl)-4-méthyl-3-(2-pipéridin-4-ylméthyl-pyrimidin-4-yloxy)-benzamide ;
N-(5-tert-butyl-3-méthanesulfonylamino-2-méthoxy-phényl)-3-(2-[1,4]diazépan-1-ylméthyl)-pyridin-4-yloxy)-4-méthyl-benzamide ;
N-(5-tert-butyl-2-méthoxy-3-méthylsulfanylméthyl-phényl)-4-méthyl-3-(2-pipéridin-4-ylméthyl-pyridin-4-yloxy)-benzamide ;
N-(5-tert-butyl-3-méthanesulfonylméthyl-2-méthoxy-phényl)-4-méthyl-3-(2-pipéridin-4-ylméthyl-pyridin-4-yloxy-benzamide ;
N-(5-tert-butyl-3-méthanesulfinylméthyl-2-méthoxy-phényl)-4-méthyl-3-(2-pipéridin-4-ylméthyl-pyridin-4-yloxy-benzamide ;
N-[5-tert-butyl-3-(cyclopropanecarbonyl-amino)-2-méthoxy-phényl]-4-méthyl-3-(2-pipéridin-4-ylméthyl-pyridin-4-yloxy-benzamide ;
N-(5-tert-butyl-2-isopropoxy-3-méthanesulfonylamino-phényl)-4-méthyl-3-(2-pipéridin-4-ylméthyl-pyridin-4-yloxy)-benzamide ;
N-(5-tert-butyl-2-éthoxy-3-méthanesulfonylamino-phényl)-4-méthyl-3-(2-pipéridin-4-ylméthyl-pyridin-4-yloxy)-benzamide ;
N-[5-tert-butyl-2-méthoxy-3-(2-méthyl-propane-1-sulfinylamino)-phényl]-4-méthyl-3-(2-pipéridin-4-ylméthyl-pyridin-4-yloxy)-benzamide ;
N-[3-(butane-1-sulfonylamino)-5-tert-butyl-2-méthoxy-phényl]-4-méthyl-3-(2-pipéridin-4-ylméthyl-pyridin-4-yloxy)-benzamide ;
N-(5-tert-butyl-2-méthoxy-3-pentanoylamino-phényl)-4-méthyl-3-(2-pipéridin-4-ylméthyl-pyridin-4-yloxy) - benzamide ;
N-(5-tert-butyl-3-méthanesulfonyl-2-méthoxy-phényl)-4-méthyl-3-(2-pipéridin-4-ylméthyl-pyridin-4-yloxy)-benzamide ;
N-[5-tert-butyl-2-méthoxy-3-(3-méthyl-butylamino)-phényl]-4-méthyl-3-(2-pipéridin-4-ylméthyl-pyridin-4-yloxy)-benzamide ;
N-(5-tert-butyl-3-isobutyrylamino-2-méthoxy-phényl)-4-méthyl-3-(2-pipéridin-4-ylméthyl-pyridin-4-yloxy)-benzamide ;
N-(5-tert-butyl-3-méthanesulfinyl-2-méthoxy-phényl)-4-méthyl-3-(2-pipéridin-4-ylméthyl-pyridin-4-yloxy)-benzamide ;
N-(5-tert-butyl-3-méthanesulfinylméthyl-2-méthoxy-phényl)-4-méthyl-3-(2-pipéridin-4-ylméthyl-pyridin-4-yloxy)-benzamide ;
N-(5-tert-butyl-3-méthanesulfinylméthyl-2-méthoxy-phépyl)-4-méthyl-3-(2-pipéridin-4-ylméthyl-pyridin-4-yloxy)-benzamide ;
N-(5-tert-butyl-3-cyclopropanesulfonylamino-2-méthoxy-phényl)-4-méthyl-3-(2-pipéridin-4-ylméthyl-pyridin-4-yloxy)-benzamide ;
3-(2-azépan-4-ylméthyl-pyridin-4-yloxy)-N-(5-tert-butyl-3-méthanesulfonylamino-2-méthoxy-phényl)-4-méthyl-benzamide ;
3-(2-azépan-4-ylméthyl-pyrimidin-4-yloxy)-N-(5-tert-butyl-3-méthanesulfonylamino-2-méthoxy-phényl)-4-méthyl-benzamide ;
N-(5-tert-butyl-3-méthanesulfonylamino-2-méthoxy-phényl)-4-méthyl-3-[2-(1-méthyl-azépan-4-ylméthyl)-pyridin-4-yloxy]-benzamide ;
N-(5-tert-butyl-3-méthanesulfonylamino-2-méthoxy-phényl)-4-méthyl-3-[2-(1-méthyl-azépan-4-ylméthyl)-pyrimidin-4-yloxy]-benzamide ;
3-(6-azépan-4-ylméthyl-pyrimidin-4-yloxy)-N-(5-tert-butyl-3-méthanesulfonylamino-2-méthoxy-phényl)-4-méthyl-benzamide ;
N-(5-tert-butyl-3-méthanesulfonylamino-2-méthoxy-phényl)-4-méthyl-3-[6-(1-méthyl-azépan-4-ylméthyl)-pyrimidin-4-yloxy]-benzamide ;
N-(3-méthanesulfonylamino-2-méthoxy-5-pentafluoroéthyl-phényl)-4-méthyl-3-(2-pipéridin-4-ylméthyl)-pyridin-4-yloxy]-benzamide ;
N-(5-tert-butyl-3-hydroxyméthyl-2-méthoxy-phényl)-4-méthyl-3-[2-(4-méthyl-pipérazin-1-ylméthyl)-pyrimidin-4-yloxy]-benzamide ;
N-(5-tert-butyl-3-méthanesulfinyl-2-méthoxy-phényl)-4-méthyl-3-[2-(4-méthyl-pipérazin-1-ylméthyl)-pyrimidin-4-yloxy]-benzamide ;
N-(5-tert-butyl-3-diméthylaminoéthyl-2-méthoxy-phényl)-4-méthyl-3-(2-pipéridin-4-ylméthyl-pyridin-4-yloxy)-benzamide ;
N-(5-tert-butyl-3-méthanesulfonylamino-2-méthoxy-phényl)-4-méthyl-3-{2-[4-(2,2,2-trifluoro-éthyl)pipérazin-1-ylméthyl]-pyridin-4-yloxy}-benzamide ;
N-(5-tert-butyl-3-méthanesulfonylamino-2-méthoxy-phényl)-3-[2-(4-cyanométhyl-pipérazin-1-ylméthyl)-pyridin-4-yloxy]-4-méthyl-benzamide ;
N-(5-tert-butyl-2-méthoxy-3-pyrrolidin-1-ylméthyl-phényl)-4-méthyl-3-(2-pipéridin-4-ylméthyl-pyridin-4-yloxy}-benzamide ;
N-(5-tert-butyl-3-hydroxyméthyl-2-méthoxy-phényl)-4-méthyl-3-(2-pipérazin-1-ylméthyl-pyridin-4-yl-oxy)-benzamide ;
N-(5-tert-butyl-3-hydroxyméthyl-2-méthoxy-phényl)-4-méthyl-3-[2-(4-méthyl-pipérazin-1-ylméthyl)-pyridin-4-yloxy]-benzamide ;
N-(5-tert-butyl-3-hydroxyméthyl-2-méthoxy-phényl)-3-(2-[1,4]-diazépan-1-ylméthyl-pyridin-4-yloxy)-4-méthyl-benzamide ;
N-(5-tert-butyl-3-hydroxyméthyl-2-méthoxy-phényl)-4-méthyl-3-[2-(4-méthyl)-[1,4]-diazépan-1-ylméthyl)-pyridin-4-yloxy]-benzamide ;
N-(5-tert-butyl-3-cyclopropylaminométhyl-2-méthoxy-phényl)-4-méthyl-3-(2-pipéridin-4-ylméthyl-pyridin-4-yloxy)-benzamide
leur tautomères, leurs stéréoisomères et leurs mélanges.

12. Sels physiologiquement acceptables des composés selon une ou plusieurs des revendications 1 à 11, avec des acides inorganiques ou organiques.

13. Composé selon une ou plusieurs des revendications 1 à 11, ou sels physiologiquement acceptables selon la revendication 12, pour son utilisation comme médicament.

14. Médicament contenant un composé selon une ou plusieurs des revendications 1 à 11 ou un sel physiologiquement acceptable selon la revendication 12, outre éventuellement un ou plusieurs véhicules, conservateurs et/ou diluants inertes.

15. Utilisation d'au moins un composé selon une ou plusieurs des revendications 1 à 11, ou d'un sel physiologiquement acceptable selon la revendication 12, pour la production d'un médicament qui est approprié pour le traitement ou la prophylaxie de maladies des voies respiratoires.

16. Utilisation selon la revendication 15, **caractérisée en ce que** la maladie des voies respiratoires est la BPCO ou l'asthme.

17. Procédé de production d'un médicament selon la revendication 14, **caractérisé en ce que**, de façon non chimique, un composé selon au moins l'une des revendications 1 à 11 ou un sel physiologiquement acceptable selon la revendication 12 est incorporé dans un ou plusieurs véhicules, conservateurs et/ou diluants inertes.

18. Procédé de production des composés de formule générale I selon les revendications 1 à 11, **caractérisé en ce que** l'on fait réagir un composé de formule générale II 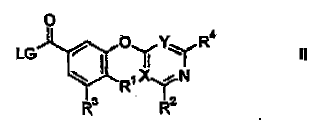 dans laquelle R¹ à R⁴, X et Y ont les significations indiquées dans les revendications 1 à 6 et 10 et LG est un atome de fluor, de chlore, de brome, un groupe cyano, alcoxy en C₁ à C₁₀, alkylsulfanyle en C₁ à C₆, alcényloxy en C₂ à C₄, alcinyloxy en C₂ à C₄, benzotriazol-1-yloxy, [1,2,3]triazolo[4,5-b]pyridin-3-yloxy, hétéroaryle en C₅ à C₁₀ (lié par N au composé II), succinyl-N-oxy, alkylcarbonyloxy en C₁ à C₄, di-(alkyle en C₁ à C₄)-aminocarbonyloxy, pyrrol-1-ylcarbonyloxy, pipéridin-1-yl-carbonyloxy, morpholin-4-ylcarbonyloxy, tri-(alkyle en C₁ à C₄)-carbamimidoyloxy, N,N,N',N'-tétra-(alkyle en C₁ à C₄)-uronium-O-yl, N,N'-dicyclohexyluron-O-yle, N-(3-diméthylaminopropyl)-N'-éthyl-uronyle, di-(alkyloxy en C₁ à C₄)-phosphoryloxy, bis(di-alkylamino en C₁ à C₄)-phosphoryloxy, dipyrrolidinyl-phosphoryloxy, arylsulfanyle en C₅ à C₁₀, hétéroarylsulfanyle en C₅ à C₁₀, aryloxy en C₅ à C₁₀ ou hétéroaryloxy en C₅ à C₁₀
où tous les groupes alkyle, alcényle et alcinyle mentionnés dans la définition peuvent être mono- ou polysubstitués par un atome de fluor, de chlore, un groupe alkyle en C₁ à C₃ et/ou alcoxy en C₁ à C₃,
où tous les groupes aryle mentionnés dans la définition désignent des groupes phényle ou naphtyle et tous les groupes hétéroaryle désignent des groupes pyridinyle, pyrimidinyle, triazinyle, imidazolyle, pyrazolyle, triazolyle, tétrazolyle qui, ainsi que les groupes aryle et hétéroaryle, sont éventuellement mono- ou polysubstitués par des radicaux identiques ou différents choisis parmi des atomes de fluor, de chlore, de brome, un groupe alkyle en C₁ à C₃, alkyloxy en C₁ à C₃, nitro, cyano et di(alkyle en C₁ à C₃)amino,
avec une aniline de formule générale 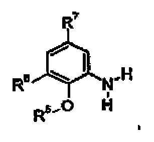 dans laquelle R⁵, R⁶ et R⁷ ont les significations indiquées dans les revendications 1, 2, 7, 8 et 9,
éventuellement en présence d'une base et/ou d'un additif tel que par exemple la triéthylamine, la pyridine, l'éthyldiisopropylamine, la 4-diméthylaminopyridine, le carbonate de potassium ou le 1-hydroxybenzotriazole ;
et
si nécessaire, un radical protecteur utilisé dans les réactions décrites précédemment aux points a) et b) est clivé et/ou
si on le souhaite, un composé de formule générale I ainsi obtenu est séparé en ses stéréoisomères et/ou si on le souhaite, un composé de formule générale I ainsi obtenu est converti en ses sels, en particulier en ses sels physiologiquement acceptables pour l'utilisation pharmaceutique.

19. Procédé de production de composés de formule générale I selon les revendications 1 à 11, **caractérisé en ce que** l'on fait réagir un composé de formule général III 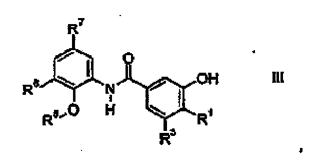 dans laquelle R¹, R³, R⁵, R⁶ et R⁷ ont les significations indiquées dans les revendications 1 à 3, 5 et 7 à 9,
avec un composé de formule générale IV 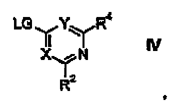 dans laquelle R² et R⁴ ont les significations indiquées dans les revendications 1, 2, 4, 6 et 10 et LG représente un groupe partant, en particulier, LG désigne F, Cl, Br, I, un groupe O-alkyle en C₁ à C₆, O-aryle en C₅ à C₁₀, S(O)ₙ-alkyle en C₁ à C₄, S(O)ₘ-aryle en C₅ à C₁₀, OSO₂-alkyle en C₁ à C₄, OSO₂-aryle en C₅ à C₁₀, NO₂,
où tous les groupes alkyle mentionnés sont éventuellement mono- ou polysubstitués par un atome de fluor, un groupe alkyle en C₁ à C₃ et/ou alcoxy en C₁ à C₃, et où tous les groupes aryle mentionnés désignent des groupes phényle ou naphtyle qui sont éventuellement mono- ou polysubstitués par des radicaux identiques ou différents choisis parmi le fluor, le chlore, les groupes alkyle en C₁ à C₃, alkyloxy en C₁ à C₃, nitro et cyano,
où n et m peuvent être indépendamment l'un, de l'autre, 0, 1 ou 2,
en présence d'une base, par exemple NaH, KH, KOtBu, NaOtBu, NaOMe, NaOEt, NaO*i*Pr, KF, K₂CO₃, Cs₂CO₃, pyridine, 4-diméthylaminopyridine, NEt₃ ou EtN/Pr₂,
éventuellement en présence d'un catalyseur, par exemple d'un complexe de Cu ou Pd ; et
si nécessaire, un radical protecteur utilisé dans les réactions décrites précédemment aux points a) et b) est clivé et/ou
si on le souhaite, un composé de formule générale I ainsi obtenu est séparé en ses stéréoisomères et/ou si on le souhaite, un composé de formule générale I ainsi obtenu est converti en ses sels, en particulier en ses sels physiologiquement acceptables pour l'utilisation pharmaceutique.
